(19) Europäisches Patentamt / European Patent Office / Office européen des brevets

(11) **EP 4 455 164 A1**

(12) **EUROPEAN PATENT APPLICATION**
published in accordance with Art. 153(4) EPC


(43) Date of publication:
**30.10.2024 Bulletin 2024/44**

(21) Application number: **22910217.3**

(22) Date of filing: **23.12.2022**

(51) International Patent Classification (IPC):
***C07K 16/32*** *(2006.01)* ***A61K 47/68*** *(2017.01)*
***A61K 31/4745*** *(2006.01)* ***A61P 35/00*** *(2006.01)*

(52) Cooperative Patent Classification (CPC):
**A61K 31/4745; A61K 47/68; A61P 35/00; C07K 16/00; C07K 16/28; C07K 16/32; C12N 5/10; C12N 15/85**

(86) International application number:
**PCT/CN2022/141572**

(87) International publication number:
**WO 2023/116911 (29.06.2023 Gazette 2023/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.12.2021 CN 202111602429**
**10.11.2022 CN 202211408028**



(71) Applicant: **Bio-Thera Solutions, Ltd.**
**Guangzhou, Guangdong 510530 (CN)**

(72) Inventors:
- **TANG, Weijia**
  **Guangzhou, Guangdong 510530 (CN)**
- **MAI, Siqi**
  **Guangzhou, Guangdong 510530 (CN)**
- **ZHOU, Xin**
  **Guangzhou, Guangdong 510530 (CN)**
- **YAN, Hui**
  **Guangzhou, Guangdong 510530 (CN)**
- **LI, Shuoxu**
  **Guangzhou, Guangdong 510530 (CN)**
- **FAN, Jianjun**
  **Guangzhou, Guangdong 510530 (CN)**
- **SHEN, Jingyi**
  **Guangzhou, Guangdong 510530 (CN)**
- **LIANG, Shide**
  **Guangzhou, Guangdong 510530 (CN)**
- **YU, Jin-Chen**
  **Guangzhou, Guangdong 510530 (CN)**
- **LI, Shengfeng**
  **Guangzhou, Guangdong 510530 (CN)**

(74) Representative: **Engelhard, Markus**
**Boehmert & Boehmert**
**Anwaltspartnerschaft mbB**
**Pettenkoferstrasse 22**
**80336 München (DE)**


Remarks:
The complete document including Reference Table(s) and the Sequence Listing(s) can be downloaded from the EPO website

(54) **ANTI-FRalpha ANTIBODY, AND ANTIBODY-DRUG CONJUGATE AND USE THEREOF**


(57) An anti-FRα antibody and an antibody-drug conjugate thereof, and uses of the anti-FRα antibody and the antibody-drug conjugate in the treatment of diseases related to FRα expression.


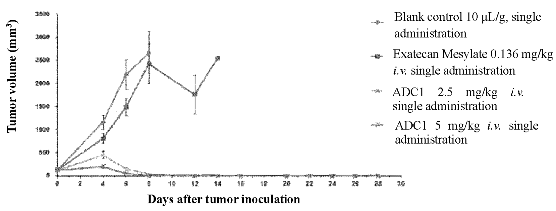


FIG. 5

EP 4 455 164 A1

## Description

### TECHNICAL FIELD

The present invention relates to an anti-FRα antibody, an antibody-drug conjugate comprising the anti-FRα antibody, and uses thereof.

### BACKGROUND

Folate, as an essential vitamin required for DNA synthesis and repair as well as the process of cell division, is transported through endocytosis by binding to folate receptors on the cell surface, which are internalized and then recycled back to the cell membrane. The folate receptor is a transmembrane single-chain glycoprotein linked to glycosylated phosphatidylinositol and has a high affinity for folate. The folate receptor includes three subtypes, i.e., folate receptor α (FOLR1, FRα), folate receptor β (FOLR2) and folate receptor y (FOLR3). The expression of the folate receptor is highly restricted in normal cells and is significantly highly expressed on tumor cells, wherein the folate receptor α is found to be overexpressed in various malignant tumors, and thus becomes one of hot targets of anti-cancer drugs.

### SUMMARY

The present invention provides an antibody that specifically recognizes and binds to folate receptor α (also referred to as "anti-folate receptor α antibody" or "anti-FRα antibody"). In one or more embodiments, the antibody of the present invention is capable of recognizing and binding to human folate receptor α.

In one or more embodiments, the present invention provides an antibody or an antigen-binding unit thereof that specifically binds to folate receptor α and comprises one or more of (a)-(f):

(a) a VH CDR1 comprising or consisting of an amino acid sequence set forth in SEQ ID NO: 5 or an amino acid sequence having substitutions, deletions or insertions at one or more sites compared with the amino acid sequence set forth in SEQ ID NO: 5;
(b) a VH CDR2 comprising or consisting of an amino acid sequence set forth in SEQ ID NO: 6 or an amino acid sequence having substitutions, deletions or insertions at one or more sites compared with the amino acid sequence set forth in SEQ ID NO: 6;
(c) a VH CDR3 comprising or consisting of an amino acid sequence set forth in SEQ ID NO: 7 or an amino acid sequence having substitutions, deletions or insertions at one or more sites compared with the amino acid sequence set forth in SEQ ID NO: 7;
(d) a VL CDR1 comprising or consisting of an amino acid sequence set forth in SEQ ID NO: 8 or an amino acid sequence having substitutions, deletions or insertions at one or more sites compared with the amino acid sequence set forth in SEQ ID NO: 8;
(e) a VL CDR2 comprising or consisting of an amino acid sequence set forth in SEQ ID NO: 9 or an amino acid sequence having substitutions, deletions or insertions at one or more sites compared with the amino acid sequence set forth in SEQ ID NO: 9; and
(f) a VL CDR3 comprising or consisting of an amino acid sequence set forth in SEQ ID NO: 10 or an amino acid sequence having substitutions, deletions or insertions at one or more sites compared with the amino acid sequence set forth in SEQ ID NO: 10.

In one or more embodiments, the antibody or the antigen-binding unit thereof specifically binds to folate receptor α and comprises:

(a) a VH CDR1 comprising or consisting of an amino acid sequence set forth in SEQ ID NO: 5 or an amino acid sequence having substitutions, deletions or insertions at one or more sites compared with the amino acid sequence set forth in SEQ ID NO: 5; and
(b) a VH CDR2 comprising or consisting of an amino acid sequence set forth in SEQ ID NO: 6 or an amino acid sequence having substitutions, deletions or insertions at one or more sites compared with the amino acid sequence set forth in SEQ ID NO: 6; and
(c) a VH CDR3 comprising or consisting of an amino acid sequence set forth in SEQ ID NO: 7 or an amino acid sequence having substitutions, deletions or insertions at one or more sites compared with the amino acid sequence set forth in SEQ ID NO: 7.

In one or more embodiments, the antibody or the antigen-binding unit thereof comprises a VH CDR1 set forth

in SEQ ID NO: 5, a VH CDR2 set forth in SEQ ID NO: 6 and a VH CDR3 set forth in SEQ ID NO: 7.

In one or more embodiments, the antibody or the antigen-binding unit thereof specifically binds to folate receptor α and comprises:

(d) a VL CDR1 comprising or consisting of an amino acid sequence set forth in SEQ ID NO: 8 or an amino acid sequence having substitutions, deletions or insertions at one or more sites compared with the amino acid sequence set forth in SEQ ID NO: 8; and
(e) a VL CDR2 comprising or consisting of an amino acid sequence set forth in SEQ ID NO: 9 or an amino acid sequence having substitutions, deletions or insertions at one or more sites compared with the amino acid sequence set forth in SEQ ID NO: 9; and
(f) a VL CDR3 comprising or consisting of an amino acid sequence set forth in SEQ ID NO: 10 or an amino acid sequence having substitutions, deletions or insertions at one or more sites compared with the amino acid sequence set forth in SEQ ID NO: 10.

In one or more embodiments, the antibody or the antigen-binding unit thereof comprises a VL CDR1 set forth in SEQ ID NO: 8, a VL CDR2 set forth in SEQ ID NO: 9 and a VL CDR3 set forth in SEQ ID NO: 10.

In one or more embodiments, the antibody or the antigen-binding unit thereof specifically binds to folate receptor α and comprises:

(a) a VH CDR1 comprising or consisting of an amino acid sequence set forth in SEQ ID NO: 5 or an amino acid sequence having substitutions, deletions or insertions at one or more sites compared with the amino acid sequence set forth in SEQ ID NO: 5; and
(b) a VH CDR2 comprising or consisting of an amino acid sequence set forth in SEQ ID NO: 6 or an amino acid sequence having substitutions, deletions or insertions at one or more sites compared with the amino acid sequence set forth in SEQ ID NO: 6; and
(c) a VH CDR3 comprising or consisting of an amino acid sequence set forth in SEQ ID NO: 7 or an amino acid sequence having substitutions, deletions or insertions at one or more sites compared with the amino acid sequence set forth in SEQ ID NO: 7; and
(d) a VL CDR1 comprising or consisting of an amino acid sequence set forth in SEQ ID NO: 8 or an amino acid sequence having substitutions, deletions or insertions at one or more sites compared with the amino acid sequence set forth in SEQ ID NO: 8; and
(e) a VL CDR2 comprising or consisting of an amino acid sequence set forth in SEQ ID NO: 9 or an amino acid sequence having substitutions, deletions or insertions at one or more sites compared with the amino acid sequence set forth in SEQ ID NO: 9; and
(f) a VL CDR3 comprising or consisting of an amino acid sequence set forth in SEQ ID NO: 10 or an amino acid sequence having substitutions, deletions or insertions at one or more sites compared with the amino acid sequence set forth in SEQ ID NO: 10.

In one or more embodiments, the substitutions, deletions or insertions at sites are independently one, two or three.

In one or more embodiments, the antibody or the antigen-binding unit thereof comprises a VH CDR1 set forth in SEQ ID NO: 5, a VH CDR2 set forth in SEQ ID NO: 6, a VH CDR3 set forth in SEQ ID NO: 7, a VL CDR1 set forth in SEQ ID NO: 8, a VL CDR2 set forth in SEQ ID NO: 9 and a VL CDR3 set forth in SEQ ID NO: 10.

In one or more embodiments, the antibody or the antigen-binding unit thereof further comprises one or more of (g)-(n):

(g) a VH FR1 comprising an amino acid sequence set forth in SEQ ID NO: 1 or SEQ ID NO: 29, or an amino acid sequence having at least 90% sequence identity compared with the amino acid sequence set forth in SEQ ID NO: 1 or SEQ ID NO: 29, or an amino acid sequence having substitutions, deletions or insertions at one or more sites compared with the amino acid sequence set forth in SEQ ID NO: 1 or SEQ ID NO: 29;
(h) a VH FR2 comprising an amino acid sequence set forth in SEQ ID NO: 2 or SEQ ID NO: 30, or an amino acid sequence having at least 90% sequence identity compared with the amino acid sequence set forth in SEQ ID NO: 2 or SEQ ID NO: 30, or an amino acid sequence having substitutions, deletions or insertions at one or more sites compared with the amino acid sequence set forth in SEQ ID NO: 2 or SEQ ID NO: 30;
(i) a VH FR3 comprising an amino acid sequence set forth in SEQ ID NO: 3 or SEQ ID NO: 31, or an amino acid sequence having at least 90% sequence identity compared with the amino acid sequence set forth in SEQ ID NO: 3 or SEQ ID NO: 31, or an amino acid sequence having substitutions, deletions or insertions at one or more sites compared with the amino acid sequence set forth in SEQ ID NO: 3 or SEQ ID NO: 31;

(j) a VH FR4 comprising an amino acid sequence set forth in SEQ ID NO: 11, or an amino acid sequence having at least 90% sequence identity compared with the amino acid sequence set forth in SEQ ID NO: 11, or an amino acid sequence having substitutions, deletions or insertions at one or more sites compared with the amino acid sequence set forth in SEQ ID NO: 11;

(k) a VL FR1 comprising an amino acid sequence set forth in SEQ ID NO: 12, or an amino acid sequence having at least 90% sequence identity compared with the amino acid sequence set forth in SEQ ID NO: 12, or an amino acid sequence having substitutions, deletions or insertions at one or more sites compared with the amino acid sequence set forth in SEQ ID NO: 12;

(l) a VL FR2 comprising an amino acid sequence set forth in SEQ ID NO: 13, or an amino acid sequence having at least 90% sequence identity compared with the amino acid sequence set forth in SEQ ID NO: 13, or an amino acid sequence having substitutions, deletions or insertions at one or more sites compared with the amino acid sequence set forth in SEQ ID NO: 13;

(m) a VL FR3 comprising an amino acid sequence set forth in SEQ ID NO: 4 or SEQ ID NO: 32, or an amino acid sequence having at least 90% sequence identity compared with the amino acid sequence set forth in SEQ ID NO: 4 or SEQ ID NO: 32, or an amino acid sequence having substitutions, deletions or insertions at one or more sites compared with the amino acid sequence set forth in SEQ ID NO: 4 or SEQ ID NO: 32; and

(n) a VL FR4 comprising an amino acid sequence set forth in SEQ ID NO: 14, or an amino acid sequence having at least 90% sequence identity compared with the amino acid sequence set forth in SEQ ID NO: 14, or an amino acid sequence having substitutions, deletions or insertions at one or more sites compared with the amino acid sequence set forth in SEQ ID NO: 14.

In one or more embodiments, the antibody or the antigen-binding unit thereof specifically binds to folate receptor $\alpha$ and comprises:

(g) a VH FR1 comprising an amino acid sequence set forth in SEQ ID NO: 1 or SEQ ID NO: 29, or an amino acid sequence having at least 90% sequence identity compared with the amino acid sequence set forth in SEQ ID NO: 1 or SEQ ID NO: 29, or an amino acid sequence having substitutions, deletions or insertions at one or more sites compared with the amino acid sequence set forth in SEQ ID NO: 1 or SEQ ID NO: 29; and

(h) a VH FR2 comprising an amino acid sequence set forth in SEQ ID NO: 2 or SEQ ID NO: 30, or an amino acid sequence having at least 90% sequence identity compared with the amino acid sequence set forth in SEQ ID NO: 2 or SEQ ID NO: 30, or an amino acid sequence having substitutions, deletions or insertions at one or more sites compared with the amino acid sequence set forth in SEQ ID NO: 2 or SEQ ID NO: 30; and

(i) a VH FR3 comprising an amino acid sequence set forth in SEQ ID NO: 3 or SEQ ID NO: 31, or an amino acid sequence having at least 90% sequence identity compared with the amino acid sequence set forth in SEQ ID NO: 3 or SEQ ID NO: 31, or an amino acid sequence having substitutions, deletions or insertions at one or more sites compared with the amino acid sequence set forth in SEQ ID NO: 3 or SEQ ID NO: 31; and

(j) a VH FR4 comprising an amino acid sequence set forth in SEQ ID NO: 11, or an amino acid sequence having at least 90% sequence identity compared with the amino acid sequence set forth in SEQ ID NO: 11, or an amino acid sequence having substitutions, deletions or insertions at one or more sites compared with the amino acid sequence set forth in SEQ ID NO: 11; and/or

(k) a VL FR1 comprising an amino acid sequence set forth in SEQ ID NO: 12, or an amino acid sequence having at least 90% sequence identity compared with the amino acid sequence set forth in SEQ ID NO: 12, or an amino acid sequence having substitutions, deletions or insertions at one or more sites compared with the amino acid sequence set forth in SEQ ID NO: 12; and

(l) a VL FR2 comprising an amino acid sequence set forth in SEQ ID NO: 13, or an amino acid sequence having at least 90% sequence identity compared with the amino acid sequence set forth in SEQ ID NO: 13, or an amino acid sequence having substitutions, deletions or insertions at one or more sites compared with the amino acid sequence set forth in SEQ ID NO: 13; and

(m) a VL FR3 comprising an amino acid sequence set forth in SEQ ID NO: 4 or SEQ ID NO: 32, or an amino acid sequence having at least 90% sequence identity compared with the amino acid sequence set forth in SEQ ID NO: 4 or SEQ ID NO: 32, or an amino acid sequence having substitutions, deletions or insertions at one or more sites compared with the amino acid sequence set forth in SEQ ID NO: 4 or SEQ ID NO: 32; and

(n) a VL FR4 comprising an amino acid sequence set forth in SEQ ID NO: 14, or an amino acid sequence having at least 90% sequence identity compared with the amino acid sequence set forth in SEQ ID NO: 14, or an amino acid sequence having substitutions, deletions or insertions at one or more sites compared with the amino acid sequence set forth in SEQ ID NO: 14.

In one or more embodiments, the antibody or the antigen-binding unit thereof comprises a VH FR1 set forth in

SEQ ID NO: 1, a VH FR2 set forth in SEQ ID NO: 2, a VH FR3 set forth in SEQ ID NO: 3 and a VH FR4 set forth in SEQ ID NO: 11.

In one or more embodiments, the antibody or the antigen-binding unit thereof comprises a VL FR1 set forth in SEQ ID NO: 12, a VL FR2 set forth in SEQ ID NO: 13, a VL FR3 set forth in SEQ ID NO: 4 and a VL FR4 set forth in SEQ ID NO: 14.

In one or more embodiments, the antibody or the antigen-binding unit thereof comprises a VH FR1 set forth in SEQ ID NO: 1, a VH FR2 set forth in SEQ ID NO: 2, a VH FR3 set forth in SEQ ID NO: 3, a VH FR4 set forth in SEQ ID NO: 11, a VL FR1 set forth in SEQ ID NO: 12, a VL FR2 set forth in SEQ ID NO: 13, a VL FR3 set forth in SEQ ID NO: 4 and a VL FR4 set forth in SEQ ID NO: 14.

In one or more embodiments, the antibody or the antigen-binding unit thereof comprises a VH FR1 set forth in SEQ ID NO: 29, a VH FR2 set forth in SEQ ID NO: 30, a VH FR3 set forth in SEQ ID NO: 31 and a VH FR4 set forth in SEQ ID NO: 11.

In one or more embodiments, the antibody or the antigen-binding unit thereof comprises a VL FR1 set forth in SEQ ID NO: 12, a VL FR2 set forth in SEQ ID NO: 13, a VL FR3 set forth in SEQ ID NO: 32 and a VL FR4 set forth in SEQ ID NO: 14.

In one or more embodiments, the antibody or the antigen-binding unit thereof comprises a VH FR1 set forth in SEQ ID NO: 29, a VH FR2 set forth in SEQ ID NO: 30, a VH FR3 set forth in SEQ ID NO: 31, a VH FR4 set forth in SEQ ID NO: 11, a VL FR1 set forth in SEQ ID NO: 12, a VL FR2 set forth in SEQ ID NO: 13, a VL FR3 set forth in SEQ ID NO: 32 and a VL FR4 set forth in SEQ ID NO: 14.

In one or more embodiments, the present invention provides an antibody or an antigen-binding unit thereof that specifically binds to folate receptor $\alpha$ and comprises a heavy chain variable region (VH) and/or a light chain variable region (VL), wherein:

the heavy chain variable region comprises an amino acid sequence set forth in SEQ ID NO: 15, or an amino acid sequence having at least 90% sequence identity compared with the amino acid sequence set forth in SEQ ID NO: 15, or an amino acid sequence having substitutions, deletions or insertions at one or more sites compared with the amino acid sequence set forth in SEQ ID NO: 15; and/or

the light chain variable region comprises an amino acid sequence set forth in SEQ ID NO: 16, or an amino acid sequence having at least 90% sequence identity compared with the amino acid sequence set forth in SEQ ID NO: 16, or an amino acid sequence having substitutions, deletions or insertions at one or more sites compared with the amino acid sequence set forth in SEQ ID NO: 16.

In one or more embodiments, the antibody or the antigen-binding unit thereof comprises a heavy chain variable region set forth in SEQ ID NO: 15 and a light chain variable region set forth in SEQ ID NO: 16.

In one or more embodiments, the antibody or the antigen-binding unit thereof further comprises a heavy chain constant region, a light chain constant region, an Fc region or a combination thereof. In one or more embodiments, the light chain constant region is a $\kappa$ or $\lambda$ chain constant region. In one or more embodiments, the antibody or the antigen-binding unit thereof is one of the isotypes IgG, IgM, IgA, IgE or IgD. In one or more embodiments, the isotype is IgG1, IgG2, IgG3 or IgG4. In one or more embodiments, the antibody or the antigen-binding unit thereof is a murine antibody, a chimeric antibody, a humanized antibody or a fully human antibody.

In one or more embodiments, the antibody or the antigen-binding unit thereof is an scFv, a Fab, a Fab' or a $F(ab)_2$. In one or more embodiments, the antibody or the antigen-binding unit thereof is a monoclonal antibody.

In one or more embodiments, the antibody or the antigen-binding unit thereof comprises a heavy chain constant region (CH) and/or a light chain constant region (CL), wherein:

the heavy chain constant region comprises an amino acid sequence set forth in SEQ ID NO: 17, or an amino acid sequence having at least 90% sequence identity compared with the amino acid sequence set forth in SEQ ID NO: 17, or an amino acid sequence having substitutions, deletions or insertions at one or more sites compared with the amino acid sequence set forth in SEQ ID NO: 17; and/or

the light chain constant region comprises an amino acid sequence set forth in SEQ ID NO: 18, or an amino acid sequence having at least 90% sequence identity compared with the amino acid sequence set forth in SEQ ID NO: 18, or an amino acid sequence having substitutions, deletions or insertions at one or more sites compared with the amino acid sequence set forth in SEQ ID NO: 18.

In one or more embodiments, the antibody or the antigen-binding unit thereof comprises a heavy chain constant region set forth in SEQ ID NO: 17 and a light chain constant region set forth in SEQ ID NO: 18.

In one or more embodiments, the present invention provides an antibody that specifically binds to folate receptor $\alpha$, a heavy chain of the antibody comprises a heavy chain variable region set forth in SEQ ID NO: 15 and a heavy chain

constant region set forth in SEQ ID NO: 17; a light chain of the antibody comprises a light chain variable region set forth in SEQ ID NO: 16 and a light chain constant region set forth in SEQ ID NO: 18.

In one or more embodiments, the present invention provides an antibody that specifically binds to folate receptor α and comprises a heavy chain (H) and a light chain (L), wherein:

the heavy chain comprises an amino acid sequence set forth in SEQ ID NO: 19, or an amino acid sequence having at least 90% sequence identity compared with the amino acid sequence set forth in SEQ ID NO: 19, or an amino acid sequence having substitutions, deletions or insertions at one or more sites compared with the amino acid sequence set forth in SEQ ID NO: 19; and/or

the light chain comprises an amino acid sequence set forth in SEQ ID NO: 20, or an amino acid sequence having at least 90% sequence identity compared with the amino acid sequence set forth in SEQ ID NO: 20, or an amino acid sequence having substitutions, deletions or insertions at one or more sites compared with the amino acid sequence set forth in SEQ ID NO: 20.

In one or more embodiments, the antibody comprises a heavy chain set forth in SEQ ID NO: 19 and a light chain set forth in SEQ ID NO: 20.

In one or more embodiments, the substitution is a conservative amino acid substitution.

In one or more embodiments, the antibody or the antigen-binding unit thereof is chimeric, humanized or fully human.

In one or more embodiments, the antibody or the antigen-binding unit thereof is a monoclonal antibody (including a full-length monoclonal antibody) or a multispecific antibody or antigen-binding unit thereof (e.g., a bispecific antibody or antigen-binding unit thereof).

In one or more embodiments, the antibody has two heavy chains with the same sequence and two light chains with the same sequence. In one or more embodiments, the Fc regions pair to form disulfide bonds. In one or more embodiments, the antibody or the antigen-binding unit thereof of the present invention is an isolated antibody or an antigen-binding unit thereof.

In one or more embodiments, the antibody or the antigen-binding unit thereof of the present invention is a monoclonal antibody or a fragment thereof.

In one or more embodiments, the present invention further provides a biomaterial, which is

(1) a polynucleotide encoding the antibody or the antigen-binding unit thereof described herein; or a portion thereof,
(2) an expression vector comprising a polynucleotide encoding the antibody or the antigen-binding unit thereof described herein; or
(3) a cell comprising one or more polynucleotides encoding the antibody or the antigen-binding unit thereof described herein.

In one or more embodiments, the present invention further provides a pharmaceutical composition comprising the antibody or the antigen-binding unit thereof provided by the present invention. These compositions can be contained in a kit, such as a diagnostic kit.

In one or more embodiments, the present invention further provides a method for alleviating or treating symptoms of cancer or other disorders by administering to a patient in need thereof one or more antibodies that bind to folate receptor α or antigen-binding units thereof. The antibody should be administered at a dose sufficient to alleviate or treat the symptoms of the cancer or other disorders in the patient. In one or more embodiments, the patient is a human. In one or more embodiments, the anti-FRα antibody of the present invention is used in combination with one or more additional therapies. Suitable additional therapies include existing drugs, radiotherapy and/or surgical therapies for specific applications (such as cancer). For example, the anti-FRα antibody is used in combination with one or more additional chemotherapeutic or anti-tumor agents. Alternatively, the additional chemotherapy is radiotherapy. In one or more embodiments, the chemotherapeutic agent is an apoptosis inducer.

In one or more embodiments, the anti-FRα antibody and the additional agent are prepared as a single therapeutic composition, and the anti-FRα antibody and the additional agent are administered simultaneously. Alternatively, the anti-FRα antibody and the additional agent are separated from each other, for example, are each prepared as a separate therapeutic composition, and the anti-FRα antibody and the additional agent are administered simultaneously, or the anti-FRα antibody and the additional agent are administered at different time points during a treatment regimen. For example, the anti-FRα antibody is administered prior to the administration of the additional agent, the anti-FRα antibody is administered subsequent to the administration of the additional agent, or the anti-FRα antibody and the additional agent are administered in an alternating manner. In the present invention, the anti-FRα antibody and the additional agent are administered at a single dose or at multiple doses. In one or more embodiments, the present invention further provides an antibody-drug conjugate (ADC) comprising the antibody or the antigen-binding unit thereof described herein

conjugated to a drug via a linker, or a pharmaceutically acceptable salt or solvate thereof.

In one or more embodiments, the linker is a cleavable linker.

In one or more embodiments, the drug is an anti-cancer drug, a cytotoxic drug, a cell differentiation factor, a stem cell trophic factor, a steroid drug, a drug for treating autoimmune diseases, an anti-inflammatory drug or a drug for treating infectious diseases.

In one or more embodiments, the drug is an anti-cancer drug.

In one or more embodiments, the drug is a tubulin inhibitor, a DNA damaging agent or a DNA topoisomerase inhibitor.

In one or more embodiments, the tubulin inhibitor is selected from dolastatin, auristatins and maytansinoids.

In one or more embodiments, the drug is an auristatin selected from monomethyl auristatin E (MMAE), monomethyl auristatin F (MMAF) or auristatin F (AF).

In one or more embodiments, the drug is a DNA damaging agent, e.g., a calicheamicin, a duocarmycin or an anthramycin derivative PBD (pyrrolobenzodiazepine).

In one or more embodiments, the drug is a DNA topoisomerase inhibitor or a salt thereof, e.g., irinotecan, irinotecan hydrochloride, an exatecan derivative, camptothecin, 9-aminocamptothecin, 9-nitrocamptothecin, 10-hydroxy camptothecin, 9-chloro-10-hydroxycamptothecin, a camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-N-(phenylmethyl)-(2*E*)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, *N*-[2-(dimethylamino)ethyl]-4-acridinecarboxamide dihydrochloride or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine or exatecan. In one or more embodiments, the drug has an amino group or an amino group substituted with one alkyl group, and links to the linker via an amide bond.

In one or more embodiments, the drug is

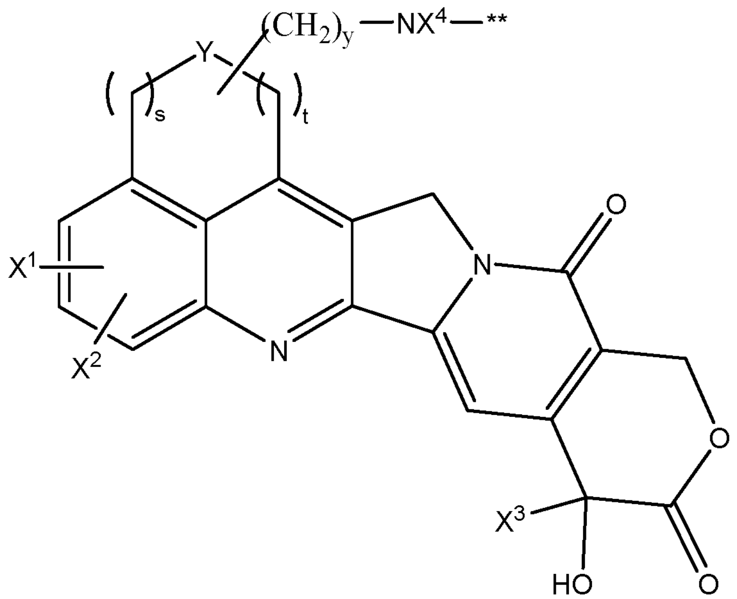

or a stereoisomer thereof, wherein $X^1$ and $X^2$ are each independently:

H,
hydroxy,
C1-C6 alkyl,
C1-C6 alkyl substituted with one or more hydroxy, halogen, nitro or cyano groups,
C2-C6 alkenyl,
C2-C6 alkynyl,
C1-C6 alkoxy,
C1-C6 aminoalkoxy,
halogen,
nitro,

cyano,
thiol,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocyclic ring, wherein the heterocyclic ring is optionally substituted with one or more C1-C6 alkyl, C1-C6 alkoxy, amino, halogen, nitro or cyano groups,
C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or a protecting group,
amino-substituted heterocyclyl optionally substituted at a nitrogen atom of the heterocyclyl moiety or at the amino moiety with a protecting group or one or more C1-C6 alkyl groups, heterocyclylamino optionally substituted at a nitrogen atom of the heterocyclyl moiety or at the amino moiety with a protecting group or C1-C6 alkyl,
carbamoyl optionally substituted with a carbamoyl-protecting group or C1-C6 alkyl, morpholin-1-yl, or piperidin-1-yl;
$X^3$ is C1-C6 alkyl;
$X^4$ is H, $-(CH_2)_q-CH_3$, $-(CHR^n)_q-CH_3$, C3-C8 carbocyclyl, $-O-(CH_2)_q-CH_3$, arylene-$CH_3$, - $(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_{qC}(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, - $(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or - $(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;
** links to the linker;
y is 0, 1 or 2;
Y is O, S or $CR^1R^2$, wherein $R^1$ and $R^2$ are each independently H or C1-C6 alkyl;
s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, t-butoxycarbonyl, benzyl, or *p*-methoxybenzyloxycarbonyl.

In one or more embodiments, the drug is

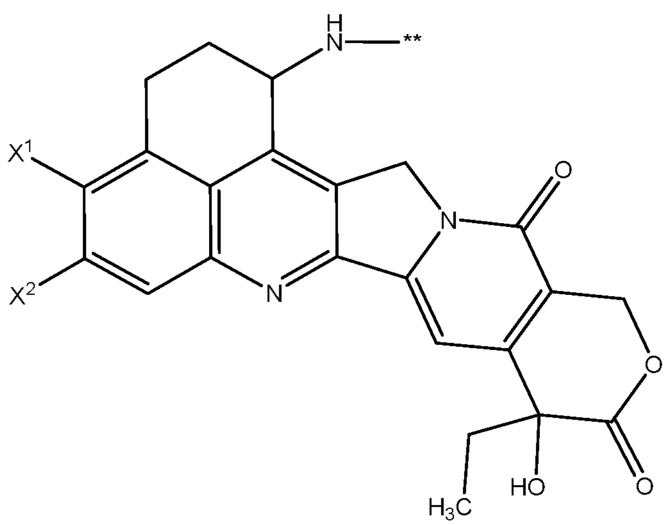

or

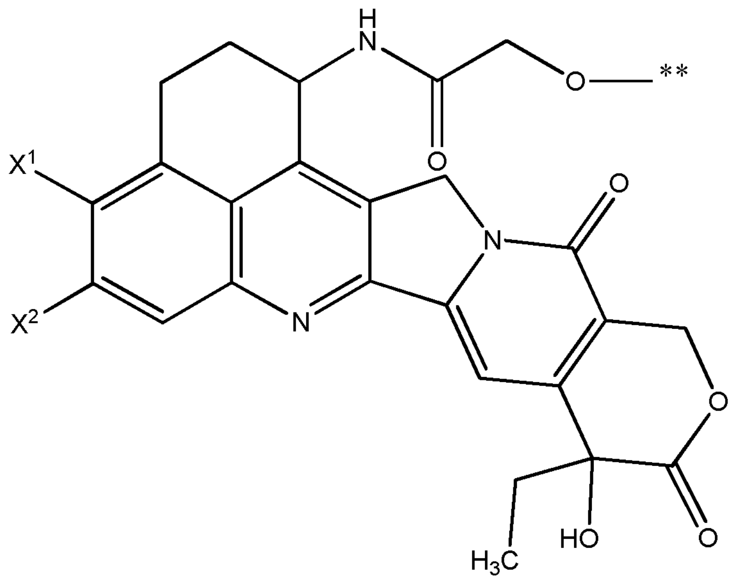

or a stereoisomer thereof, wherein $X^1$ and $X^2$ are each independently C1-C6 alkyl, halogen or -OH; ** links to the linker.

In one or more embodiments, the drug is

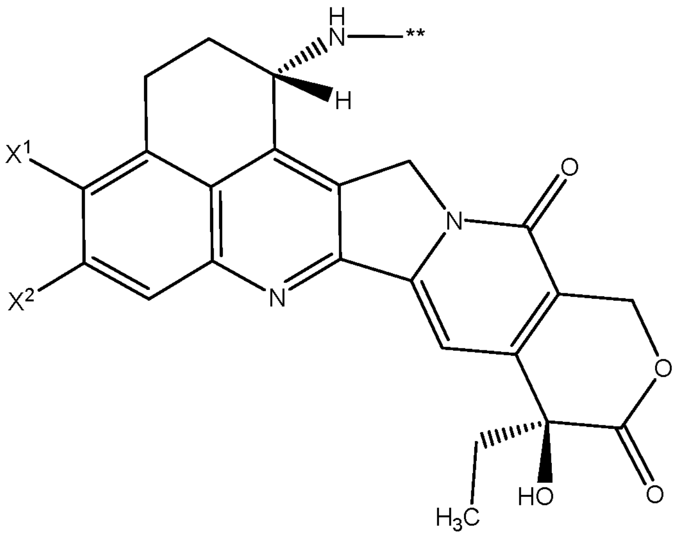

or

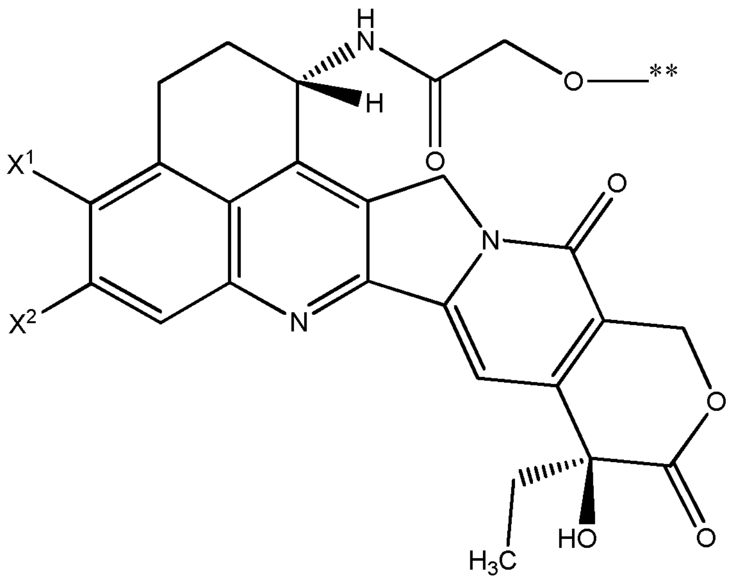

or a stereoisomer thereof, wherein

$X^1$ and $X^2$ are each independently C1-C6 alkyl, halogen or -OH; ** links to the linker.

In one or more embodiments, $X^1$ and $X^2$ are each $-CH_3$.

In one or more embodiments, $X^1$ and $X^2$ are each independently F, Cl, Br or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$, and $X^2$ is F.

In one or more embodiments, the antibody-drug conjugate provided by the present invention has a structure shown as Formula I or a stereoisomer, or a pharmaceutically acceptable salt or solvate thereof:

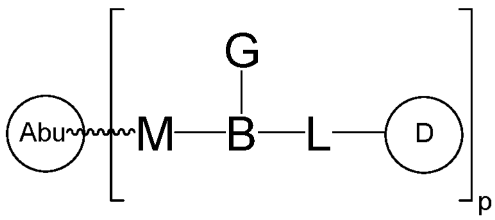

Formula I

wherein

Abu is an antibody or an antigen-binding unit thereof; the antibody or the antigen-binding unit thereof specifically binds to folate receptor α;
D is a drug;
M is

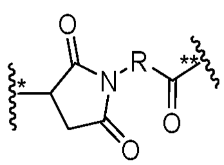

,

wherein * links to Abu, ** links to B, and R is selected from: $-(CH_2)_r-$, $-(CHR^m)_r-$, C3-C8 carbocyclyl, $-O-(CH_2)_r-$, arylene, $-(CH_2)_r$-arylene-, -arylene-$(CH_2)_r$-, - $(CH_2)_r$-(C3-C8 carbocyclyl)-, -(C3-C8 carbocyclyl)-$(CH_2)_r$-, C3-C8 heterocyclyl, $-(CH_2)_r$-(C3-C8 heterocyclyl)-, -(C3-C8 heterocyclyl)-$(CH_2)_r$-, $-(CH_2)_rC(O)NR^m(CH_2)_r-$, - $(CH_2CH_2O)_r-$,

$-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

B is

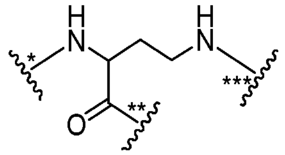

,

e.g.,

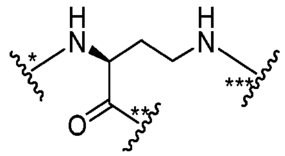

;

wherein * links to M, ** links to L, and *** links to G;

L is -(AA)i-(FF)f-, wherein AA is an amino acid or polypeptide, and i is 1, 2, 3, 4, 5, 6, 7, 8, 9, 10, 11, 12, 13, 14, 15, 16, 17, 18, 19 or 20; each FF is independently

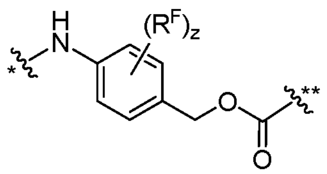

, 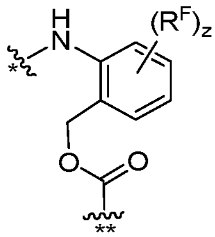 or 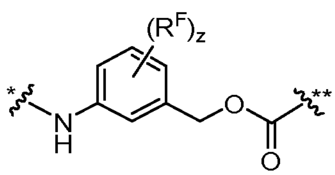 ,

wherein each $R^F$ is independently C1-C6 alkyl, C1-C6 alkoxy, $-NO_2$ or halogen; z is 0, 1, 2, 3 or 4; f is 1, 2, 3, 4, 5, 6, 7, 8, 9 or 10; wherein * links to AA, and ** links to D;

Gis

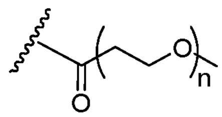

,

wherein n is an integer from 1 to 24, e.g., 1, 2, 3, 4, 5, 6, 7, 8, 9, 10, 11, 12, 13, 14, 15, 16, 17, 18, 19, 20, 21, 22, 23 or 24; p is an integer from 1 to 10, e.g., 1, 2, 3, 4, 5, 6, 7, 8, 9 or 10.

In one or more embodiments, the drug is an anti-cancer drug, a cytotoxic drug, a cell differentiation factor, a stem cell trophic factor, a steroid drug, a drug for treating autoimmune diseases, an anti-inflammatory drug or a drug for treating infectious diseases.

In one or more embodiments, the drug is an anti-cancer drug.

In one or more embodiments, the drug is a tubulin inhibitor, a DNA damaging agent or a DNA topoisomerase inhibitor.

In one or more embodiments, the tubulin inhibitor is selected from dolastatin, auristatins and maytansinoids.

In one or more embodiments, the drug is an auristatin, e.g., MMAE, MMAF or AF.

In one or more embodiments, the drug is a DNA damaging agent, e.g., a calicheamicin, a duocarmycin or an

anthramycin derivative PBD (pyrrolobenzodiazepine).

In one or more embodiments, the drug is a DNA topoisomerase inhibitor or a salt thereof, e.g., irinotecan, irinotecan hydrochloride, camptothecin, 9-aminocamptothecin, 9-nitrocamptothecin, 10-hydroxy camptothecin, 9-chloro-10-hydroxy camptothecin, a camptothecin derivative SN-38, 22-hydroxyacuminatine, topotecan, lurtotecan, belotecan, exatecan, an exatecan derivative, homosilatecan, 6,8-dibromo-2-methyl-3-[2-(D-xylopyranosylamino)phenyl]-4(3*H*)-quinazolinone, 2-cyano-3-(3,4-dihydroxyphenyl)-N-(phenylmethyl)-(2E)-2-propenamide, 2-cyano-3-(3,4-dihydroxyphenyl)-N-(3-hydroxyphenylpropyl)-(E)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione,*N*-[2-(dimethylamino)ethyl]-4-acridinecarboxamide dihydrochloride or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine or exatecan. In one or more embodiments, the drug is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan or a chlorambucil derivative.

In one or more embodiments, the drug has an amino group or an amino group substituted with one alkyl group, and links to FF via an amide bond.

In one or more embodiments, the drug is

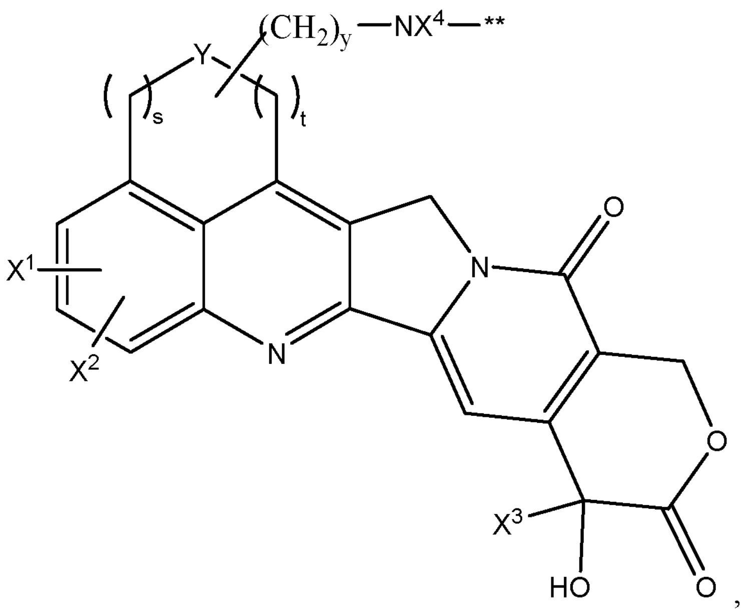

wherein $X^1$ and $X^2$ are each independently:

H,
hydroxy,
C1-C6 alkyl,
C1-C6 alkyl substituted with one or more hydroxy, halogen, nitro or cyano groups,
C2-C6 alkenyl,
C2-C6 alkynyl,
C1-C6 alkoxy,
C1-C6 aminoalkoxy,
halogen,
nitro,
cyano,
thiol,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocyclic ring, wherein the heterocyclic ring is optionally substituted with one or more

C1-C6 alkyl, C1-C6 alkoxy, amino, halogen, nitro or cyano groups,
C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or a protecting group,
amino-substituted heterocyclyl optionally substituted at a nitrogen atom of the heterocyclyl moiety or at the amino moiety with a protecting group or one or more C1-C6 alkyl groups, heterocyclylamino optionally substituted at a nitrogen atom of the heterocyclyl moiety or at the amino moiety with a protecting group or C1-C6 alkyl,
carbamoyl optionally substituted with a carbamoyl-protecting group or C1-C6 alkyl, morpholin-1-yl, or piperidin-1-yl;
$X^3$ is C1-C6 alkyl;
$X^4$ is H, $-(CH_2)_q-CH_3$, $-(CHR^n)_q-CH_3$, C3-C8 carbocyclyl, $-O-(CH_2)_q-CH_3$, arylene-$CH_3$, - $(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, - $(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or - $(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;
** links to L;
y is 0, 1 or 2;
Y is O, S or $CR^1R^2$, wherein $R^1$ and $R^2$ are each independently H or C1-C6 alkyl;
s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, t-butoxycarbonyl, benzyl, or *p*-methoxybenzyloxycarbonyl.

In one or more embodiments, the drug is

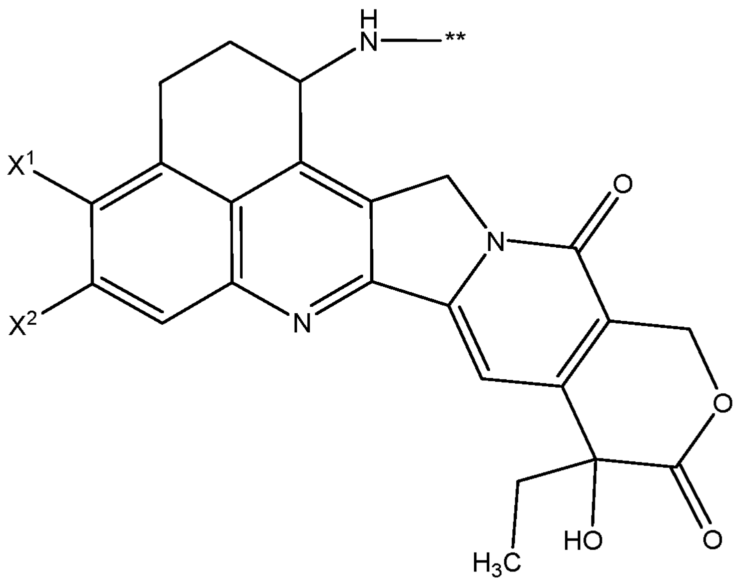

or

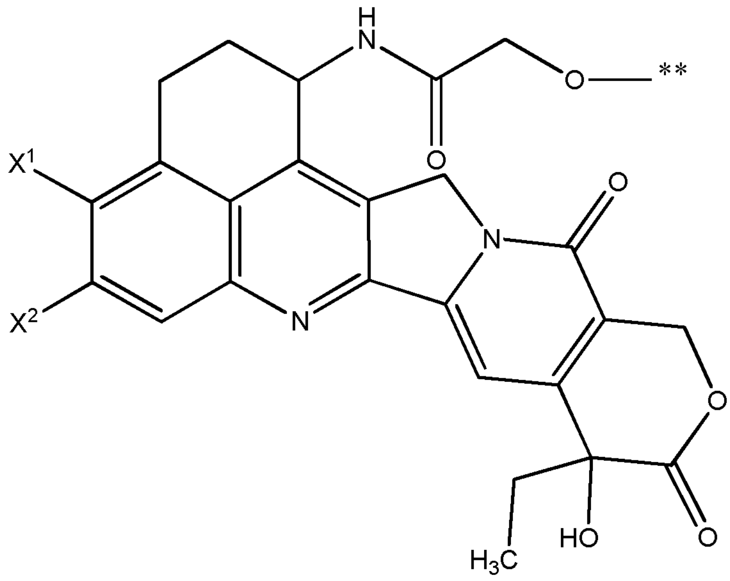

,

wherein $X^1$ and $X^2$ are each independently C1-C6 alkyl, halogen or -OH; ** links to L.

In one or more embodiments, the drug is

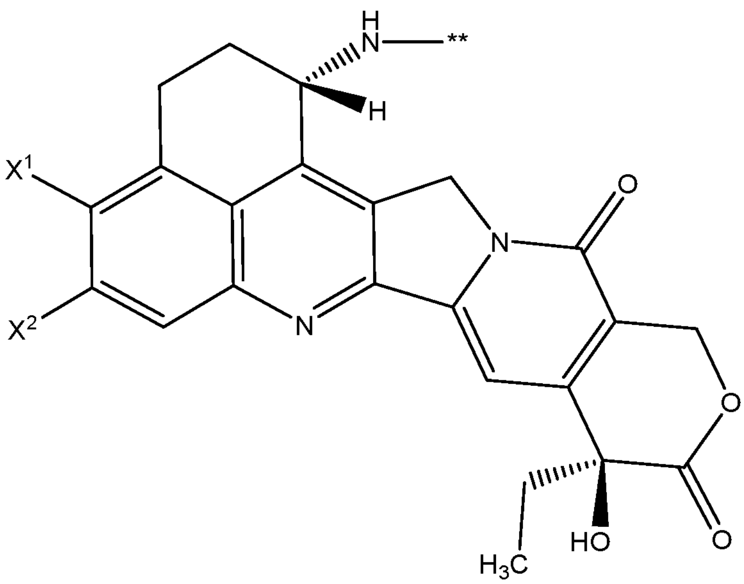

or

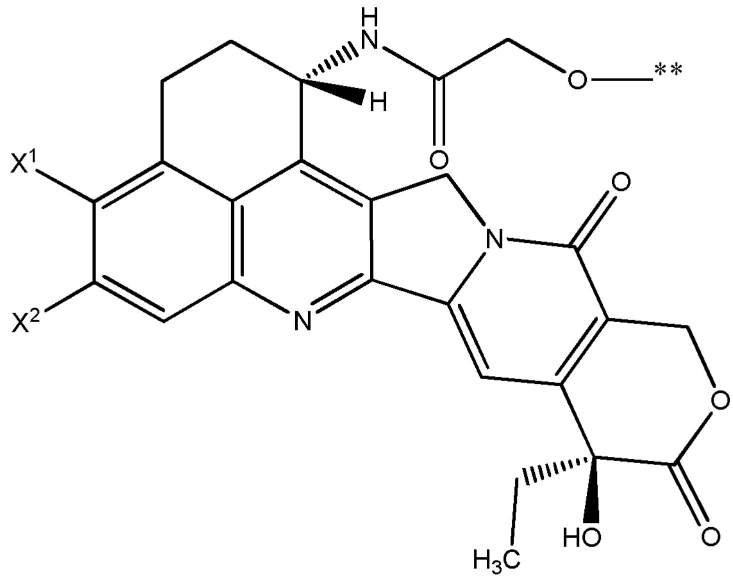

,

wherein $X^1$ and $X^2$ are each independently C1-C6 alkyl, halogen or -OH; ** links to L.

In one or more embodiments, $X^1$ and $X^2$ are each $-CH_3$.

In one or more embodiments, $X^1$ and $X^2$ are each independently F, Cl, Br or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$, and $X^2$ is F.

In one or more embodiments, R is $-(CH_2)_r-$.

In one or more embodiments, R is $-(CH_2)_r-$, wherein r is 1 or 5.

In one or more embodiments, each AA is independently selected from the following amino acid or peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu and Gly-Phe-Leu-Gly.

In one or more embodiments, AA is Val-Cit, and i is 1.

In one or more embodiments, each FF is independently

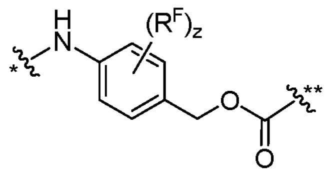

,

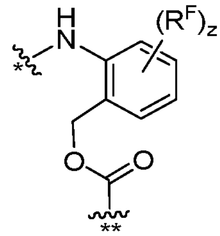

or

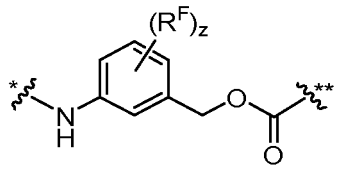

,

wherein each $R^F$ is independently C1-C6 alkyl, C1-C6 alkoxy, $-NO_2$ or halogen; wherein * links to AA, and ** links to D. In one or more embodiments, the halogen is F, and z is 0, 1, 2, 3 or 4.

In one or more embodiments, $R^F$ is $-CH_3$, F, $-NO_2$ or $-OCH_3$.

In one or more embodiments, z is 0.

In one or more embodiments, z is 1 or 2.

In one or more embodiments, each FF is independently

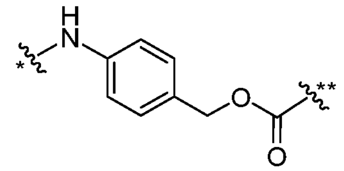,

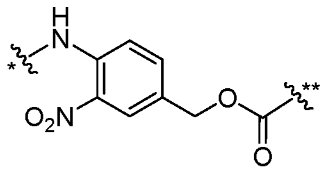, 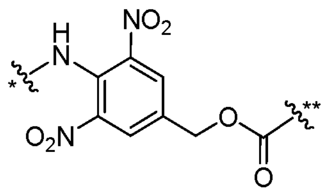, 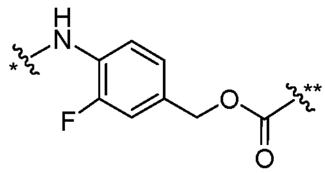,

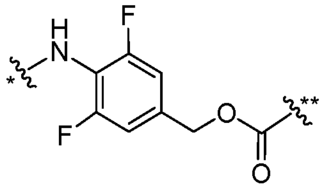, 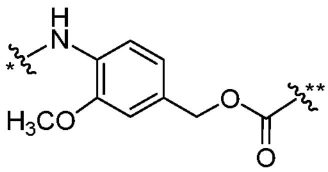, 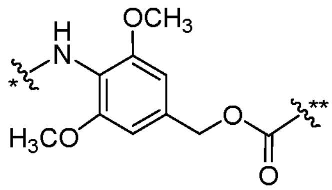,

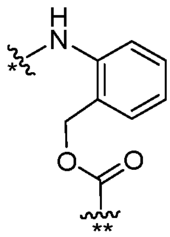, 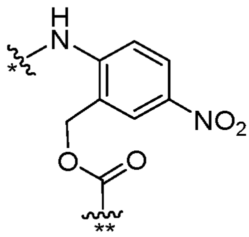, 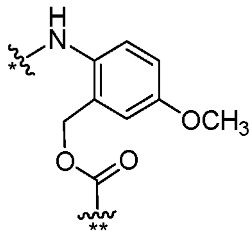, 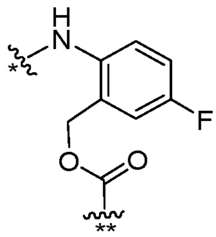,

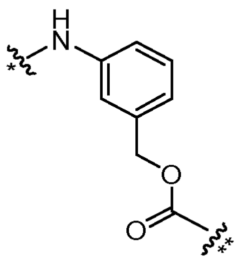, 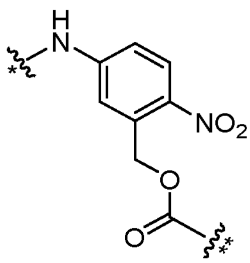, 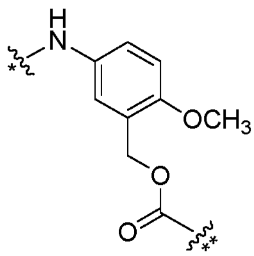 or 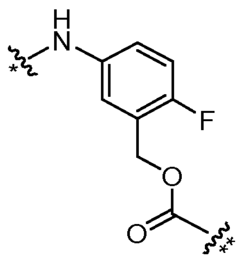;

wherein * links to AA, and ** links to D.

In one or more embodiments, f is 1.

In one or more embodiments, FF is

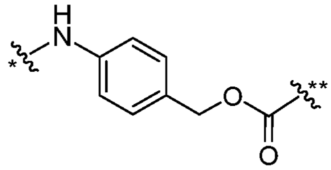,

and f is 1; wherein * links to AA, and ** links to D.

In one or more embodiments, L is

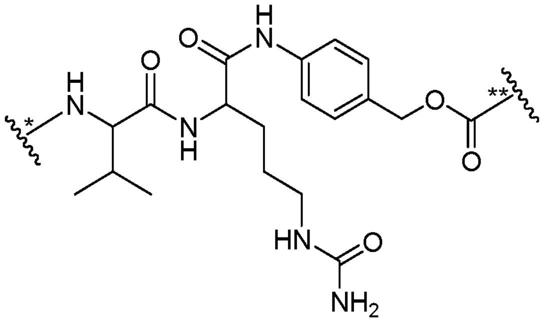

,

wherein * links to B, and ** links to D.

In one or more embodiments, L is

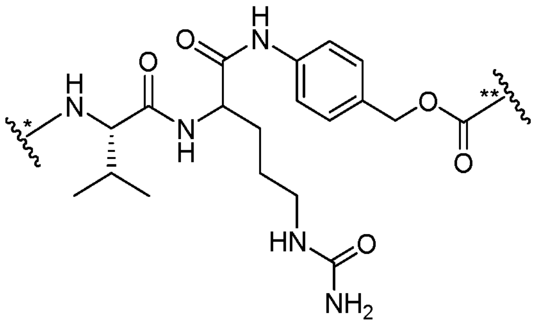

or

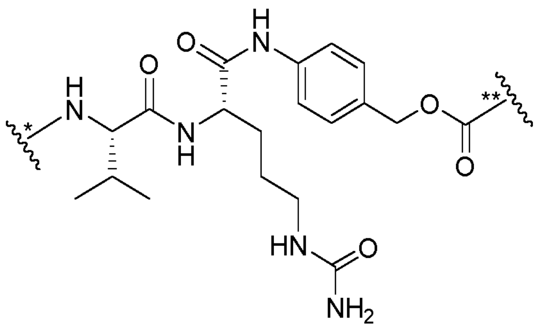

,

wherein * links to B, and ** links to D.

In one or more embodiments, G is

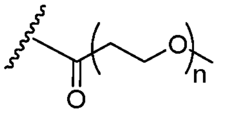

,

and n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, p is 7.

In one or more embodiments, p is 7.4.

In one or more embodiments, p is 8.

In one or more embodiments, Abu is the antibody or the antigen-binding unit thereof provided by the present invention.

In one or more embodiments, the antibody-drug conjugate has a structure shown as Formula I-1 or a stereoisomer thereof, or a pharmaceutically acceptable salt or solvate thereof:

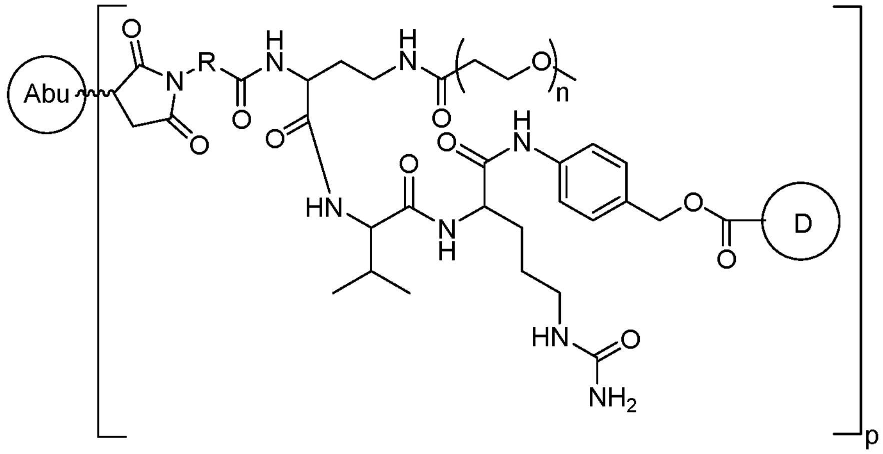

Formula I-1

wherein

Abu is the antibody or the antigen-binding unit thereof provided by the present invention;

R is selected from: $-(CH_2)_r-$, $-(CHR^m)_r-$, C3-C8 carbocyclyl, $-O-(CH_2)_r-$, arylene, $-(CH_2)_r$-arylene-, -arylene-$(CH_2)_r-$, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

D is a drug;

n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;

p is 1-10, e.g., 1, 2, 3, 4, 5, 6, 7, 7.4, 8, 9 or 10.

In one or more embodiments, the drug is an anti-cancer drug, a cytotoxic drug, a cell differentiation factor, a stem cell trophic factor, a steroid drug, a drug for treating autoimmune diseases, an anti-inflammatory drug or a drug for treating infectious diseases.

In one or more embodiments, the drug is a tubulin inhibitor, a DNA damaging agent or a DNA topoisomerase inhibitor.

In one or more embodiments, the tubulin inhibitor is selected from dolastatin, auristatins and maytansinoids.

In one or more embodiments, the drug is an auristatin, e.g., MMAE, MMAF or AF.

In one or more embodiments, the drug is a DNA damaging agent, e.g., a calicheamicin, a duocarmycin or an anthramycin derivative PBD (pyrrolobenzodiazepine).

In one or more embodiments, the drug is a DNA topoisomerase inhibitor or a salt thereof, e.g., irinotecan, irinotecan hydrochloride, camptothecin, 9-aminocamptothecin, 9-nitrocamptothecin, 10-hydroxy camptothecin, 9-chloro-10-hydroxy camptothecin, a camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2E)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, N-[2-(dimethylamino)ethyl]-4-acridinecarboxamide dihydrochloride or N-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine or exatecan. In one or more embodiments, the drug is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan or a chlorambucil derivative.

In one or more embodiments, the drug is

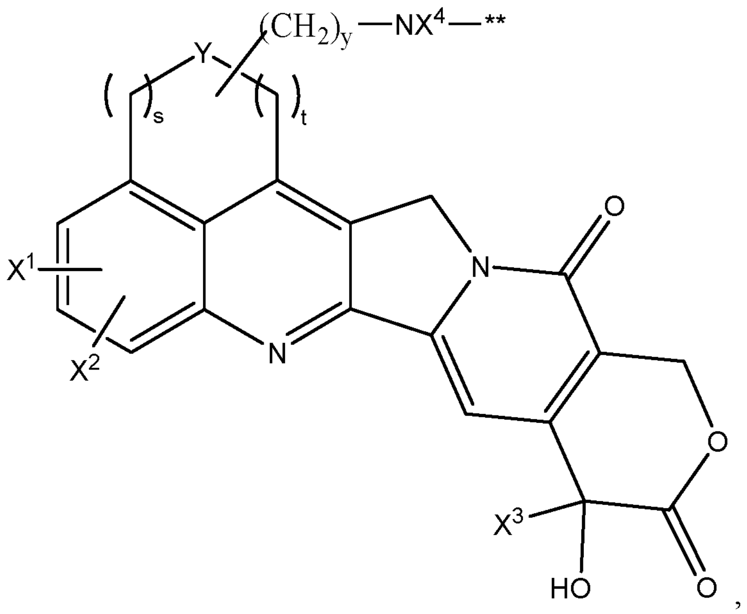

wherein $X^1$ and $X^2$ are each independently:

H,
hydroxy,
C1-C6 alkyl,
C1-C6 alkyl substituted with one or more hydroxy, halogen, nitro or cyano groups,
C2-C6 alkenyl,
C2-C6 alkynyl,
C1-C6 alkoxy,
C1-C6 aminoalkoxy,
halogen,
nitro,
cyano,
thiol,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocyclic ring, wherein the heterocyclic ring is optionally substituted with one or more C1-C6 alkyl, C1-C6 alkoxy, amino, halogen, nitro or cyano groups,
C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or a protecting group,
amino-substituted heterocyclyl optionally substituted at a nitrogen atom of the heterocyclyl moiety or at the amino moiety with a protecting group or one or more C1-C6 alkyl groups, heterocyclylamino optionally substituted at a nitrogen atom of the heterocyclyl moiety or at the amino moiety with a protecting group or C1-C6 alkyl,
carbamoyl optionally substituted with a carbamoyl-protecting group or C1-C6 alkyl, morpholin-1-yl, or piperidin-1-yl;
$X^3$ is C1-C6 alkyl;
$X^4$ is H, $-(CH_2)_q-CH_3$, $-(CHR^n)_q-CH_3$, C3-C8 carbocyclyl, $-O-(CH_2)_q-CH_3$, arylene-$CH_3$, - $(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_qC(O)NR^n(CH_2)_q-CH_3$,

$-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or $-(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;

** links to other moieties of the antibody-drug conjugate;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, t-butoxycarbonyl, benzyl, or *p*-methoxybenzyloxycarbonyl.

In one or more embodiments, the drug is

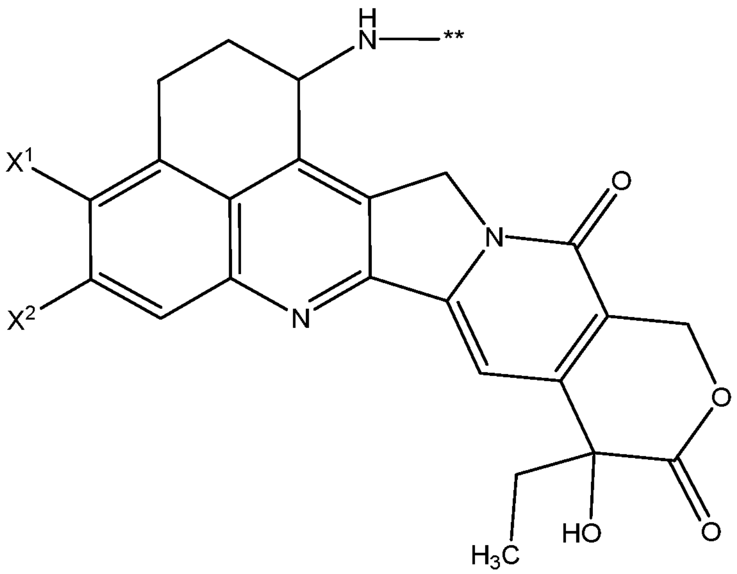

or

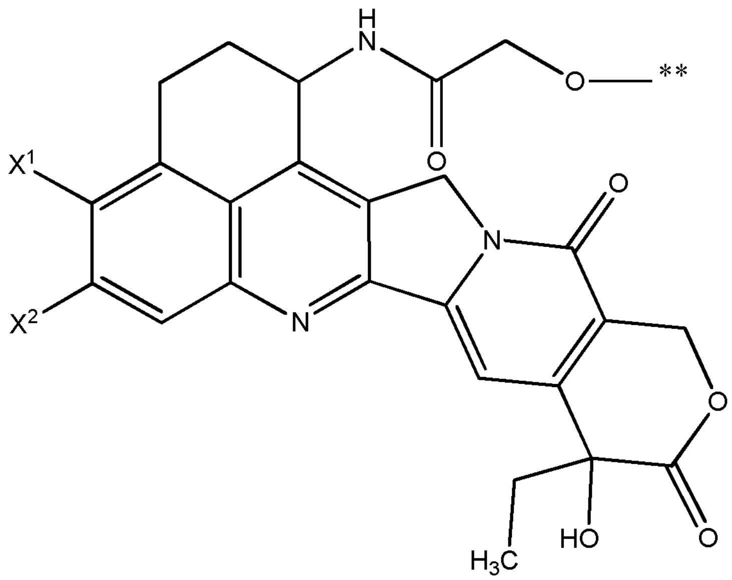

,

wherein $X^1$ and $X^2$ are each independently C1-C6 alkyl, halogen or -OH; ** links to other moieties of the antibody-drug conjugate.

In one or more embodiments, the drug is

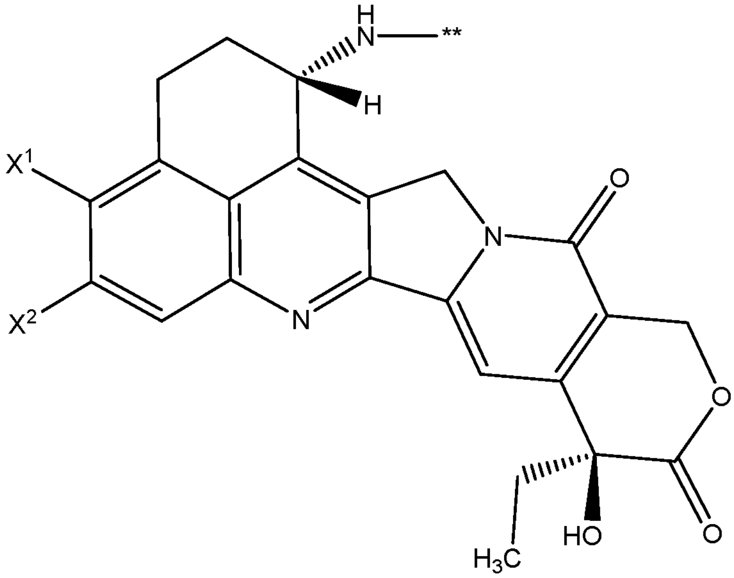

or

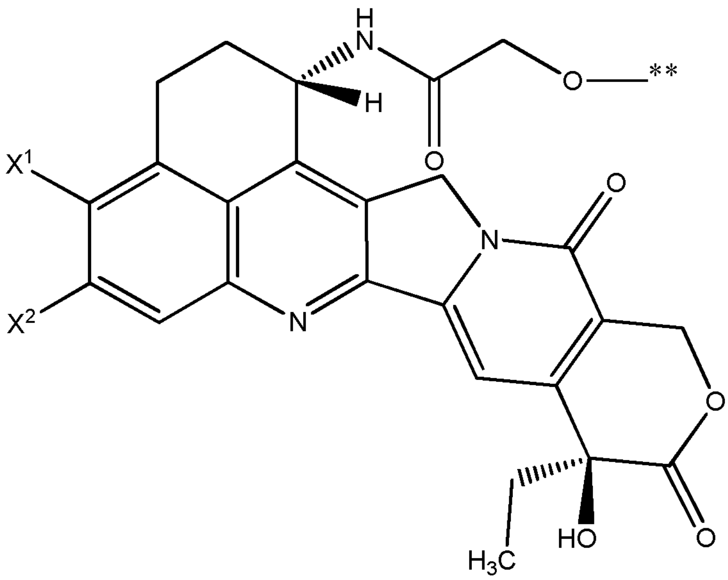

,

wherein $X^1$ and $X^2$ are each independently C1-C6 alkyl, halogen or -OH; ** links to other moieties of the antibody-drug conjugate.

In one or more embodiments, $X^1$ and $X^2$ are each $-CH_3$.

In one or more embodiments, $X^1$ and $X^2$ are each independently F, Cl, Br or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$, and $X^2$ is F.

In one or more embodiments, R is $-(CH_2)_r-$.

In one or more embodiments, R is $-(CH_2)_r-$, wherein r is 1 or 5.

In one or more embodiments, n is 4-12.

In one or more embodiments, n is 4-8.

In one or more embodiments, n is 4.
In one or more embodiments, n is 8.
In one or more embodiments, p is 2-8.
In one or more embodiments, p is 4-8.
In one or more embodiments, p is 6-8.
In one or more embodiments, p is 7-8.
In one or more embodiments, p is 7.
In one or more embodiments, p is 7.4.
In one or more embodiments, p is 8.
In one or more embodiments, the antibody-drug conjugate has a structure shown as Formula I-2 or I-2-1 or a stereoisomer thereof, or a pharmaceutically acceptable salt or solvate thereof:

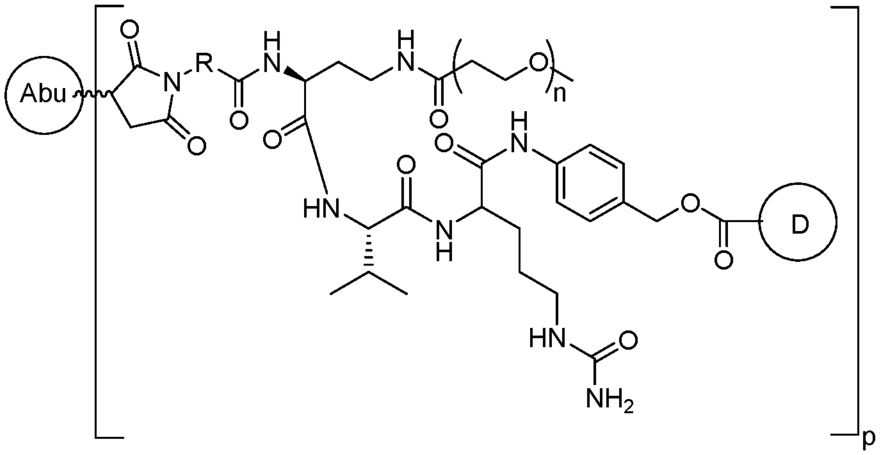

Formula I-2

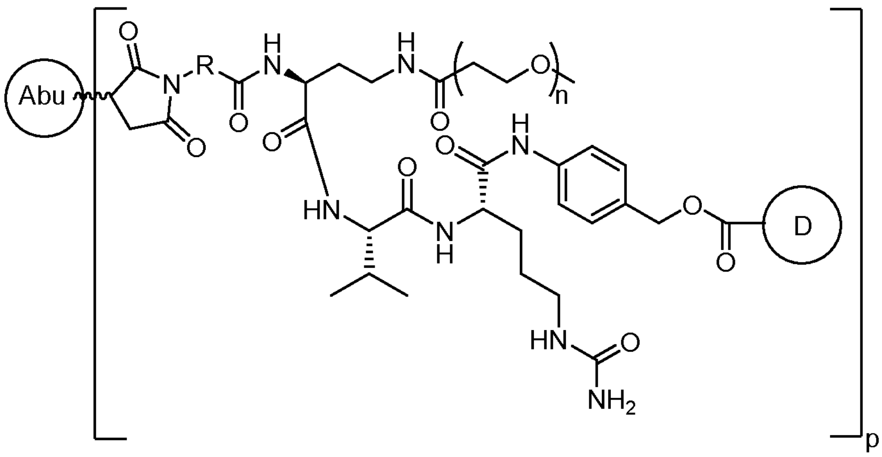

Formula I-2-1

wherein

Abu is the antibody or the antigen-binding unit thereof provided by the present invention;
R is selected from: $-(CH_2)_r-$, $-(CHR^m)_r-$, C3-C8 carbocyclyl, $-O-(CH_2)_r-$, arylene, $-(CH_2)_r$-arylene-, -arylene-$(CH_2)_r-$, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;
D is a drug;
n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;

p is 1-10, e.g., 1, 2, 3, 4, 5, 6, 7, 7.4, 8, 9 or 10.

In one or more embodiments, the drug is an anti-cancer drug, a cytotoxic drug, a cell differentiation factor, a stem cell trophic factor, a steroid drug, a drug for treating autoimmune diseases, an anti-inflammatory drug or a drug for treating infectious diseases.

In one or more embodiments, the drug is a tubulin inhibitor, a DNA damaging agent or a DNA topoisomerase inhibitor.

In one or more embodiments, the tubulin inhibitor is selected from dolastatin, auristatins and maytansinoids.

In one or more embodiments, the drug is an auristatin, e.g., MMAE, MMAF or AF.

In one or more embodiments, the drug is a DNA damaging agent, e.g., a calicheamicin, a duocarmycin or an anthramycin derivative PBD (pyrrolobenzodiazepine).

In one or more embodiments, the drug is a DNA topoisomerase inhibitor or a salt thereof, e.g., irinotecan, irinotecan hydrochloride, camptothecin, 9-aminocamptothecin, 9-nitrocamptothecin, 10-hydroxy camptothecin, 9-chloro-10-hydroxy camptothecin, a camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2E)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, *N*-[2-(dimethylamino)ethyl]-4-acridinecarboxamide dihydrochloride or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine or exatecan. In one or more embodiments, the drug is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan or a chlorambucil derivative.

In one or more embodiments, the drug is

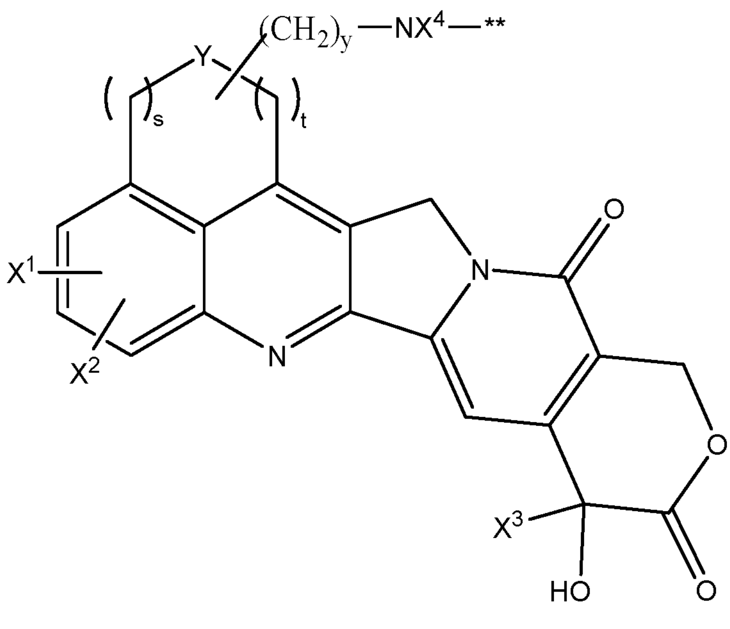,

wherein $X^1$ and $X^2$ are each independently:

H,
hydroxy,
C1-C6 alkyl,
C1-C6 alkyl substituted with one or more hydroxy, halogen, nitro or cyano groups,
C2-C6 alkenyl,
C2-C6 alkynyl,
C1-C6 alkoxy,
C1-C6 aminoalkoxy,
halogen,

nitro,
cyano,
thiol,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocyclic ring, wherein the heterocyclic ring is optionally substituted with one or more C1-C6 alkyl, C1-C6 alkoxy, amino, halogen, nitro or cyano groups,
C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or a protecting group,
amino-substituted heterocyclyl optionally substituted at a nitrogen atom of the heterocyclyl moiety or at the amino moiety with a protecting group or one or more C1-C6 alkyl groups, heterocyclylamino optionally substituted at a nitrogen atom of the heterocyclyl moiety or at the amino moiety with a protecting group or C1-C6 alkyl,
carbamoyl optionally substituted with a carbamoyl-protecting group or C1-C6 alkyl, morpholin-1-yl, or piperidin-1-yl;
$X^3$ is C1-C6 alkyl;
$X^4$ is H, $-(CH_2)_q-CH_3$, $-(CHR^n)_q-CH_3$, C3-C8 carbocyclyl, $-O-(CH_2)_q-CH_3$, arylene-$CH_3$, - $(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_{qC}(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, - $(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or - $(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;
** links to other moieties of the antibody-drug conjugate;
y is 0, 1 or 2;
Y is O, S or $CR^1R^2$, wherein $R^1$ and $R^2$ are each independently H or C1-C6 alkyl;
s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, t-butoxycarbonyl, benzyl, or *p*-methoxybenzyloxycarbonyl.

In one or more embodiments, the drug is

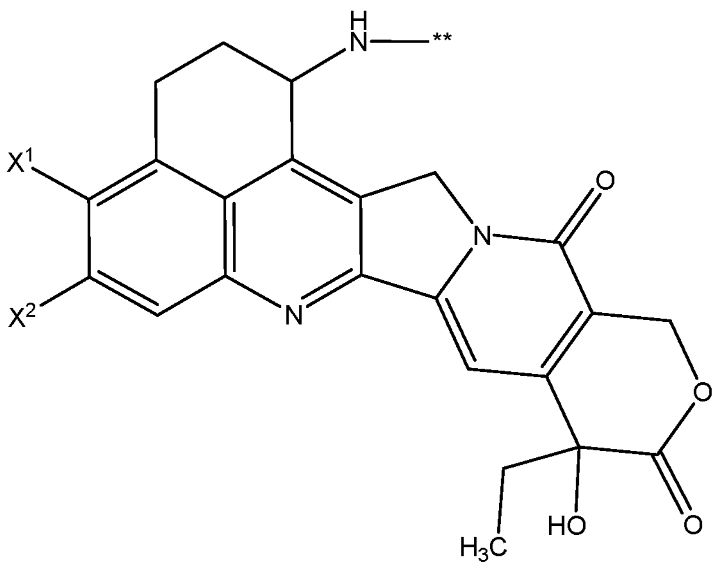

or

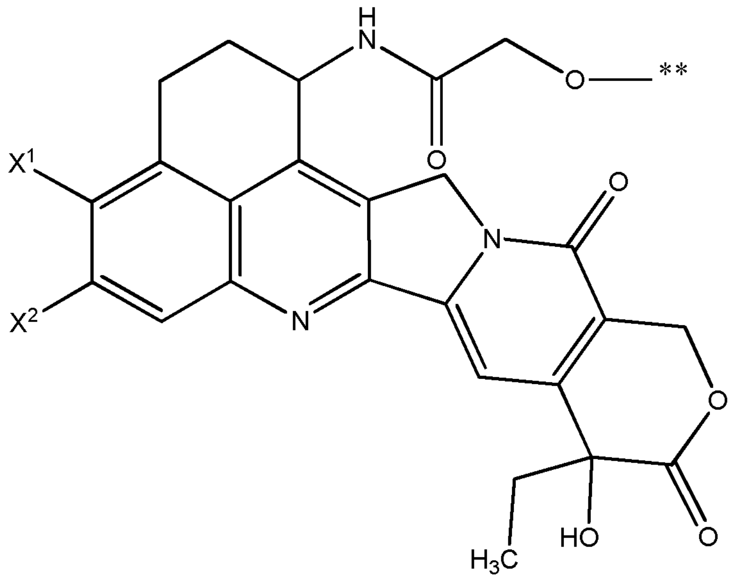

,

wherein $X^1$ and $X^2$ are each independently C1-C6 alkyl, halogen or -OH; ** links to other moieties of the antibody-drug conjugate.

In one or more embodiments, the drug is

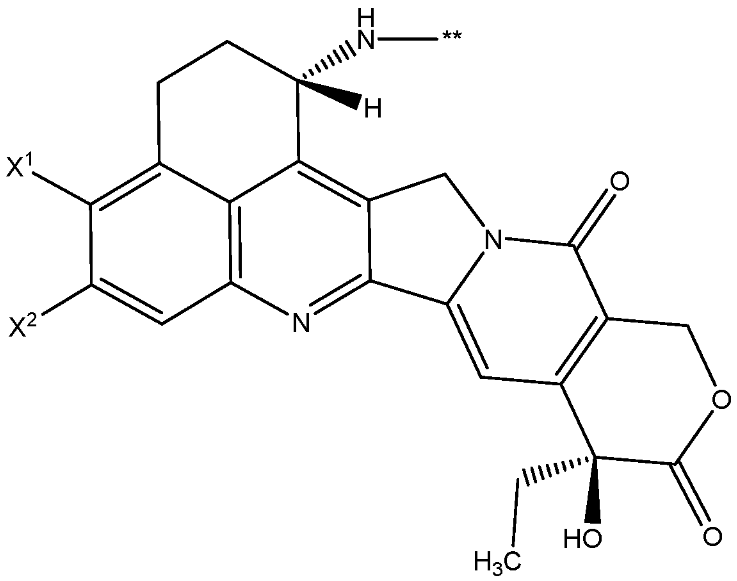

or

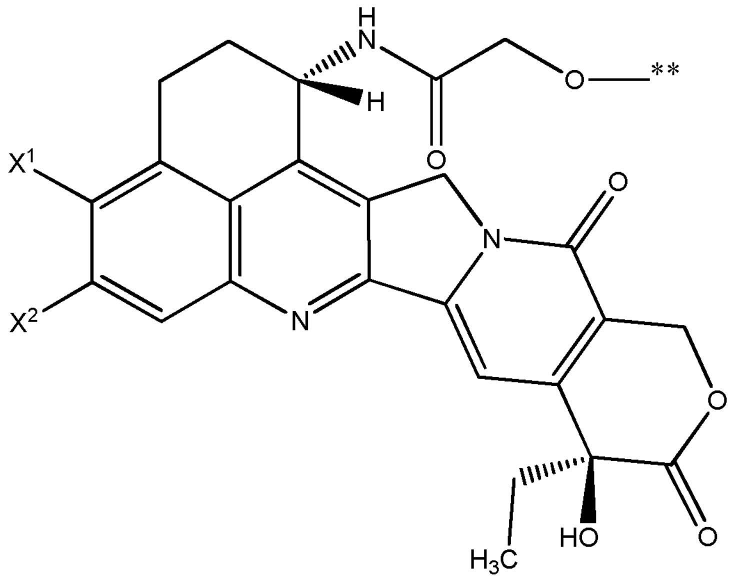

,

wherein $X^1$ and $X^2$ are each independently C1-C6 alkyl, halogen or -OH; ** links to other moieties of the antibody-drug conjugate.

In one or more embodiments, $X^1$ and $X^2$ are each $-CH_3$.

In one or more embodiments, $X^1$ and $X^2$ are each independently F, Cl, Br or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$, and $X^2$ is F.

In one or more embodiments, R is $-(CH_2)_r-$.

In one or more embodiments, R is $-(CH_2)_r-$, wherein r is 1 or 5.

In one or more embodiments, n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, p is 7.

In one or more embodiments, p is 7.4.

In one or more embodiments, p is 8.

In one or more embodiments, the antibody-drug conjugate has a structure shown as Formula I-3 or a stereoisomer thereof, or a pharmaceutically acceptable salt or solvate thereof:

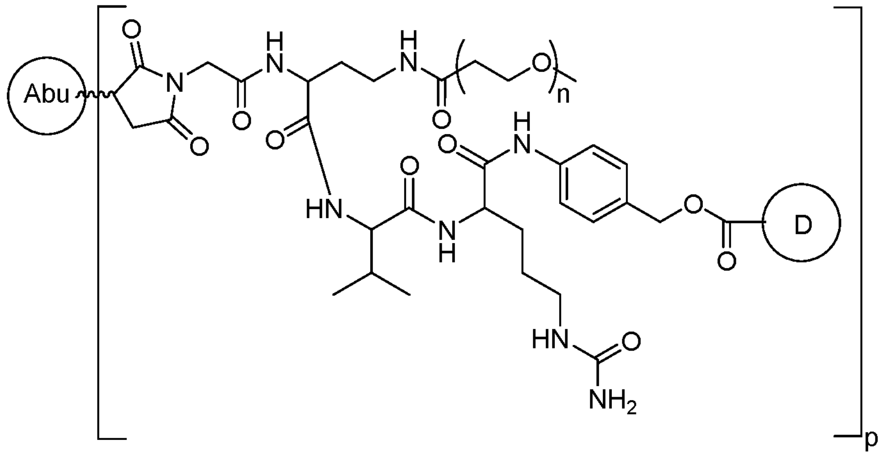

Formula I-3

wherein

Abu is the antibody or the antigen-binding unit thereof provided by the present invention;
D is a drug;
n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;
p is 1-10, e.g., 1, 2, 3, 4, 5, 6, 7, 7.4, 8, 9 or 10.

In one or more embodiments, the drug is an anti-cancer drug, a cytotoxic drug, a cell differentiation factor, a stem cell trophic factor, a steroid drug, a drug for treating autoimmune diseases, an anti-inflammatory drug or a drug for treating infectious diseases.

In one or more embodiments, the drug is a tubulin inhibitor, a DNA damaging agent or a DNA topoisomerase inhibitor.

In one or more embodiments, the tubulin inhibitor is selected from dolastatin, auristatins and maytansinoids.

In one or more embodiments, the drug is an auristatin, e.g., MMAE, MMAF or AF.

In one or more embodiments, the drug is a DNA damaging agent, e.g., a calicheamicin, a duocarmycin or an anthramycin derivative PBD (pyrrolobenzodiazepine).

In one or more embodiments, the drug is a DNA topoisomerase inhibitor or a salt thereof, e.g., irinotecan, irinotecan hydrochloride, camptothecin, 9-aminocamptothecin, 9-nitrocamptothecin, 10-hydroxycamptothecin, 9-chloro-10-hydroxycamptothecin, a camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2E)-2-propenamide, 2-cyano-3-(3,4-dihydroxyphenyl)-A-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, *N*-[2-(dimethylamino)ethyl]-4-acridinecarboxamide dihydrochloride or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine or exatecan. In one or more embodiments, the drug is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan or a chlorambucil derivative.

In one or more embodiments, the drug is

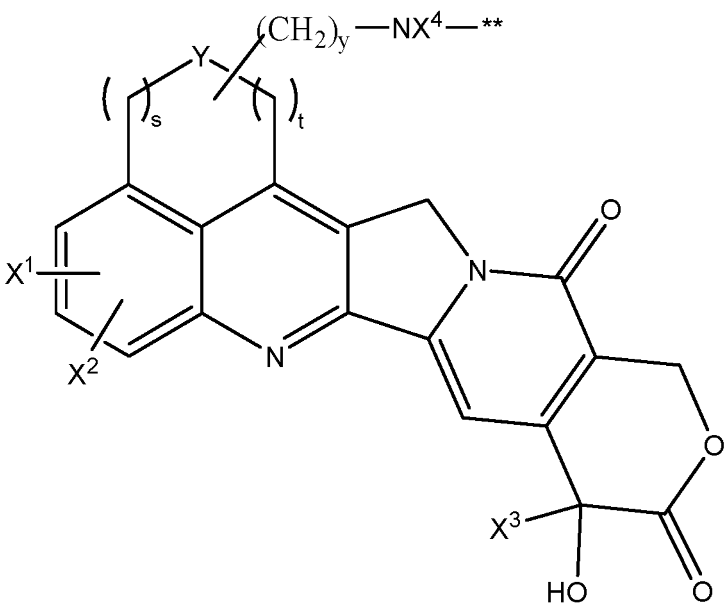

,

wherein $X^1$ and $X^2$ are each independently:

H,
hydroxy,
C1-C6 alkyl,
C1-C6 alkyl substituted with one or more hydroxy, halogen, nitro or cyano groups,
C2-C6 alkenyl,
C2-C6 alkynyl,
C1-C6 alkoxy,
C1-C6 aminoalkoxy,
halogen,
nitro,
cyano,
thiol,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocyclic ring, wherein the heterocyclic ring is optionally substituted with one or more C1-C6 alkyl, C1-C6 alkoxy, amino, halogen, nitro or cyano groups,
C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or a protecting group,
amino-substituted heterocyclyl optionally substituted at a nitrogen atom of the heterocyclyl moiety or at the amino moiety with a protecting group or one or more C1-C6 alkyl groups, heterocyclylamino optionally substituted at a nitrogen atom of the heterocyclyl moiety or at the amino moiety with a protecting group or C1-C6 alkyl,
carbamoyl optionally substituted with a carbamoyl-protecting group or C1-C6 alkyl, morpholin-1-yl, or piperidin-1-yl;
$X^3$ is C1-C6 alkyl;
$X^4$ is H, $-(CH_2)_q-CH_3$, $-(CHR^n)_q-CH_3$, C3-C8 carbocyclyl, $-O-(CH_2)_q-CH_3$, arylene-$CH_3$, - $(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_{qC}(O)NR^n(CH_2)_q-CH_3$, $-(CH_2CH_2O)_q-CH_3$, $-(CH_{2C}H_{20})_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, - $(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or - $(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;
** links to other moieties of the antibody-drug conjugate;

y is 0, 1 or 2;
Y is O, S or $CR^1R^2$, wherein $R^1$ and $R^2$ are each independently H or C1-C6 alkyl;
s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, t-butoxycarbonyl, benzyl, or *p*-methoxybenzyloxycarbonyl.

In one or more embodiments, the drug is

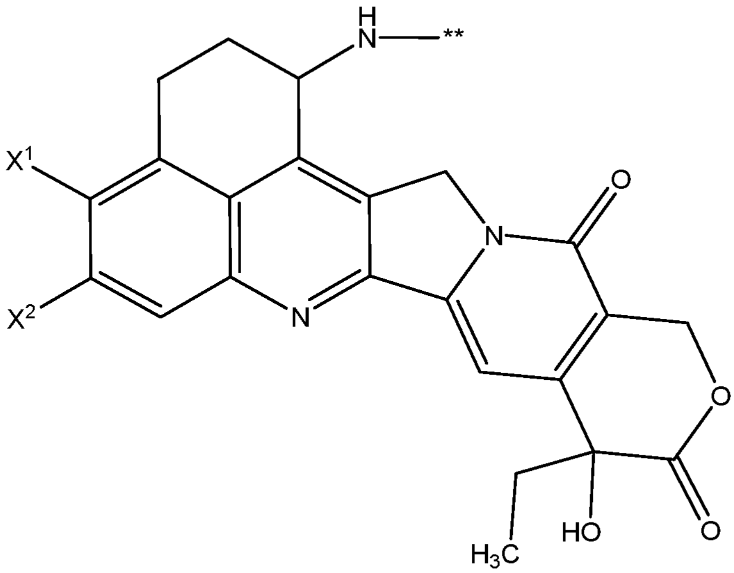

or

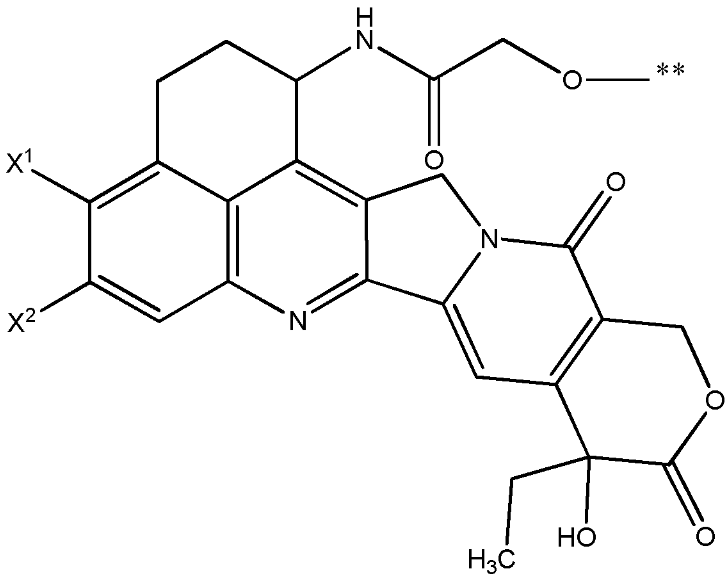

,

wherein $X^1$ and $X^2$ are each independently C1-C6 alkyl, halogen or -OH; ** links to other moieties of the antibody-drug conjugate.

In one or more embodiments, the drug is

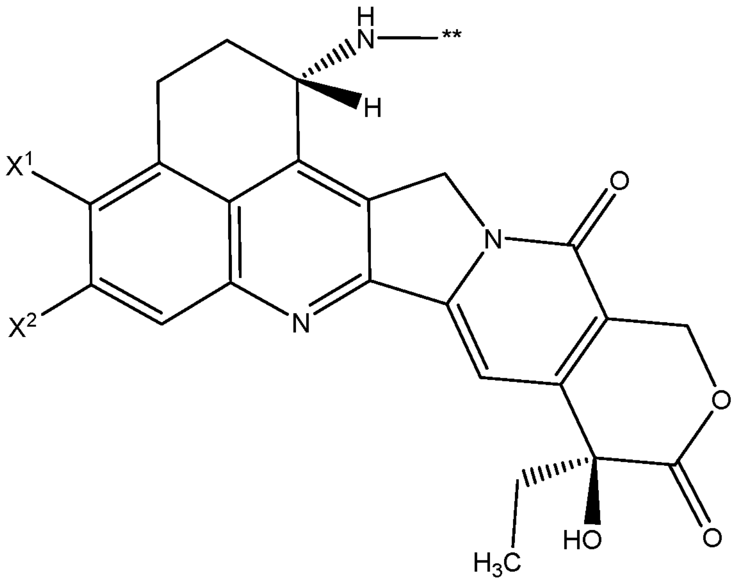

or

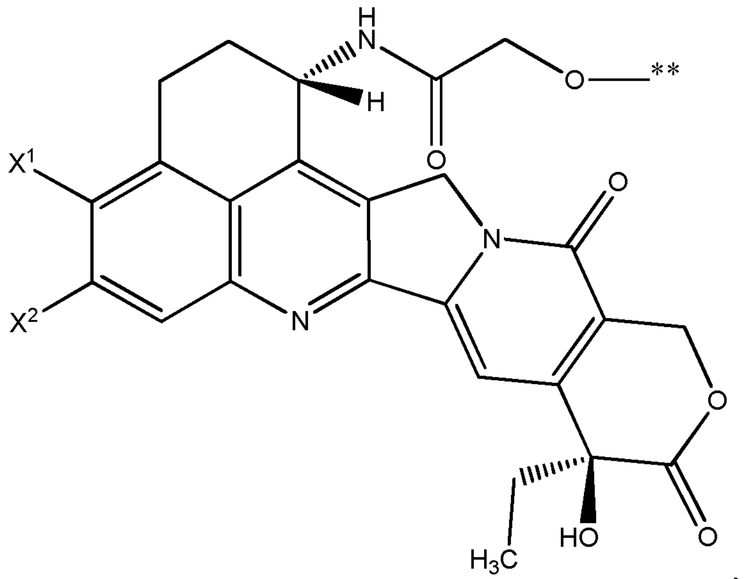

,

wherein $X^1$ and $X^2$ are each independently C1-C6 alkyl, halogen or -OH; ** links to other moieties of the antibody-drug conjugate.

In one or more embodiments, $X^1$ and $X^2$ are each $-CH_3$.

In one or more embodiments, $X^1$ and $X^2$ are each independently F, Cl, Br or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$, and $X^2$ is F.

In one or more embodiments, n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, p is 7.
In one or more embodiments, p is 7.4.
In one or more embodiments, p is 8.
In one or more embodiments, the antibody-drug conjugate has a structure shown as Formula I-4 or I-4-1 or a stereoisomer thereof, or a pharmaceutically acceptable salt or solvate thereof:

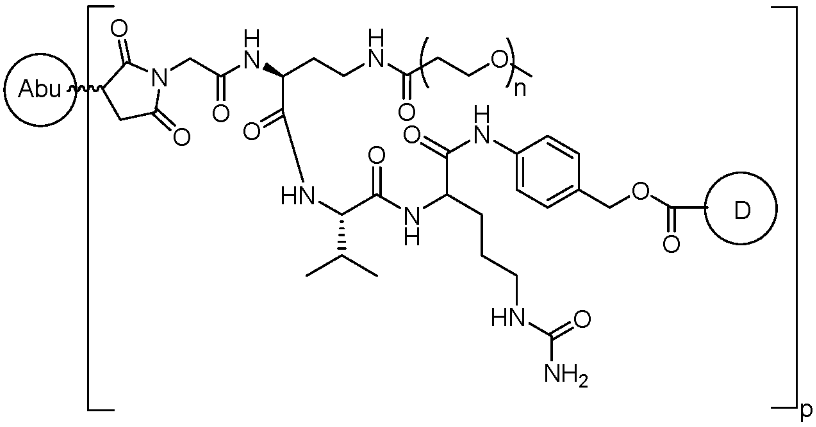

Formula I-4

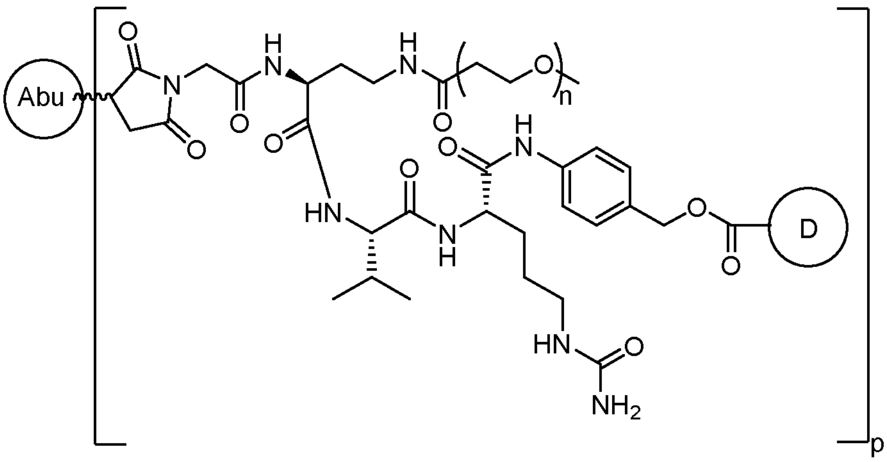

Formula I-4-1

wherein

Abu is the antibody or the antigen-binding unit thereof provided by the present invention;
D is a drug;
n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;
p is 1-10, e.g., 1, 2, 3, 4, 5, 6, 7, 7.4, 8, 9 or 10.

In one or more embodiments, the drug is an anti-cancer drug, a cytotoxic drug, a cell differentiation factor, a stem cell trophic factor, a steroid drug, a drug for treating autoimmune diseases, an anti-inflammatory drug or a drug for treating infectious diseases.
In one or more embodiments, the drug is a tubulin inhibitor, a DNA damaging agent or a DNA topoisomerase inhibitor.
In one or more embodiments, the tubulin inhibitor is selected from dolastatin, auristatins and maytansinoids.
In one or more embodiments, the drug is an auristatin, e.g., MMAE, MMAF or AF.
In one or more embodiments, the drug is a DNA damaging agent, e.g., a calicheamicin, a duocarmycin, an anthramycin derivative PBD (pyrrolobenzodiazepine) or a DNA topoisomerase inhibitor.
In one or more embodiments, the drug is a DNA topoisomerase inhibitor or a salt thereof, e.g., irinotecan,

irinotecan hydrochloride, camptothecin, 9-aminocamptothecin, 9-nitrocamptothecin, 10-hydroxycamptothecin, 9-chloro-10-hydroxycamptothecin, a camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2E)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, *N*-[2-(dimethylamino)ethyl]-4-acridinecarboxamide dihydrochloride or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine or exatecan. In one or more embodiments, the drug is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan or a chlorambucil derivative.

In one or more embodiments, the drug is

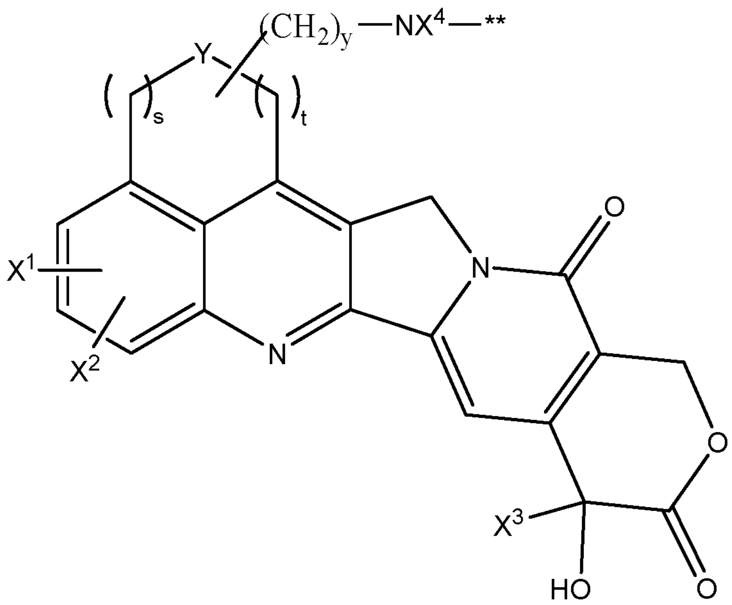
,

wherein $X^1$ and $X^2$ are each independently:

- H,
- hydroxy,
- C1-C6 alkyl,
- C1-C6 alkyl substituted with one or more hydroxy, halogen, nitro or cyano groups,
- C2-C6 alkenyl,
- C2-C6 alkynyl,
- C1-C6 alkoxy,
- C1-C6 aminoalkoxy,
- halogen,
- nitro,
- cyano,
- thiol,
- alkylthio,
- amino, amino substituted with an amino-protecting group, C1-C6 aminoalkyl optionally substituted at the amino moiety with an amino-protecting group or C1-C6 alkyl,
- C1-C6 aminoalkylamino optionally substituted at the amino moiety with an amino-protecting group or C1-C6 alkyl,
- C1-C6 alkyl linking to a heterocyclic ring, wherein the heterocyclic ring is optionally substituted with one or more C1-C6 alkyl, C1-C6 alkoxy, amino, halogen, nitro or cyano groups,
- C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or a protecting group,

amino-substituted heterocyclyl optionally substituted at a nitrogen atom of the heterocyclyl moiety or at the amino moiety with a protecting group or one or more C1-C6 alkyl groups, heterocyclylamino optionally substituted at a nitrogen atom of the heterocyclyl moiety or at the amino moiety with a protecting group or C1-C6 alkyl, carbamoyl optionally substituted with a carbamoyl-protecting group or C1-C6 alkyl, morpholin-1-yl, or piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, - $(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_{qC}(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, - $(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or - $(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;

** links to other moieties of the antibody-drug conjugate;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, *t*-butoxycarbonyl, benzyl, or *p*-methoxybenzyloxycarbonyl.

In one or more embodiments, the drug is

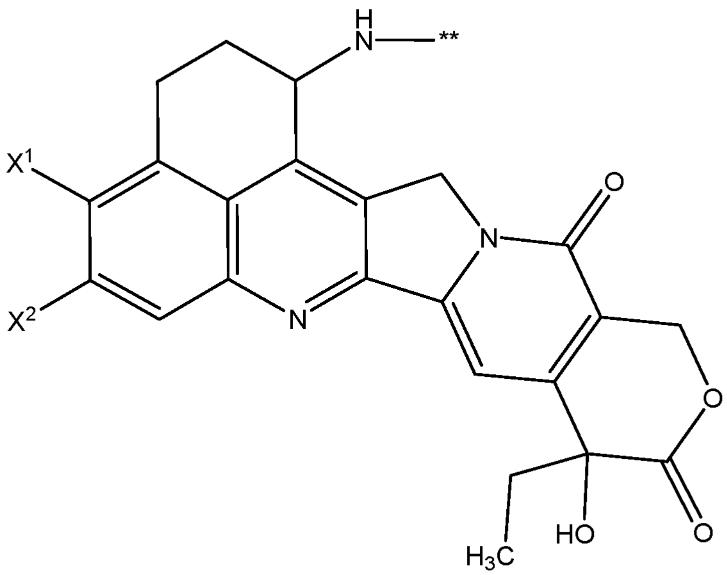

or

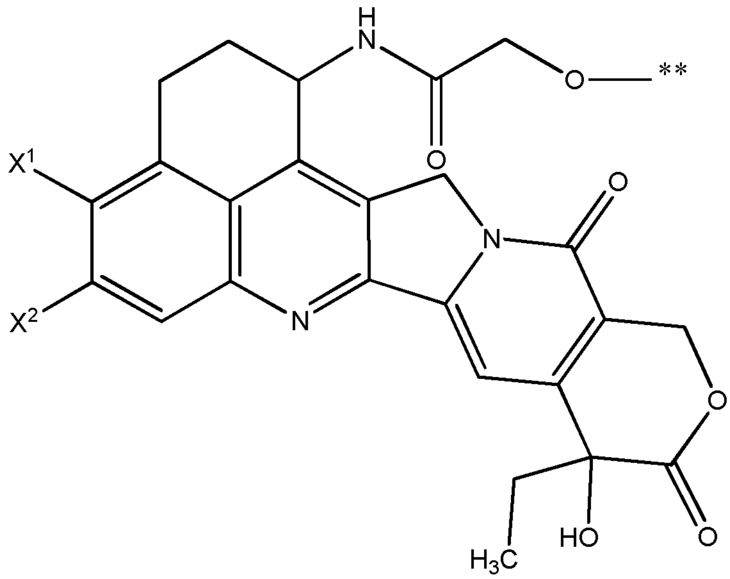

,

wherein $X^1$ and $X^2$ are each independently C1-C6 alkyl, halogen or -OH; ** links to other moieties of the antibody-drug conjugate.

In one or more embodiments, the drug is

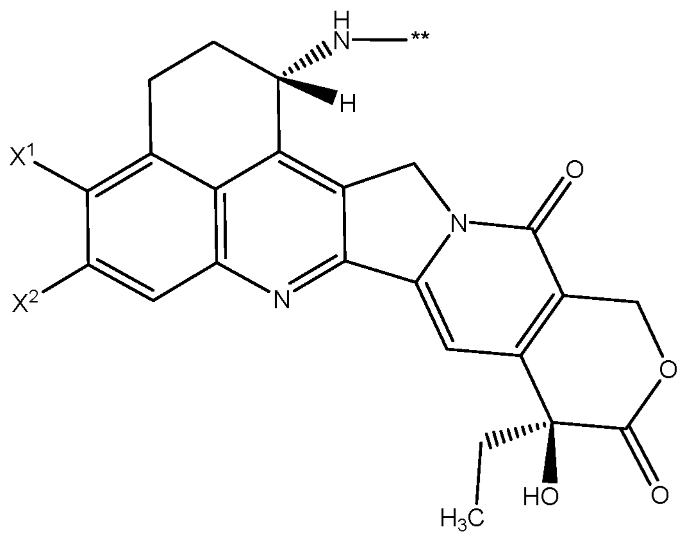

or

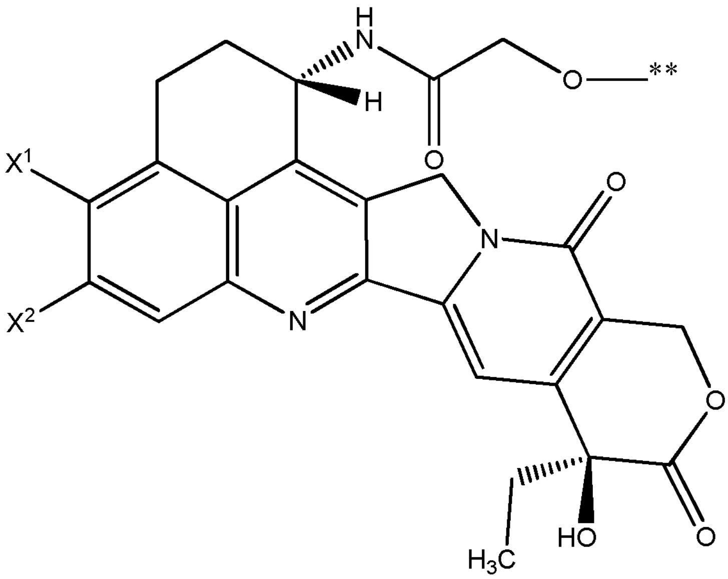

,

wherein $X^1$ and $X^2$ are each independently C1-C6 alkyl, halogen or -OH; ** links to other moieties of the antibody-drug conjugate.

In one or more embodiments, $X^1$ and $X^2$ are each independently $-CH_3$, F, Cl, Br or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each $-CH_3$.

In one or more embodiments, $X^1$ and $X^2$ are each independently F or $-CH_3$.

In one or more embodiments, $X^1$ is $-CH_3$, and $X^2$ is F.

In one or more embodiments, n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, p is 7.

In one or more embodiments, p is 7.4.

In one or more embodiments, p is 8.

In one or more embodiments, the antibody-drug conjugate has a structure shown as Formula I-5, I-5-1, I-6, I-6-1, I-7, I-7-1, I-8, I-8-1, I-9, I-9-1, I-10, I-10-1, I-11 or I-11-1 or a stereoisomer thereof, or a pharmaceutically acceptable salt or solvate thereof:

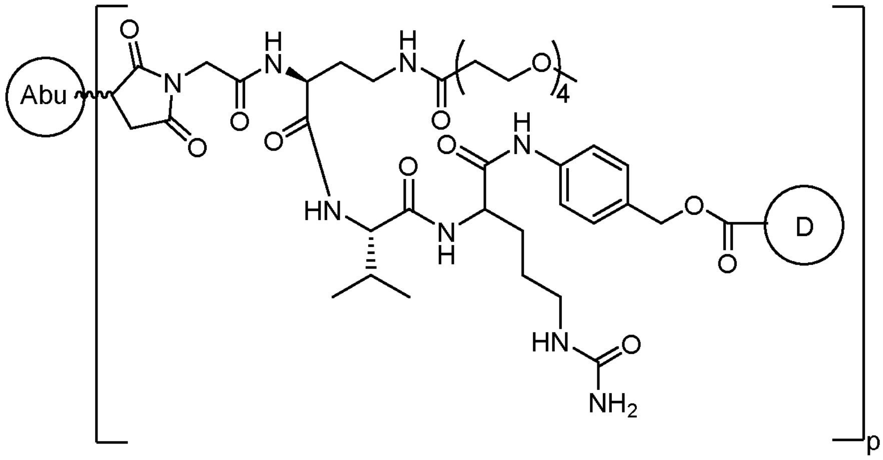

Formula I-5

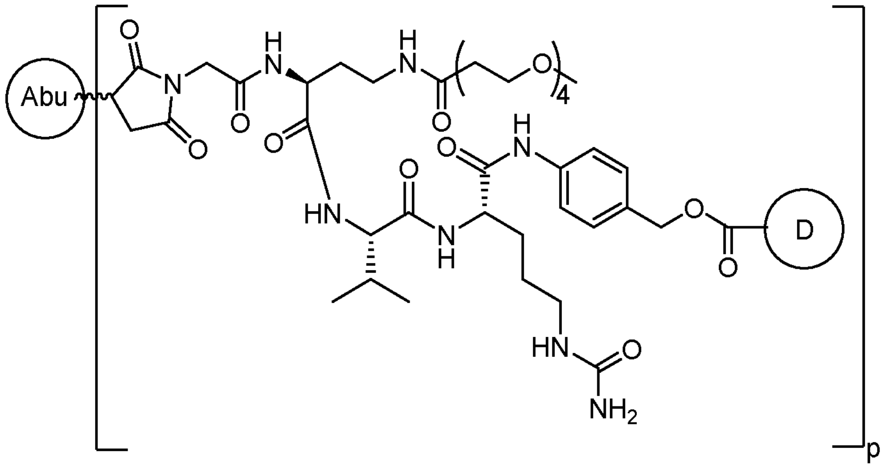

Formula I-5-1

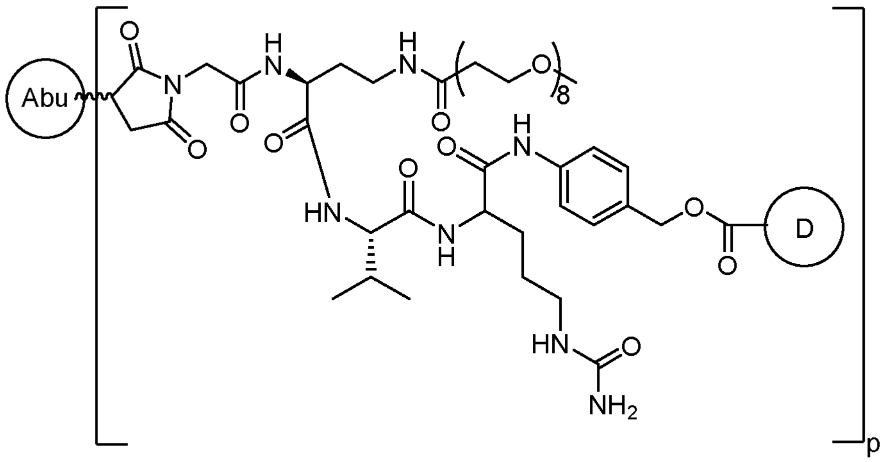

Formula I-6

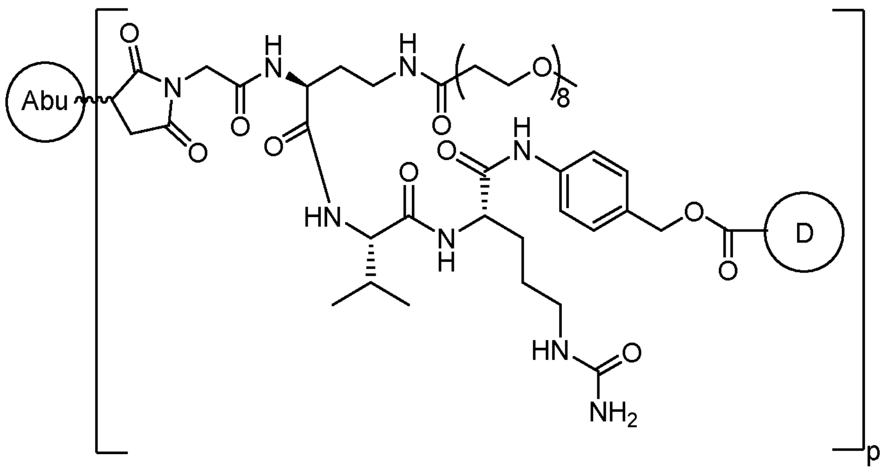

Formula I-6-1

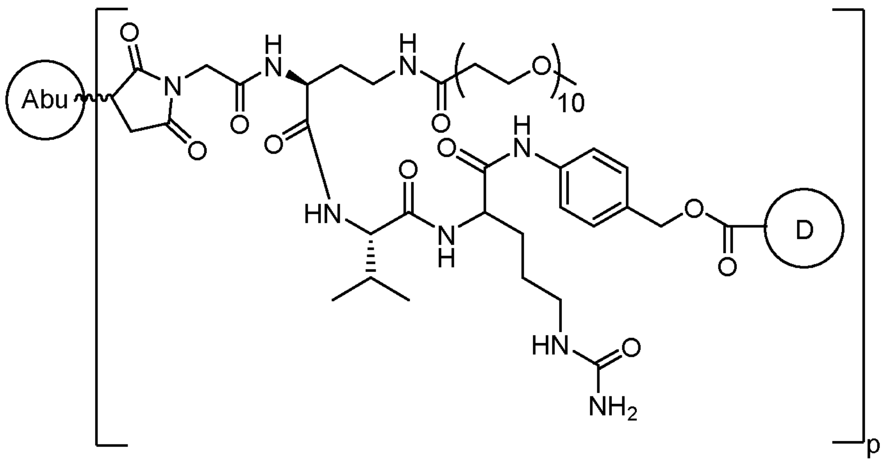

Formula I-7

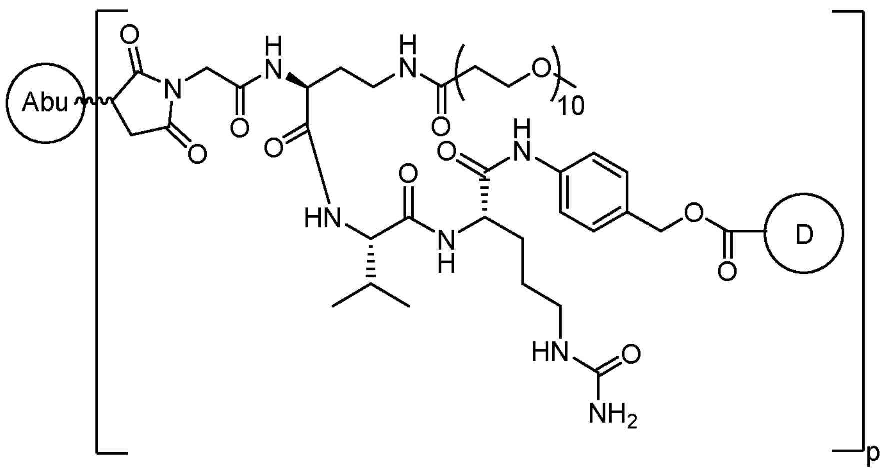

Formula I-7-1

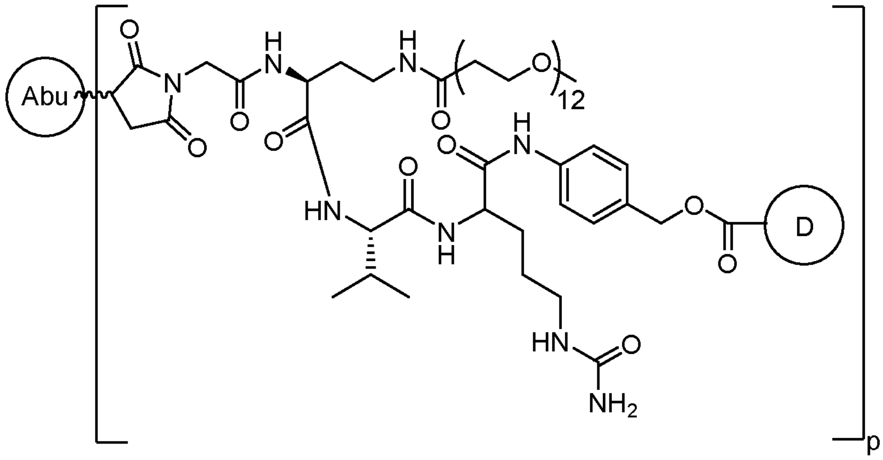

Formula I-8

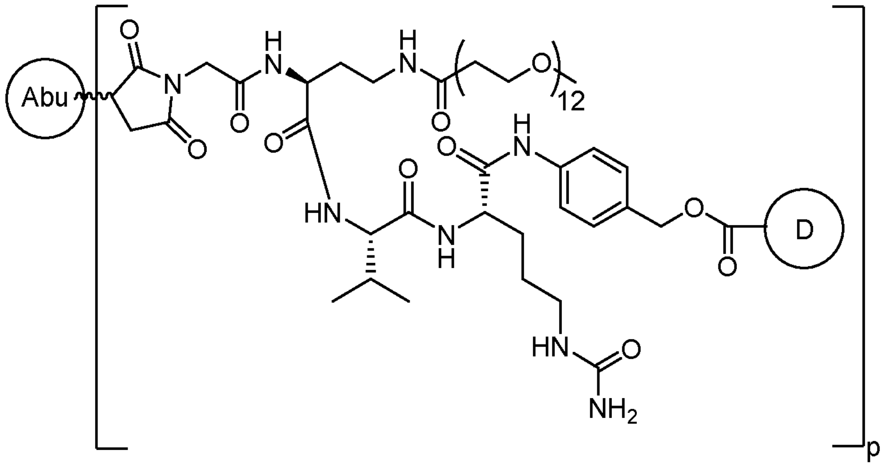

Formula I-8-1

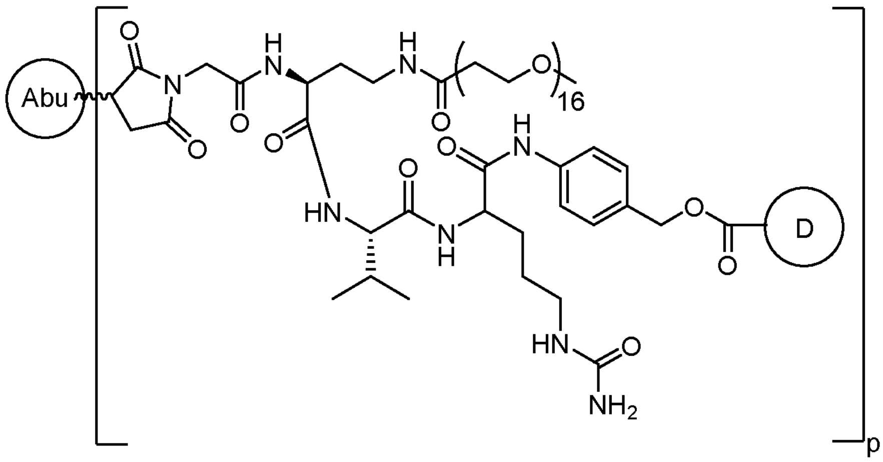

Formula I-9

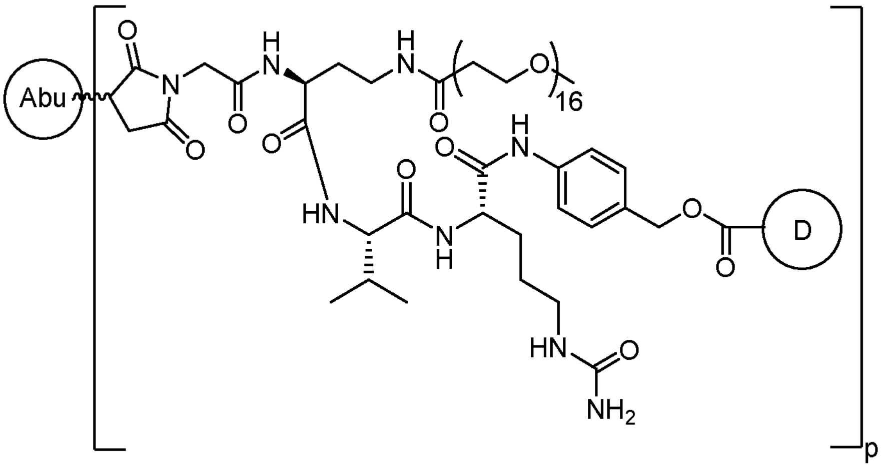

Formula I-9-1

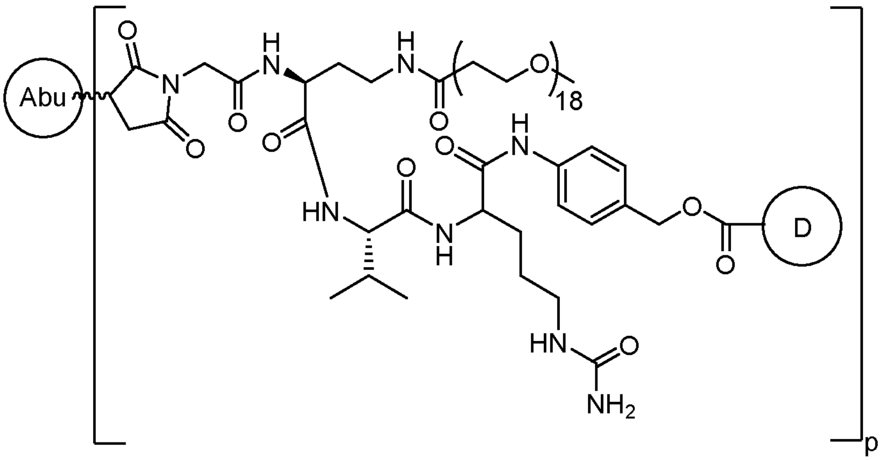

Formula I-10

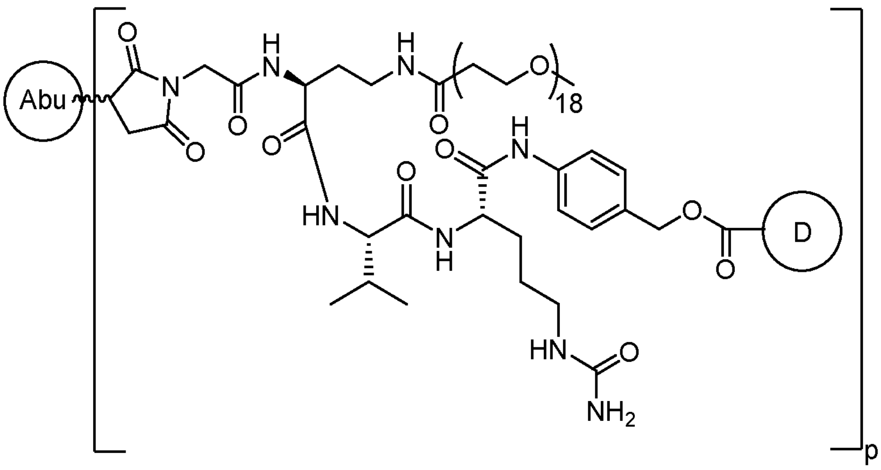

Formula I-10-1

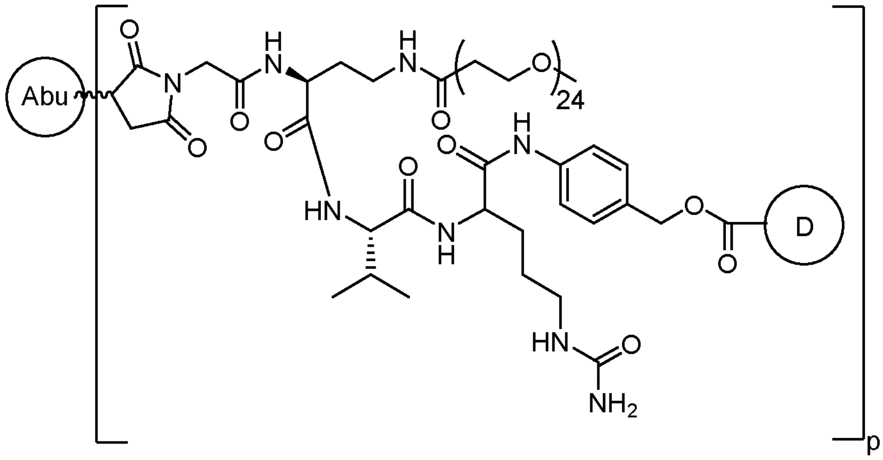

Formula I-11

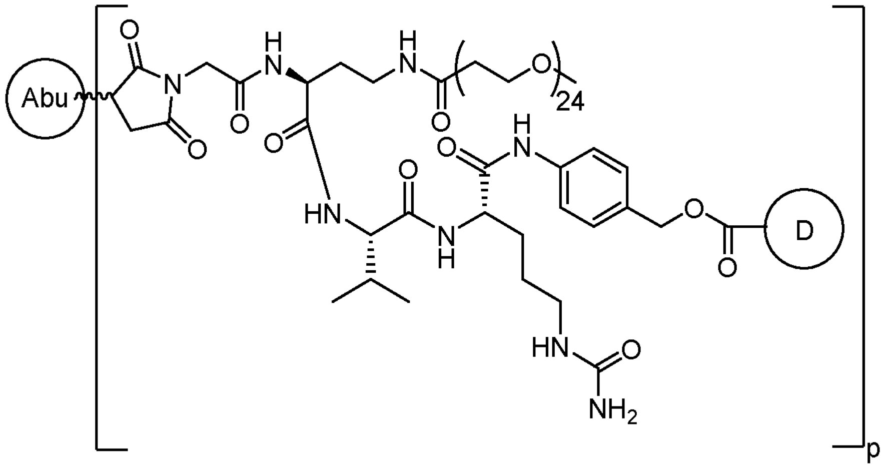

Formula I-11-1

wherein

Abu is the antibody or the antigen-binding unit thereof provided by the present invention;
D is a drug;
p is 1-10, e.g., 1, 2, 3, 4, 5, 6, 7, 7.4, 8, 9 or 10.

In one or more embodiments, the drug is an anti-cancer drug, a cytotoxic drug, a cell differentiation factor, a stem cell trophic factor, a steroid drug, a drug for treating autoimmune diseases, an anti-inflammatory drug or a drug for treating infectious diseases.

In one or more embodiments, the drug is a tubulin inhibitor, a DNA damaging agent or a DNA topoisomerase inhibitor.

In one or more embodiments, the tubulin inhibitor is selected from dolastatin, auristatins and maytansinoids.

In one or more embodiments, the drug is an auristatin, e.g., MMAE, MMAF or AF.

In one or more embodiments, the drug is a DNA damaging agent, e.g., a calicheamicin, a duocarmycin or an anthramycin derivative PBD (pyrrolobenzodiazepine).

In one or more embodiments, the drug is a DNA topoisomerase inhibitor or a salt thereof, e.g., irinotecan, irinotecan hydrochloride, camptothecin, 9-aminocamptothecin, 9-nitrocamptothecin, 10-hydroxy camptothecin, 9-chloro-10-hydroxy camptothecin, a camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, *N*-[2-(dimethylamino)ethyl]-4-acridinecarboxamide dihydrochloride or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine or exatecan. In one or more embodiments, the drug is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan or a chlorambucil derivative.

In one or more embodiments, the drug is

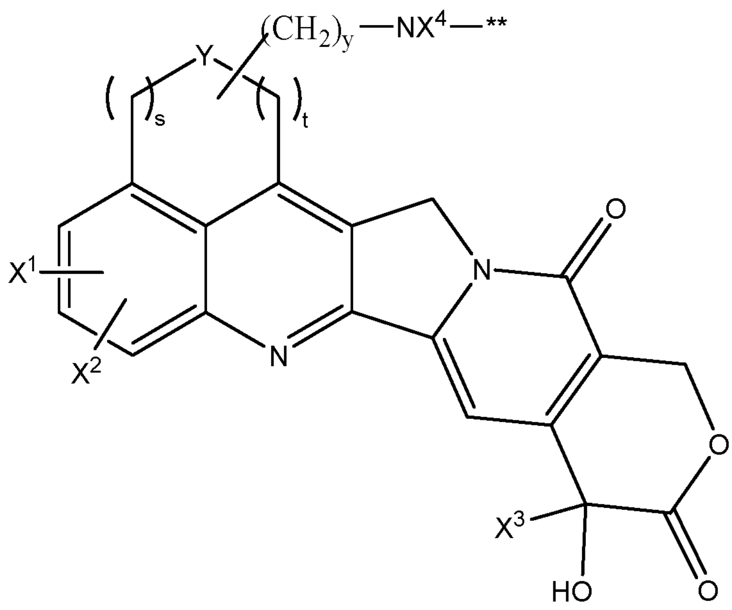
,

wherein $X^1$ and $X^2$ are each independently:

H,
hydroxy,
C1-C6 alkyl,
C1-C6 alkyl substituted with one or more hydroxy, halogen, nitro or cyano groups, C2-C6 alkenyl,
C2-C6 alkynyl,
C1-C6 alkoxy,
C1-C6 aminoalkoxy,
halogen,
nitro,
cyano,
thiol,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocyclic ring, wherein the heterocyclic ring is optionally substituted with one or more C1-C6 alkyl, C1-C6 alkoxy, amino, halogen, nitro or cyano groups,
C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or a protecting group,
amino-substituted heterocyclyl optionally substituted at a nitrogen atom of the heterocyclyl moiety or at the amino moiety with a protecting group or one or more C1-C6 alkyl groups, heterocyclylamino optionally substituted at a nitrogen atom of the heterocyclyl moiety or at the amino moiety with a protecting group or C1-C6 alkyl,
carbamoyl optionally substituted with a carbamoyl-protecting group or C1-C6 alkyl, morpholin-1-yl, or piperidin-1-yl;
$X^3$ is C1-C6 alkyl;
$X^4$ is H, $-(CH_2)_q-CH_3$, $-(CHR^n)_q-CH_3$, C3-C8 carbocyclyl, $-O-(CH_2)_q-CH_3$, arylene-$CH_3$, - $(CH_2)_q$-arylene-$CH_3$,

-arylene-$(CH_2)_q$-$CH_3$, -$(CH_2)_q$-(C3-C8 carbocyclyl)-$CH_3$, -(C3-C8 carbocyclyl)-$(CH_2)_q$-$CH_3$, C3-C8 heterocyclyl, -$(CH_2)_q$-(C3-C8 heterocyclyl)-$CH_3$, -(C3-C8 heterocyclyl)-$(CH_2)_q$-$CH_3$, -$(CH_2)_q$C(O)$NR^n$$(CH_2)_q$-$CH_3$, -$(CH_2CH_2O)_q$-$CH_3$, -$(CH_2CH_2O)_q$-$CH_2$-$CH_3$, -$(CH_2)_q$C(O)$NR^n$$(CH_2CH_2O)_q$-$CH_3$, -$(CH_2)_q$C(O)$NR^n$$(CH_2CH_2O)_q$-$CH_2$-$CH_3$, - $(CH_2CH_2O)_q$C(O)$NR^n$$(CH_2CH_2O)_q$-$CH_3$, -$(CH_2CH_2O)_q$C(O)$NR^n$$(CH_2CH_2O)_q$-$CH_2$-$CH_3$ or - $(CH_2CH_2O)_q$C(O)$NR^n$$(CH_2)_q$-$CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;
** links to other moieties of the antibody-drug conjugate;
y is 0, 1 or 2;
Y is O, S or $CR^1R^2$, wherein $R^1$ and $R^2$ are each independently H or C1-C6 alkyl;
s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, t-butoxycarbonyl, benzyl, or *p*-methoxybenzyloxycarbonyl.

In one or more embodiments, the drug is

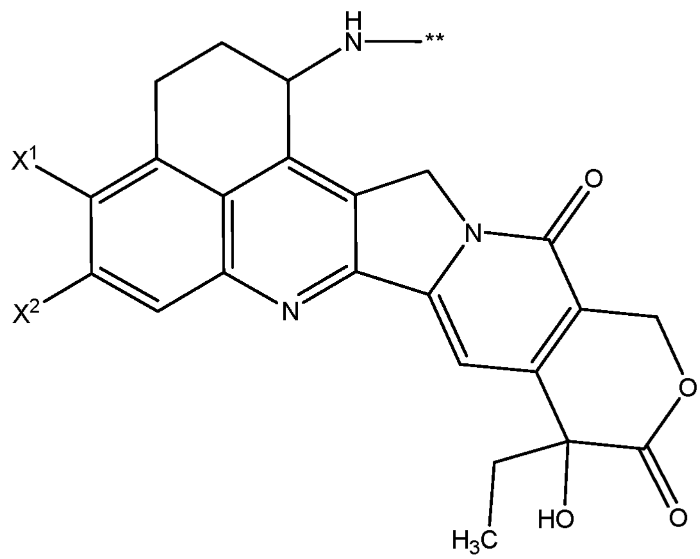

or

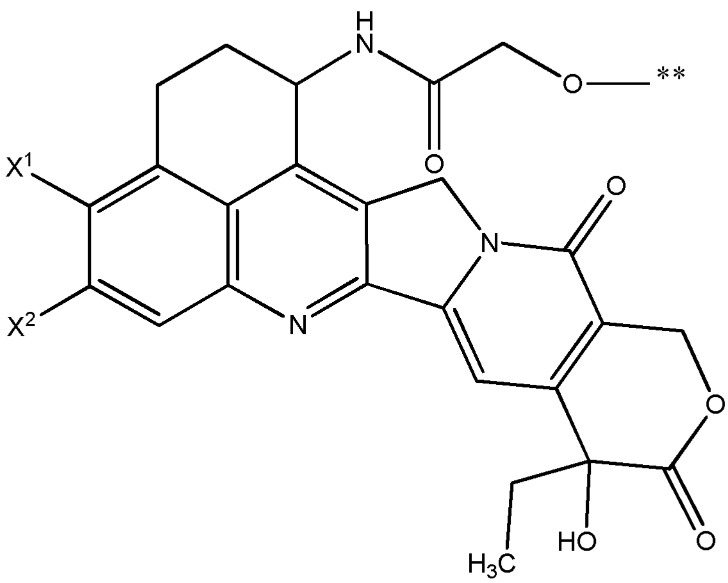

,

wherein $X^1$ and $X^2$ are each independently C1-C6 alkyl, halogen or -OH; ** links to other moieties of the antibody-drug conjugate.

In one or more embodiments, the drug is

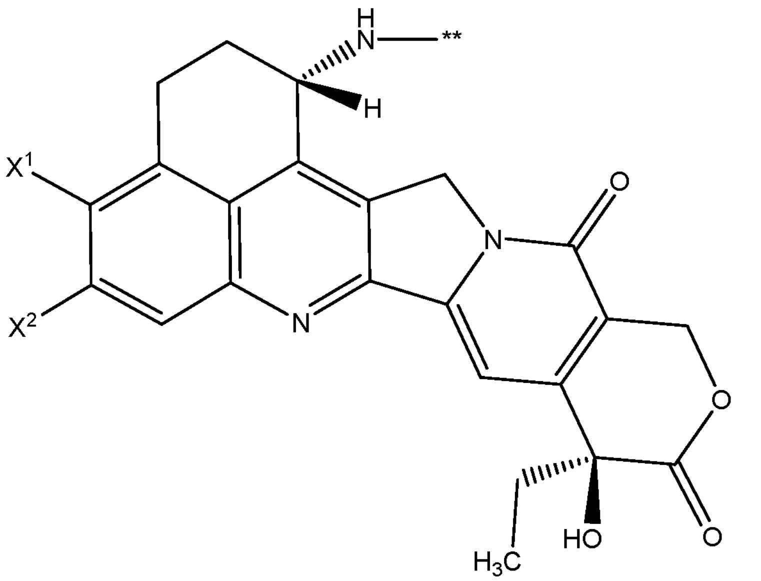

or

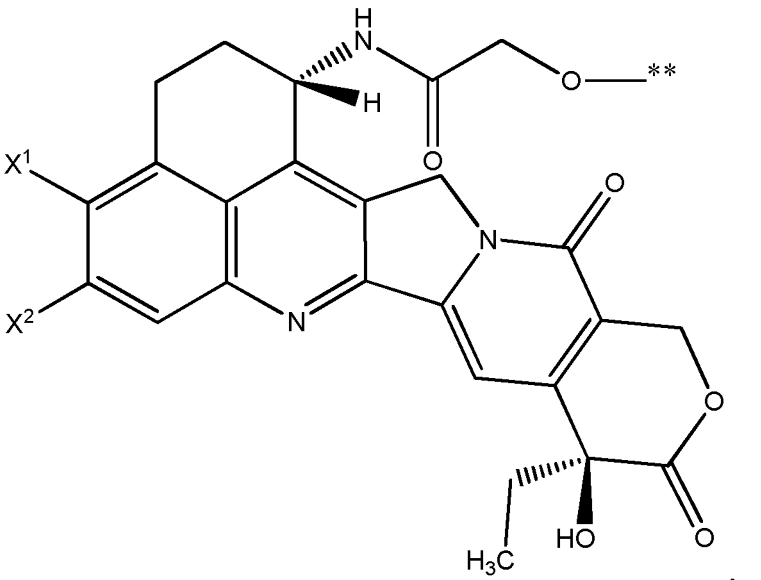

,

wherein $X^1$ and $X^2$ are each independently C1-C6 alkyl, halogen or -OH; ** links to other moieties of the antibody-drug conjugate.

In one or more embodiments, the C1-C6 alkyl is $-CH_3$.

In one or more embodiments, the halogen is F.

In one or more embodiments, $X^1$ and $X^2$ are each independently $-CH_3$, F, or -OH.

In one or more embodiments, $X^1$ and $X^2$ are each $-CH_3$.

In one or more embodiments, $X^1$ and $X^2$ are each independently F, Cl, Br or I.

In one or more embodiments, $X^1$ and $X^2$ are each F.

In one or more embodiments, $X^1$ and $X^2$ are each independently F or $-CH_3$.

In one or more embodiments, $X^1$ is $-CH_3$, and $X^2$ is F.

In one or more embodiments, p is 2-8.

In one or more embodiments, p is 4-8.

In one or more embodiments, p is 6-8.

In one or more embodiments, p is 7-8.

In one or more embodiments, p is 7.

In one or more embodiments, p is 7.4.

In one or more embodiments, p is 8.

In one or more embodiments, the antibody-drug conjugate has a structure shown as Formula I-12, I-12-1, I-13, I-13-1, I-14, I-14-1, I-15, I-15-1, I-16, I-16-1, I-17, I-17-1, I-18, I-18-1, I-19, I-19-1, I-20, I-20-1, I-21, I-21-1, I-22, I-22-1, I-23, 1-23-1, I-24, I-24-1, I-25 or I-25-1 or a stereoisomer thereof, or a pharmaceutically acceptable salt or solvate thereof:

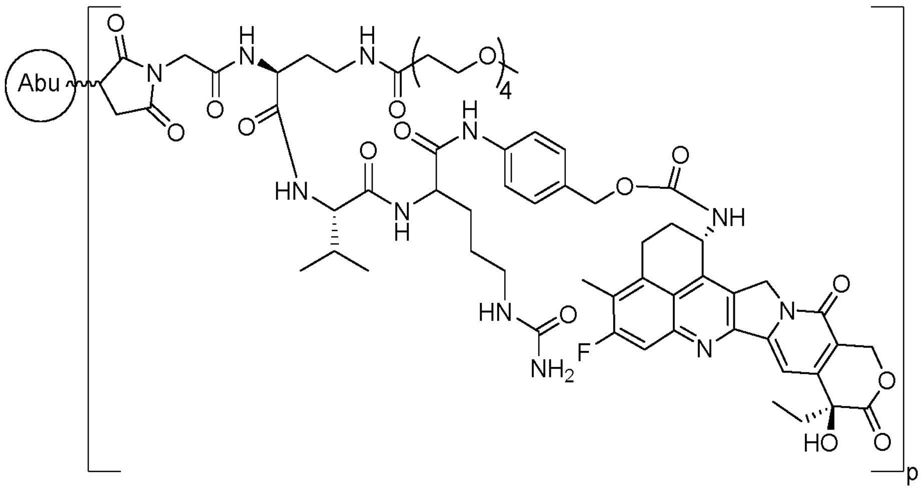

Formula I-12

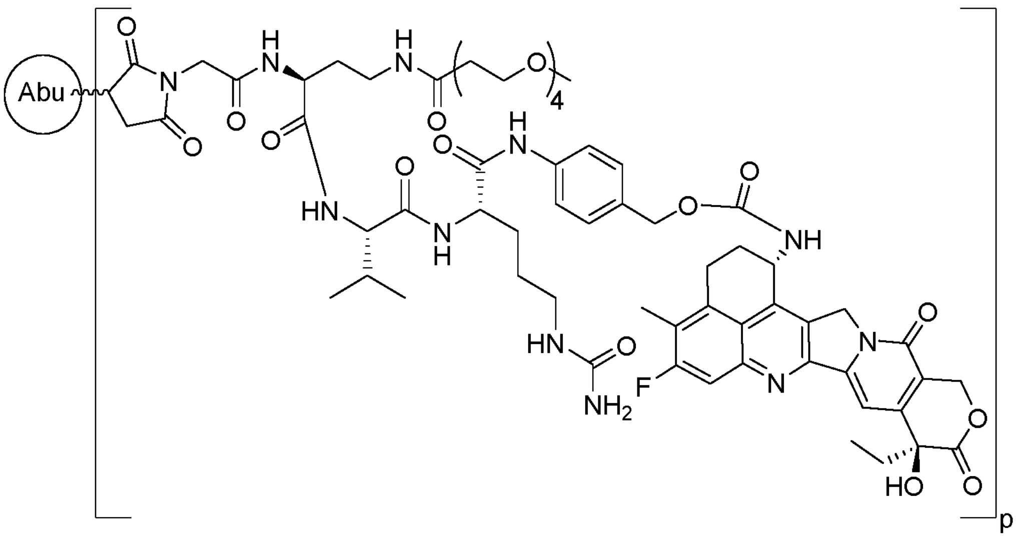

Formula I-12-1

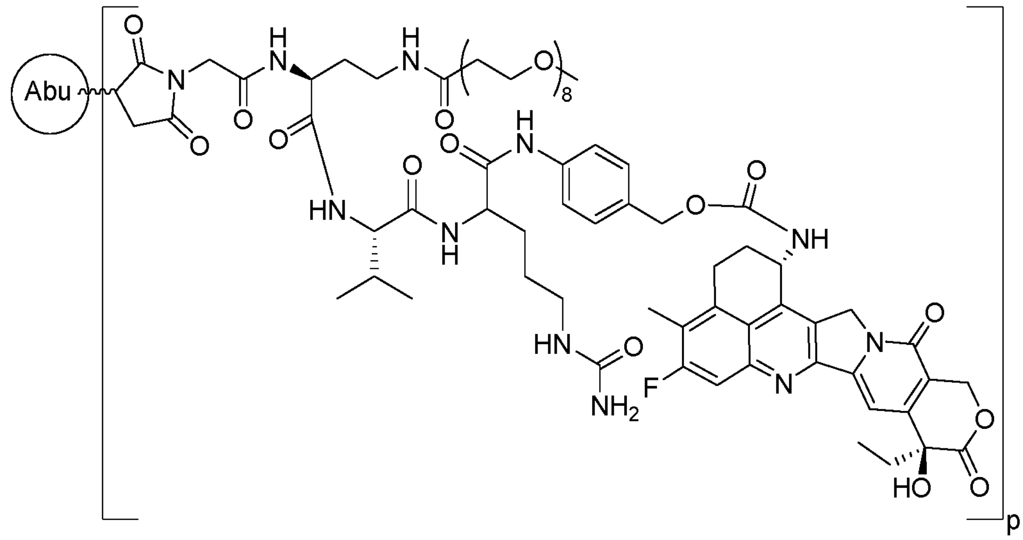

Formula I-13

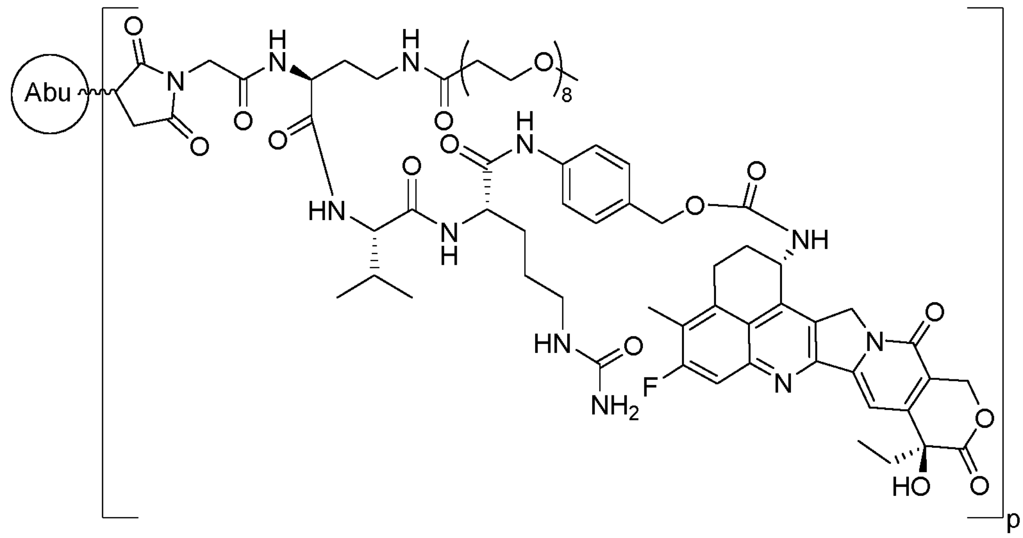

Formula I-13-1

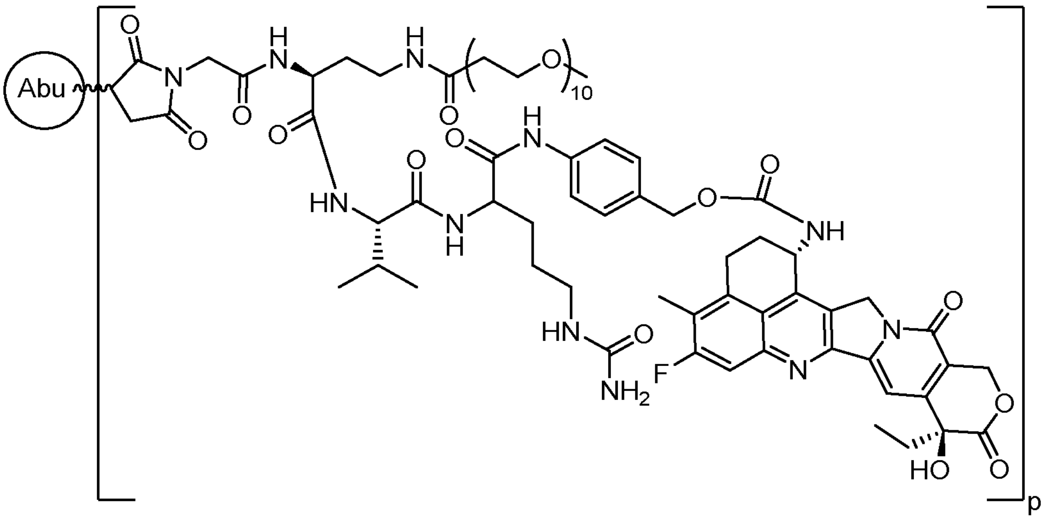

Formula I-14

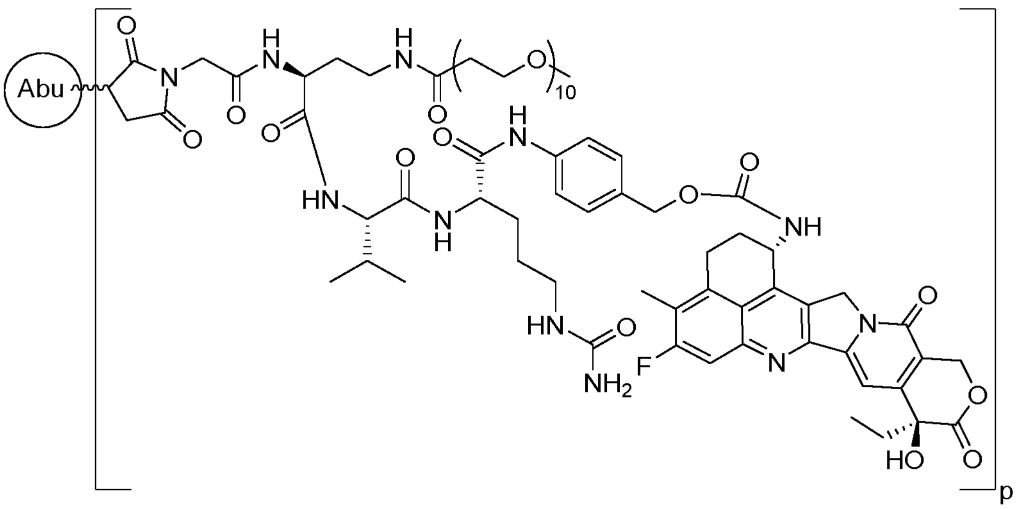

Formula I-14-1

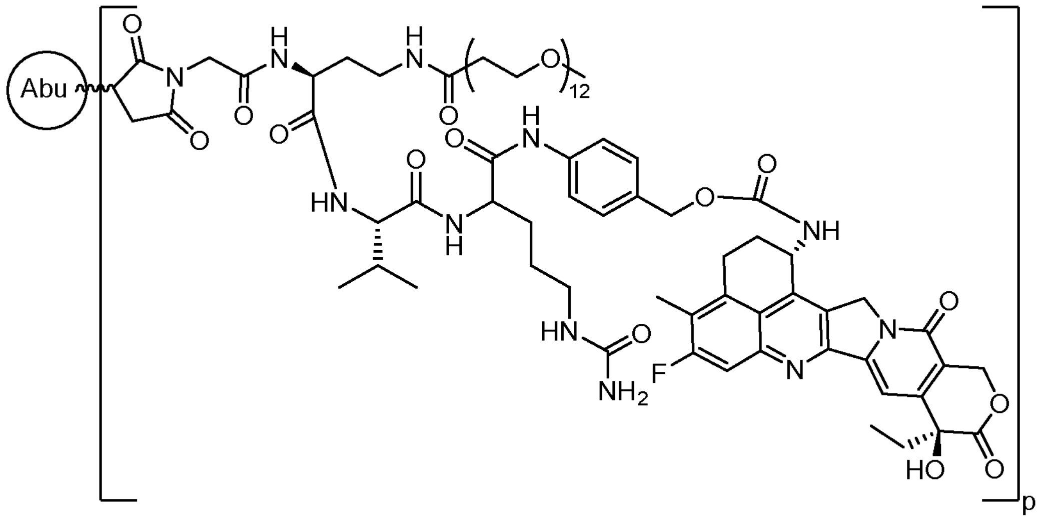

Formula I-15

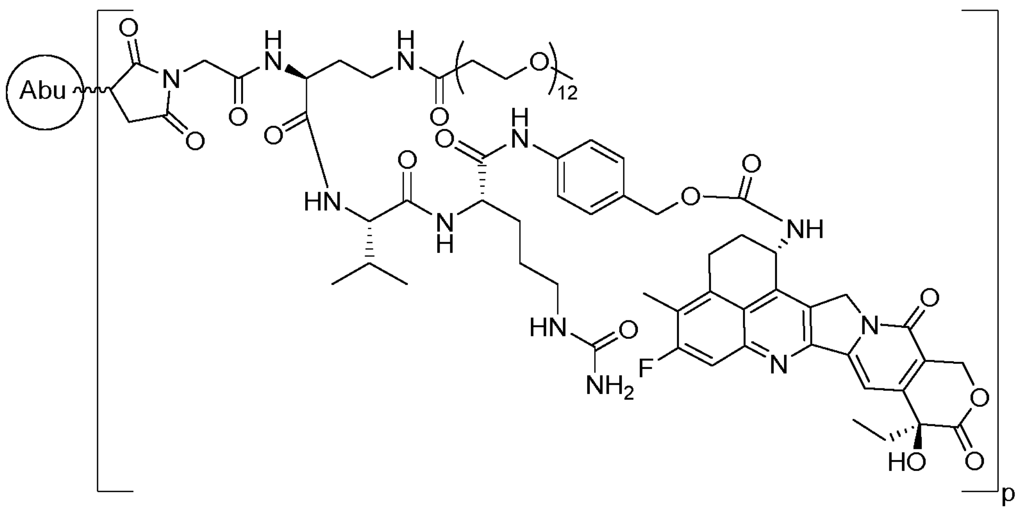

Formula I-15-1

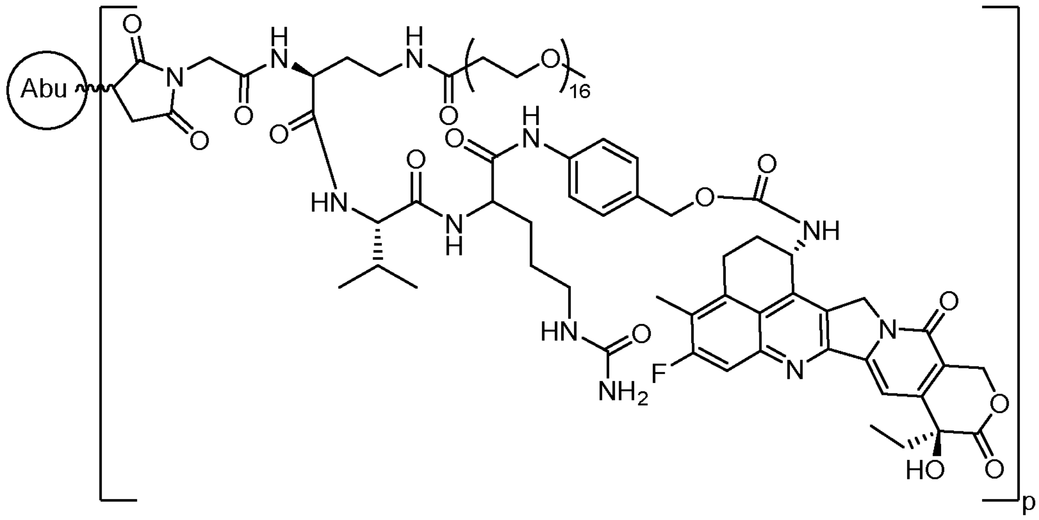

Formula I-16

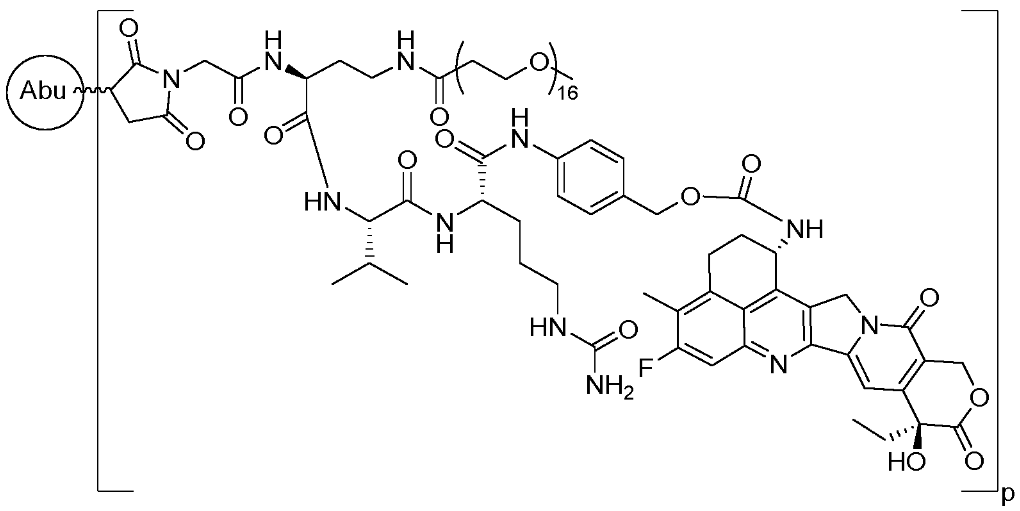

Formula I-16-1

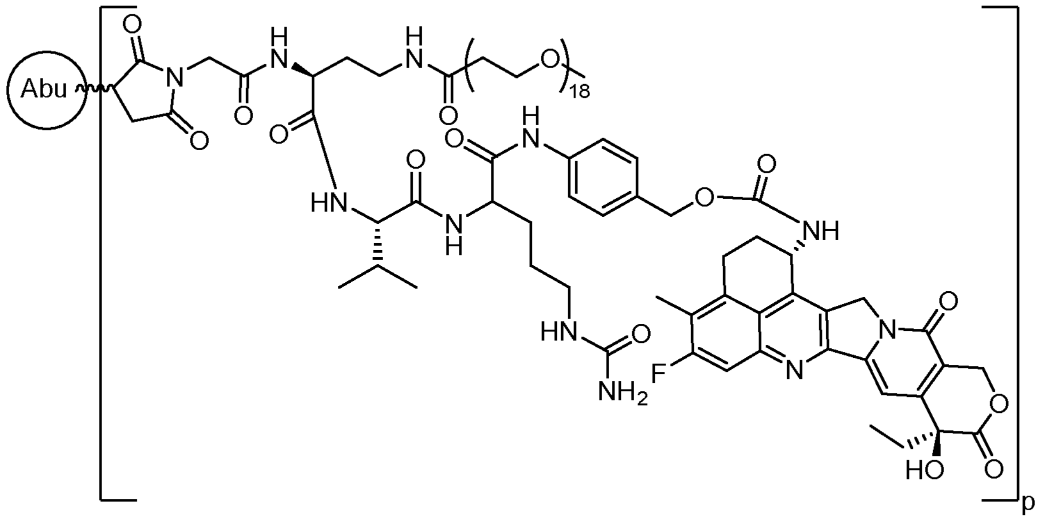

Formula I-17

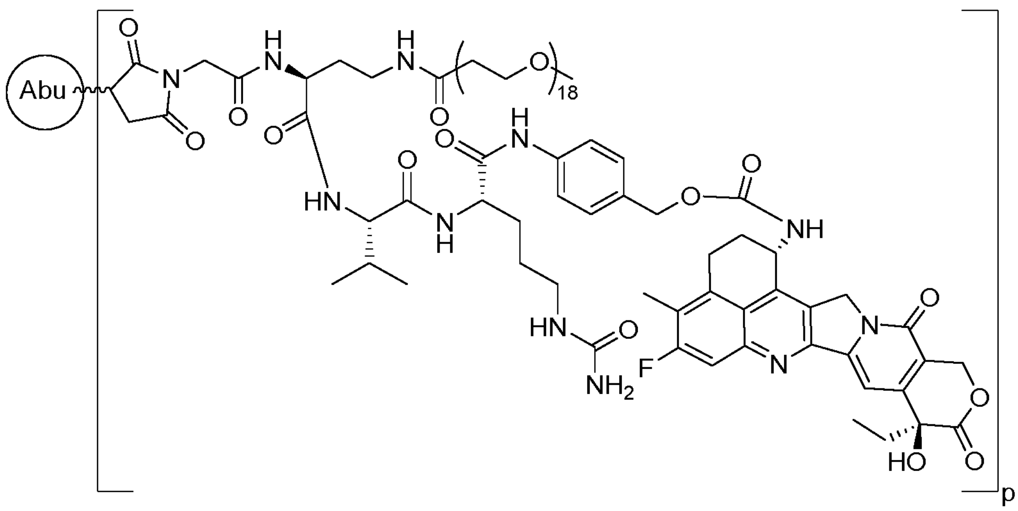

Formula I-17-1

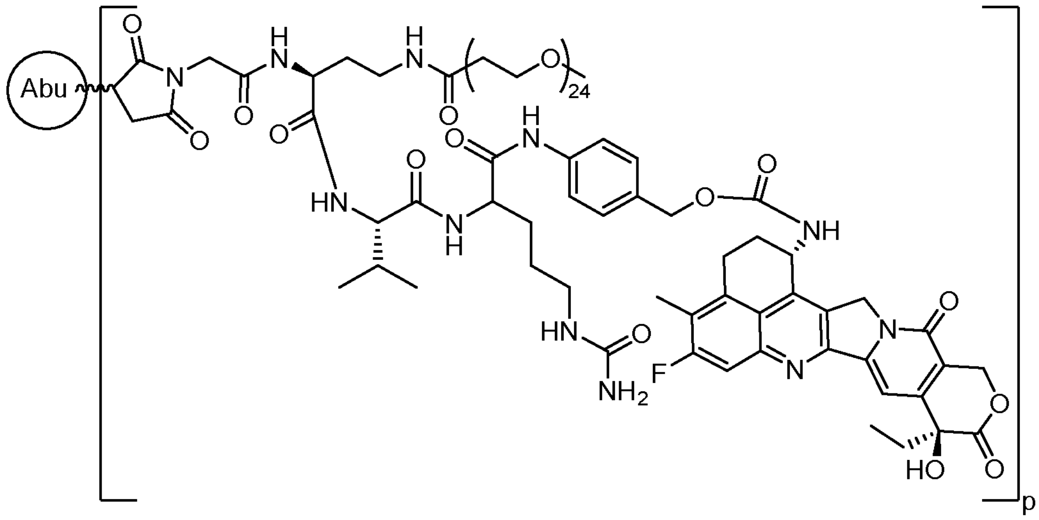

Formula I-18

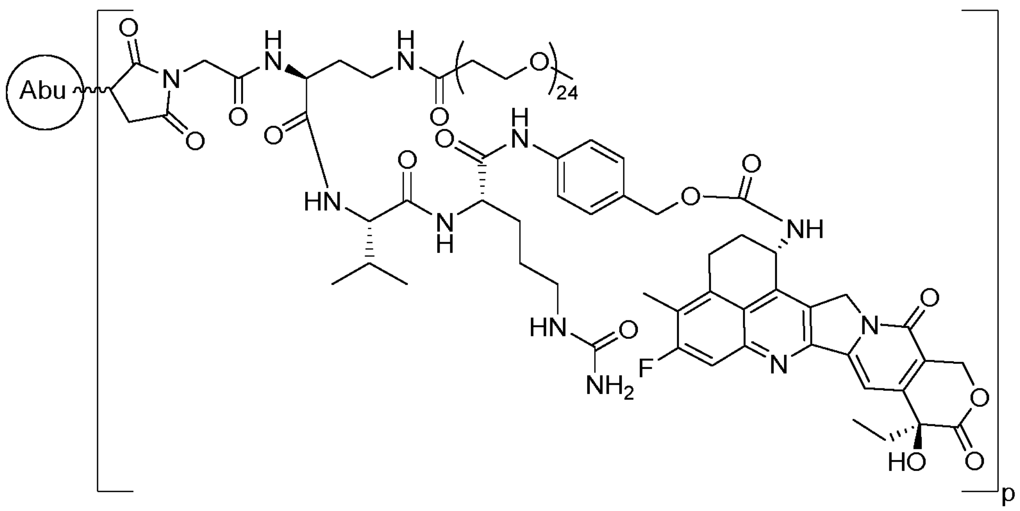

Formula I-18-1

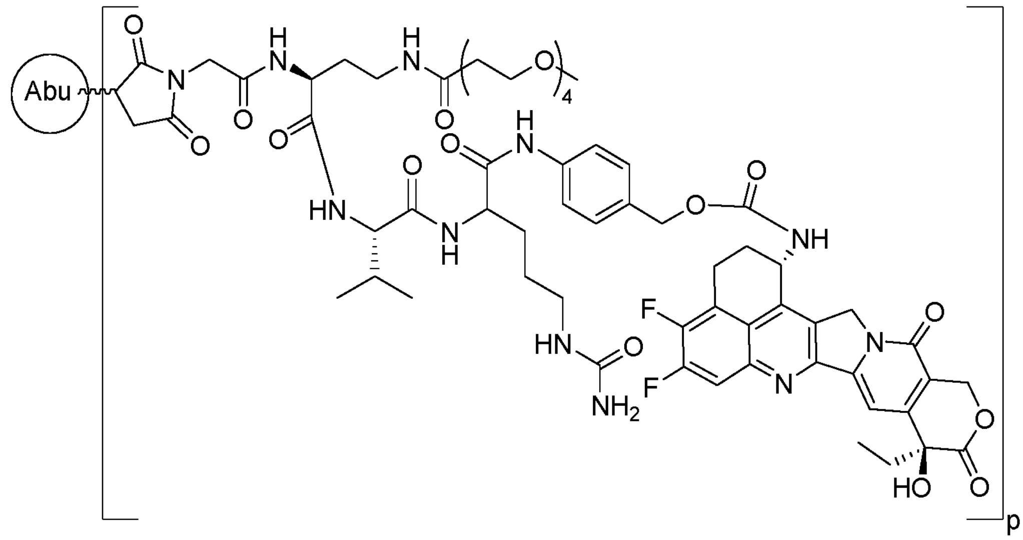

Formula I-19

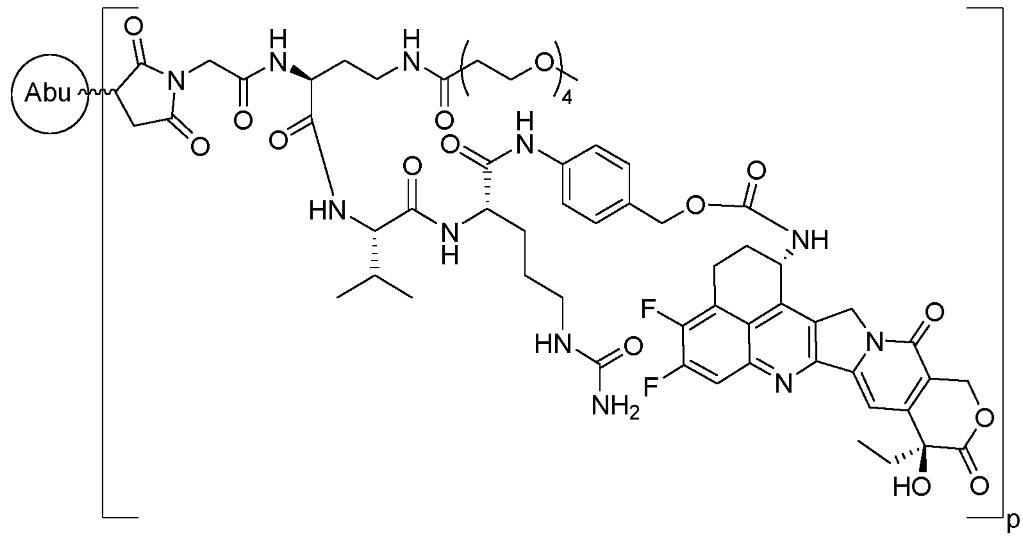

Formula I-19-1

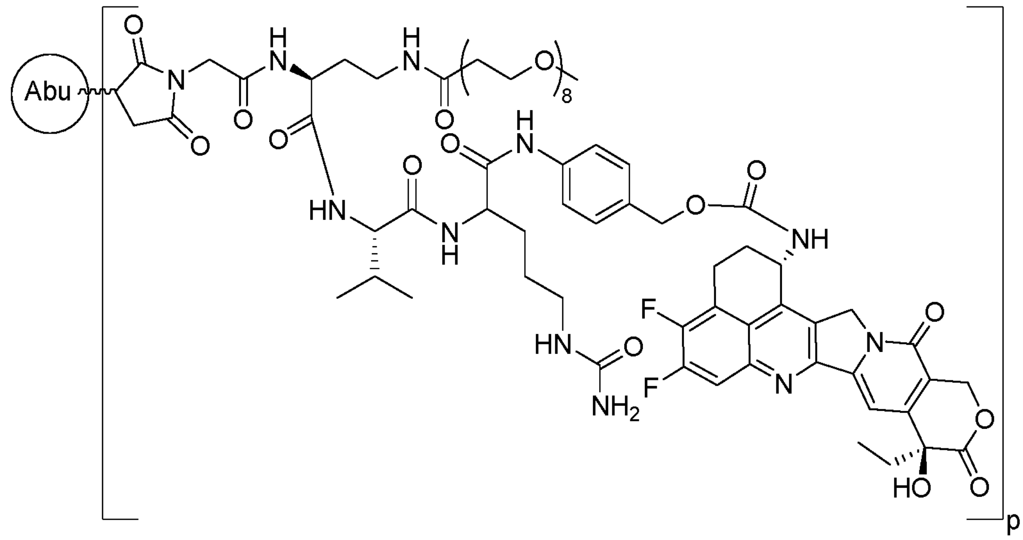

Formula I-20

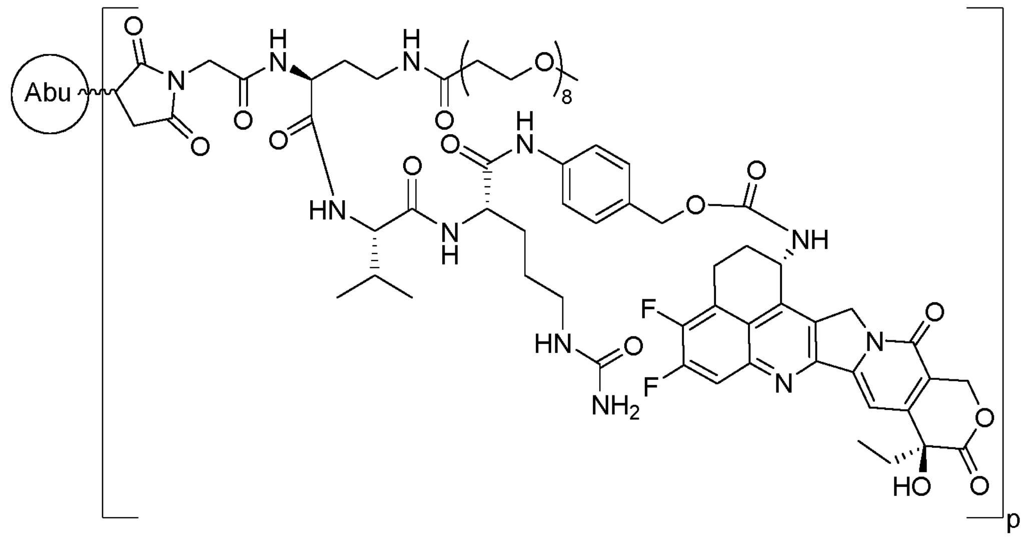

Formula I-20-1

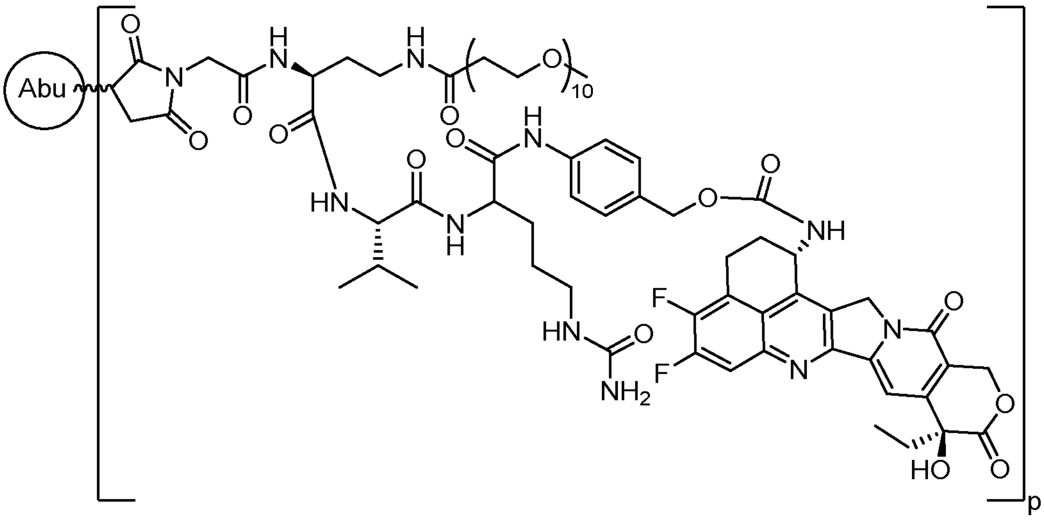

Formula I-21

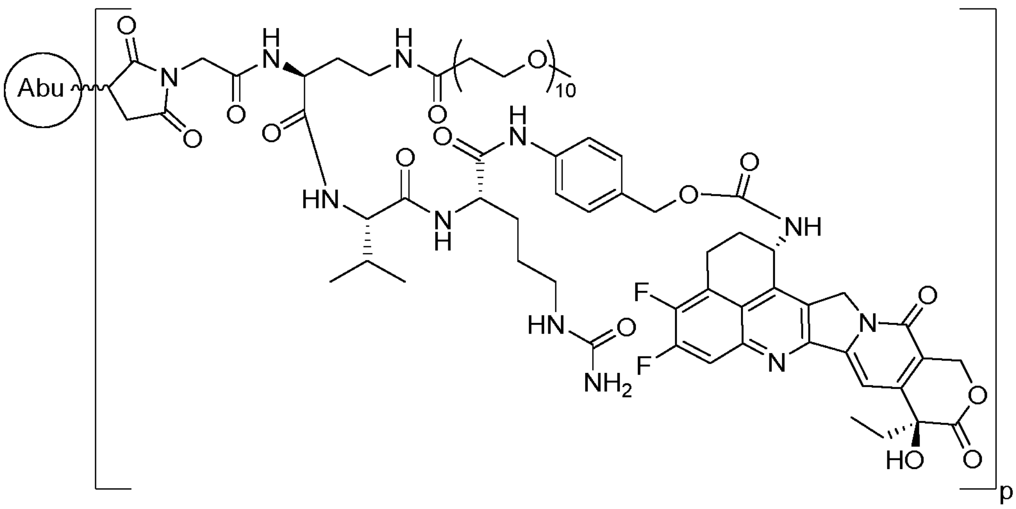

Formula I-21-1

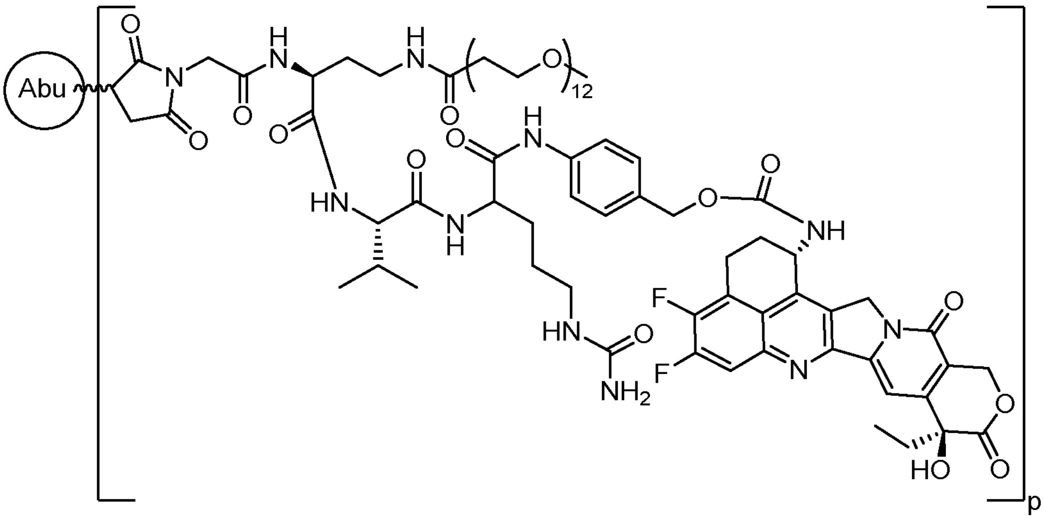

Formula I-22

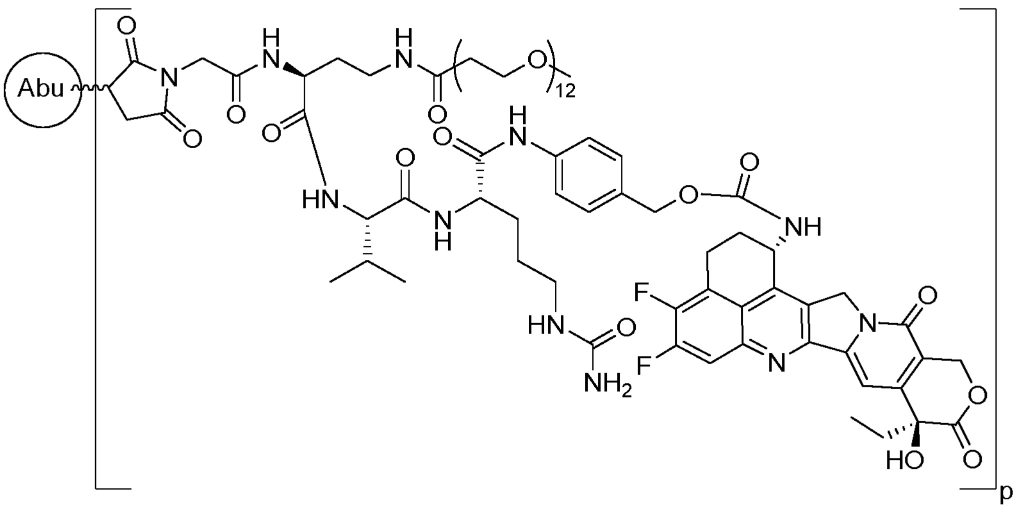

Formula I-22-1

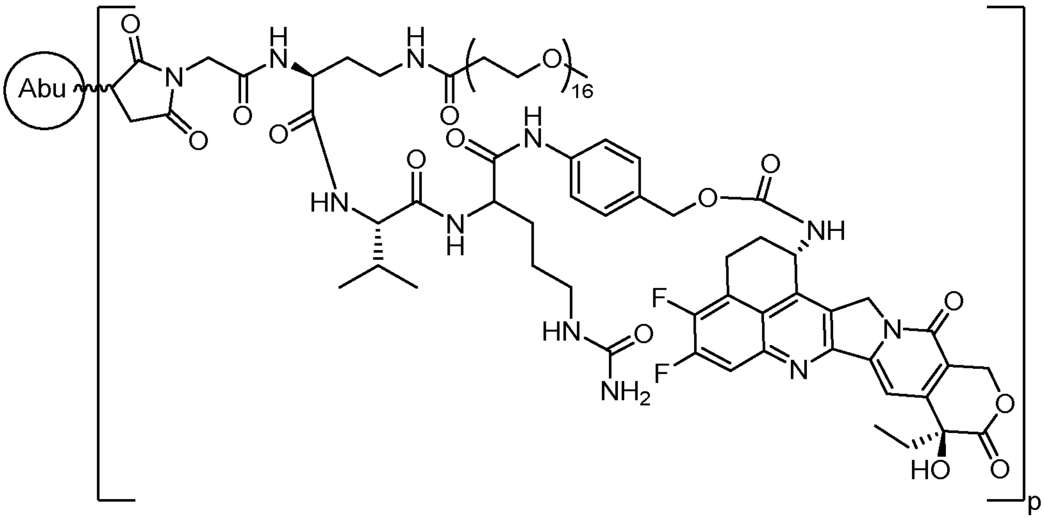

Formula I-23

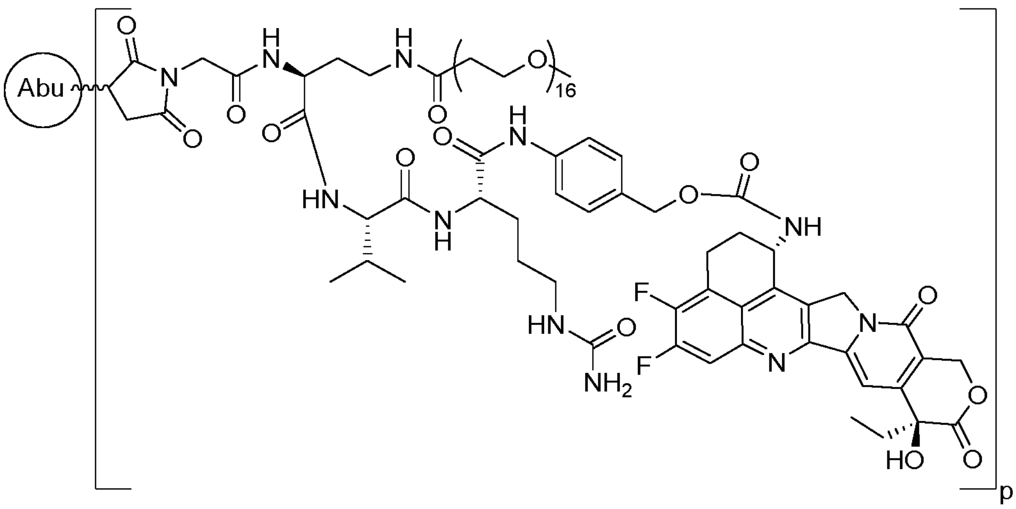

Formula I-23-1

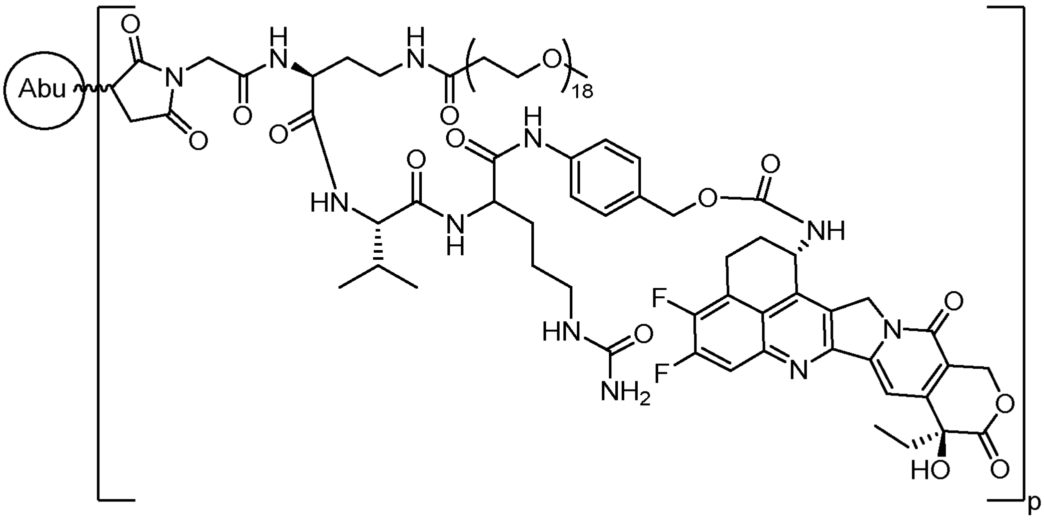

Formula I-24

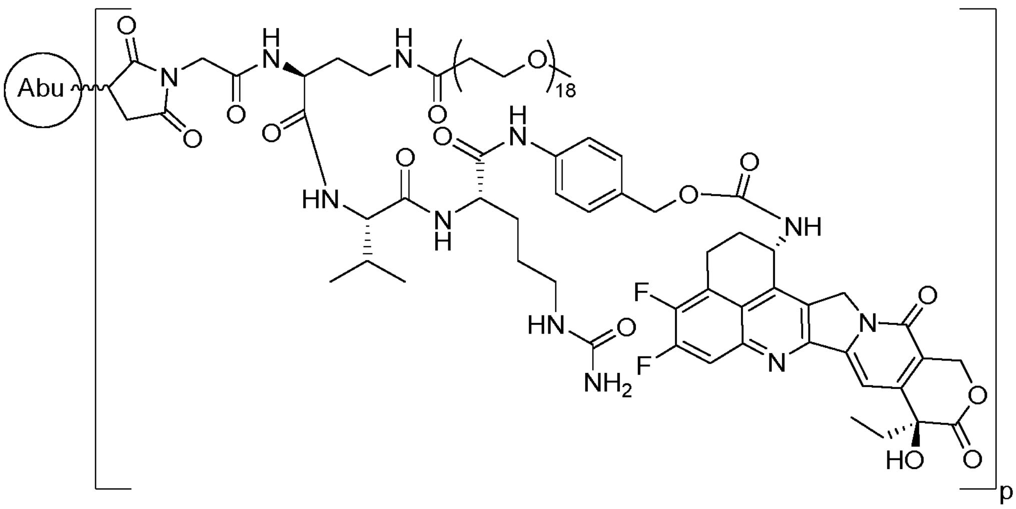

Formula I-24-1

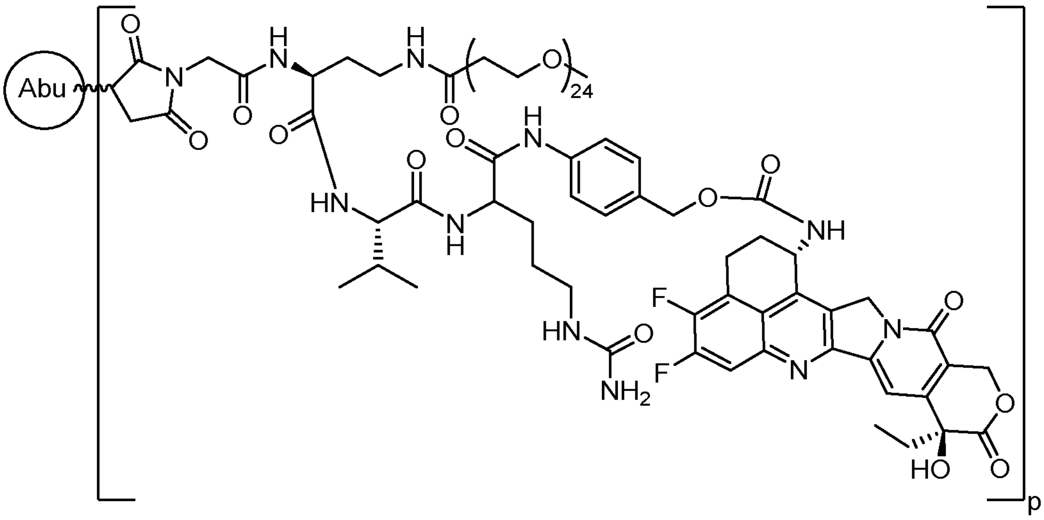

Formula I-25

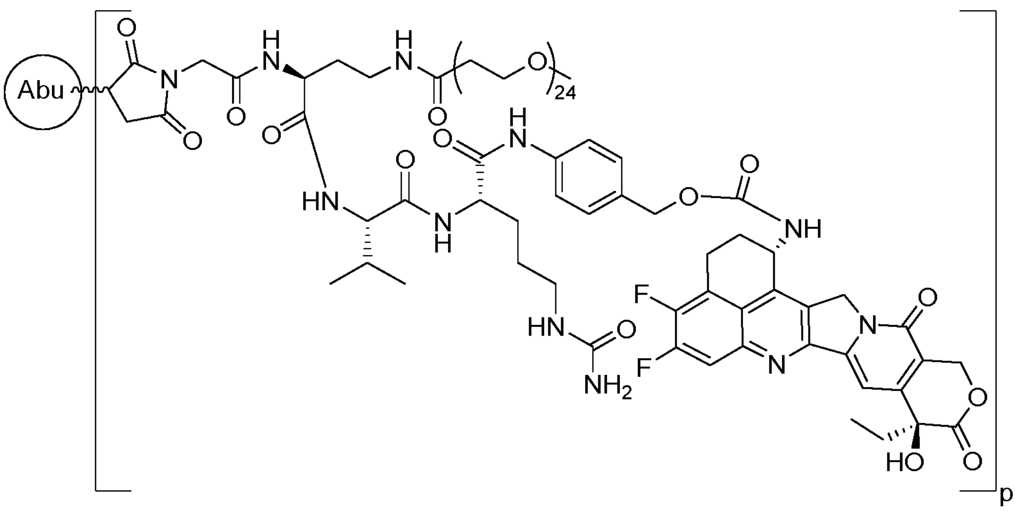

Formula I-25-1

wherein

Abu is the antibody or the antigen-binding unit thereof provided by the present invention;
p is 1-10, e.g., 1, 2, 3, 4, 5, 6, 7, 7.4, 8, 9 or 10.

In one or more embodiments, p is 2-8.
In one or more embodiments, p is 4-8.
In one or more embodiments, p is 6-8.
In one or more embodiments, p is 7-8.
In one or more embodiments, p is 7.
In one or more embodiments, p is 7.4.
In one or more embodiments, p is 8.
One or more embodiments provide an antibody-drug conjugate for use as a drug. In one or more embodiments, DAR (p) of the antibody-drug conjugate in the drug is 1, 2, 3, 4, 5, 6, 7, 8, 9 or 10. In one or more embodiments, the average DAR (p) of the antibody-drug conjugate in the drug is 2-9. In one or more embodiments, the average DAR (p) of the antibody-drug conjugate in the drug is 4-9. In one or more embodiments, the average DAR (p) of the antibody-drug conjugate in the drug is 6-8.5. In one or more embodiments, the average DAR (p) of the antibody-drug conjugate in the drug is 6-8. In one or more embodiments, the average DAR (p) of the antibody-drug conjugate in the drug is 7-8.

In one or more embodiments, the antibody-drug conjugate of the present invention is used in combination with one or more additional therapies. Suitable additional therapies include existing drugs and/or surgical therapies for specific applications (such as cancer). For example, the antibody-drug conjugate is used in combination with one or more additional chemotherapeutic or anti-tumor agents. Alternatively, the additional chemotherapeutic agent is radiotherapy. In one or more embodiments, the chemotherapeutic agent is an apoptosis inducer.

In one or more embodiments, the antibody-drug conjugate and the additional agent are prepared as a single therapeutic composition, and the antibody-drug conjugate and the additional agent are administered simultaneously. Alternatively, the antibody-drug conjugate and the additional agent are separated from each other, for example, are each prepared as a separate therapeutic composition, and the antibody-drug conjugate and the additional agent are administered simultaneously, or the antibody-drug conjugate and the additional agent are administered at different time points during a treatment regimen. For example, the antibody-drug conjugate is administered prior to administration of the additional agent, the antibody-drug conjugate is administered subsequent to the administration of the additional agent, or the antibody-drug conjugate and the additional agent are administered in an alternating manner. In the present invention, the antibody-drug conjugate and the additional agent are administered at a single dose or at multiple doses.

In one or more embodiments, the present invention further provides a pharmaceutical composition comprising the antibody or the antigen-binding unit thereof or the antibody-drug conjugate described herein, and a pharmaceutically acceptable carrier, an excipient and/or an adjuvant. In one or more embodiments, the pharmaceutical composition further comprises an optional additional anti-cancer drug. The pharmaceutical composition provided by the present invention may be administered by any convenient route, e.g., by infusion or bolus injection, by absorption through epithelial or cutaneous mucosa (e.g., oral mucosa, rectal and intestinal mucosa, etc.), and may be co-administered with other biologically active agents. Accordingly, the pharmaceutical composition may be administered intravenously, orally, rectally, parenterally, intracerebrally, intravaginally, intraperitoneally, topically (e.g., by powder, ointment, drop or transdermal patch) or buccally, or by oral or nasal spray.

In one or more embodiments, the present invention provides use of at least one of the antibody or the antigen-binding unit thereof, the biomaterial (selected from the polynucleotide, the expression vector and the cell), the antibody-drug conjugate and the pharmaceutical composition in the manufacture of a drug for treating and/or preventing a disease. In one or more embodiments, the disease is a disease associated with the expression of folate receptor α. In one or more embodiments, the disease is a disease associated with the overexpression of folate receptor α. In one or more embodiments, the disease is a tumor expressing folate receptor α. In one or more embodiments, the disease is a tumor overexpressing folate receptor α. In one or more embodiments, the disease is cancer expressing folate receptor α. In one or more embodiments, the disease is cancer overexpressing folate receptor α. In one or more embodiments, the drug for treating a tumor further comprises an additional anti-cancer drug.

In one or more embodiments, the present invention provides use of the antibody or the antigen-binding unit thereof, the biomaterial (selected from the polynucleotide, the expression vector and the cell), the antibody-drug conjugate and/or the pharmaceutical composition comprising the same described herein in the treatment and/or prevention of a disease. In one or more embodiments, the disease is a disease associated with the expression of folate receptor α. In one or more embodiments, the disease is a disease associated with the overexpression of folate receptor α. In one or more embodiments, the disease is a tumor expressing folate receptor α. In one or more embodiments, the disease is a tumor overexpressing folate receptor α. In one or more embodiments, the disease is cancer expressing folate receptor α. In one or more embodiments, the disease is cancer overexpressing folate receptor α. In one or more embodiments, the disease is cancer, an autoimmune disease, an inflammatory disease or an infectious disease.

One or more embodiments provide a method for treating and/or preventing a disease, the method comprising administering to a patient in need thereof an effective amount of the antibody or the antigen-binding unit thereof, the biomaterial (selected from the polynucleotide, the expression vector and the cell), the antibody-drug conjugate and/or the pharmaceutical composition comprising the same described herein. In one or more embodiments, the disease is a disease associated with the expression of folate receptor α. In one or more embodiments, the disease is a disease associated with the overexpression of folate receptor α. In one or more embodiments, the disease is a tumor expressing folate receptor α. In one or more embodiments, the disease is a tumor overexpressing folate receptor α. In one or more embodiments, the disease is cancer expressing folate receptor α. In one or more embodiments, the disease is cancer overexpressing folate receptor α. In one or more embodiments, the effective amount refers to the amount of an active compound or drug that results in a biological or drug response of tissues, systems, animals, individuals and humans which is being sought by researchers, vets, doctors or other clinical doctors, including the treatment of a disease.

Examples of cancers include, but are not limited to, solid tumors, hematological cancers and metastatic lesions. Specific examples of such cancers include, but are not limited to, colorectal cancer, lung cancer, ovarian cancer, uterine cancer, endometrial cancer, peritoneal cancer, fallopian tube cancer, pancreatic cancer, head and neck squamous cell carcinoma, nasopharyngeal cancer, laryngeal cancer, lung adenocarcinoma, liver cancer, breast cancer, brain cancer, renal cancer, renal cell carcinoma, colon cancer, testicular cancer, cervical cancer, bladder cancer, retinoblastoma, glioblastoma, mesothelioma, oral epithelioid cancer, choriocarcinoma and head and neck cancer.

In one or more embodiments, the antibody or the antigen-binding unit thereof or the antibody-drug conjugate may be formulated into a pharmaceutical composition and administered to a patient in a form suitable for the chosen route of administration, e.g., parenteral, intravenous (iv), intramuscular, topical or subcutaneous administration.

In one or more embodiments, the antibody or the antigen-binding unit thereof or the antibody-drug conjugate is administered at a dose (e.g., a single dose) of 1 mg/kg-10 mg/kg; or 1.2 mg/kg-8.0 mg/kg; or 1.2 mg/kg-6.5 mg/kg; or 1.2 mg/kg-5.0 mg/kg; or 1.2 mg/kg-3.5 mg/kg; or 1.2 mg/kg-2.4 mg/kg; or 2.4 mg/kg-8.0 mg/kg; or 2.4 mg/kg-6.5 mg/kg; or 2.4 mg/kg-5.0 mg/kg; or 2.4 mg/kg-3.5 mg/kg; or 3.5 mg/kg-8.0 mg/kg; or 3.5 mg/kg-6.5 mg/kg; or 3.5 mg/kg-5.0 mg/kg; or 5.0 mg/kg-8.0 mg/kg; or 5.0 mg/kg-6.5 mg/kg; or 6.5 mg/kg-8.0 mg/kg; or 1.5 mg/kg-8.5 mg/kg; or 1.5 mg/kg-6.5 mg/kg; or 1.5 mg/kg-4.5 mg/kg; or 1.5 mg/kg-3.0 mg/kg; or 3.0 mg/kg-8.5 mg/kg; or 3.0 mg/kg-6.5 mg/kg; or 3.0 mg/kg-4.5 mg/kg; or 4.5 mg/kg-8.5 mg/kg; or 4.5 mg/kg-6.5 mg/kg; or 6.5 mg/kg-8.5 mg/kg. In one or more embodiments, the antibody or the antigen-binding unit thereof or the antibody-drug conjugate is administered at a dose (e.g., a single dose) of about 1 mg/kg, 1.2 mg/kg, 1.5 mg/kg, about 2.0 mg/kg, 2.4 mg/kg, about 3.0 mg/kg, about 3.5 mg/kg, about 4.0 mg/kg, about 4.5 mg/kg, about 5.0 mg/kg, about 5.5 mg/kg, about 6.0 mg/kg, about 6.5 mg/kg, about 7.0 mg/kg, about 7.5 mg/kg, about 8.0 mg/kg, about 8.5 mg/kg, about 9 mg/kg, about 10 mg/kg, or a range between any two of these values (inclusive) or any value therein.

In one or more embodiments, the antibody or the antigen-binding unit thereof or the antibody-drug conjugate is administered at a single dose of 1 mg/kg-10 mg/kg; or 1.2 mg/kg-8.0 mg/kg; or 1.2 mg/kg-6.5 mg/kg; or 1.2 mg/kg-5.0 mg/kg; or 1.2 mg/kg-3.5 mg/kg; or 1.2 mg/kg-2.4 mg/kg; or 2.4 mg/kg-8.0 mg/kg; or 2.4 mg/kg-6.5 mg/kg; or 2.4 mg/kg-5.0 mg/kg; or 2.4 mg/kg-3.5 mg/kg; or 3.5 mg/kg-8.0 mg/kg; or 3.5 mg/kg-6.5 mg/kg; or 3.5 mg/kg-5.0 mg/kg; or 5.0 mg/kg-8.0 mg/kg; or 5.0 mg/kg-6.5 mg/kg; or 6.5 mg/kg-8.0 mg/kg; or 1.5 mg/kg-8.5 mg/kg; or 1.5 mg/kg-6.5 mg/kg; or 1.5 mg/kg-4.5 mg/kg; or 1.5 mg/kg-3.0 mg/kg; or 3.0 mg/kg-8.5 mg/kg; or 3.0 mg/kg-6.5 mg/kg; or 3.0 mg/kg-4.5 mg/kg; or 4.5 mg/kg-8.5 mg/kg; or 4.5 mg/kg-6.5 mg/kg; or 6.5 mg/kg-8.5 mg/kg. In one or more embodiments, the antibody or the antigen-binding unit thereof or the antibody-drug conjugate is administered at a single dose of about 1 mg/kg, 1.2 mg/kg, 1.5 mg/kg, about 2.0 mg/kg, 2.4 mg/kg, about 3.0 mg/kg, about 3.5 mg/kg, about 4.0 mg/kg, about 4.5 mg/kg, about 5.0 mg/kg, about 5.5 mg/kg, about 6.0 mg/kg, about 6.5 mg/kg, about 7.0 mg/kg, about 7.5 mg/kg, about 8.0 mg/kg, about 8.5 mg/kg, about 9 mg/kg, about 10 mg/kg, or a range between any two of these values (inclusive) or any value therein.

In one or more embodiments, the antibody or the antigen-binding unit thereof or the antibody-drug conjugate is administered at a dose (e.g., a single dose) of 50 mg-1000 mg; or 50 mg-600 mg; or 60 mg-600 mg; or 100 mg-600 mg; or 72 mg-480 mg; or 72 mg-390 mg; or 72 mg-300 mg; or 72 mg-210 mg; or 72 mg-144 mg; or 144 mg-480 mg; or 144 mg-390 mg; or 144 mg-300 mg; or 144 mg-210 mg; or 210 mg-480 mg; or 210 mg-390 mg; or 210 mg-300 mg; or 300 mg-480 mg; or 300 mg-390 mg; or 390 mg-480 mg; or 84 mg-560 mg; or 84 mg-455 mg; or 84 mg-350 mg; or 84 mg-245 mg; or 84 mg-168 mg; or 168 mg-560 mg; or 168 mg-455 mg; or 168 mg-350 mg; or 168 mg-245 mg; or 245 mg-560 mg; or 245 mg-455 mg; or 245 mg-350 mg; or 350 mg-560 mg; or 350 mg-455 mg; or 455 mg-560 mg; or 90 mg-595 mg; or 90 mg-510 mg; or 90 mg-390 mg; or 90 mg-270 mg; or 90 mg-180 mg; or 180 mg-510 mg; or 180 mg-390 mg; or 180 mg-270 mg; or 270 mg-510 mg; or 270 mg-390 mg; or 390 mg-510 mg; or 105 mg-595 mg; or 105 mg-455 mg; or 105 mg-315 mg; or 105 mg-210 mg; or 210 mg-595 mg; or 210 mg-455 mg; or 210 mg-315 mg; or 315 mg-595 mg; or 315 mg-455 mg; or 455 mg-595 mg. In one or more embodiments, the antibody or the antigen-binding unit thereof or the antibody-drug conjugate is administered at a dose (e.g., a single dose) of about 50 mg, about 55 mg, about 60 mg, about 65 mg, about 70 mg, about 72 mg, about 80 mg, about 84 mg, about 90 mg, about 95 mg, about 100 mg, about 105 mg, about 110 mg, about 120 mg, about 130 mg, about 140 mg, about 144 mg, about 145 mg, about 150 mg, about 155 mg, about 160 mg, about 165 mg, about 168 mg, about 170 mg, about 175 mg, about 180 mg, about 190 mg, about 200 mg, about 210 mg, about 220 mg, about 230 mg, about 240 mg, about 245 mg, about 250 mg, about 260 mg, about 270 mg, about 280 mg, about 290 mg, about 300 mg, about 310 mg, about 315 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 455 mg, about 460 mg, about 470 mg, about 480 mg, about 490 mg, about 500 mg, about 510 mg, about 550 mg, about 560 mg, about 595 mg, about 600 mg, about 650 mg, about 700 mg, about 750 mg, about 800 mg, about 850 mg, about 900 mg, about 950 mg, about 1000 mg, or a range between any two of these values (inclusive) or any value therein.

In one or more embodiments, the antibody or the antigen-binding unit thereof or the antibody-drug conjugate is administered at a single dose of 50 mg-1000 mg; or 50 mg-600 mg; or 60 mg-600 mg; or 100 mg-600 mg; or 72 mg-480 mg; or 72 mg-390 mg; or 72 mg-300 mg; or 72 mg-210 mg; or 72 mg-144 mg; or 144 mg-480 mg; or 144 mg-390 mg; or 144 mg-300 mg; or 144 mg-210 mg; or 210 mg-480 mg; or 210 mg-390 mg; or 210 mg-300 mg; or 300 mg-480 mg; or 300 mg-390 mg; or 390 mg-480 mg; or 84 mg-560 mg; or 84 mg-455 mg; or 84 mg-350 mg; or 84 mg-245 mg; or 84 mg-168 mg; or 168 mg-560 mg; or 168 mg-455 mg; or 168 mg-350 mg; or 168 mg-245 mg; or 245 mg-560 mg; or 245 mg-455 mg; or 245 mg-350 mg; or 350 mg-560 mg; or 350 mg-455 mg; or 455 mg-560 mg; or 90 mg-595 mg; or 90 mg-510 mg; or 90 mg-390 mg; or 90 mg-270 mg; or 90 mg-180 mg; or 180 mg-510 mg; or 180 mg-390 mg; or 180 mg-270 mg; or 270 mg-510 mg; or 270 mg-390 mg; or 390 mg-510 mg; or 105 mg-595 mg; or 105 mg-455 mg; or 105

mg-315 mg; or 105 mg-210 mg; or 210 mg-595 mg; or 210 mg-455 mg; or 210 mg-315 mg; or 315 mg-595 mg; or 315 mg-455 mg; or 455 mg-595 mg. In one or more embodiments, the antibody or the antigen-binding unit thereof or the antibody-drug conjugate is administered at a single dose of about 50 mg, about 55 mg, about 60 mg, about 65 mg, about 70 mg, about 72 mg, about 84 mg, about 90 mg, about 100 mg, about 105 mg, about 110 mg, about 120 mg, about 130 mg, about 140 mg, about 144 mg, about 145 mg, about 150 mg, about 155 mg, about 160 mg, about 165 mg, about 168 mg, about 170 mg, about 175 mg, about 180 mg, about 190 mg, about 200 mg, about 210 mg, about 220 mg, about 230 mg, about 240 mg, about 245 mg, about 250 mg, about 260 mg, about 270 mg, about 280 mg, about 290 mg, about 300 mg, about 310 mg, about 315 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 455 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, about 595 mg, about 600 mg, or a range between any two of these values (inclusive) or any value therein.

In one or more embodiments, the method comprises at least 1, at least 2, at least 3, at least 4, at least 5 or at least 6 treatment cycles. In one or more embodiments, one treatment cycle is at least 1 week, at least 2 weeks, at least 3 weeks, at least 4 weeks, at least 5 weeks, at least 6 weeks or at least 7 weeks. In one or more embodiments, one treatment cycle is 1 week, 2 weeks, 3 weeks, 4 weeks, 5 weeks, 6 weeks, 7 weeks, or a range between any two of these values (inclusive) or any value therein.

In one or more embodiments, the antibody or the antigen-binding unit thereof or the antibody-drug conjugate is given in single administration. In one or more embodiments, the administration is performed once every 2 days to once every 6 weeks. In one or more embodiments, the administration is performed about twice a week or about once a week or about once every 2 weeks, 3 weeks, 4 weeks, 5 weeks, 6 weeks or 7 weeks. In one or more embodiments, the administration is performed once every 2 days, once every 3 days, once every 4 days, once every 5 days, twice a week, once a week, once every 2 weeks, once every 3 weeks, once every 4 weeks, once every 5 weeks or once every 6 weeks.

In one or more embodiments, the patient receives treatment for one treatment cycle. In one or more embodiments, the patient receives treatment for multiple (e.g., 2, 3, 4, 5, 6, 7, 8, 9, 10, 11 or 12) treatment cycles. In one or more embodiments, the patient receives treatment until conditions are alleviated and no treatment is required.

In one or more embodiments, the antibody or the antigen-binding unit thereof or the antibody-drug conjugate or the pharmaceutical composition comprising the same is administered by injection. In one or more embodiments, the antibody or the antigen-binding unit thereof or the antibody-drug conjugate or the pharmaceutical composition comprising the same is administered by subcutaneous (s.c.) injection, intraperitoneal (i.p.) injection, parenteral injection, intraarterial injection, intravenous (i.v.) injection, or the like. In one or more embodiments, the antibody or the antigen-binding unit thereof or the antibody-drug conjugate or the pharmaceutical composition comprising the same is administered by infusion. In one or more embodiments, the antibody or the antigen-binding unit thereof or the antibody-drug conjugate or the pharmaceutical composition comprising the same is administered by bolus injection. In one or more embodiments, the antibody or the antigen-binding unit thereof or the antibody-drug conjugate or the pharmaceutical composition comprising the same is administered by intravenous injection. In one or more embodiments, the antibody or the antigen-binding unit thereof or the antibody-drug conjugate or the pharmaceutical composition comprising the same is administered by intravenous infusion. The dose of the antibody or the antigen-binding unit thereof or the antibody-drug conjugate will depend on the properties of the drug, the extent to which the internalization, transport and release of the drug are triggered at the cell surface, and the disease being treated and the conditions of the patient (e.g., age, sex and body weight).

In one or more embodiments, the antibody or the antigen-binding unit thereof or the antibody-drug conjugate or the pharmaceutical composition comprising the same is administered by infusing intravenously (i.v.) (i.e., intravenous infusion). In one or more embodiments, the duration of the intravenous infusion is about 10 minutes, about 15 minutes, about 20 minutes, about 25 minutes, about 30 minutes, about 40 minutes, about 50 minutes, about 55 minutes, about 60 minutes, about 65 minutes, about 70 minutes, about 75 minutes, about 81 minutes, about 87 minutes, about 90 minutes, about 95 minutes, or a range between any two of these values (inclusive) or any value therein. In one or more embodiments, the duration of the intravenous infusion is less than or equal to 30 minutes. In one or more embodiments, the duration of the intravenous infusion is greater than or equal to 60 minutes. In one or more embodiments, the duration of the intravenous infusion is greater than or equal to 90 minutes. In one or more embodiments, the present invention provides a pharmaceutical composition comprising the antibody or the antigen-binding unit thereof or the antibody-drug conjugate and suitable for injection, such as a pharmaceutical composition for bolus injection or a pharmaceutical composition for infusion (dripping). The pharmaceutical composition suitable for injection includes a sterile aqueous solution or dispersion and sterile powders for the instant preparation of a sterile injectable solution or dispersion. For intravenous administration, a suitable carrier includes normal saline, bacteriostatic water or phosphate-buffered saline (PBS), ethanol, a solvent or dispersion medium for polyol (e.g., glycerol, propylene glycol and liquid polyethylene glycol), and a suitable mixture thereof. In one or more embodiments, the pharmaceutical composition further comprises a

pharmaceutically acceptable carrier. In one or more embodiments, the pharmaceutically acceptable carrier may comprise an antibacterial and/or antifungal agent, e.g., p-hydroxylbenzoate, chlorobutanol, phenol, ascorbic acid, thimerosal, etc. In one or more embodiments, the pharmaceutically acceptable carrier may comprise an isotonic agent, such as sugar, polyol (such as mannitol and sorbitol) and sodium chloride. In one or more embodiments, the pharmaceutical composition comprises at least 0.1% of the antibody or the antigen-binding unit thereof or the antibody-drug conjugate. The percentage of antibody may vary and is between about 2% and 90% by weight of a given dosage form. The amount of the antibody or the antigen-binding unit thereof or the antibody-drug conjugate in such a pharmaceutical composition may be an effective amount for administration.

In one or more embodiments, the present invention provides a method for preparing a pharmaceutical composition: mixing the antibody or the antigen-binding unit thereof or the antibody-drug conjugate described herein with a pharmaceutically acceptable carrier (e.g., water for injection, normal saline, etc.). Methods for mixing the antibody or the antigen-binding unit thereof or the antibody-drug conjugate described above with a pharmaceutically acceptable carrier are generally known in the art.

In one or more embodiments, the present invention provides a kit comprising the antibody or the antigen-binding unit thereof, the antibody-drug conjugate or the pharmaceutical composition comprising the same described herein and instructions guiding administration to a patient.

One or more embodiments further provide an article of manufacture comprising the anti-FRα antibody or the antigen-binding unit thereof, the antibody-drug conjugate or the pharmaceutical composition comprising the same described herein;

a container; and
a package insert, instructions or label indicating that the anti-FRα antibody, the antibody-drug conjugate or the pharmaceutical composition comprising the same described herein is for use in treating and/or preventing a disease associated with the expression of folate receptor α.

It will be understood by those skilled in the art that the antibodies of the present invention can be widely used. For example, the antibodies of the present invention can be used as therapeutic agents, reagents in diagnostic kits or diagnostic tools, or reagents in competitive experiments to produce therapeutic agents.

## BRIEF DESCRIPTION OF THE DRAWINGS

FIG. 1 shows a conjugation schematic diagram for an ADC.
FIG. 2 shows the endocytosis of ADC1.
FIG. 3 shows the bystander effect of ADC1.
FIG. 4 shows the *in vivo* inhibitory effect of ADC1 on tumors.
FIG. 5 shows the *in vivo* inhibitory effect of ADC1 on tumors.
FIG. 6 shows the growth curves (mean ± standard error) of tumor volumes of LU11554 xenograft model mice in each group.
FIG. 7 shows the growth curves (mean ± standard error) of tumor volumes of LU5197 xenograft model mice in each group.

## DETAILED DESCRIPTION

Unless otherwise defined, scientific and technical terms used in the present invention have the meanings that are commonly understood by those skilled in the art. Generally, the nomenclature and techniques used in cell culture, molecular biology and protein purification described herein are well known and commonly used in the art. Standard techniques are used for recombinant DNA, oligonucleotide synthesis and cell culture and transformation (e.g., electroporation and lipofection). Enzymatic reactions and purification techniques are performed according to the manufacturer's instructions, or methods commonly used in the art or described herein. The aforementioned techniques and methods are generally used as described in various comprehensive and specific documents that are well known in the art and that are cited and discussed in this specification. See, e.g., Sambrook et al., Molecular Cloning: A Laboratory Manual (the 2nd edition, Cold Spring Harbor Laboratory Press, Cold Spring Harbor, N.Y. (1989)).

### *Definitions*

It should be noted that the term "an" entity refers to one or more of the entities. For example, "an antibody"

should be interpreted as one or more antibodies. As such, the terms "a" (or "an"), "one or more" and "at least one" can be used interchangeably herein.

As used herein, the term "contain" or "comprise" means that the antibodies, compositions, methods or the like comprise the recited elements, such as components or steps and do not exclude the others. "Consisting essentially of..." means that the antibodies, compositions, methods or the like exclude other elements that have a fundamental impact on the characteristics of the combination, but do not exclude elements that do not substantially affect the characteristics of the antibodies, compositions, methods or the like. "Consisting of..." means that elements not specifically listed are excluded.

The term "antibody" as used herein refers to immunoglobulin (Ig) molecules and immunologically active portions of immunoglobulin molecules, i.e., molecules that contain an antigen-binding site that specifically binds to (immunoreacts with) an antigen. By "specifically bind" or "immunoreact" or "directed against" is meant that the antibody reacts with one or more antigenic determinants of the target antigen and does not react with other polypeptides, or binds to other polypeptides with very low affinity (KD > $10^{-6}$ g/mL). Antibodies include, but are not limited to, monoclonal antibodies, chimeric antibodies, dAbs (domain antibodies), single-chain antibodies, Fab, Fab- and $F(ab')_2$ fragments, Fv and Fab expression libraries.

The term "antibody" includes a wide variety of polypeptides that can be biochemically distinguished. Those skilled in the art will appreciate that the classes of heavy chains include gamma, mu, alpha, delta, or epsilon ($\gamma$, $\mu$, $\alpha$, $\delta$, or $\varepsilon$), and some subclasses (e.g., $\gamma$1-$\gamma$4). The nature of this chain determines the "type" of the antibody as IgG, IgM, IgA, IgG or IgE. Immunoglobulin subclasses (isotypes), such as IgG1, IgG2, IgG3, IgG4, IgG5, etc., have been well characterized and the functional specificity imparted is also known. All types of immunoglobulins are within the scope of the present invention. In one or more embodiments, the immunoglobulin molecule is of the IgG class. Two heavy chains and two light chains are connected in a "Y" configuration through disulfide bonds, wherein the light chain starts at the opening of "Y" configuration and extends through the variable region to surround the heavy chain.

The antibodies, antigen-binding units or derivatives disclosed herein include, but are not limited to, polyclonal antibodies, monoclonal antibodies, multispecific antibodies, fully human antibodies, humanized antibodies, primatized antibodies, chimeric antibodies, single-chain antibodies, epitope-binding fragments (e.g., Fab, Fab' and $F(ab')_2$), and single-chain Fv (scFv).

Light chains can be classified into kappa ($\kappa$) or lambda ($\lambda$). Each heavy chain may bind to a $\kappa$ or $\lambda$ light chain. In general, when an immunoglobulin is produced by a hybridoma, a B cell or a genetically engineered host cell, the light and heavy chains are connected by covalent bonds, and the "tail" portions of the two heavy chains are connected by covalent disulfide bonds or non-covalent bonds. In the heavy chains, the amino acid sequence extends from the N terminus at the forked end of the Y configuration to the C terminus at the bottom of each chain. The immunoglobulin $\kappa$ light chain variable region is $V_K$; and the immunoglobulin $\lambda$ light chain variable region is $V_\lambda$.

The terms "constant" and "variable" are used in accordance with function. The light chain variable region (VL) and the heavy chain variable region (VH) determine the antigen recognition and specificity. The light chain constant region (CL) and the heavy chain constant region (CH) impart important biological properties such as secretion, transplacental movement, Fc receptor binding, complement fixation, etc. By convention, the numbering of amino acids in the constant regions increases as they become further away from the antigen-binding site of the antibody or amino terminus. The N-terminal portion is the variable region, and the C-terminal portion is the constant region; the CH3 and CL domains actually comprise the carboxyl termini of the heavy chain and light chain, respectively.

In naturally occurring antibodies, the six "complementarity determining regions" or "CDRs" present in each antigen-binding domain are short, non-contiguous, antigen-specific binding amino acid sequences that form the antigen-binding domain, assuming that the antibody is present in its three-dimensional configuration in an aqueous environment. The remaining amino acids in the antigen-binding domain, referred to as the "framework" region, exhibit little intermolecular variability. Most of the framework regions adopt a $\beta$-sheet conformation, with the CDRs forming a loop structure connected to, or in some cases forming part of the $\beta$-sheet structure. Thus, the framework regions position the CDRs in a correct orientation by interchain non-covalent interactions through forming a scaffold. The antigen-binding domain with the specifically positioned CDRs forms a surface complementary to an epitope on the antigen that facilitates non-covalent binding of the antibody to its antigenic epitope. For a given heavy or light chain variable region, amino acids comprising the CDRs and the framework regions may be identified by one of ordinary skill in the art according to known methods (see, Kabat, E., et al., U.S. Department of Health and Human Services, Sequences of Proteins of Immunological Interest, (1983) and Chothia and Lesk, J. Mol. Biol., 196:901-917 (1987)). As used herein, the term "monoclonal antibody" (mAb) refers to a population of antibody molecules that contain only one molecular species of the antibody molecules consisting of a unique light chain gene product and a unique heavy chain gene product. In particular, the complementarity determining regions (CDRs) of the monoclonal antibody are identical in all the molecules of the population. MAbs contain an antigen-binding site capable of immunoreacting with a particular epitope of an antigen.

The term "single-chain antibody" (scFv) refers to an antibody in which its heavy chain variable region (VH) and light chain variable region (VL) are linked by a linker of 15-20 amino acids. The linker may be enriched with glycine to

improve flexibility, and enriched with serine or threonine to improve solubility, and may link the N terminus of VH and the C terminus of VL, or vice versa. Although the protein has the constant region removed and the linker introduced, it retains the specificity of the original immunoglobulin. ScFv molecules are generally known in the art and are described, for example, in U.S. patent No. 5,892,019.

The term "antigen-binding site" or "binding portion" refers to the portion of an immunoglobulin molecule that is involved in antigen binding. The antigen-binding site is formed from amino acid residues of the N-terminal variable (V) region of the heavy (H) chain and the light (L) chain. Three highly differentiated branches (referred to as "hypervariable regions") in the heavy chain variable region (VH) and light chain variable region (VL) are located between more conserved branches (referred to as "framework regions" or "FRs"). Thus, the term "FR" refers to amino acid sequences that naturally occur between or adjacent to hypervariable regions in immunoglobulins. In an antibody molecule, the three hypervariable regions of a light chain and the three hypervariable regions of a heavy chain are arranged relative to each other in three-dimensional space to form an antigen-binding surface. The antigen-binding surface is complementary to the three-dimensional surface of the bound antigen, and the three hypervariable regions of each of the heavy chains and light chains are referred to as "complementarity determining regions" or "CDRs". The heavy chain variable region comprises, in positional order, a VH FR1, a VH CDR1, a VH FR2, a VH CDR2, a VH FR3, a VH CDR3 and a VH FR4. The light chain variable region comprises, in positional order, a VL FR1, a VL CDR1, a VL FR2, a VL CDR2, a VL FR3, a VL CDR3 and a VL FR4. The alignment of amino acids to each domain can be in accordance with the definitions of Kabat Sequences of Proteins of Immunological Interest (National Institutes of Health (1987 and 1991)) or Chothia and Lesk's articles (J. Mol. Biol. 196:901-917 (1987); Chothia et al., Nature 342:878-883 (1989)).

The framework and CDR regions of a humanized antibody need not correspond precisely to the parental sequences, e.g., the donor antibody CDR or the consensus framework may be mutagenized by substitution, insertion and/or deletion of at least one amino acid residue so that the CDR or framework residue at that site does not correspond to either the donor antibody or the consensus framework. Usually, at least 80%, at least 85%, at least 90% or at least 95% of the humanized antibody residues will correspond to those of the parental FR and CDR sequences. As used herein, the term "consensus framework" refers to the framework region in the consensus immunoglobulin sequence. As used herein, the term "consensus immunoglobulin sequence" refers to the sequence formed from the most frequently occurring amino acids (or nucleotides) in a family of related immunoglobulin sequences (See e.g., Winnaker, From Genes to Clones (Verlagsgesellschaft, Weinheim, Germany 1987)). In a family of immunoglobulins, each position in the consensus sequence is occupied by the amino acid occurring most frequently at that position in the family. If two amino acids occur equally frequently, either can be included in the consensus sequence.

Where the terms used and/or accepted in the art have two or more definitions, the definitions of the terms used herein include all of these meanings unless explicitly indicated as opposite. One specific example is the use of the term "complementarity determining regions" ("CDRs") to describe non-contiguous antigen-binding sites found within the variable regions of heavy and light chain polypeptides. This specific region is described in Kabat et al., U.S. Dept. of Health and Human Services, Sequences of Proteins of Immunological Interest (1983) and Chothia et al., J. Mol. Biol. 196:901-917 (1987), which are incorporated herein by reference in their entirety.

Kabat et al. also define a numbering scheme applicable to the variable region sequence of any antibody. One of ordinary skills in the art can apply the "Kabat numbering" scheme to any variable region sequence without depending on other experimental data beyond the sequence itself. "Kabat numbering" refers to the numbering system proposed by Kabat et al., U.S. Dept. of Health and Human Services in Sequence of Proteins of Immunological Interest (1983). EU or Chothia numbering scheme can also be applicable to the antibody.

The antibodies disclosed herein may be derived from any animal, including fish, birds and mammals. Preferably, the antibody is derived from a human being, a mouse, a donkey, a rabbit, a goat, a camel, a llama, a horse, or a chicken source. In another embodiment, the variable region may be derived from a condricthoid source (e.g., from a shark).

The "heavy chain constant region" comprises at least one of a CH1 domain, a hinge (e.g., upper, middle, and/or lower hinge region) domain, a CH2 domain, a CH3 domain, or a variant or a fragment. The heavy chain constant regions of the antibody may be derived from different immunoglobulin molecules. For example, the heavy chain constant regions of the polypeptide may comprise a CH1 domain derived from an IgG1 molecule and a hinge region derived from an IgG3 molecule. In another embodiment, the heavy chain constant region may comprise a hinge region derived partially from an IgG1 molecule and partially from an IgG3 molecule. In another embodiment, a portion of the heavy chain may comprise a chimeric hinge region derived partially from an IgG1 molecule and partially from an IgG4 molecule.

"Light chain constant region" includes a part of amino acid sequence from the light chain of an antibody. Preferably, the light chain constant region comprises at least one of a constant κ domain or a constant λ domain. "Light chain-heavy chain pair" refers to a collection of light and heavy chains that can form dimers through disulfide bonds between the CL domain of the light chain and the CH1 domain of the heavy chain.

The "VH domain" includes the variable domain at the amino terminus of an immunoglobulin heavy chain. The "CH1 domain" includes the first constant region of an immunoglobulin heavy chain. The CH2 domain is not closely paired with other domains, but rather two N-linked branched carbohydrate chains are inserted between the two CH2 domains

of an intact native IgG molecule. The CH3 domain extends from the CH2 domain to the C terminus of the IgG molecule and comprises about 108 residues. "Hinge region" includes a portion of the heavy chain region connecting the CH1 domain and CH2 domain. The hinge region comprises about 25 residues and is flexible, thereby enabling independent movement of the two N-terminal antigen-binding regions. The hinge region can be subdivided into three distinct domains: upper, middle and lower hinge domains (Roux et al., J. Immunol., 161:4083 (1998)).

"Disulfide bond" refers to a covalent bond formed between two sulfur atoms. The thiol group of cysteine can form a disulfide bond or a bridge with a second thiol group. In most naturally occurring IgG molecules, the CH1 and CL regions are linked by a disulfide bond.

"Chimeric antibody" refers to any antibody in which the variable region of the antibody is obtained or derived from a first species, and the constant region thereof (which may be intact, partial or modified) is derived from a second species. In certain embodiments, the variable region is derived from a non-human source (e.g., mouse or primate) and the constant region is derived from a human source.

The term "epitope" as used herein includes any protein determining region that can specifically bind to an immunoglobulin or a fragment thereof or a T cell receptor. The epitope determinant generally consists of chemically active surface groups of molecules (such as amino acids or sugar side chains), and generally has particular three-dimensional structural properties and specific charge properties.

As used herein, the term "specific binding" refers to the type of non-covalent interactions that occur between an immunoglobulin molecule and an antigen for which the immunoglobulin is specific. The strength or affinity of an immunological binding interaction can be expressed in terms of the equilibrium dissociation constant (KD) of the interaction, where a smaller KD represents a greater affinity. The immunological binding properties of the selected polypeptides may be quantified using methods well known in the art. One such method requires the measurement of the rates of antigen-binding site/antigen complex formation and dissociation, where those rates depend on the concentration of the complex partners, the affinity of the interaction and geometric parameters that affect the rates equally in both directions. Thus, both "association rate constant" ($k_{on}$) and "dissociation rate constant" ($k_{off}$) can be determined by calculating the concentration and the actual association and dissociation rates (see Nature 361: 186-87 (1993)). The ratio $k_{off}/k_{on}$ is capable of eliminating all the parameters that are independent of affinity and is equal to the equilibrium dissociation constant KD (generally see Davies et al., (1990) Annual Rev Biochem 59:439-473). Specific binding can be measured by radioligand binding assays, surface plasmon resonance (SPR), flow cytometry binding assay or similar assays known to those skilled in the art.

The term "isolated" as used herein with respect to cells, nucleic acids, polypeptides and the like, e.g., "isolated" DNA, RNA or polypeptides, refers to molecules that are separated from one or more other components, e.g., DNA or RNA, in the natural environment of the cell. The term "isolated" as used herein also refers to nucleic acids or peptides that are substantially free of cellular materials, viral materials or cell media when produced by recombinant DNA techniques, or of chemical precursors or other chemicals when chemically synthesized. In addition, "isolated nucleic acid" is intended to include nucleic acid fragments that do not and will not occur in nature. The term "isolated" is also used herein to refer to cells or polypeptides that are separated from other cellular proteins or tissues. Isolated polypeptides are intended to include both purified and recombinant polypeptides. Isolated polypeptides and the like are usually prepared by at least one purification step. In one or more embodiments, the purity of the isolated nucleic acids, polypeptides and the like is at least about 50%, about 60%, about 70%, about 80%, about 90%, about 95%, about 99%, or a range between any two of these values (inclusive) or any value therein.

An "isolated" antibody is one that is isolated and/or recovered from a component of its natural environment. Some components of its natural environment are substances that would interfere with diagnostic or therapeutic uses of the antibody, and can include enzymes, hormones and other proteinaceous or non-proteinaceous solutes. In some embodiments, the antibody is purified to the following extent: (1) the percentage, by weight, of the antibody is greater than 95%, e.g., greater than 99%, as measured by the Lowry method; (2) at least 15 residues of the N-terminal or internal amino acid sequence can be obtained by using a spinning cup sequenator; or (3) the antibody is homogeneous, as determined by reducing or non-reducing SDS-PAGE with Coomassie blue or silver staining. Isolated antibodies include antibodies *in situ* within recombinant cells. Typically, an isolated antibody will be prepared through at least one or more purification steps. In some embodiments, the purity of the isolated antibody is at least about 50%, about 60%, about 70%, about 80%, about 90%, about 95%, about 99%, or a range between any two of these values (inclusive) or any value therein.

The term "encoding" as applied to a polynucleotide refers to a polynucleotide known to "encode" a polypeptide. When in its native state or manipulated by methods well known to those skilled in the art, the polynucleotide can be transcribed and/or translated to produce the polypeptide and/or a fragment thereof.

The term "recombinant", with regard to a polypeptide or polynucleotide, is intended to refer to a polypeptide or polynucleotide that does not occur in nature, and non-limiting examples can be combined to produce a polynucleotide or polypeptide that does not normally occur. The term "sequence identity" means that two polynucleotides or amino acid sequences are identical (i.e., are identical on a nucleotide-by-nucleotide or residue-by-residue basis) over the comparison

window. The term "percentage of sequence identity" is calculated by comparing two optimally aligned sequences over the comparison window, determining the number of positions at which the identical amino acid residue occurs in both sequences to yield the number of matched positions, dividing the number of matched positions by the total number of positions in the comparison window (i.e., the window size), and then multiplying the result by 100 to yield the percentage of sequence identity. "At least 90% sequence identity" refers to about 90% sequence identity, about 91% sequence identity, about 92% sequence identity, about 93% sequence identity, about 95% sequence identity, about 96% sequence identity, about 97% sequence identity, about 98% sequence identity, about 99% sequence identity, or a range between any two of these values (inclusive) or any value therein.

"Amino acid" refers to an organic compound containing both an amino group and a carboxyl group, such as an α-amino acid that can be encoded by a nucleic acid, either directly or in the form of a precursor. A single amino acid is encoded by a nucleic acid consisting of three nucleotides (so-called codon or base triplet). Each amino acid is encoded by at least one codon. The encoding of the same amino acid by different codons is known as "codon degeneracy". Amino acids include natural amino acids and non-natural amino acids.

As used herein, the twenty conventional amino acids and their abbreviations follow conventional usage. See Immunology-A Synthesis (second edition, Eds. E. S. Golub and D. R. Gren, Sinauer Associates, Sunderland 7 Mass. (1991)). Stereoisomers (e.g., D-amino acids) of the twenty conventional amino acids, unnatural amino acids (such as α- or α-disubstituted amino acids), N-alkyl amino acids, lactic acid, and other unconventional amino acids can also be suitable components for the polypeptides of the present disclosure. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine and other similar amino acids and imino acids (e.g., 4-hydroxyproline). In the polypeptide notation used herein, the left-hand direction is the amino-terminal direction and the right-hand direction is the carboxy-terminal direction, in accordance with standard usage and convention. The conventional (natural) amino acids include alanine (three-letter symbol: Ala, one-letter symbol: A), arginine (Arg, R), asparagine (Asn, N), aspartic acid (Asp, D), cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), and valine (Val, V).

The term "polypeptide" is intended to encompass both the singular form "polypeptide" and the plural form "polypeptides", and refers to a molecule consisting of amino acid monomers linearly linked by amide bonds (also known as peptide bonds). The term "polypeptide" refers to any single chain or multiple chains of two or more amino acids and does not refer to a specific length of the product. Thus, included within the definition of "polypeptide" are peptides, dipeptides, tripeptides, oligopeptides, "proteins", "amino acid chains" or any other term used to refer to chains of two or more amino acids, and the term "polypeptide" may be used in place of, or interchangeably with, any of the above terms. The term "polypeptide" is also intended to refer to a product of post-expression polypeptide modification, including but not limited to glycosylation, acetylation, phosphorylation, amidation, derivatization by known protecting/blocking groups, proteolytic cleavage, or non-naturally occurring amino acid modification. The polypeptide may be derived from a natural biological source or produced by recombinant techniques, but it is not necessarily translated from a specified nucleic acid sequence. It may be produced in any manner, including chemical synthesis.

The term "substantially identical" when applied to polypeptides means that two peptide sequences, when optimally aligned, such as by the program GAP or BESTFIT using default GAP weights, share at least 80% sequence identity, preferably at least 90% sequence identity, more preferably at least 95% sequence identity, and most preferably at least 99% sequence identity.

It is understood by those skilled in the art that when an amino acid or polypeptide is used as a component of a molecule (e.g., an antibody or an ADC), the amino acid or polypeptide refers to the amino acid residue or polypeptide residue (whether or not explicitly described), i.e., the remainder after some of groups of the amino acid or polypeptide (e.g., one hydrogen atom of an amino group and/or hydroxy group of a carboxyl group) are lost due to the formation of a covalent bond (e.g., an amide bond) with other portions of the molecule when the amino acid or polypeptide binds to other portions of the molecule.

A polynucleotide consists of a specific sequence of four bases: adenine (A), cytosine (C), guanine (G) and thymine (T)/uracil (U, instead of thymine when the polynucleotide is RNA). A "polynucleotide sequence" can be a letter representation of a polynucleotide molecule. The letter representation can be input into a database in a computer with a central processing unit and used for bioinformatics applications, such as functional genomics and homology searches.

The terms "polynucleotide" and "oligonucleotide" are used interchangeably to refer to a polymeric form of nucleotides of any length, whether deoxyribonucleotides or ribonucleotides or analogs thereof. The polynucleotide may have any three-dimensional structure and may perform any function, known or unknown. The following are non-limiting examples of polynucleotides: genes or gene fragments (such as probes, primers, EST or SAGE tags), exons, introns, messenger RNA (mRNA), transfer RNA, ribosomal RNA, ribozymes, cDNA, dsRNA, siRNA, miRNA, recombinant polynucleotides, branched polynucleotides, plasmids, vectors, isolated DNA of any sequence, isolated RNA of any sequence, and nucleic acid probes and primers. Polynucleotides can include modified nucleotides, such as methylated nucleotides

and nucleotide analogs. If present, structural modifications to the nucleotide can be made before or after the assembly of the polynucleotide. The sequence of nucleotides can be interrupted by non-nucleotide components. The polynucleotide may be further modified after polymerization, such as by conjugation with a labeling component. This term also refers to double-stranded and single-stranded molecules. Unless otherwise stated or required, examples of any polynucleotide disclosed herein include a double-stranded form and each of the two complementary single-stranded forms known or predicted to constitute the double-stranded form.

A nucleic acid or a polynucleotide sequence (or a polypeptide or an antibody sequence) having a certain percentage (e.g., 90%, 95%, 98% or 99%) of "identity or sequence identity" to another sequence refers to that when the sequences are aligned, the percentage of bases (or amino acids) in the compared sequences are the same. This alignment and identity percentage or sequence identity can be determined using visual inspection or software programs known in the art, such as the software programs described in Ausubel et al. eds., (2007), Current Protocols in Molecular Biology. Preferably, the alignment is performed using default parameters. One alignment program is BLAST using default parameters, such as BLASTN and BLASTP, both using the following default parameters: Genetic code = standard; filter = none; strand = both; cutoff = 60; expect = 10; Matrix = BLOSUM62; Descriptions = 50 sequences; sort by = HIGH SCORE; Databases = non-redundant; GenBank + EMBL + DDBJ + PDB + GenBank CDS translations + SwissProtein + SPupdate + PIR. Biologically equivalent polynucleotides are polynucleotides having the above-specified percentage identity and encoding polypeptides having identical or similar biological activity.

Unless otherwise stated, the left-hand end of a single-stranded polynucleotide sequence is the 5' end, and the left-hand direction of a double-stranded polynucleotide sequence is referred to as the 5' direction. The direction of 5' to 3' addition of nascent RNA transcripts is referred to as the transcription direction; sequence regions on the DNA strand which have the same sequence as the RNA and are 5' to the 5' end of the RNA transcript are referred to as "upstream sequences"; sequence regions on the DNA strand which have the same sequence as the RNA and are 3' to the 3' end of the RNA transcript are referred to as "downstream sequences".

In some embodiments, residue positions that are not identical differ in conservative amino acid substitutions.

Minor variations in the amino acid sequences of antibodies or immunoglobulin molecules are contemplated by the present disclosure, provided that the identity of the amino acid sequences is maintained to be at least 90%, such as at least 92%, 95%, 98% or 99%. In some embodiments, the variations are conservative amino acid substitutions. Conservative amino acid substitutions are those that occur within a family of amino acids that are related in their side chains. Genetically encoded amino acids are generally classified into the following categories: (1) acidic amino acids: aspartate, and glutamate; (2) basic amino acids: lysine, arginine, and histidine; (3) non-polar amino acids: alanine, valine, leucine, isoleucine, proline, phenylalanine, methionine, and tryptophan; and (4) uncharged polar amino acids: glycine, asparagine, glutamine, cysteine, serine, threonine, and tyrosine. Amino acids of the other families include (i) serine and threonine of the aliphatic-hydroxy family; (ii) asparagine and glutamine of the amide-containing family; (iii) alanine, valine, leucine, and isoleucine of the aliphatic family; and (iv) phenylalanine, tryptophan, and tyrosine of the aromatic family. In some embodiments, conservative amino acid substitution groups are valine-leucine-isoleucine, phenylalanine-tyrosine, lysine-arginine, alanine-valine, glutamic acid-aspartic acid, and asparagine-glutamine. For example, it is reasonable to expect that the single replacement of leucine with isoleucine or valine, aspartate with glutamate, threonine with serine, or the similar replacement of an amino acid with a structurally related amino acid, will not have a major effect on the binding or properties of the resulting molecule, particularly when the replacement does not involve an amino acid within a binding site. Whether an amino acid change results in a functional peptide can be readily determined by measuring the specific activity of the polypeptide derivative. The measurement is described in detail herein. Fragments or analogs of antibodies or immunoglobulin molecules can be readily prepared by those of ordinary skill in the art.

In some embodiments, the amino acid substitutions have the following effects: (1) reducing susceptibility to proteolysis, (2) reducing susceptibility to oxidation, (3) altering the binding affinity for formation of protein complexes, (4) altering binding affinity, and (5) conferring or improving other physicochemical or functional properties of such analogs. Analogs can include various mutant proteins with sequences different from those of the naturally occurring peptide sequences. For example, single or multiple amino acid substitutions (preferably conservative amino acid substitutions) may be made in the naturally occurring sequence (preferably in a portion of the polypeptide outside the domains that form intermolecular contacts). A conservative amino acid substitution should not significantly alter the structural characteristics of the parent sequence (e.g., an amino acid for the replacement should not tend to disrupt a helix that occurs in the parent sequence, or disrupt other types of secondary structures that characterize the parent sequence). Examples of secondary and tertiary structures of artificially recognized polypeptides are described in Proteins: Structures and Molecular Principles (Ed. Creighton, W. H. Freeman and Company, New York (1984)); Introduction to Protein Structure (Eds. C. Branden and J. Tooze, Garland Publishing, New York, N.Y (1991)); and Thornton et al., Nature 354:105 (1991).

The number of amino acids of conservative amino acid substitutions of VL or VH may be about 1, about 2, about 3, about 4, about 5, about 6, about 8, about 9, about 10, about 11, about 13, about 14, about 15, or a range between any two of these values (inclusive) or any value therein. The number of amino acids of conservative amino acid substitutions of a heavy chain constant region, a light chain constant region, a heavy chain or a light chain may be

about 1, about 2, about 3, about 4, about 5, about 6, about 8, about 9, about 10, about 11, about 13, about 14, about 15, about 18, about 19, about 22, about 24, about 25, about 29, about 31, about 35, about 38, about 41, about 45 conservative amino acid substitutions, or a range between any two of these values (inclusive) or any value therein.

The term "agent" as used herein means a chemical compound, a mixture of chemical compounds, a biological macromolecule, or an extract made from biomaterial.

As used herein, the term "label" or "labeled" refers to incorporation of a detectable marker, e.g., by incorporation of a radiolabeled amino acid or attachment to a polypeptide of biotinyl moieties that can be detected by marked avidin (e.g., streptavidin containing a fluorescent marker or having enzymatic activity that can be detected by optical or calorimetric methods). In certain instances, the label or labels may also be therapeutic. Various methods for labeling polypeptides and glycoproteins are known in the art and can be used. Examples of labels for polypeptides include, but are not limited to: radioisotopes or radionuclides (e.g., $^{3}H$, $^{14}C$, $^{15}N$, $^{35}S$, $^{90}Y$, $^{99}Tc$, $^{111}In$ $^{125}I$ and $^{131}I$), fluorescent labels (e.g., FITC, rhodamine and lanthanide phosphor), enzymatic labels (e.g., horseradish peroxidase, β-galactosidase, luciferase and alkaline phosphatase), chemiluminescent labels, biotinyl groups and predetermined polypeptide epitopes recognized by secondary reporter genes (e.g., leucine zipper pair sequences, secondary antibody-binding sites, metal binding domains and epitope tags). In some embodiments, the labels are connected by spacer arms of various lengths to reduce potential steric hindrance. The term "medicament" or "drug" refers to a compound or composition that is capable of inducing the desired therapeutic effect when properly administered to a patient.

"About" refers to a general error range for corresponding values as readily understood by those skilled in the relevant art. In some embodiments, "about" mentioned herein refers to the values described and ranges thereof of ±10%, ±5% or ±1%.

"$EC_{50}$", i.e., concentration for 50% of maximal effect, refers to a concentration that causes 50% of maximal effect.

"$IC_{50}$" represents 50% inhibitory concentration, i.e., a concentration of drug or inhibitor required to inhibit half of a given biological process.

"Treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of extent of disease, stabilized (i.e., not worsening) state of disease, delay or slowing of disease progression, amelioration, palliation, alleviation, or elimination (whether partial or total) of state of disease, prolongation of life expectancy as compared with that in the absence of treatment, and the like, as either detectable or undetectable. Patients in need of treatment include patients already with a condition or disorder, patients susceptible to a condition or disorder, or patients in need of prevention of the condition or disorder, or patients who may or are expected to benefit from administration of the antibody or the pharmaceutical composition disclosed herein for detection, diagnostic procedures, and/or treatment.

The term "cancer" means or is intended to describe the physiological state of a mammal, which is typically characterized by uncontrolled cell growth. Examples of cancers include, but are not limited to, carcinoma, lymphoma, blastoma, sarcoma or leukemia. More specific examples of such cancers include, but are not limited to, colorectal cancer, lung cancer, ovarian cancer, uterine cancer, endometrial cancer, salivary gland cancer, peritoneal cancer, fallopian tube cancer, pancreatic cancer, thyroid cancer, head and neck squamous cell carcinoma, nasopharyngeal cancer, laryngeal cancer, lung adenocarcinoma, lung squamous cell carcinoma, liver cancer, hepatocellular carcinoma, gastrointestinal cancer, glioblastoma, breast cancer, brain cancer, renal cancer, renal cell carcinoma, colon cancer, rectal cancer, prostate cancer, vulval cancer, testicular cancer, squamous cell carcinoma, small cell lung cancer, cervical cancer, bladder cancer, retinoblastoma, glioblastoma, mesothelioma, oral epithelioid cancer, choriocarcinoma and head and neck cancer.

The term "overexpression" or "overexpressed" interchangeably refers to a gene that is transcribed or translated at a detectably greater level, usually in certain cells, e.g., a cancer cell, in comparison to a normal cell. Overexpression may be overexpression of a protein and RNA (due to increased transcription, post transcriptional processing, translation, post translational processing, altered stability, and altered protein degradation), as well as local overexpression due to altered protein traffic patterns (increased nuclear localization), and augmented functional activity, e.g., increased enzyme hydrolysis of substrate. Overexpression may be by 5%, 10%, 20%, 30%, 50%, 60%, 70%, 80%, 90% or more in comparison to a normal cell or control cell. In certain embodiments, the anti-FRα antibody and the antibody-drug conjugate of the present invention are used to treat solid tumors likely to overexpress folate receptor α.

As used herein, the term "tumor overexpressing folate receptor α" refers to a tumor overexpressing folate receptor α (including benign tumors and cancers). In some embodiments, the expression of folate receptor α in a tumor sample above background levels of immune tissue (e.g., as determined by immunohistochemical staining) indicates that the tumor is a tumor overexpressing folate receptor α. Methods for detecting the expression of folate receptor α in a tumor are known in the art, such as immunohistochemical assays. In some embodiments, an "FRα-negative cell" is a cell that lacks folate receptor α above background in a cell sample (e.g., as determined by immunohistochemical techniques).

As used herein, the term "administering" means delivering a substance (e.g., an anti-folate receptor α antibody

or ADC) for therapeutic purposes (e.g., to treat a disorder associated with folate receptor α). The mode of administration can be parenteral, enteral, and topical. Parenteral administration is typically performed by injection, including but not limited to, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

As used herein, the term "effective amount" or "therapeutically effective amount" refers to the amount of a drug, e.g., an antibody or ADC, that is sufficient to reduce or ameliorate the severity and/or duration of a disorder (e.g., cancer) or one or more symptoms thereof, prevent the progression of a disorder, cause regression of a disorder, prevent the recurrence, development, onset or progression of one or more symptoms associated with a disorder, detect a disorder, or enhance or improve the prophylactic or therapeutic effect of another therapy (e.g., a prophylactic or therapeutic agent). For example, the effective amount of an antibody may inhibit tumor growth (e.g., inhibit an increase in tumor volume), decrease tumor growth (e.g., decrease tumor volume), reduce the number of cancer cells, and/or relieve to some extent one or more of the symptoms associated with the cancer. For example, the effective amount may improve disease free survival (DFS), improve overall survival (OS), or decrease likelihood of recurrence.

The terms "patient" and "subject" are used interchangeably and refer to any mammal in need of diagnosis, prognosis or treatment, including, but not limited to, humans, dogs, cats, guinea pigs, rabbits, rats, mice, horses, cattle, and the like. In some embodiments, the patient is a human.

As used herein, the term "in need" means that the patient has been identified as in need of a particular method or treatment. In some embodiments, identification may be made by any diagnostic mode. The patient may be in need of any methods and treatments described herein. The term "administration" as used herein refers to administering a substance for therapeutic purposes (e.g., treating a tumor).

The term "drug for treating a tumor" as used herein refers to an agent having the functional property of inhibiting in a human the development or progression of a tumor, particularly a malignant (cancerous) lesion, such as cancer, sarcoma, lymphoma or leukemia. Inhibition of metastasis is a property of antineoplastic drugs in many cases.

The term "pharmaceutically acceptable carrier" refers generally to any type of non-toxic solid, semi-solid, or liquid filler, diluent, encapsulating material, formulation additive, etc.

The term "carrier" refers to a diluent, an adjuvant, an excipient or a carrier that can be administered to a patient together with the active ingredient. Such pharmaceutical carriers can be sterile liquids, such as water and oils, including oils originating from petroleum, animal, plant or synthesis, such as peanut oil, soybean oil, mineral oil, sesame oil, etc. Water is a preferred carrier when the pharmaceutical composition is administered intravenously. A saline aqueous solution, a glucose aqueous solution, and a glycerol solution can also be used as a liquid carrier, especially for injection. Suitable pharmaceutical excipients include starch, glucose, lactose, sucrose, gelatin, malt, rice, flour, chalk, silica gel, sodium stearate, glycerol monostearate, talc, sodium chloride, skimmed milk powder, glycerol, propylene, glycol, water, ethanol, and the like. If desired, the composition may also comprise a small amount of a wetting agent, an emulsifier, or a pH buffering agent such as acetate, citrate or phosphate. Antibacterials such as benzyl alcohol or methylparaben, antioxidants such as ascorbic acid or sodium bisulfite, chelating agents such as ethylenediamine tetraacetic acid, and tonicity adjusting agents such as sodium chloride or dextrose are also contemplated. Such compositions may be in the form of solutions, suspensions, emulsions, tablets, pills, capsules, pulvises, sustained-release formulations and the like. The composition may be formulated as a suppository using conventional binders and carriers such as triglycerides. Oral formulations may comprise standard carriers such as mannitol, lactose, starch, magnesium stearate, sodium saccharin, cellulose and magnesium carbonate of pharmaceutical grade. Examples of suitable pharmaceutical carriers are described in Remington's Pharmaceutical Sciences by E. W. Martin, which is hereby incorporated herein by reference. Such compositions will contain a clinically effective dose of an antibody or an antigen-binding fragment, preferably in purified form, together with an appropriate amount of a carrier, to provide a form suitable for administration to a patient. The formulation should be suitable for the administration mode. The formulation may be encapsulated in an ampoule bottle, a disposable syringe, or a multidose vial made of glass or plastic.

The term "antibody-drug conjugate" or "ADC" refers to a binding protein (e.g., an antibody or an antigen-binding unit thereof) that is linked to one or more chemical drugs, which may optionally be a therapeutic agent or a cytotoxic agent. In a preferred embodiment, the ADC comprises an antibody, a drug (e.g., a cytotoxic drug), and a linker capable of attaching or conjugating the drug to the antibody. Non-limiting examples of drugs that can be included in the ADC are a mitotic inhibitor, an anti-tumor antibiotic, an immunomodulator, a vector for gene therapy, an alkylating agent, an anti-angiogenic agent, an anti-metabolite, a boron-containing agent, a chemoprotective agent, a hormone, an anti-hormonal agent, a corticosteroid, a photoactive therapeutic agent, an oligonucleotide, a radionuclide agent, a topoisomerase inhibitor, a kinase inhibitor (e.g., a TEC-family kinase inhibitor and a serine/threonine kinase inhibitor) and a radiosensitizer.

The terms "antibody-drug conjugate" and "ADC" are used interchangeably. The terms "anti-folate receptor α antibody-drug conjugate", "anti-FRα antibody-drug conjugate", "anti-FRα ADC" and "anti-folate receptor α ADC" are used interchangeably and refer to an ADC comprising an antibody that specifically binds to folate receptor α, wherein

the antibody is conjugated to one or more drugs. In one or more embodiments, the anti-FRα ADC comprises an antibody conjugated to exatecan. In one or more embodiments, the anti-FRα antibody or ADC binds to folate receptor α (e.g., human folate receptor α).

The term "drug to antibody ratio" or "DAR" refers to the number of drugs (e.g., exatecan) that are attached to the antibody in the ADC. The DAR of an ADC may be in the range of 1 to 10, but a higher load (e.g., 20) is also possible depending on the number of connection sites on the antibody. The term DAR may be used when referring to the number of drugs loaded onto a single antibody, or alternatively, when referring to an average or mean DAR for a set of ADCs. In some embodiments, the value is selected from 1, 2, 3, 4, 5, 6, 7, 8, 9, or 10. In considering the mean binding number of small molecule drugs, i.e., the mean drug binding number of an antibody, or the mean drug to antibody ratio, the value is selected from about 0 to about 10, or about 2 to about 8. In some embodiments, the drug to antibody ratio is about 3 to about 6. In other embodiments, the drug to antibody ratio is about 6 to about 8, or about 7 to about 8. DAR values may be denoted herein by p. It is understood by those skilled in the art that the average DAR values (p) of different batches of products from the same ADC can vary slightly. The DAR value of ADC can be measured by ultraviolet-visible absorption spectroscopy (UV-Vis), high performance liquid chromatography-hydrophobic interaction chromatography (HPLC-HIC), reversed-phase high performance liquid chromatography (RP-HPLC), liquid chromatography-mass spectrometry (LC-MS), etc. These techniques are described in Ouyang, J. Methods Mol Biol, 2013, 1045: p. 275-83.

Various substituents are defined below. In some cases, the number of carbon atoms in a substituent (e.g., alkyl, alkenyl, alkynyl, alkoxy, aminoalkoxy, aminoalkyl, aminoalkylamino, alkylamino, heterocyclyl, heterocyclylamino and aryl) is indicated by the prefix "Cx-Cy" or "Cx-y", wherein x is the minimum number and y is the maximum number of carbon atoms. Thus, for example, "C1-C6 alkyl" refers to an alkyl containing 1 to 6 carbon atoms. If a substituent is described as "substituted with...", a hydrogen atom on a carbon or nitrogen is substituted with a non-hydrogen group. For example, a substituted alkyl substituent is an alkyl substituent in which at least one hydrogen atom on the alkyl is substituted with a non-hydrogen group. To illustrate, monofluoroalkyl is an alkyl substituted with a fluoro group, and difluoroalkyl is an alkyl substituted with two fluoro groups. It should be recognized that if there is more than one substitution on a substituent, each substitution may be identical or different (unless otherwise stated). If a substituent is described as "optionally substituted with...", the substituent may be (1) unsubstituted or (2) substituted. Possible substituents include, but are not limited to, hydroxy, C1-C6 alkyl, C2-C6 alkenyl, C2-C6 alkynyl, C1-C6 alkoxy, C1-C6 aminoalkoxy, halogen, nitro, cyano, sulfhydryl, alkylthio, amino, C1-C6 aminoalkyl, C1-C6 aminoalkylamino, C1-C6 alkyl linking to a heterocycle, C1-C6 alkylamino linking to a heterocycle, heterocyclyl, amino-substituted heterocyclyl, heterocyclylamino, carbamoyl, morpholin-1-yl, piperidin-1-yl, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, - $(CH_2)_q$-(C3-C8 heterocyclyl)-$CH_3$, -(C3-C8 heterocyclyl)-$(CH_2)_q-CH_3$, - $(CH_2)_qC(O)NR^n(CH_2)_q-CH_3$, $-(CH_2CH_2O)_q-CH_3$, $-(CH_2CH_2O)_q-CH_2-CH_3$, - $(CH_2)_qC(O)NR^n(CH_2CH_2O)_q-CH_3$, $-(CH_2)_qC(O)NR^n(CH_2CH_2O)_q-CH_2-CH_3$, - $(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or - $(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10.

The "alkyl" refers to a saturated aliphatic hydrocarbyl group, and this term includes linear and branched hydrocarbyl groups. For example, C1-C20 alkyl, such as C1-C6 alkyl. C1-C20 alkyl refers to an alkyl group containing 1 to 20 carbon atoms, e.g., an alkyl group containing 1 carbon atom, 2 carbon atoms, 3 carbon atoms, 4 carbon atoms, 5 carbon atoms, 6 carbon atoms, 7 carbon atoms, 8 carbon atoms, 9 carbon atoms, 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, or 20 carbon atoms. Non-limiting examples of alkyl include methyl, ethyl, n-propyl, isopropyl, n-butyl, isobutyl, sec-butyl, tert-butyl, *n*-pentyl, neopentyl, n-hexyl, and the like. The alkyl may be unsubstituted or substituted with one or more substituents including, but not limited to, alkyl, alkoxy, cyano, hydroxy, carbonyl, carboxy, aryl, heteroaryl, amino, halogen, sulfonyl, sulfinyl, phosphonyl, and the like.

The term "alkenyl", by itself or as part of another substituent, refers to an unsaturated branched, linear or cyclic alkyl having at least one carbon-carbon double bond derived by the removal of one hydrogen atom from a single carbon atom of a parent olefin. Typical alkenyl includes, but is not limited to, ethenyl; propenyl (e.g., prop-1-en-1-yl, prop-1-en-2-yl, prop-2-en-1-yl, prop-2-en-2-yl, cycloprop-1-en-1-yl); cycloprop-2-en-1-yl; butenyl (e.g., but-1-en-1-yl, but-1-en-2-yl, 2-methyl-prop-1-en-1-yl, but-2-en-1-yl, but-2-en-2-yl, but-1,3-dien-1-yl, but-1,3-dien-2-yl, cyclobut-1-en-1-yl, cyclobut-1-en-3-yl, cyclobut-1,3-dien-1-yl, etc.), and the like.

The term "alkynyl", by itself or as part of another substituent, refers to an unsaturated branched, linear or cyclic alkyl having at least one carbon-carbon triple bond derived by the removal of one hydrogen atom from a single carbon atom of a parent alkyne. Typical alkynyl includes, but is not limited to, ethynyl; propynyl (e.g., prop-1-yn-1-yl, prop-2-yn-1-yl, etc.); butynyl (e.g., but-1-yn-1-yl, but-1-yn-3-yl, but-3-yn-1-yl, etc.), and the like.

The "carbocyclyl" refers to a stable non-aromatic monocyclic or polycyclic hydrocarbyl radical consisting of carbon and hydrogen atoms only, and may comprise a fused or bridged ring system, contains 3 to 15 carbon atoms, for example, 3 to 10 (e.g., 3, 4, 5, 6, 7, 8, 9 or 10) carbon atoms. It is saturated or unsaturated, and links to the remainder

of the molecule through a single bond. Monocyclic radicals include, for example, cyclopropyl, cyclobutyl, cyclopentyl, cyclohexyl, cycloheptyl and cyclooctyl. Polycyclic radicals include, for example, adamantyl, norbornyl, and decahydronaphthyl. When specifically stated, the carbocyclyl may be optionally substituted with one or more substituents independently selected from the following: alkyl, halogen, haloalkyl, cyano, nitro, oxo, aryl, aralkyl, carbocyclyl, carbocyclylalkyl, heterocyclyl, heterocyclylalkyl, heteroaryl and heteroarylalkyl.

The "aryl" refers to an all-carbon monocyclic or all-carbon fused ring having a completely conjugated $\pi$-electron system, and typically has 5-14 carbon atoms, e.g., 6, 10, 12, or 14 carbon atoms. Aryl may be unsubstituted or substituted with one or more substituents including, but not limited to, alkyl, alkoxy, cyano, hydroxy, carboxy, aryl, aralkyl, amino, halogen, sulfonyl, sulfinyl, phosphonyl. Examples of unsubstituted aryl include, but are not limited to, phenyl, naphthyl, and anthracenyl.

The "heterocyclyl" refers to a stable 3 to 18 membered aromatic or nonaromatic ring group consisting of 2 to 8 (e.g., 2, 3, 4, 5, 6, 7 or 8) carbon atoms and 1 to 6 (1, 2, 3, 4, 5 or 6) heteroatoms selected from nitrogen, oxygen and sulfur. Unless otherwise specifically stated, heterocyclyl may be a monocyclic, bicyclic, tricyclic or tetracyclic ring system, and may include fused or bridged ring systems; and the nitrogen, carbon or sulfur atoms in heterocyclyl may be optionally oxidized; the nitrogen atoms are optionally quaternized; and heterocyclyl may be partially or fully saturated. Examples of such heterocyclyl include, but are not limited to, dioxolanyl, dioxinyl, thienyl[1,3]dithianyl, decahydroisoquinolinyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidinonyl, pyrrolidinyl, pyrazolidinyl, quinuclidinyl, thiazolidinyl, 1,2,4-thiadiazol-5(4*H*)-ylidene, tetrahydrofuranyl, trioxacyclohexyl, trithianyl, triazinanyl, tetrahydropyranyl, thiomorpholinyl, thiamorpholinyl, 1-oxo-thiomorpholinyl and 1,1-dioxo-thiomorpholinyl. When specifically stated in the specification, heterocyclyl may be optionally substituted with one or more substituents selected from: alkyl, alkenyl, halogen, haloalkyl, cyano, oxo, thioxo, nitro, aryl, aralkyl, cycloalkyl, cycloalkylalkyl, optionally substituted heterocyclyl, optionally substituted heterocyclylalkyl, optionally substituted heteroaryl, and optionally substituted heteroarylalkyl.

The "alkoxy" refers to formula -O-(alkyl), wherein alkyl is the alkyl defined herein. Non-limiting examples of alkoxy are methoxy, ethoxy, n-propoxy, 1-methylethoxy (isopropoxy), n-butoxy, isobutoxy, sec-butoxy, and tert-butoxy. Alkoxy may be substituted or unsubstituted. The "halogen" refers to fluoro (F), chloro (Cl), bromo (Br), or iodo (I).

The "amino" refers to $-NH_2$.

The "cyano" refers to -CN.

The "nitro" refers to $-NO_2$.

The "hydroxy" refers to -OH.

The "carboxyl" refers to -COOH.

The "thiol" refers to -SH.

The "carbonyl" refers to C=O.

"Stereoisomers" refer to isomeric compounds having the same linking order of atoms but different spatial arrangements of the atoms. Stereoisomers may have one or more stereocenters and each center may exist as R or S, and stereoisomers may also be cis-trans isomers. Stereoisomers of the compounds provided herein include any one of their all diastereomeric, enantiomeric and cis-trans isomeric forms, or suitable mixtures thereof.

The pharmaceutically acceptable salt includes those derived from the compound with a variety of organic and inorganic counterions well known in the art, and salts as examples only include organic or inorganic salts such as lithium salt, sodium salt, potassium salt, calcium salt, magnesium salt, ammonium salt, isopropylamine, trimethylamine, diethylamine, triethylamine, tripropylamine, ethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethyl, piperidine, polyamine resin, and tetraalkylammonium salt when the molecule contains an acidic functional group; and organic or inorganic acid salts such as hydrochloride salt, hydrobromide salt, tartrate salt, mesylate salt, acetate salt, maleate salt and oxalate salt when the molecule contains a basic functional group. Other non-limiting examples of acids include sulfuric acid, nitric acid, phosphoric acid, propionic acid, glycolic acid, pyruvic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. These salts can generally be prepared by conventional methods by reacting, for example, an appropriate acid or base with the compound. The solvate includes hydrates.

Other chemical terms herein are used according to conventional usage in the art, such as the McGraw-Hill Dictionary of Chemical Terms (Ed. Parker, S., McGraw-Hill, San Francisco (1985)).

All the publications and patents cited herein are incorporated herein by reference in their entirety for all purposes.

***Anti-FR$\alpha$ antibody***

The antibody provided by the present invention is an anti-folate receptor $\alpha$ antibody or an antigen-binding unit

thereof. In one or more embodiments, the antibody of the present invention is capable of specifically binding to human folate receptor α. In one or more embodiments, the present invention provides a humanized anti-FRα antibody or an antigen-binding unit thereof. These antibodies are collectively referred to herein as anti-FRα antibodies. The anti-FRα antibody or the antigen-binding unit thereof of the present invention has properties including, but not limited to, binding to FRα (e.g., human FRα) *in vitro,* binding to cells expressing FRα, high affinity and strong internalization capability.

In one or more embodiments, the anti-FRα antibody of the present invention is capable of specifically binding to folate receptor α (FOLR1), and does not bind to folate receptor β (FOLR2) or folate receptor γ (FOLR3).

In one or more embodiments, the antigen-binding fragment or antigen-binding unit of the anti-FRα antibody is Fab, Fab', $F(ab')_2$, Fv, disulfide bond-linked Fv, scFv, single-domain antibody or diabody. In one or more embodiments, the anti-FRα antibody is a multispecific antibody (e.g., bispecific antibody).

In one or more embodiments, the anti-FRα antibody may be a monoclonal antibody.

The binding specificity of the antibody or the antigen-binding unit thereof disclosed herein can be detected by *in-vitro* assays, such as co-immunoprecipitation, radioimmunoassay (RIA), surface plasmon resonance, flow cytometry (Facs), or enzyme-linked immunosorbent assay (ELISA).

The present invention further comprises an antibody that binds to the same epitope as the anti-FRα antibody described herein. For example, the antibody of the present invention specifically binds to an epitope comprising one or more amino acid residues on human FRα. In one or more embodiments, the antibody comprises a heavy chain constant region, such as an IgG1, IgG2, IgG3, IgG4, IgA, IgE, IgM or IgD constant region. In one or more embodiments, the anti-FRα antibody or the antigen-binding unit thereof comprises an immunoglobulin heavy chain constant domain selected from a human IgG constant domain, a human IgA constant domain, a human IgE constant domain, a human IgM constant domain and a human IgD constant domain. In one or more embodiments, the anti-FRα antibody or the antigen-binding unit thereof comprises an IgG1 heavy chain constant region, an IgG2 heavy chain constant region, an IgG3 heavy chain constant region or an IgG4 heavy chain constant region. In one or more embodiments, the heavy chain constant region is an IgG1 heavy chain constant region or an IgG4 heavy chain constant region. In one or more embodiments, the antibody or the antigen-binding unit thereof comprises a light chain constant region, e.g., a κ light chain constant region or a λ light chain constant region. In one or more embodiments, the antibody or the antigen-binding unit thereof comprises a κ light chain constant region. The Fc portion of an antibody mediates several important effector functions (e.g., cytokine induction, antibody-dependent cell-mediated cytotoxicity (ADCC), phagocytosis, complement-dependent cytotoxicity (CDC), and half-life/clearance of an antibody and an antigen-antibody complex). In some cases, these effector functions are desirable for therapeutic antibodies, but in other cases may be unnecessary or even detrimental depending on the therapeutic targets. Certain human IgG isotypes, particularly IgG1 and IgG3, mediate ADCC and CDC via binding to FcyRs and complement C1q, respectively. Neonatal Fc receptors (FcRns) are the critical components determining the circulating half-life of antibodies. In other embodiments, at least one amino acid residue is replaced in the constant region of the antibody (e.g., the Fc region of the antibody), such that effector functions of the antibody are altered. Replacements of amino acid residues in the Fc region of an antibody to alter antibody effector functions are described in U.S. Patent Nos. 5,648,260 and 5,624,821, which are incorporated herein by reference.

In one or more embodiments, the present invention includes a labeled anti-FRα antibody or an antigen-binding unit thereof, wherein the antibody is derived or linked to one or more functional molecules (e.g., another peptide or protein). For example, the labeled antibody can be derived by functionally linking an antibody or an antigen-binding unit thereof of the present invention (by chemical conjugation, genetic fusion, noncovalent binding or otherwise) to one or more other molecular entities, such as an additional antibody (e.g., a bispecific antibody or a bifunctional antibody), a detectable agent, a pharmaceutical agent, a protein or peptide that can mediate the binding of the antibody or the antigen-binding unit thereof to another molecule (such as a streptavidin core region or a polyhistidine tag), and/or a cytotoxic or therapeutic agent selected from the group consisting of a mitotic inhibitor, an anti-tumor antibiotic, an immunomodulator, a vector for gene therapy, an alkylating agent, an anti-angiogenic agent, an anti-metabolite, a boron-containing agent, a chemoprotective agent, a hormone, an anti-hormonal agent, a corticosteroid, a photoactive therapeutic agent, an oligonucleotide, a radionuclide agent, a topoisomerase inhibitor, a kinase inhibitor, a radiosensitizer and combinations thereof.

In one or more embodiments, the antibody or the antigen-binding unit thereof of the present invention is linked to a detectable agent, e.g., by incorporation of a radiolabeled amino acid or attachment to a polypeptide of biotinyl moieties that can be detected by marked avidin (e.g., streptavidin containing a fluorescent marker or having enzymatic activity that can be detected by optical or calorimetric methods). In certain instances, the label or labels may also be therapeutic. Various methods for labeling polypeptides and glycoproteins are known in the art and can be used. Examples of labels for polypeptides include, but are not limited to: radioisotopes or radionuclides (e.g., $^{3}H$, $^{14}C$, $^{15}N$, $^{35}S$, $^{90}Y$, $^{99}Tc$, $^{111}In$, $^{125}I$ and $^{131}I$), fluorescent labels (e.g., FITC, rhodamine and lanthanide phosphor), enzymatic labels (e.g., horseradish peroxidase, β-galactosidase, luciferase and alkaline phosphatase), chemiluminescent labels, biotinyl groups and predetermined polypeptide epitopes recognized by secondary reporter genes (e.g., leucine zipper pair sequences, secondary antibody-binding sites, metal binding domains and epitope tags). In one or more embodiments, the labels

are connected by spacer arms of various lengths to reduce potential steric hindrance.

In one or more embodiments, the antibody or the antigen-binding unit thereof of the present invention can be used to detect whether FRα (e.g., human FRα) is present in a sample or not. In one or more embodiments, the antibody comprises a detectable agent. The antibody is a polyclonal antibody, or more preferably, a monoclonal antibody. An intact antibody or antigen-binding unit thereof (e.g., Fab, scFv or $F(ab')_2$) can be used. The detection method of the embodiments can be used to detect an analyte mRNA, protein or genomic DNA in a biological sample *in vitro* as well as *in vivo.* For example, *in-vitro* techniques for detection of an analyte mRNA include Northern hybridization and in situ hybridization. *In-vitro* techniques for detection of an analyte protein include enzyme linked immunosorbent assay (ELISA), Western blots, immunoprecipitation, and immunofluorescence. *In-vitro* techniques for detection of an analyte genomic DNA include Southern hybridization. (ELISA: Theory and Practice: Methods in Molecular Biology, Vol. 42, J. R. Crowther Human Press, Totowa, N. J., 1995; Immunoassay, E. Diamandis and T. Christopoulus, Academic Press, Inc., San Diego, Calif., 1996; Practice and Theory of Enzyme Immunoassays, P. Tijssen, Elsevier Science Publishers, Amsterdam, 1985). In addition, *in-vivo* techniques for detection of an analyte protein include introducing a labeled anti-analyte protein antibody into a patient. For example, the antibody can be labeled with a radioactive marker, the presence and location of which in the patient can then be detected by standard imaging techniques.

In one or more embodiments, folate receptor α may be detected in a biological sample as part of a clinical testing procedure, e.g., to determine the efficacy of a given treatment regimen. Conjugating (e.g., physically linking) the antibody to a detectable substance can facilitate detection. Examples of detectable substances include various enzymes, prosthetic groups, fluorescent materials, luminescent materials, bioluminescent materials and radioactive materials. Examples of suitable enzymes include horseradish peroxidase, alkaline phosphatase, β-galactosidase or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; one example of a luminescent material includes luminol; examples of bioluminescent materials include luciferase, luciferin and aequorin; examples of suitable radioactive materials include $^{125}I$, $^{131}I$, $^{35}S$ or $^{3}H$.

### *Humanized antibody*

The anti-FRα antibody of the present invention includes a humanized antibody or an antigen-binding unit thereof. These antibodies or antigen-binding units thereof are suitable for administration to humans without causing detrimental immune responses in humans to the administered immunoglobulins.

In one or more embodiments, the humanized anti-FRα antibody or the antigen-binding unit thereof of the present invention comprises a VH CDR1 set forth in SEQ ID NO: 5; a VH CDR2 set forth in SEQ ID NO: 6; and a VH CDR3 set forth in SEQ ID NO: 7.

In one or more embodiments, the humanized anti-FRα antibody or the antigen-binding unit thereof of the present invention comprises a VL CDR1 set forth in SEQ ID NO: 8; a VL CDR2 set forth in SEQ ID NO: 9; and a VL CDR3 set forth in SEQ ID NO: 10.

In one or more embodiments, the humanized anti-FRα antibody or the antigen-binding unit thereof of the present invention comprises a VH CDR1 set forth in SEQ ID NO: 5; a VH CDR2 set forth in SEQ ID NO: 6; a VH CDR3 set forth in SEQ ID NO: 7; a VL CDR1 set forth in SEQ ID NO: 8; a VL CDR2 set forth in SEQ ID NO: 9; and a VL CDR3 set forth in SEQ ID NO: 10.

In one or more embodiments, the humanized anti-FRα antibody or the antigen-binding unit thereof of the present invention further comprises a framework region. The framework regions of the exemplary antibody VH and VL herein are shown in Table 1.

Table 1. Framework regions of antibody VH and VL

| Type | Amino acid sequence | SEQ ID NO: |
|---|---|---|
| VH FR 1 | QVQLVQSGVEVKKPGASVKVSCKASGYTFT | 1 |
| | QVQLVQSGVEVKKPGASVKVSCKASGYSFT | 29 |
| VH FR2 | WVRQAPGQGLEWMG | 2 |
| | WVRQAPGQGLEWIG | 30 |
| VH FR3 | RVTLTTDSSTTTAYMELKSLQFDDTAVYYCAR | 3 |
| | KATLTVDKSTTTAYMELKSLQFDDTAVYYCTR | 31 |
| VH FR4 | WGQGTTVTVSS | 11 |

(continued)

| Type | Amino acid sequence | SEQ ID NO: |
|---|---|---|
| VLFR1 | EIVLTQSPATLSLSPGERATLSC | 12 |
| VL FR2 | WYQQKPGQAPRLLIY | 13 |
| VL FR3 | GVPARFSGSGSGTDFTLTISSLEPEDFAVYYC | 4 |
| | GVPARFSGSGSKTDFTLTISSLEPEDFAVYYC | 32 |
| VL FR4 | FGGGTKVEIK | 14 |

In one or more embodiments, the humanized anti-FRα antibody or the antigen-binding unit thereof of the present invention comprises a VH FR1 set forth in SEQ ID NO: 1; a VH CDR1 set forth in SEQ ID NO: 5; a VH FR2 set forth in SEQ ID NO: 2; a VH CDR2 set forth in SEQ ID NO: 6; a VH FR3 set forth in SEQ ID NO: 3; a VH CDR3 set forth in SEQ ID NO: 7; and a VH FR4 set forth in SEQ ID NO: 11.

In one or more embodiments, the humanized anti-FRα antibody or the antigen-binding unit thereof of the present invention comprises a VH FR1 set forth in SEQ ID NO: 29; a VH CDR1 set forth in SEQ ID NO: 5; a VH FR2 set forth in SEQ ID NO: 30; a VH CDR2 set forth in SEQ ID NO: 6; a VH FR3 set forth in SEQ ID NO: 31; a VH CDR3 set forth in SEQ ID NO: 7; and a VH FR4 set forth in SEQ ID NO: 11.

In one or more embodiments, the humanized anti-FRα antibody or the antigen-binding unit thereof of the present invention comprises a VL FR1 set forth in SEQ ID NO: 12; a VL CDR1 set forth in SEQ ID NO: 8; a VL FR2 set forth in SEQ ID NO: 13; a VL CDR2 set forth in SEQ ID NO: 9; a VL FR3 set forth in SEQ ID NO: 4; a VL CDR3 set forth in SEQ ID NO: 10; and a VL FR4 set forth in SEQ ID NO: 14.

In one or more embodiments, the humanized anti-FRα antibody or the antigen-binding unit thereof of the present invention comprises a VL FR1 set forth in SEQ ID NO: 12; a VL CDR1 set forth in SEQ ID NO: 8; a VL FR2 set forth in SEQ ID NO: 13; a VL CDR2 set forth in SEQ ID NO: 9; a VL FR3 set forth in SEQ ID NO: 32; a VL CDR3 set forth in SEQ ID NO: 10; and a VL FR4 set forth in SEQ ID NO: 14.

In one or more embodiments, the humanized anti-FRα antibody or the antigen-binding unit thereof of the present invention comprises: a heavy chain variable region comprising a VH FR1 set forth in SEQ ID NO: 1; a VH CDR1 set forth in SEQ ID NO: 5; a VH FR2 set forth in SEQ ID NO: 2; a VH CDR2 set forth in SEQ ID NO: 6; a VH FR3 set forth in SEQ ID NO: 3; a VH CDR3 set forth in SEQ ID NO: 7; and a VH FR4 set forth in SEQ ID NO: 11; and a light chain variable region comprising a VL FR1 set forth in SEQ ID NO: 12; a VL CDR1 set forth in SEQ ID NO: 8; a VL FR2 set forth in SEQ ID NO: 13; a VL CDR2 set forth in SEQ ID NO: 9; a VL FR3 set forth in SEQ ID NO: 4; a VL CDR3 set forth in SEQ ID NO: 10; and a VL FR4 set forth in SEQ ID NO: 14.

In one or more embodiments, the humanized anti-FRα antibody or the antigen-binding unit thereof of the present invention comprises: a heavy chain variable region comprising a VH FR1 set forth in SEQ ID NO: 29; a VH CDR1 set forth in SEQ ID NO: 5; a VH FR2 set forth in SEQ ID NO: 30; a VH CDR2 set forth in SEQ ID NO: 6; a VH FR3 set forth in SEQ ID NO: 31; a VH CDR3 set forth in SEQ ID NO: 7; and a VH FR4 set forth in SEQ ID NO: 11; and a light chain variable region comprising a VL FR1 set forth in SEQ ID NO: 12; a VL CDR1 set forth in SEQ ID NO: 8; a VL FR2 set forth in SEQ ID NO: 13; a VL CDR2 set forth in SEQ ID NO: 9; a VL FR3 set forth in SEQ ID NO: 32; a VL CDR3 set forth in SEQ ID NO: 10; and a VL FR4 set forth in SEQ ID NO: 14.

In one or more embodiments, the heavy chain variable region of the humanized anti-FRα antibody or the antigen-binding unit thereof of the present invention comprises an amino acid sequence set forth in SEQ ID NO: 15 or an amino acid sequence having suitable sequence identity, such as at least 80% sequence identity, or at least 90% sequence identity, or at least 95% sequence identity, or at least 99% sequence identity, compared with the amino acid sequence set forth in SEQ ID NO: 15; and/or its light chain variable region comprises an amino acid sequence set forth in SEQ ID NO: 16 or an amino acid sequence having suitable sequence identity, such as at least 80% sequence identity, or at least 90% sequence identity, or at least 95% sequence identity, or at least 99% sequence identity, compared with the amino acid sequence set forth in SEQ ID NO: 16. In one or more embodiments, these amino acid sequences having sequence identity are at least CDR invariant.

In one or more embodiments, the humanized anti-FRα antibody or the antigen-binding unit thereof of the present invention comprises a heavy chain variable region set forth in SEQ ID NO: 15 and a light chain variable region set forth in SEQ ID NO: 16.

In one or more embodiments, the humanized anti-FRα antibody or the antigen-binding unit thereof of the present invention further comprises a heavy chain constant region comprising an amino acid sequence set forth in SEQ ID NO: 17 or an amino acid sequence having suitable sequence identity, such as at least 80% sequence identity, or at least 90% sequence identity, or at least 95% sequence identity, or at least 99% sequence identity, compared with the amino

acid sequence set forth in SEQ ID NO: 17.

In one or more embodiments, the humanized anti-FRα antibody or the antigen-binding unit thereof of the present invention further comprises a light chain constant region comprising an amino acid sequence set forth in SEQ ID NO: 18 or an amino acid sequence having suitable sequence identity, such as at least 80% sequence identity, or at least 90% sequence identity, or at least 95% sequence identity, or at least 99% sequence identity, compared with the amino acid sequence set forth in SEQ ID NO: 18.

In one or more embodiments, a heavy chain of the humanized anti-FRα antibody or the antigen-binding unit thereof of the present invention comprises an amino acid sequence set forth in SEQ ID NO: 19 or an amino acid sequence having suitable sequence identity, such as at least 80% sequence identity, or at least 90% sequence identity, or at least 95% sequence identity, or at least 99% sequence identity, compared with the amino acid sequence set forth in SEQ ID NO: 19.

In one or more embodiments, a light chain of the humanized anti-FRα antibody or the antigen-binding unit thereof of the present invention comprises an amino acid sequence set forth in SEQ ID NO: 20 or an amino acid sequence having suitable sequence identity, such as at least 80% sequence identity, or at least 90% sequence identity, or at least 95% sequence identity, or at least 99% sequence identity, compared with the amino acid sequence set forth in SEQ ID NO: 20.

In one or more embodiments, the heavy chain of the humanized anti-FRα antibody or the antigen-binding unit thereof of the present invention further comprises a signal peptide, such as MDWTWRILFLVAAATGAHS (SEQ ID NO: 21), of which the nucleotide sequence is atggattggacctggagaatcctgttcctggtggccgccgccaccggcgctcattct (SEQ ID NO: 25).

In one or more embodiments, the light chain of the humanized anti-FRα antibody or the antigen-binding unit thereof of the present invention further comprises a signal peptide, such as MEAPAQLLFLLLLWLPDTTG (SEQ ID NO: 22), of which the nucleotide sequence is atggaggcccctgcccagctgctgttcctgctgctgctgtggctgcctgataccaccggc (SEQ ID NO: 26).

In one or more embodiments, the humanized anti-FRα antibody or the antigen-binding unit thereof of the present invention binds to FRα with an equilibrium dissociation constant (KD) of about 1 μM or less. In one or more embodiments, the humanized anti-FRα antibody or the antigen-binding unit thereof of the present invention binds to FRα with a KD of between about 100 nM and about 1 pM or less. In one or more embodiments, the humanized anti-FRα antibody or the antigen-binding unit thereof of the present invention binds to FRα with a KD of between about 10 nM and about 1 pM or less. In one or more embodiments, the humanized anti-FRα antibody or the antigen-binding unit thereof of the present invention binds to FRα with a KD of between about 1 nM and about 0.1 nM or less.

In one or more embodiments, the humanized anti-FRα antibody or the antigen-binding unit thereof of the present invention binds to human FRα (e.g., an antigen FRα-His set forth in SEQ ID NO: 28) with a KD of between about 1 μM and about 1 pM or less. In one or more embodiments, the humanized anti-FRα antibody or the antigen-binding unit thereof of the present invention binds to human FRα with a KD of between about 100 nM and about 1 pM or less, or between about 10 nM and about 1 pM or less, or between about 1 nM and about 1 pM or less.

### *Antibody-drug conjugate*

The anti-FRα antibody or the antigen-binding unit thereof of the present invention can be conjugated to a drug to form an anti-FRα antibody-drug conjugate (anti-FRα ADC). Antibody-drug conjugates (ADCs) can increase the therapeutic efficacy of antibodies in treating diseases (e.g., cancers) due to their ability to selectively deliver one or more drugs to a target tissue (e.g., a tumor expressing FRα). In one or more embodiments, the antibody-drug conjugate (ADC) of the present invention comprises the anti-FRα antibody or the antigen-binding unit thereof described herein and at least one drug (e.g., exatecan). The ADC of the present invention has properties including, but not limited to, binding to FRα (e.g., human FRα) *in vitro,* binding to cells expressing FRα, high affinity, strong internalization capability, and reducing or inhibiting the growth of cancer cells or tumors.

The antigen-binding fragment or antigen-binding unit of the anti-FRα antibody of the present invention may be conjugated to the drug described herein. Thus, in one or more embodiments, the antigen-binding fragment or antigen-binding unit of the anti-FRα antibody described herein is conjugated to a drug via a linker to form an anti-FRα ADC.

The anti-FRα ADC of the present invention comprises an antibody or an antigen-binding unit thereof that is linked to one or more drugs and specifically binds to FRα (e.g., human FRα). The specificity of the ADC may be determined by the specificity of the antibody (e.g., anti-FRα antibody). In one or more embodiments, the anti-FRα antibody is linked to one or more drugs (e.g., DNA topoisomerase inhibitors) that are delivered to cells expressing FRα, particularly cancer cells expressing FRα. In one or more embodiments, the anti-FRα antibody-drug conjugate comprises an anti-FRα antibody conjugated to a drug (e.g., exatecan) via a linker. The anti-FRα antibody described herein provides the ADC with the ability to bind to FRα, such that the drug attached to the antibody can be delivered to cells expressing FRα, particularly cancer cells expressing FRα.

In one or more embodiments, the ADC has a structure shown as Formula I or a stereoisomer thereof, or a

pharmaceutically acceptable salt or solvate thereof.

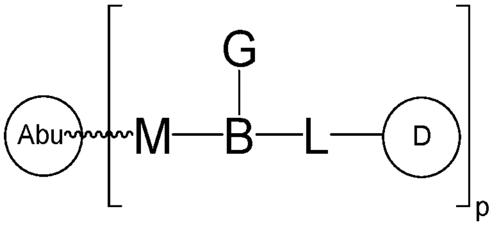

Formula I

wherein Abu, M, B, G, L, D and p are as defined herein.

In one or more embodiments, Abu is Antibody 1.

In one or more embodiments, the ADC is ADC1 or ADC2, or a pharmaceutically acceptable salt or solvate thereof.

In one or more embodiments, the antibody-drug conjugate of the present invention has a significant bystander effect.

In one or more embodiments, the ADC of the present invention binds to FRα with an equilibrium dissociation constant (KD) of about 1 μM or less. In one or more embodiments, the ADC of the present invention binds to FRα with a KD of between about 100 nM and about 1 pM or less. In one or more embodiments, the ADC of the present invention binds to FRα with a KD of between about 10 nM and about 1 pM or less. In one or more embodiments, the ADC of the present invention binds to FRα with a KD of between about 1 nM and about 1 pM or less.

In one or more embodiments, the ADC of the present invention binds to human FRα (e.g., the antigen FRα-His set forth in SEQ ID NO: 28) with a KD of about 1 μM or less. In one or more embodiments, the ADC of the present invention binds to human FRα with a KD of between about 100 nM and about 1 pM or less, or between about 10 nM and about 1 pM or less, or between about 1 nM and about 1 pM or less, or between about 1 nM and about 0.1 nM or less, or between about 0.5 nM and about 0.1 nM or less.

***Synthesis method***

The present invention further provides preparation method for intermediates and antibody-drug conjugates. The intermediates and the antibody-drug conjugates of the present invention can be prepared by using known formulations and methods. In one or more embodiments, the methods for preparing intermediate Compound 1-7 and Compound 7 are described below.

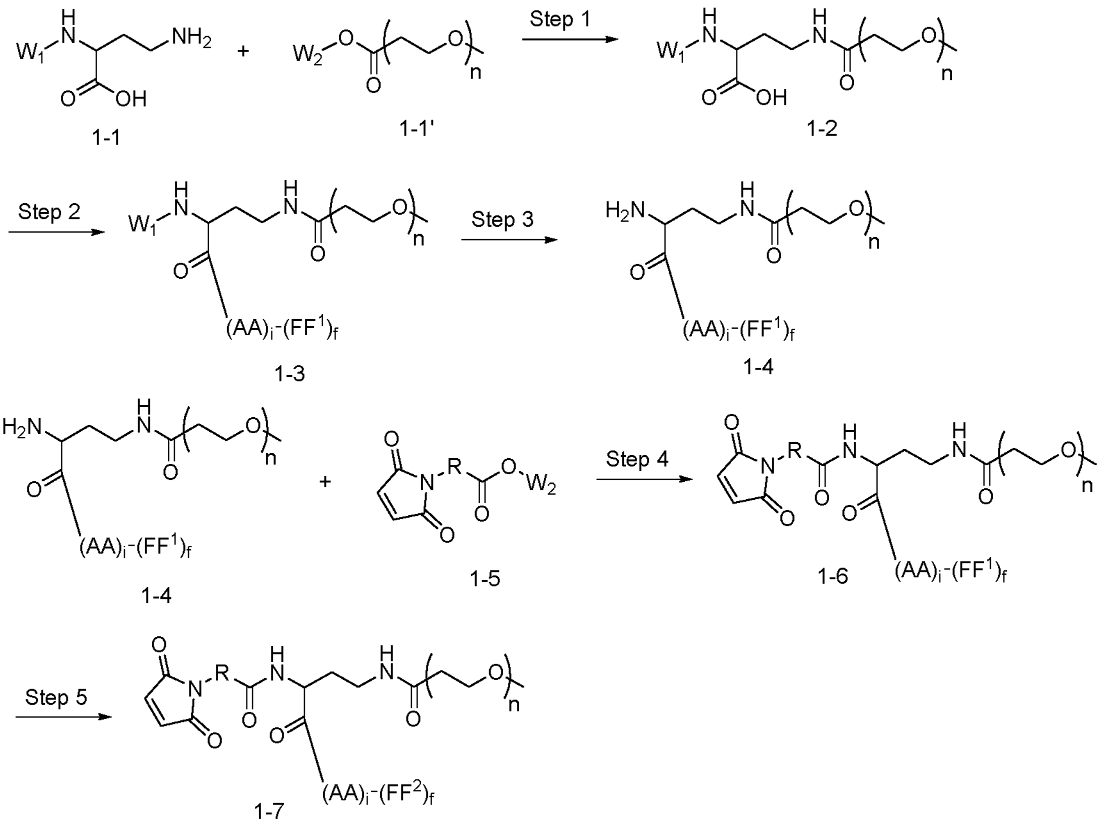


1-1 1-1' 1-2

1-3 1-4

1-4 1-5 1-6

1-7

Step 1: reacting a compound of general Formula 1-1 with a compound of general Formula 1-1' under a basic condition to obtain a compound of general Formula 1-2;
Step 2: reacting the compound of general Formula 1-2 with general Formula $(AA)_i$-$(FF^1)_f$ in the presence of a condensing agent under a basic condition to obtain a compound of general Formula 1-3;
Step 3: removing the amino-protecting group $W_1$ of the compound of general Formula 1-3 to obtain a compound of general Formula 1-4;
Step 4: reacting the compound of general Formula 1-4 with a compound of general Formula 1-5 under a basic condition to obtain a compound of general Formula 1-6; and
Step 5: reacting the compound of general Formula 1-6 with bis(*p*-nitrophenyl)carbonate under a basic condition to obtain a compound of general Formula 1-7.

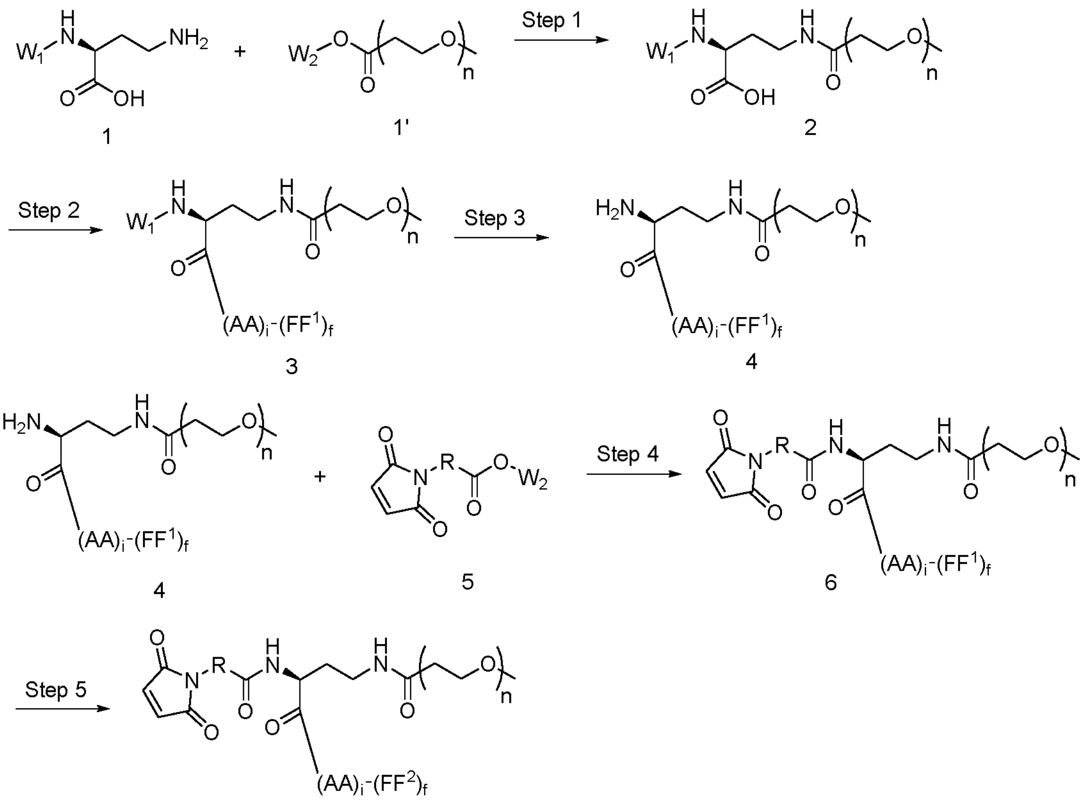


1 1' 2 3 4 4 5 6 7

Step 1: reacting a compound of general Formula 1 with a compound of general Formula 1' under a basic condition to obtain a compound of general Formula 2;
Step 2: reacting the compound of general Formula 2 with general Formula $(AA)_i$-$(FF^1)_f$ in the presence of a condensing agent under a basic condition to obtain a compound of general Formula 3;
Step 3: removing the amino-protecting group $W_1$ of the compound of general Formula 3 to obtain a compound of general Formula 4;
Step 4: reacting the compound of general Formula 4 with a compound of general Formula 5 under a basic condition to obtain a compound of general Formula 6; and Step 5: reacting the compound of general Formula 6 with bis(p-nitrophenyl)carbonate under a basic condition to obtain a compound of general Formula 7;
wherein
$W_1$ is an amino-protecting group, e.g., 9-fluorenylmethyloxycarbonyl; $W_2$ is a carboxylic acid active ester, for example, a succinimidyl ester;
wherein n, AA, R, i and f are as described herein, and each $FF^1$ is independently

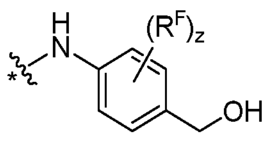 , 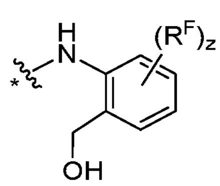 or 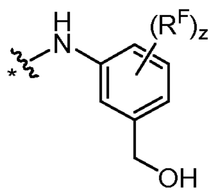 ,

wherein each $R^F$ is independently C1-C6 alkyl, C1-C6 alkoxy, $-NO_2$ or halogen; z is 0, 1, 2, 3 or 4; wherein * links to AA; each
FF' is independently

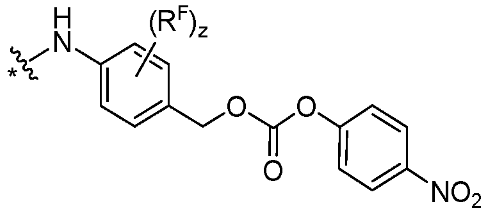, 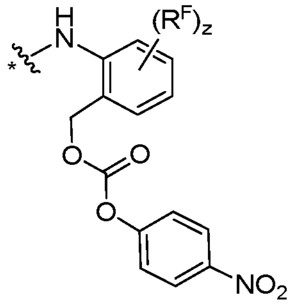

or

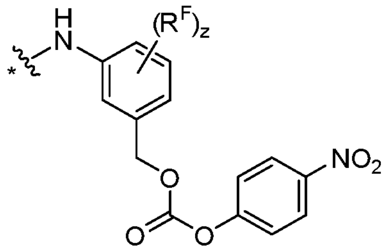,

wherein each $R^F$ is independently C1-C6 alkyl, C1-C6 alkoxy, $-NO_2$ or halogen; z is 0, 1, 2, 3 or 4; wherein * links to AA; In one or more embodiments, $R^F$ is F.

In one or more embodiments, z is 0.

In one or more embodiments, z is 1 or 2.

In one or more embodiments, the intermediate is:

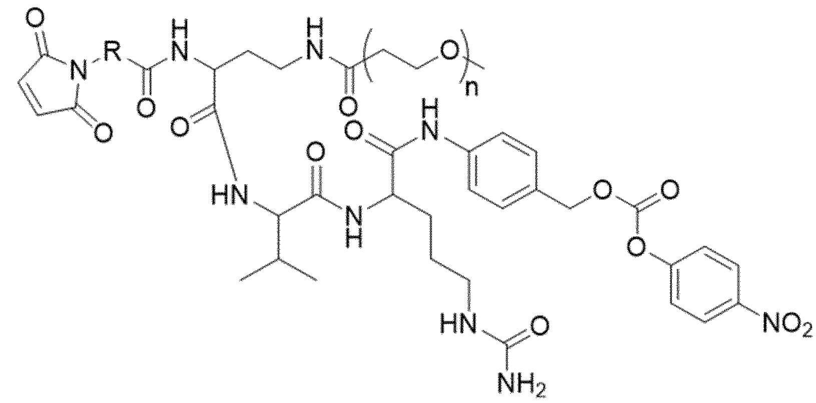

Formula II-1A

or

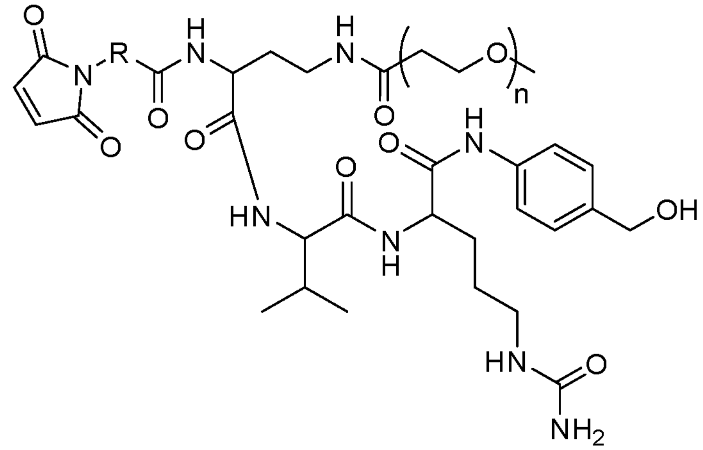

Formula II-1B

wherein Rand n are as described herein.

In one or more embodiments, the intermediate is:

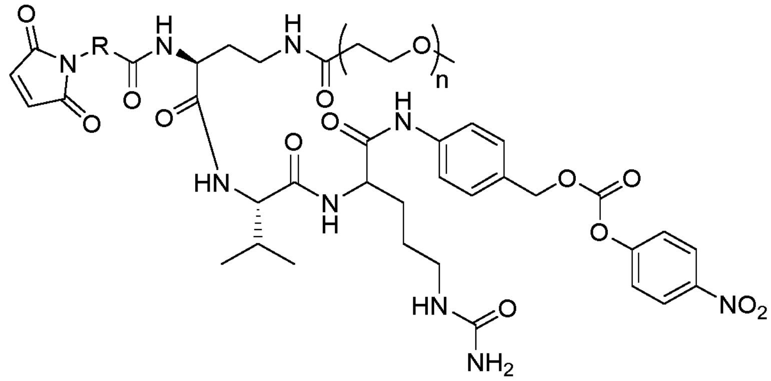

Formula II-2A

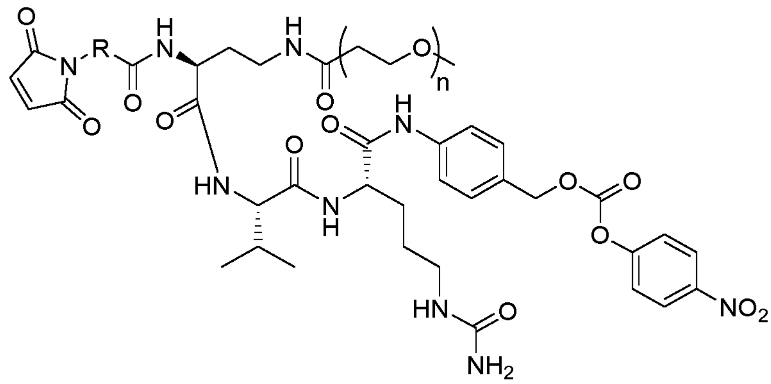

Formula II-2A-1

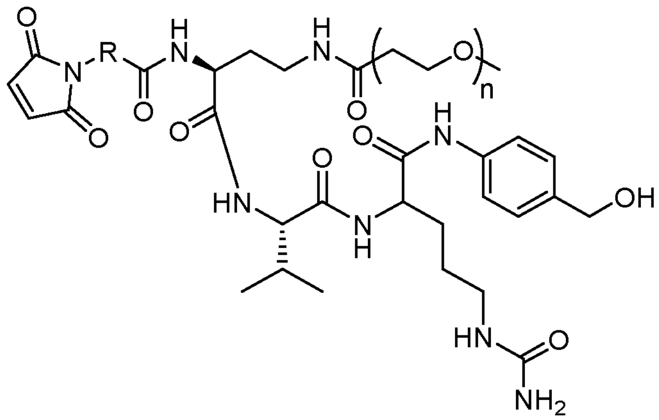

Formula II-2B

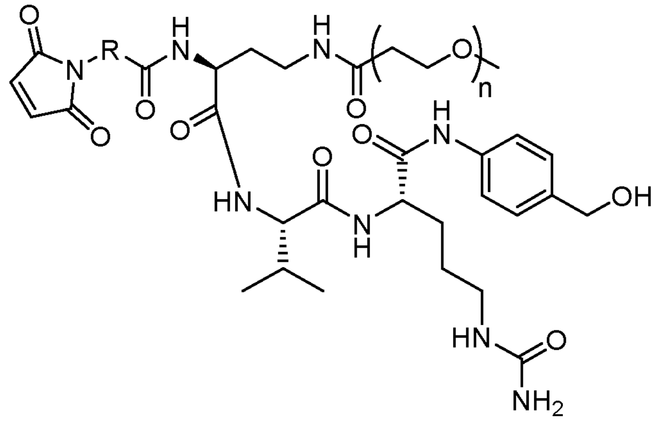

Formula II-2B-1

wherein Rand n are as described herein.

In one or more embodiments, the intermediate is:

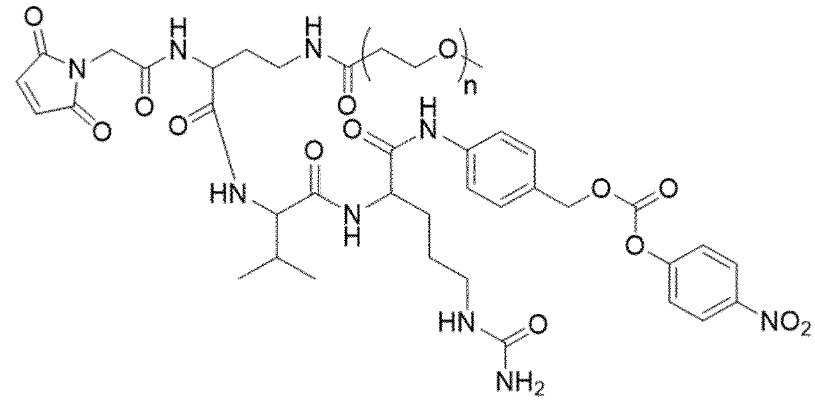

Formula II-3A

or

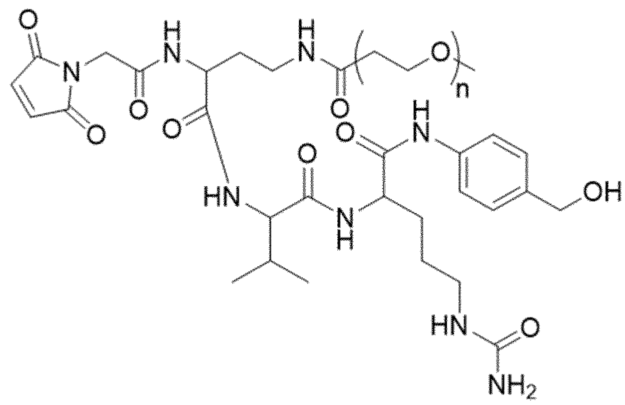

Formula II-3B

wherein n is as described herein.

In one or more embodiments, the intermediate is:

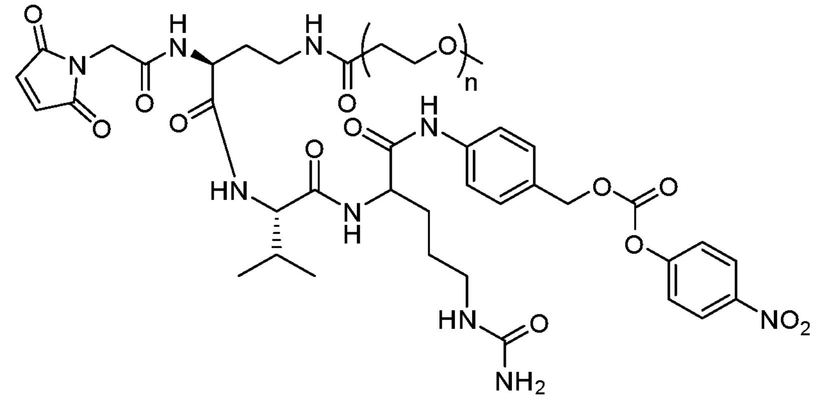

Formula II-4A

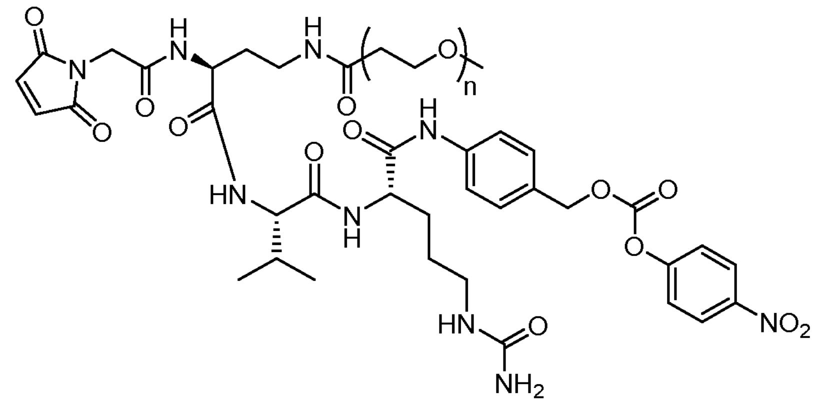

Formula II-4A-1

or

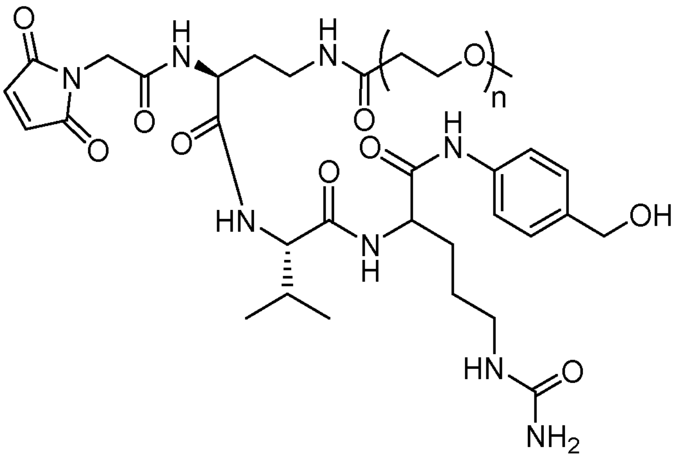

Formula II-4B

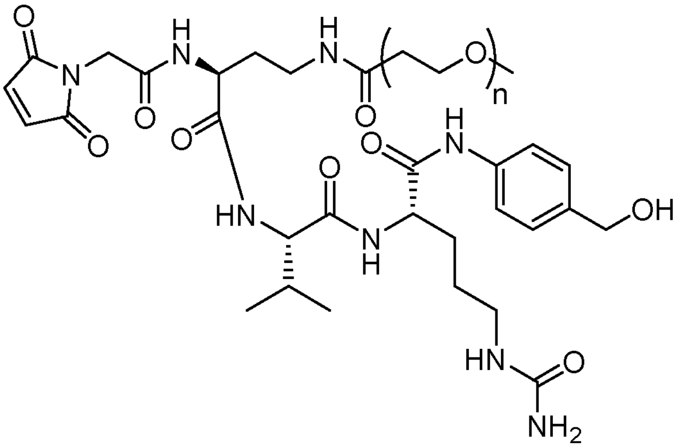

Formula II-4B-1

wherein n is as described herein.

In one or more embodiments, the intermediate is:

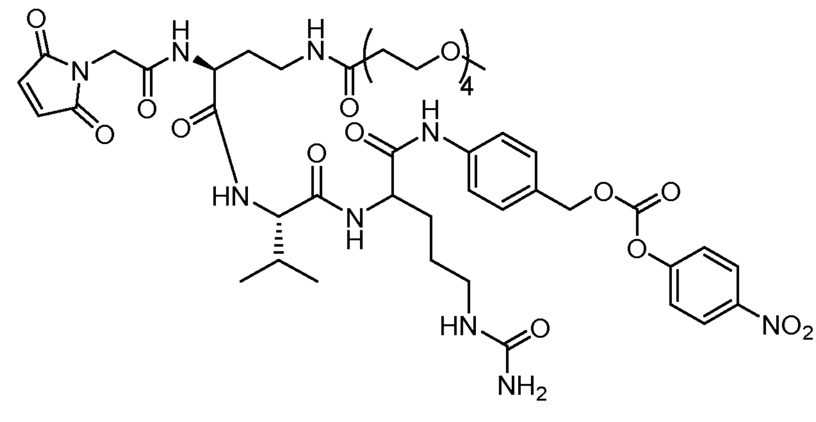

Formula II-5A

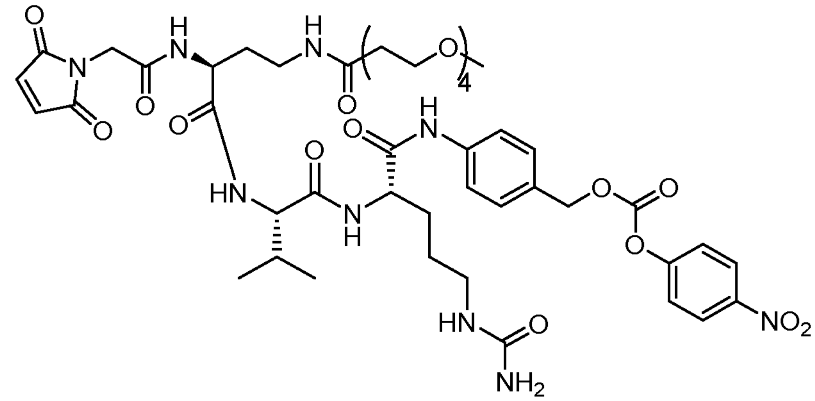

Formula II-5A-1

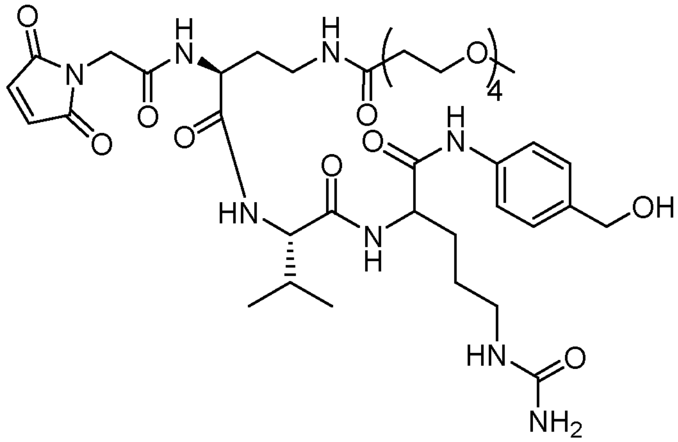

Formula II-5B

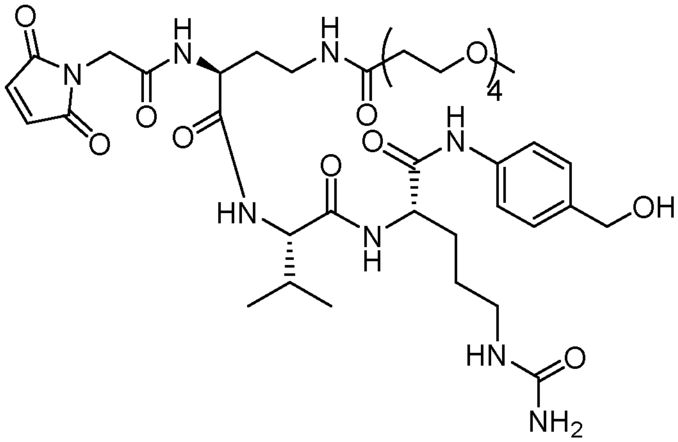

Formula II-5B-1

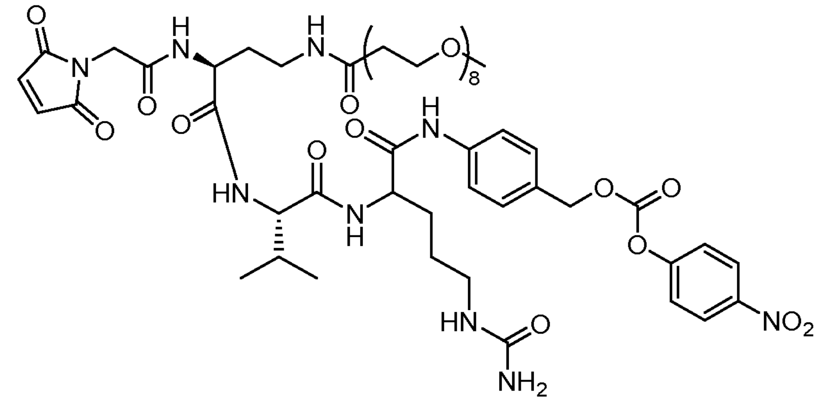

Formula II-6A

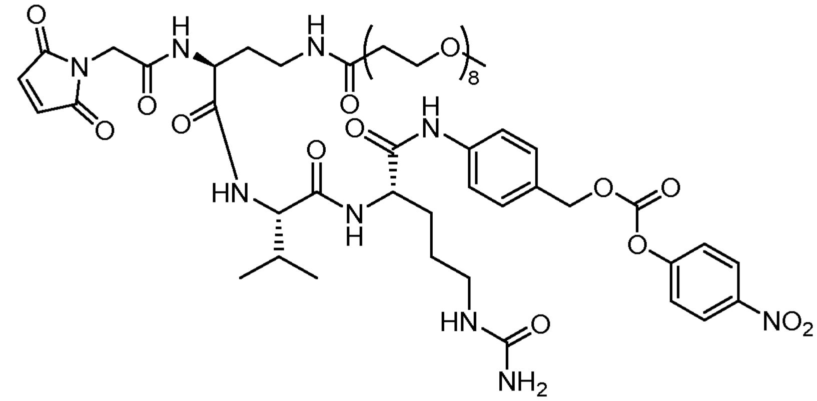

Formula II-6A-1

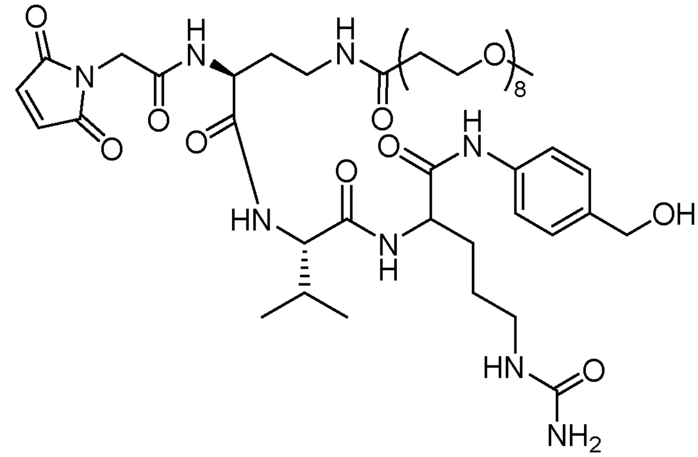

Formula II-6B

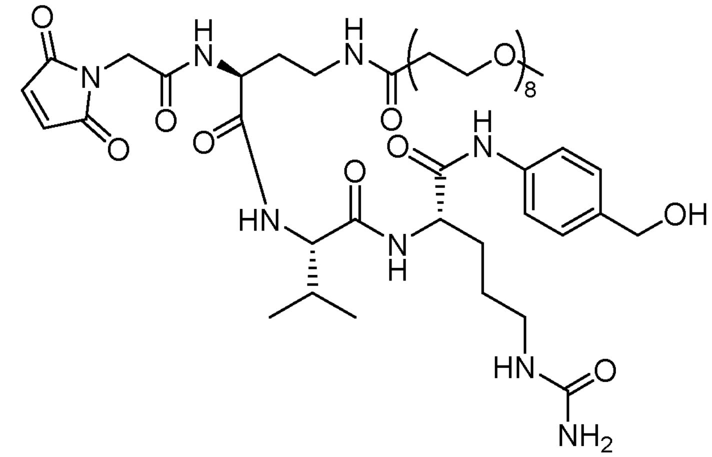

Formula II-6B-1

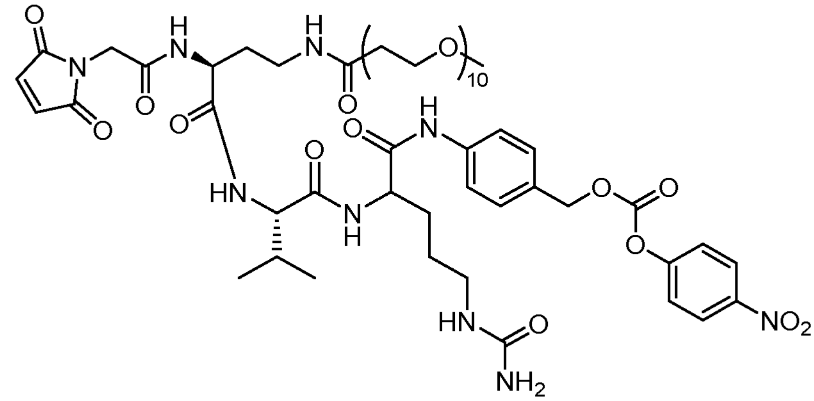

Formula II-7A

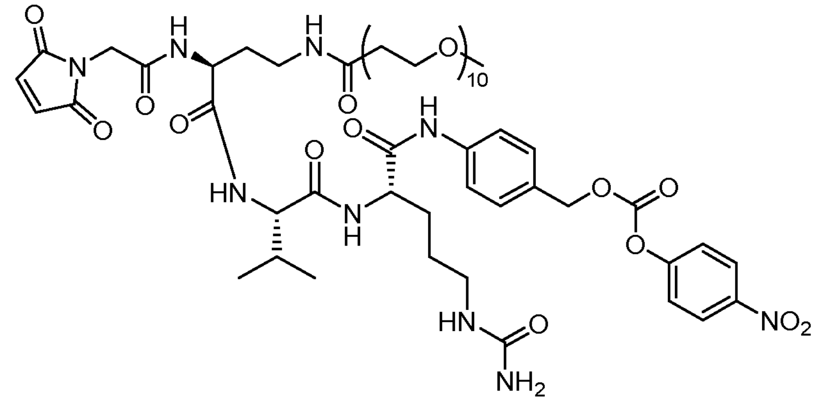

Formula II-7A-1

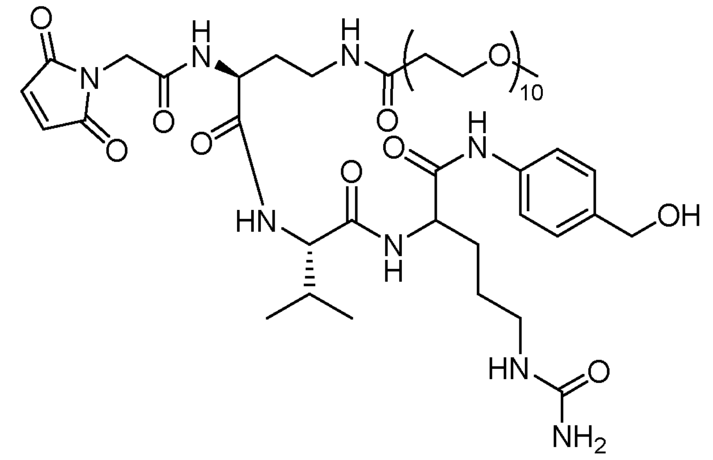

Formula II-7B

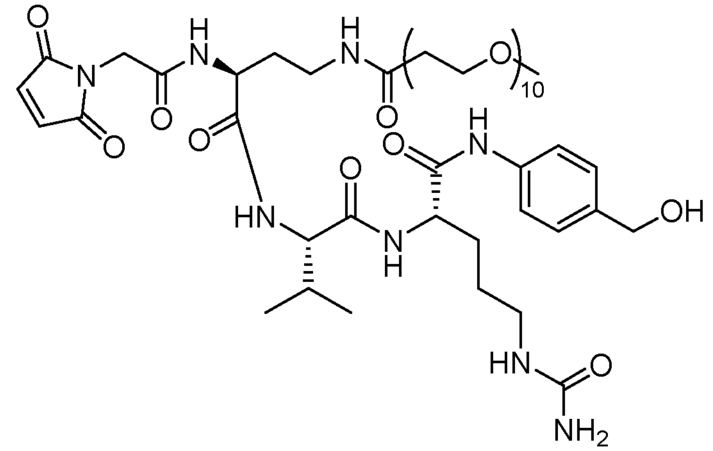

Formula II-7B-1

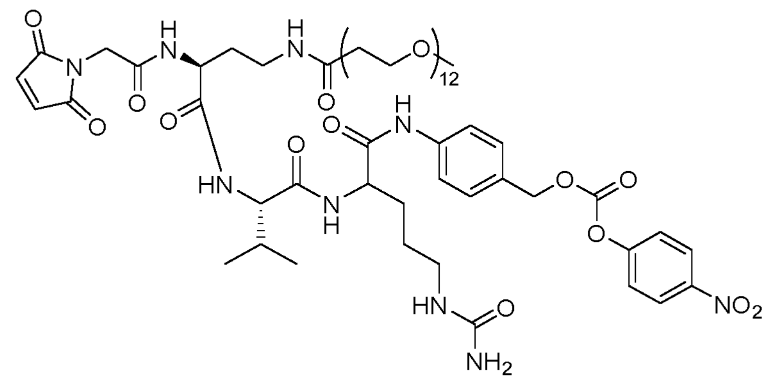

Formula II-8A

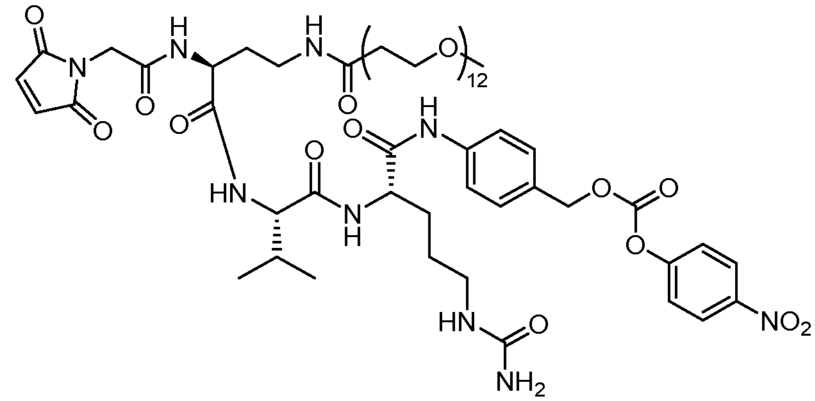

Formula II-8A-1

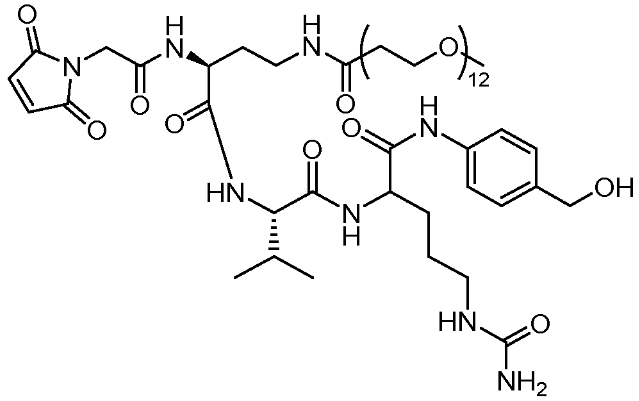

Formula II-8B

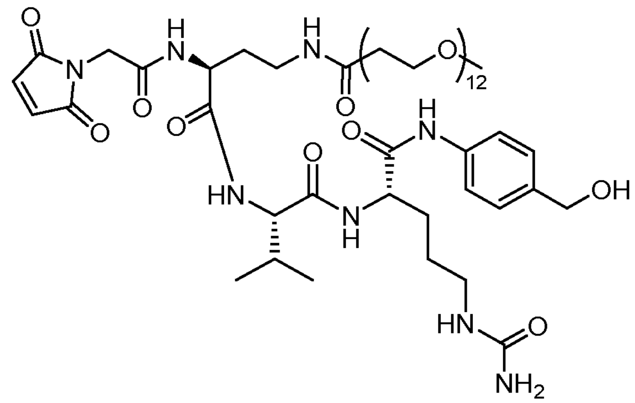

Formula II-8B-1

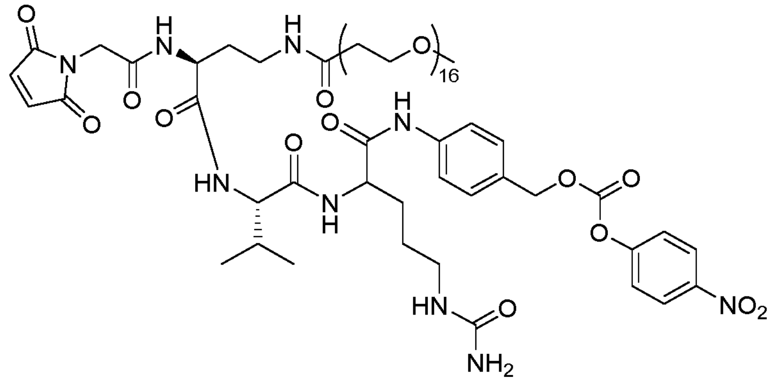

Formula II-9A

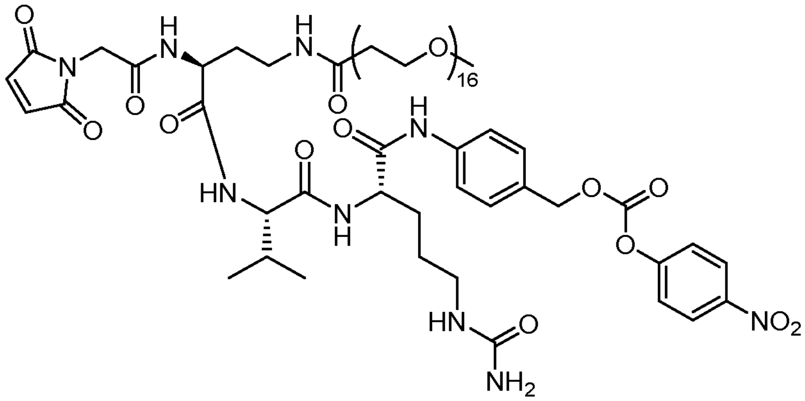

Formula II-9A-1

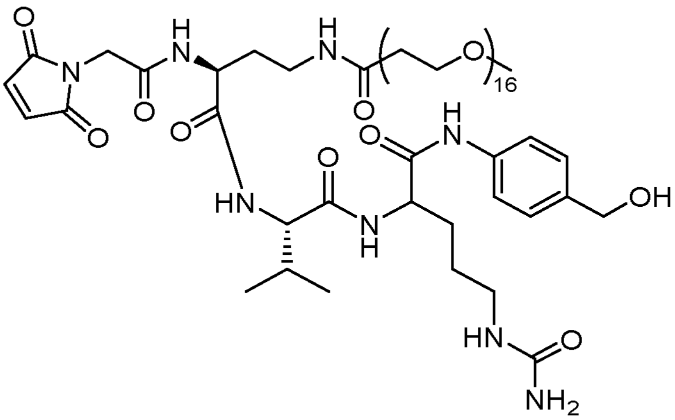

Formula II-9B

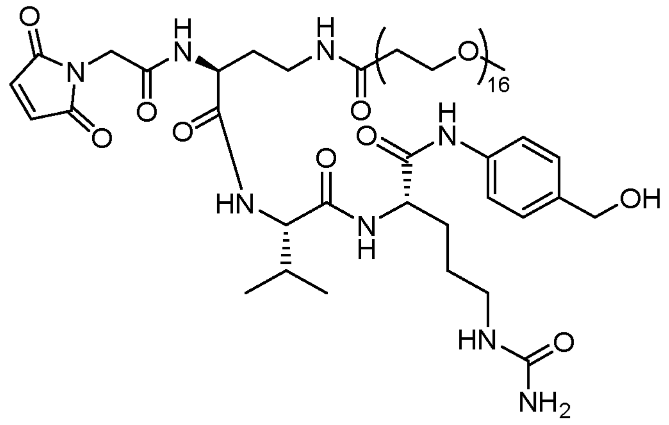

Formula II-9B-1

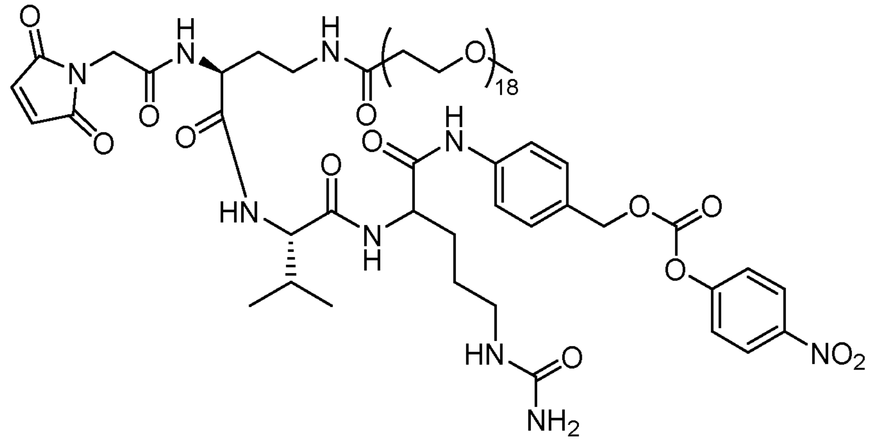

Formula II-10A

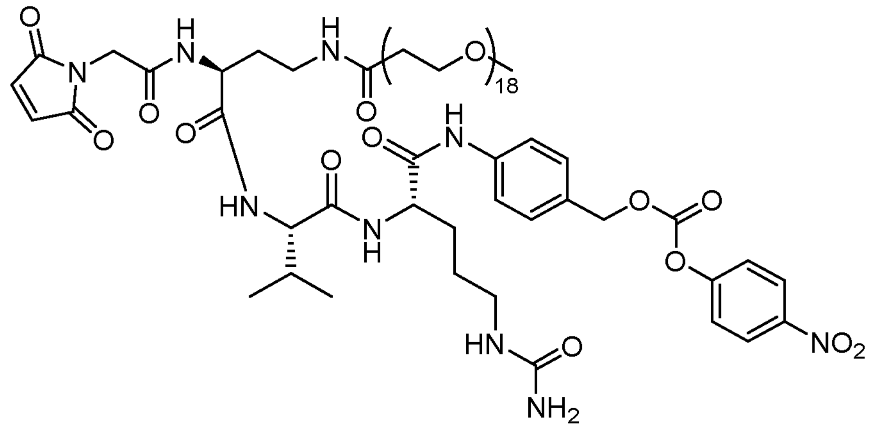

Formula II-10A-1

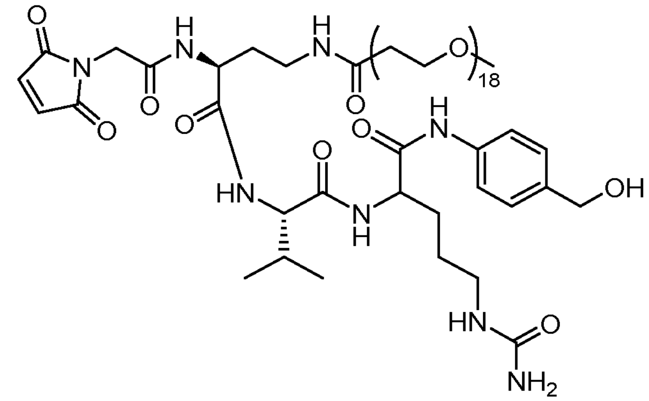

Formula II-10B

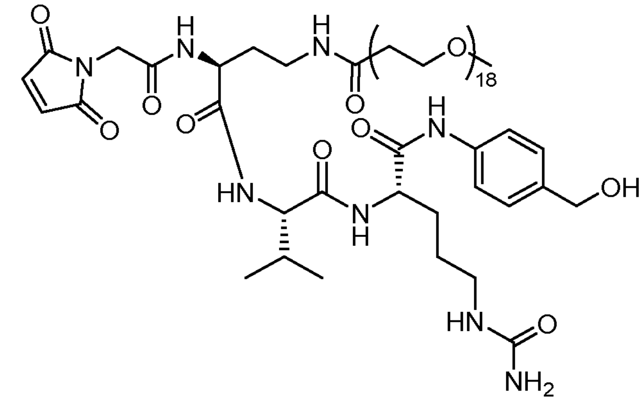

Formula II-10B-1

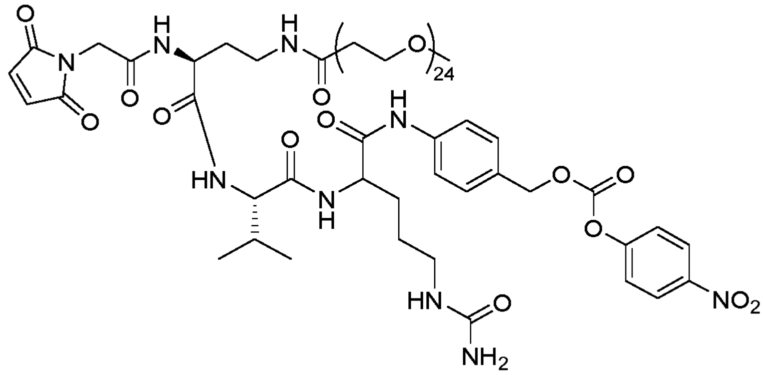

Formula II-11A

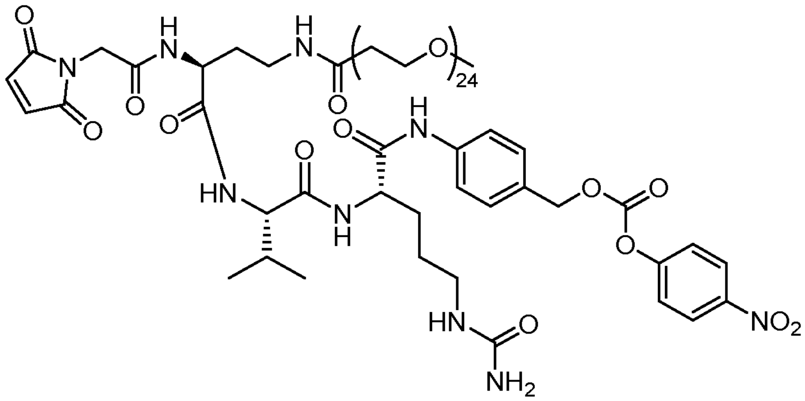

Formula II-11A-1

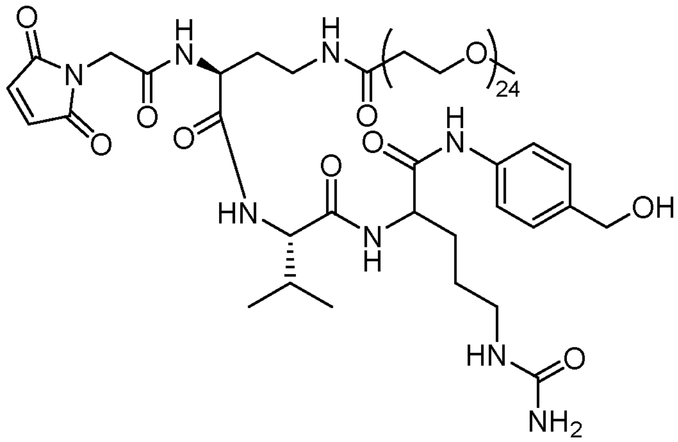

Formula II-11B

or

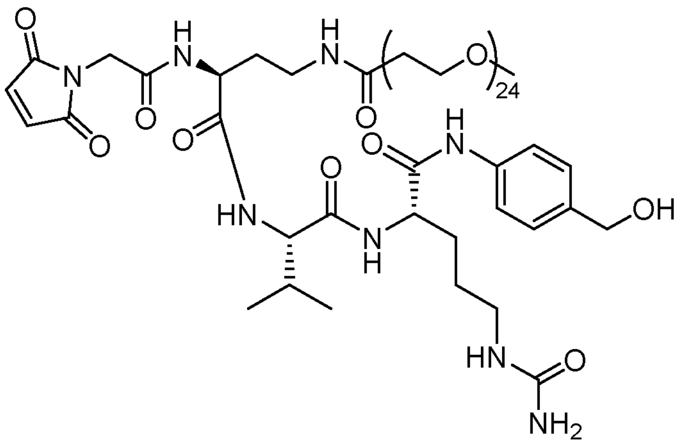

Formula II-11B-1.

The basic conditions described above can be provided using reagents including organic bases and inorganic bases, wherein the organic bases include, but are not limited to, triethylamine, diethylamine, N-methylmorpholine, pyridine, piperidine, *N*,*N*-diisopropylethylamine, *n*-butyllithium, lithium diisopropylamide, potassium acetate, sodium tert-

butoxide, or potassium tert-butoxide, and the inorganic bases include, but are not limited to, sodium hydride, potassium phosphate, sodium carbonate, potassium carbonate, cesium carbonate, sodium hydroxide, and lithium hydroxide.

The condensing agent described above may be selected from N,N,N,N-tetramethyl-O-(7-azabenzotriazol-1-yl)uronium hexafluorophosphate, 4-(4,6-dimethoxy-1,3,5-triazin-2-yl)-4-methylmorpholinium chloride, 1-hydroxybenzotriazole, 1-(3-dimethylaminopropyl)-3-ethylcarbodiimide hydrochloride, *N*,*N*-dicyclohexylcarbodiimide, *N,N*'-diisopropylcarbodiimide, *O*-benzotriazol-*N*,*N*,*N'*,*N'*-tetramethyluronium tetrafluoroborate, 1-hydroxybenzotriazole, 1-hydroxy-7-azobenzotriazol, *O*-benzotriazol-*N,N,N',N'*-tetramethyluronium hexafluorophosphate, 2-(7-azobenzotriazol)-*N*,*N*,*N'*,*N'*-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate.

In one or more embodiments, the methods for preparing intermediate Compound 1-8 and Compound 8 are described below:

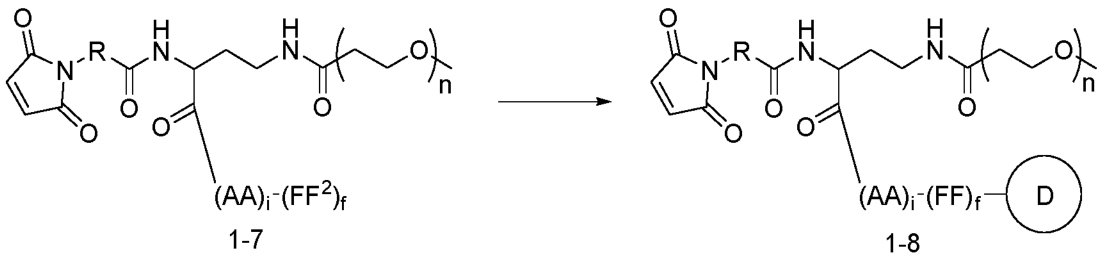

1-7 1-8

the compound of general Formula 1-7 and D are reacted in the presence of a condensing agent under a basic condition to obtain a compound of general Formula 1-8,

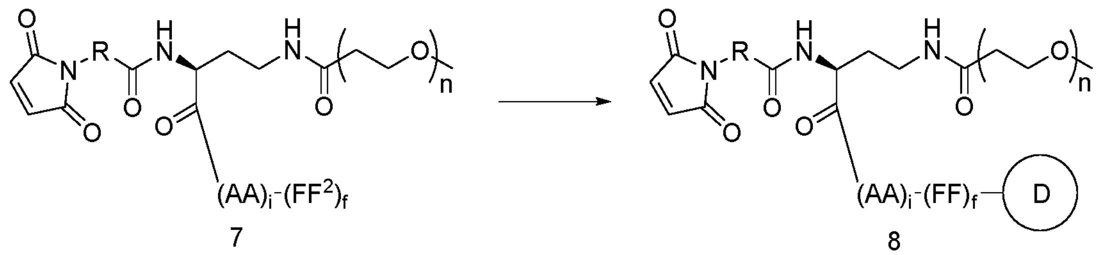

7 8

the compound of general Formula 7 and D are reacted in the presence of a condensing agent under a basic condition to obtain a compound of general Formula 8,

wherein n, AA, R, i, f, $FF^2$, FF and D are as described herein.

In one or more embodiments, the intermediate is:

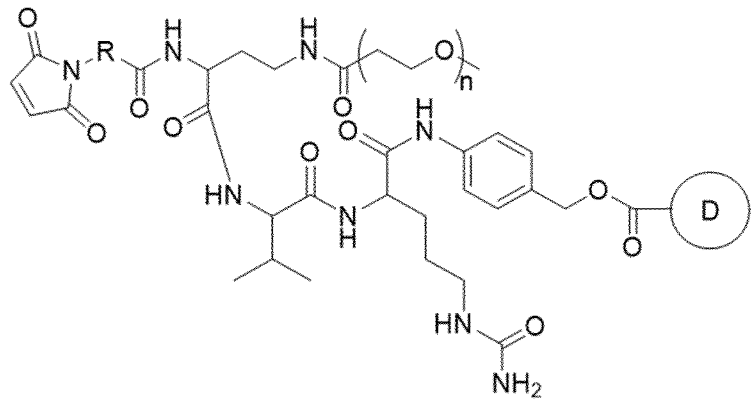

Formula III-1

wherein R, n and D are as described herein.

In one or more embodiments, the intermediate is:

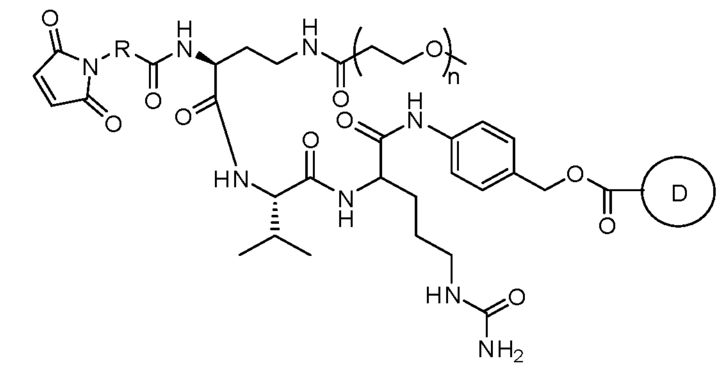

Formula III-2

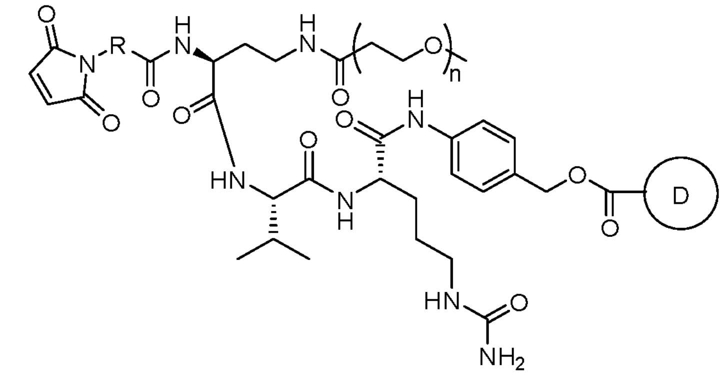

Formula III-2-1

wherein R, n and D are as described herein.

In one or more embodiments, the intermediate is:

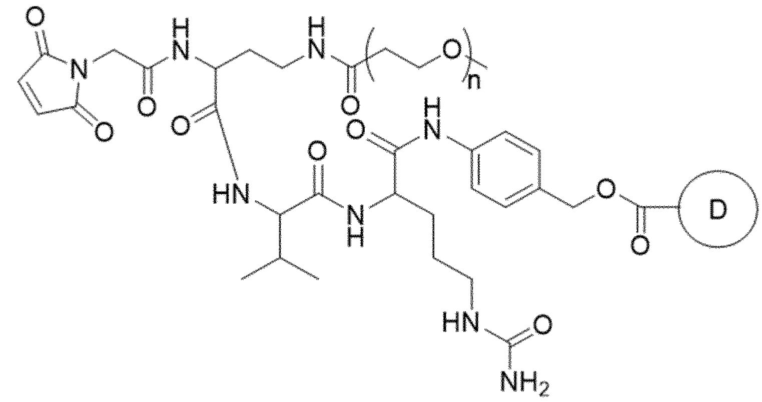

Formula III-3

wherein n and D are as described herein.

In one or more embodiments, the intermediate is:

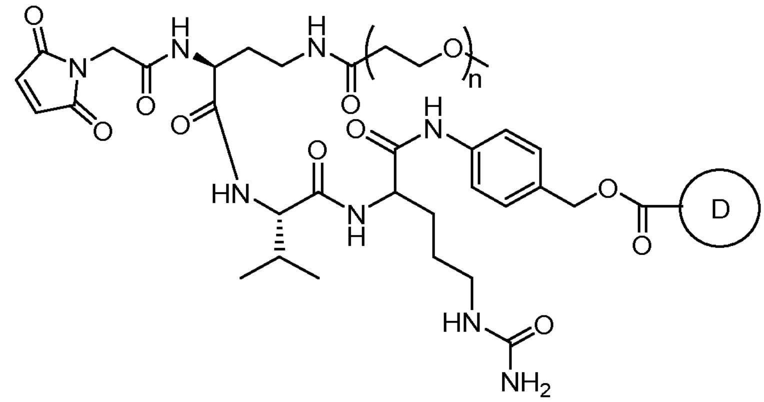

Formula III-4

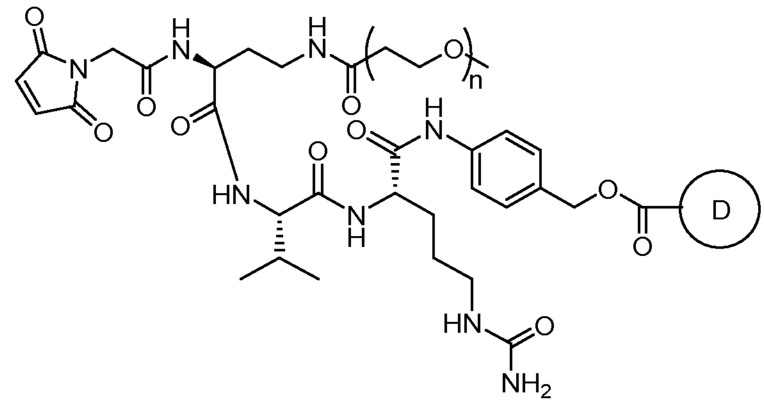

Formula III-4-1

wherein n and D are as described herein.

In one or more embodiments, the intermediate is:

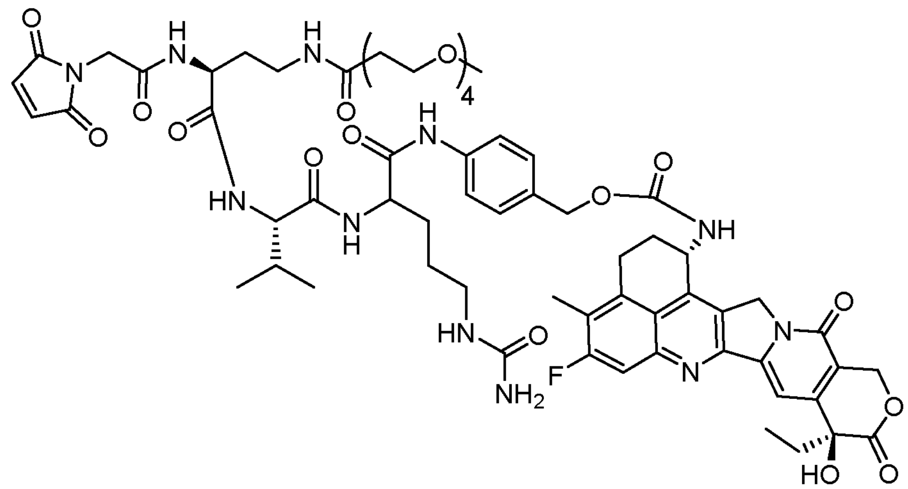

Formula III-5

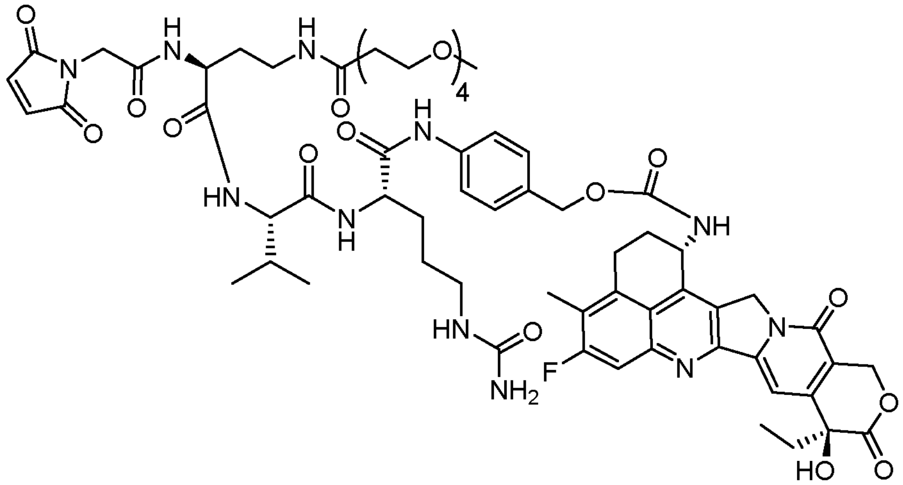

Formula III-5-1

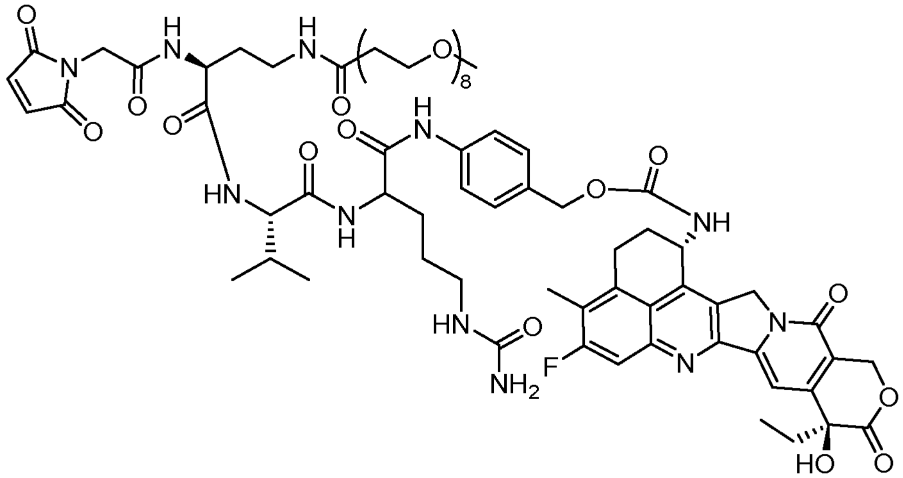

Formula III-6

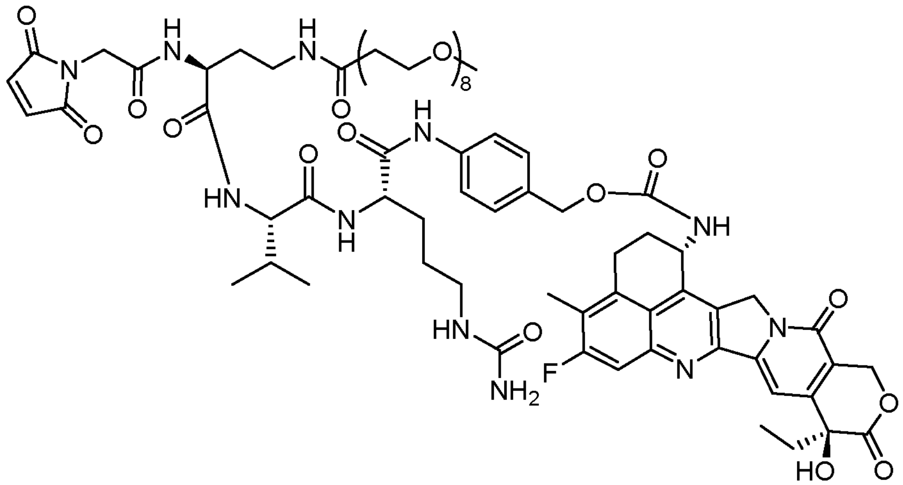

Formula III-6-1

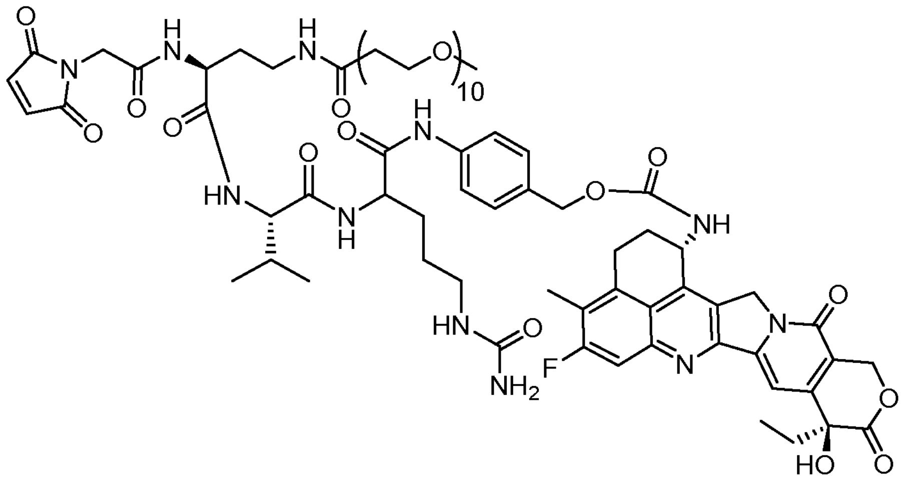

Formula III-7

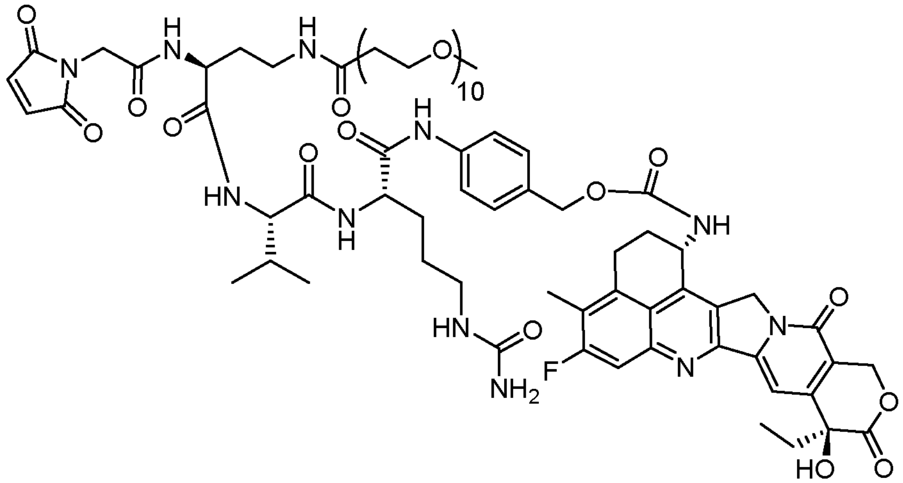

Formula III-7-1

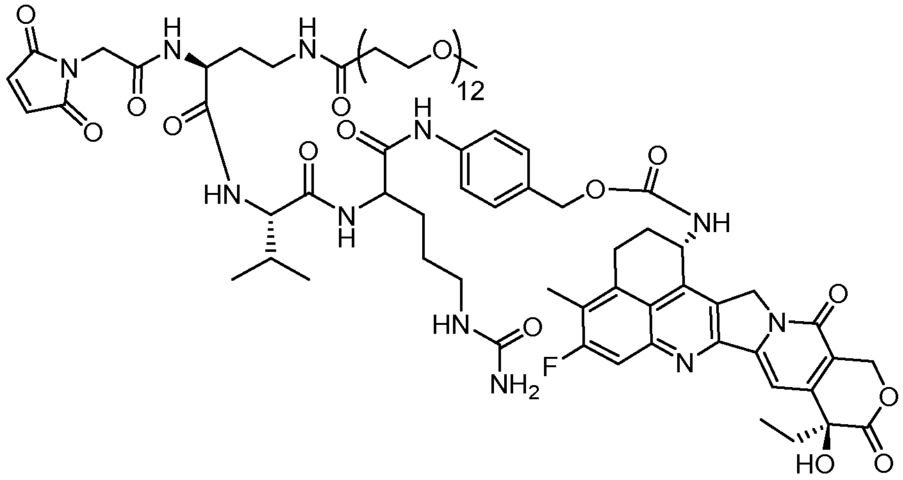

Formula III-8

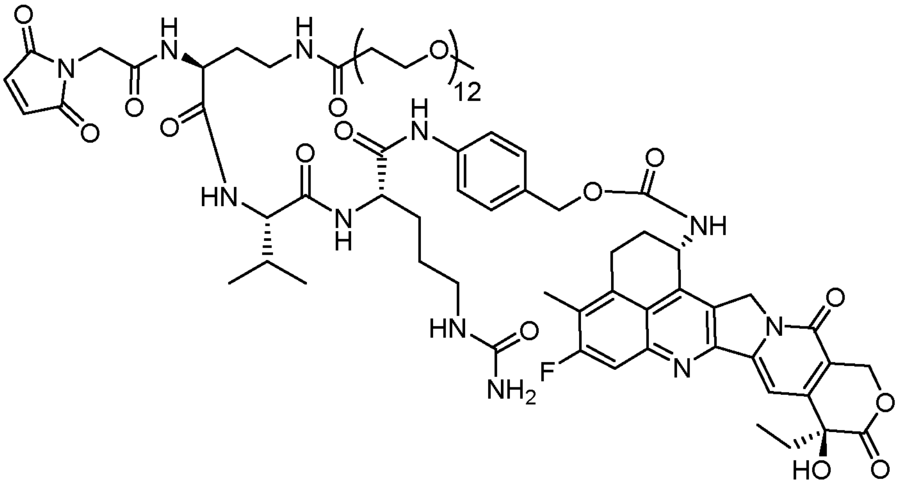

Formula III-8-1

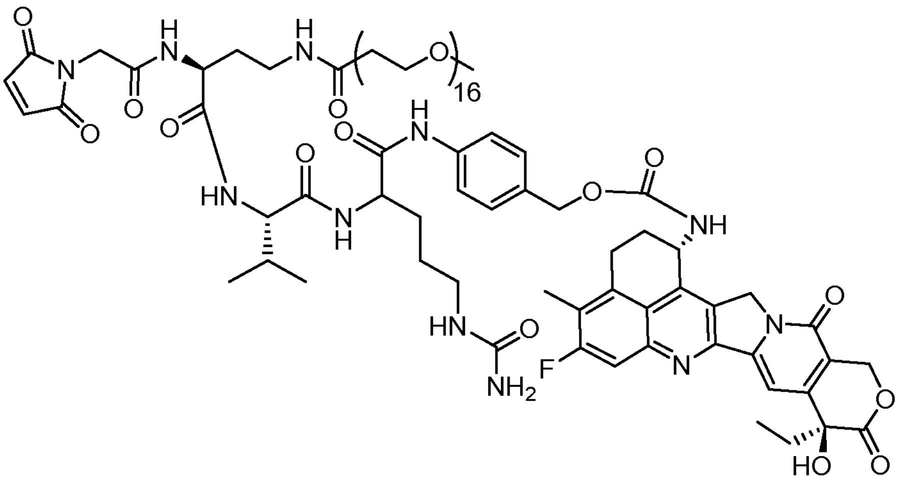

Formula III-9

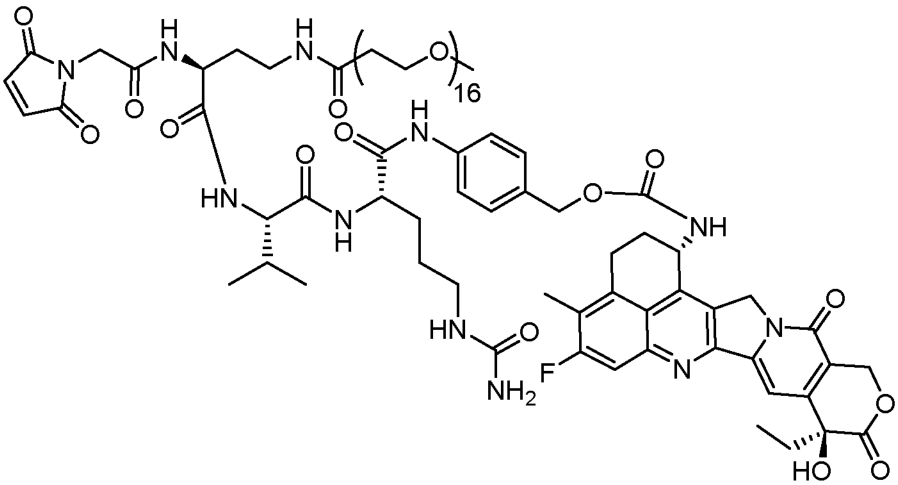

Formula III-9-1

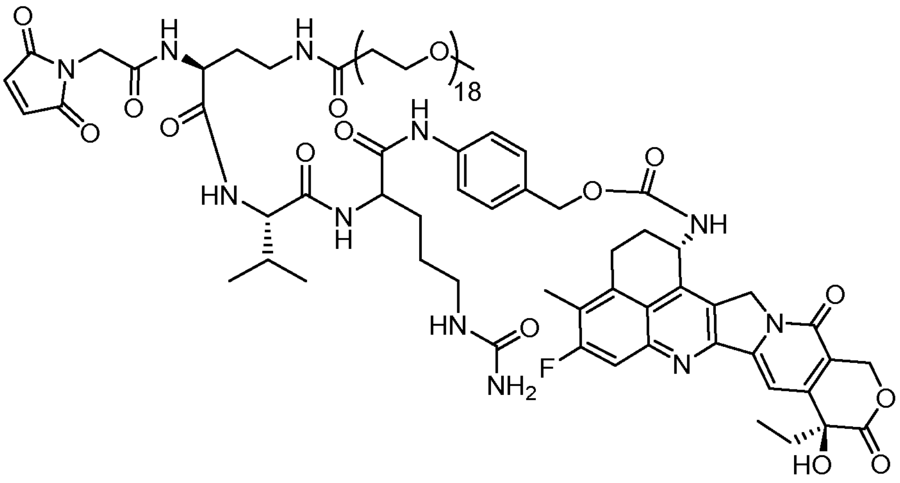

Formula III-10

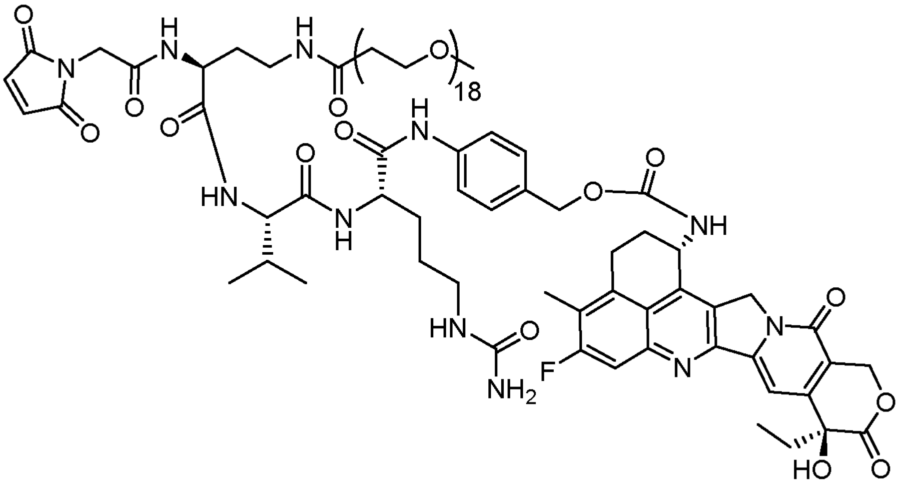

Formula III-10-1

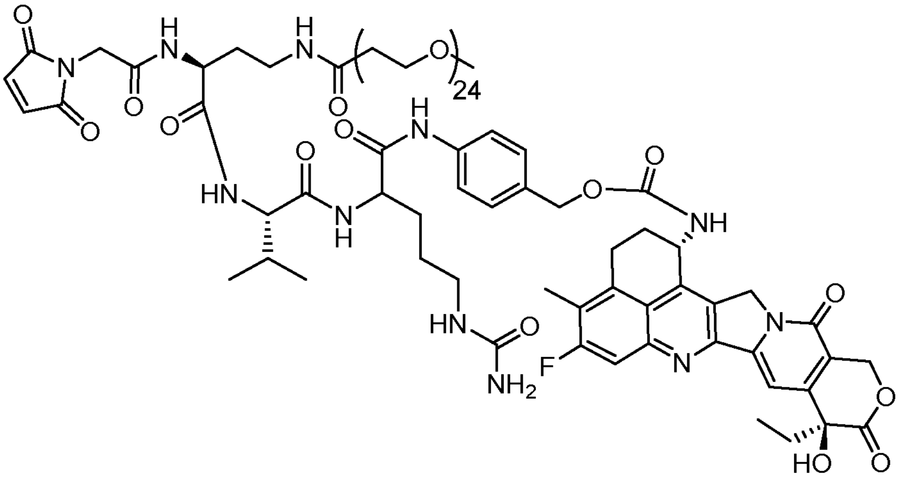

Formula III-11

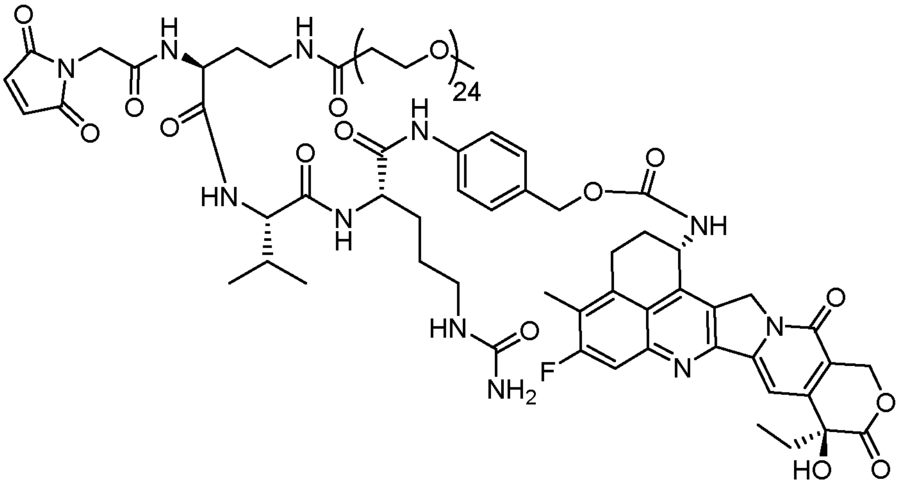

Formula III-11-1

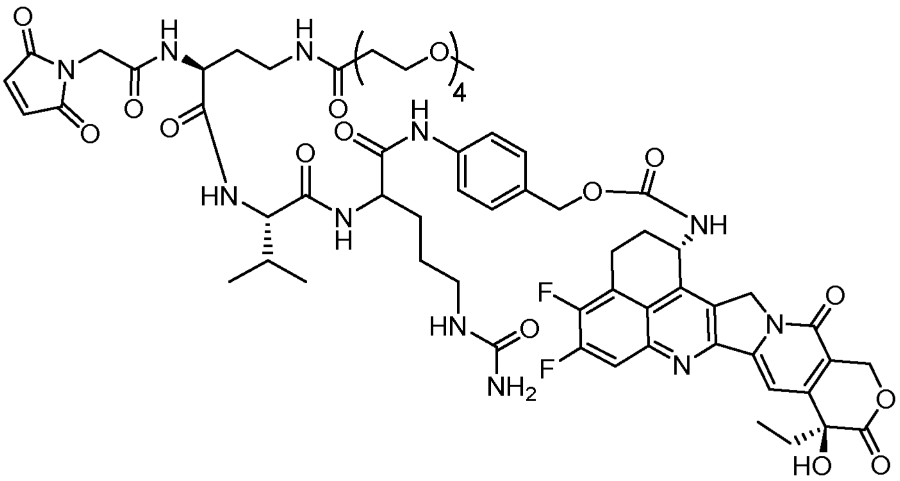

Formula III-12

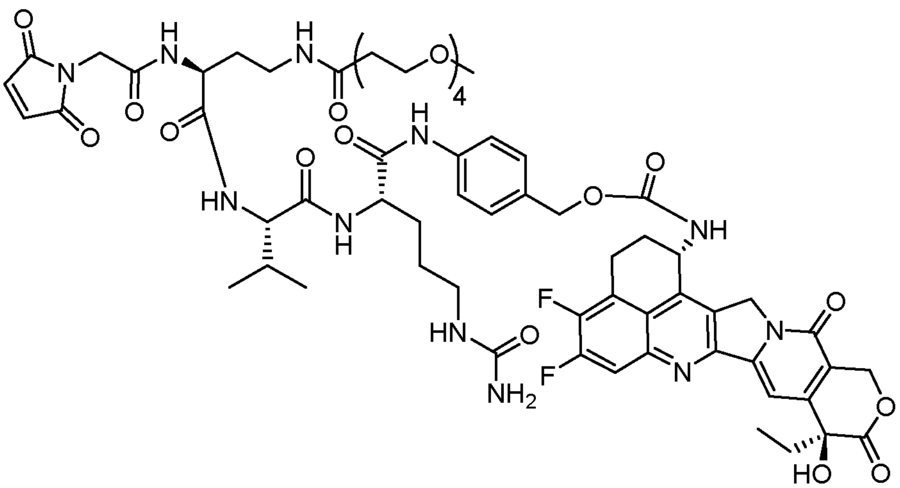

Formula III-12-1

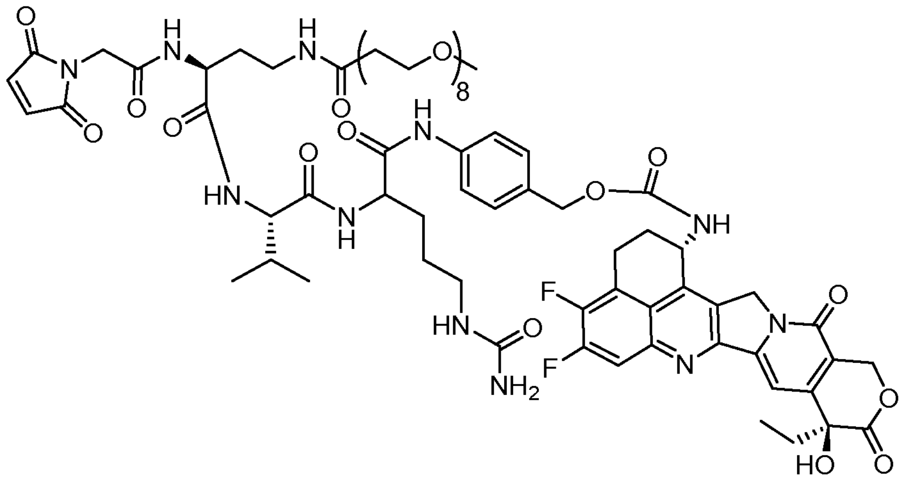

Formula III-13

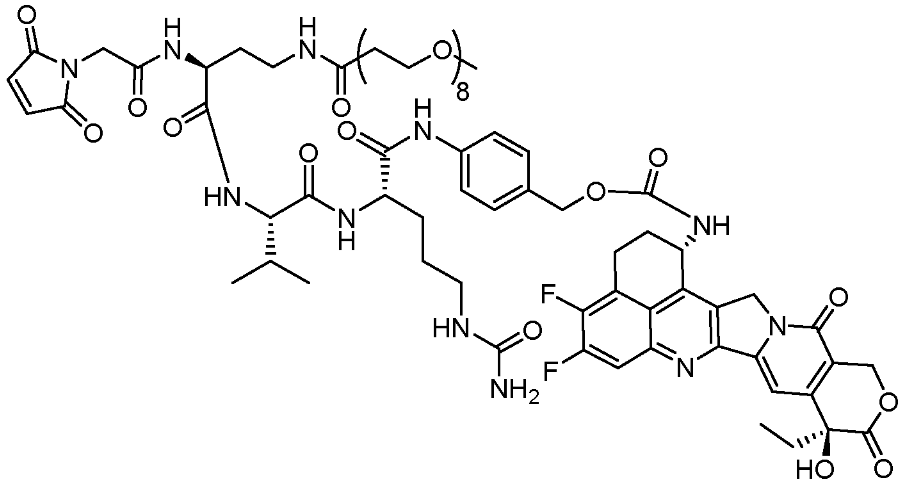

Formula III-13-1

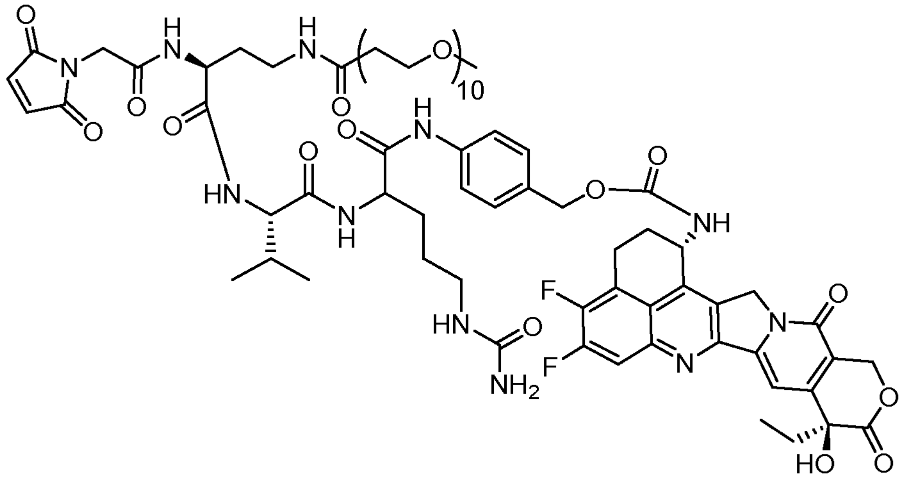

Formula III-14

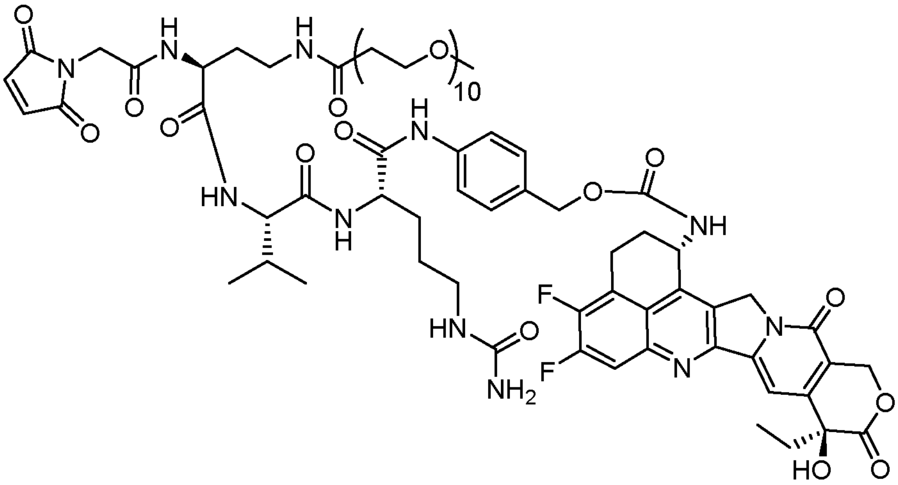

Formula III-14-1

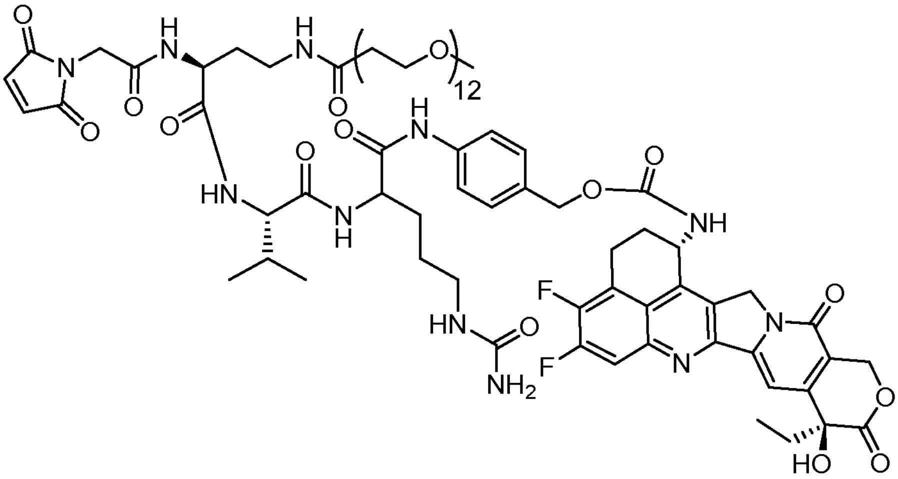

Formula III-15

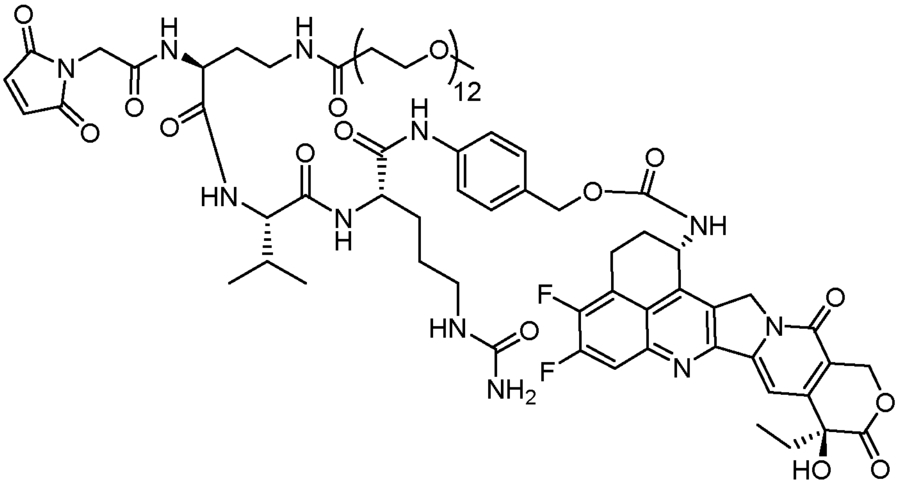

Formula III-15-1

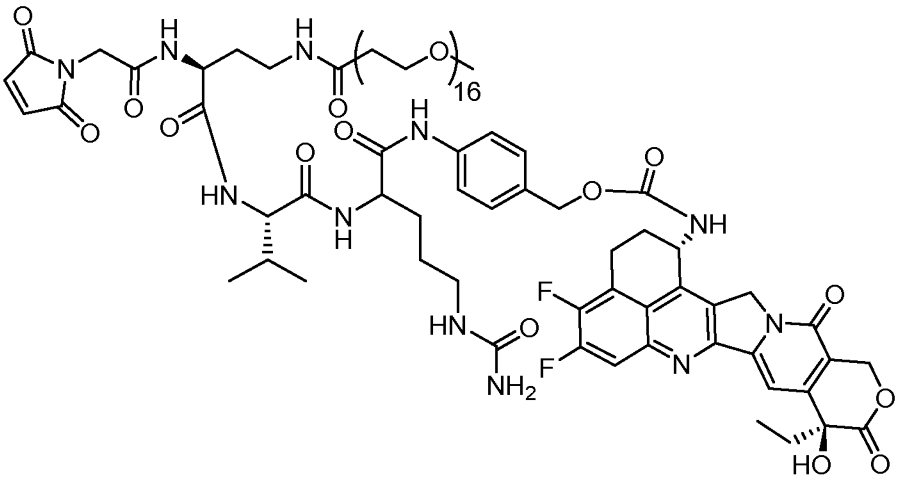

Formula III-16

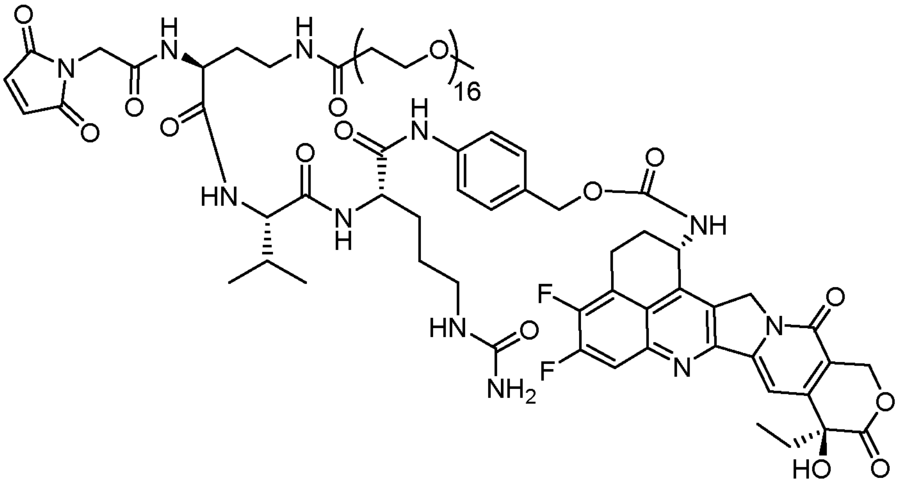

Formula III-16-1

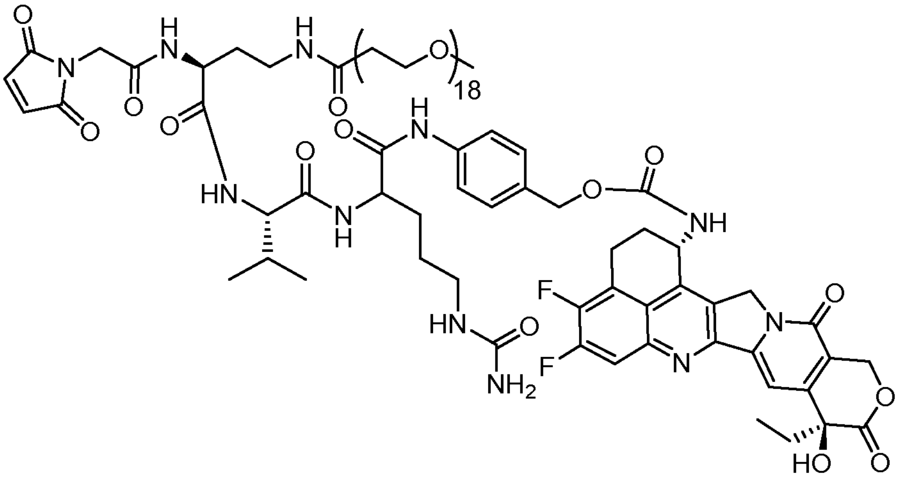

Formula III-17

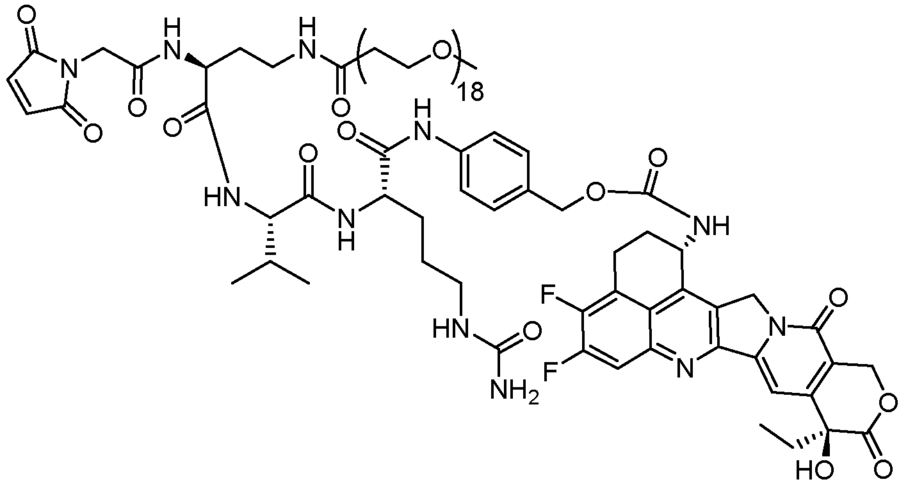

Formula III-17-1

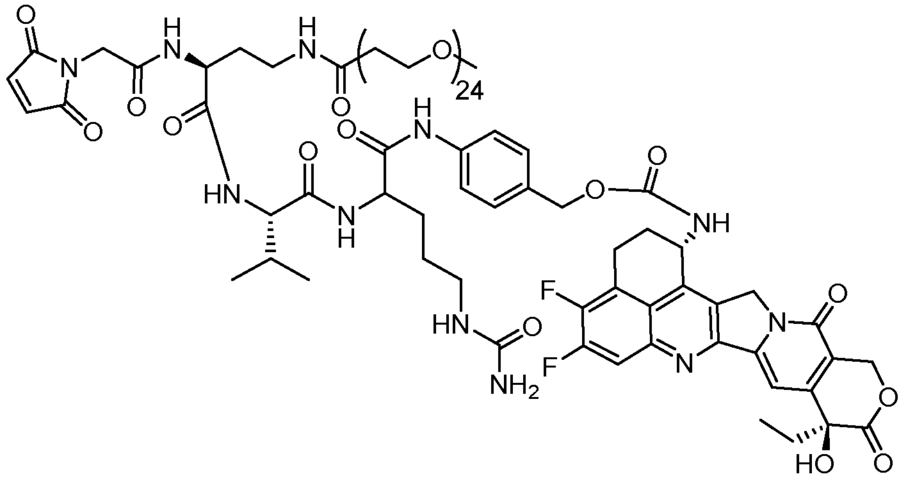

Formula III-18

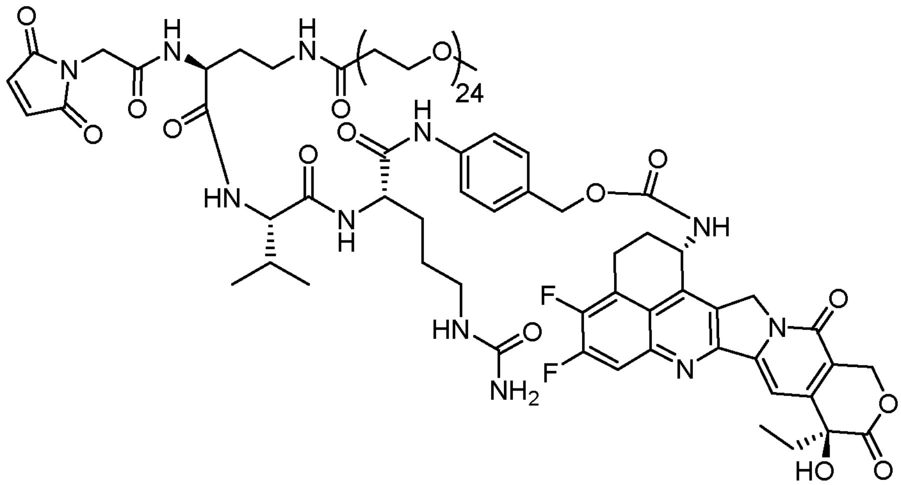

Formula III-18-1.

The basic conditions described above can be provided using reagents including organic bases and inorganic bases, wherein the organic bases include, but are not limited to, triethylamine, diethylamine, *N*-methylmorpholine, pyridine, piperidine, *N*,*N*-diisopropylethylamine, *n*-butyllithium, lithium diisopropylamide, potassium acetate, sodium *tert*-butoxide, or potassium *tert*-butoxide, and the inorganic bases include, but are not limited to, sodium hydride, potassium

phosphate, sodium carbonate, potassium carbonate, cesium carbonate, sodium hydroxide, and lithium hydroxide.

The condensing agent described above may be selected from N,N,N,N-tetramethyl-O-(7-azabenzotriazol-1-yl)uronium hexafluorophosphate, 4-(4,6-dimethoxy-1,3,5-triazin-2-yl)-4-methylmorpholinium chloride, 1-hydroxybenzotriazole, 1-(3-dimethylaminopropyl)-3-ethylcarbodiimide hydrochloride, *N,N'*-dicyclohexylcarbodiimide, *N,N'*-diisopropylcarbodiimide, O-benzotriazol-*N,N,N',N'*-tetramethyluronium tetrafluoroborate, 1-hydroxybenzotriazole, 1-hydroxy-7-azobenzotriazol, *O*-benzotriazol-*N,N,N',N'*-tetramethyluronium hexafluorophosphate, 2-(7-azobenzotriazol)-*N,N,N',N'*-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate.

In one or more embodiments, the methods for preparing intermediate Compound 1-9 and Compound 9 are described below:

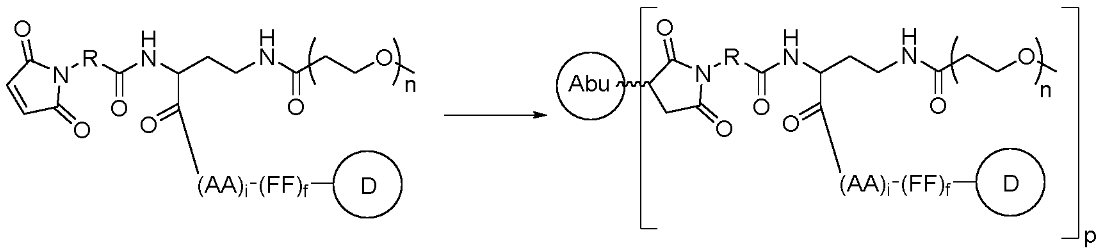

1-8 1-9

the compound of general Formula 1-8 is conjugated to Abu under a weakly acidic condition to obtain a compound of general Formula 1-9,

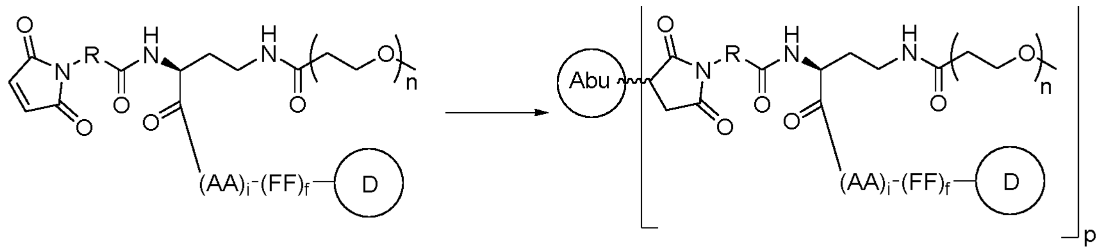

8 9

the compound of general Formula 8 is conjugated to Abu under a weakly acidic condition to obtain a compound of general Formula 9,

wherein n, AA, R, i, f, FF, D and Abu are as described herein.

Specifically, the conjugation schematic diagram is shown in FIG. 1, and the interchain disulfide bond on the anti-FRα antibody provided by the present invention is reduced and opened by a thiol reducing agent to generate free sulfhydryl, and then the antibody undergoes a conjugation reaction with the compound of general Formula 8 to obtain an antibody-drug conjugate of general Formula 9.

The weakly acidic condition described above may be provided using reagents including organic acids and inorganic acids, wherein the organic acids include, but are not limited to, acetic acid, benzoic acid, tartaric acid, oxalic acid, malic acid, citric acid, ascorbic acid, citric acid, salicylic acid, caffeic acid, sorbic acid, quinic acid, oleanolic acid, succinic acid, chlorogenic acid, formic acid, and propionic acid, and the inorganic acids include, but are not limited to, carbonic acid, nitrous acid, hypochlorous acid, hydrofluoric acid, sulfurous acid, hydrosulfuric acid, silicic acid, metasilicic acid, phosphoric acid, metaphosphoric acid, sodium bicarbonate, and sodium bisulfite.

The drug conjugates can be purified by conventional methods, e.g., preparative high performance liquid chromatography (prep-HPLC) or the like.

***Pharmaceutical composition***

The anti-FRα antibody or the antigen-binding unit thereof or the antibody-drug conjugate conjugated to the anti-FRα antibody of the present invention may be incorporated into a pharmaceutical composition suitable for administration. The principles and considerations involved in preparing such compositions and guidelines in the choice of components are well known in the art, see, e.g., Remington's Pharmaceutical Sciences: The Science And Practice Of Pharmacy, the 19th edition, Mack Pub. Co., Easton, Pa.:1995; Drug Absorption Enhancement: Concepts, Possibilities,

Limitations, And Trends, Harwood Academic Publishers, Langhorne, Pa., 1994; and Peptide And Protein Drug Delivery, Advances In Parenteral Sciences, Vol. 4, 1991, M. Dekker, New York.

Such compositions typically comprise the antibody or the antigen-binding unit thereof or the antibody-drug conjugate and a pharmaceutically acceptable carrier. In one or more embodiments, the antigen-binding fragment or antigen-binding unit is used as the smallest inhibitory fragment that specifically binds to the binding domain of the target protein. For example, a peptide that is based on the variable region sequence of an antibody and retains the ability to bind to a target protein sequence is used. In one or more embodiments, the pharmaceutical composition further comprises an anti-cancer agent (e.g., an immune checkpoint inhibitor).

As used herein, the term "pharmaceutically acceptable carrier" is intended to include any and all solvents, stabilizers, buffers, dispersion media, coatings, antibacterial agents, isotonic and absorption delaying agents, and the like that are compatible with pharmaceutical administration. Suitable carriers are described in Remington's Pharmaceutical Sciences. Such carriers or diluents can selectively include, but are not limited to, water, normal saline, Ringer's solution, glucose solution and 5% human serum albumin.

In one or more embodiments, the formulation to be used for *in vivo* administration must be sterile, which can be readily accomplished by filtration through sterile filter membranes.

The pharmaceutical compositions can be formulated in dosage unit form for ease of administration and uniformity of dosage. As used herein, dosage unit form refers to physically separable units that are suitable as unitary dosages for a patient to be treated; each unit contains a predetermined amount of one or more of the antibodies calculated to produce the desired therapeutic effect in association with the required pharmaceutical carrier.

The pharmaceutical compositions can be placed in a container or dispenser and packaged together with instructions for administration.

The pharmaceutical compositions of the present invention can further comprise other active ingredients, preferably those with complementary activities that do not adversely affect each other, depending on the particular condition to be treated. In one or more embodiments, the compositions can comprise an agent that enhances their functions, such as a cytotoxic agent, a cytokine, a chemotherapeutic agent or a growth inhibitory agent. Such active ingredients are suitably present in combination in amounts that are effective for the intended purpose.

The pharmaceutical composition provided by the present invention may be administered by any convenient route, e.g., by infusion or bolus injection, by absorption through epithelial or cutaneous mucosa (e.g., oral mucosa, rectal and intestinal mucosa, etc.), and may be co-administered with other biologically active agents. Accordingly, the pharmaceutical composition may be administered intravenously, orally, rectally, parenterally, intracisternally, intravaginally, intraperitoneally, topically (e.g., by powder, ointment, drop or transdermal patch) or buccally, or by oral or nasal spray.

In one or more embodiments, the pharmaceutical composition is formulated into a pharmaceutical composition suitable for intravenous injection into a human body according to conventional steps. A pharmaceutical composition for intravenous administration is usually a solution in sterile isotonic aqueous buffer. The pharmaceutical composition may further comprise a solubilizer and a local anesthetic, such as lidocaine, to alleviate the pain at the injection site. In general, the active ingredients are provided in a unit dosage form individually or as a mixture. For example, the active ingredients are encapsulated in sealed containers (such as ampoule bottles or sachets) that can indicate the amount of the active agent, in the form of lyophilized powder or anhydrous concentrate. Where the pharmaceutical composition is administered by infusion, the composition can be dispensed in infusion bottles containing sterile, pharmaceutical grade water or saline. Where the pharmaceutical composition is administrated by injection, an ampoule bottle containing sterile water or saline for injection can be used, so that the active ingredients can be mixed before administration.

The composition of the present invention may be formulated in a neutral or salt form.

***Treatment method and use***

The antibody or the antigen-binding unit thereof or the ADC described herein may be used in a variety of applications including, but not limited to, treatment methods, such as treatment of tumors. In one or more embodiments, the antibody or the antigen-binding unit thereof or the ADC may be used to inhibit tumor growth, reduce tumor volume and/or reduce tumorigenicity of a tumor. The method of use may be *in vitro*, *ex vivo* or *in vivo.* In one or more embodiments, the antibody or the antigen-binding unit thereof or the ADC described herein is an antagonist of FRα to which it binds.

In one or more embodiments, the method for inhibiting tumor growth comprises contacting a tumor *in vitro* with an anti-FRα antibody or an antigen-binding unit thereof or an ADC or a pharmaceutical composition comprising the same. For example, an immortalized cell line or cancer cell expressing FRα is cultured in a medium supplemented with an anti-FRα antibody or an antigen-binding unit thereof or an ADC or a pharmaceutical composition comprising the same. In one or more embodiments, tumor cells are isolated from a patient sample and added to the medium containing the anti-FRα antibody or the antigen-binding unit thereof or the ADC or the pharmaceutical composition comprising the same for culture. In one or more embodiments, the method for inhibiting tumor growth comprises contacting a tumor or a tumor cell *in vivo* with an anti-FRα antibody or an antigen-binding unit thereof or an ADC or a pharmaceutical composition

comprising the same.

The antibody or the antigen-binding unit thereof or the ADC or the pharmaceutical composition comprising the same provided by the present invention can be used for diagnosis, prognosis, monitoring, treatment, alleviation and/or prevention of diseases and disorders associated with the abnormal expression of FRα in a patient. When a disease and a disorder associated with the abnormal expression of FRα in a patient are identified by using a conventional method, the antibody or the antigen-binding unit thereof or the ADC or the pharmaceutical composition comprising the same described herein may be administered. In one or more embodiments, the extent of FRα expression is detected by immunohistochemistry (IHC), flow cytometry, nucleic acid hybridization, etc. Exemplary antibodies, detection methods and kits for detecting FRα are provided in WO 2014/036495 and WO 2015/031815, which are incorporated herein by reference in their entirety.

In one or more embodiments, the present invention relates to: a method for treating related diseases with FRα as a therapeutic target, thereby ameliorating, slowing, inhibiting, treating or preventing any disease or disorder associated with the abnormal FRα expression (e.g., FRα overexpression); a method for treating tumors (including benign tumors and cancers) in a patient, a method for alleviating symptoms of tumors (including benign tumors and cancers) in a patient, and a method for avoiding recurrence of tumors (including benign tumors and cancers) in a patient, which comprise administering to the patient an effective amount of any of the antibodies or the antigen-binding units thereof or the ADCs described herein.

In one or more embodiments, the present invention provides a method for preventing, treating or ameliorating a disease, which comprises administering to a patient in need thereof an effective amount of the anti-FRα antibody or the antigen-binding unit thereof or the ADC or the pharmaceutical composition comprising the same. In one or more embodiments, the present invention provides use of the antibody or the antigen-binding unit thereof or the ADC in the manufacture of a drug for preventing, treating or ameliorating of a disease. In one or more embodiments, the present invention provides use of the antibody or the antigen-binding unit thereof or the ADC in the prevention, treatment or amelioration of a disease. In one or more embodiments, the disease is a disease associated with the abnormal expression of FRα. In one or more embodiments, the disease is a disease associated with the overexpression of FRα. In one or more embodiments, the disease is a tumor expressing FRα. In one or more embodiments, the disease is a tumor overexpressing FRα. In one or more embodiments, the disease is cancer overexpressing FRα. In one or more embodiments, the disease is cancer overexpressing human FRα.

In one or more embodiments, the present invention provides a method for treating a tumor (including benign tumors and cancers), which comprises administering to a patient in need thereof an effective amount of the anti-FRα antibody or the antigen-binding unit thereof or the ADC or the pharmaceutical composition comprising the same. In one or more embodiments, the present invention provides use of the antibody or the antigen-binding unit thereof or the ADC in the manufacture of a drug for treating a tumor (including benign tumors and cancers). In one or more embodiments, the present invention provides use of the antibody or the antigen-binding unit thereof or the ADC in the treatment of a tumor (including benign tumors and cancers).

Examples of cancers include, but are not limited to, solid tumors, hematological cancers and metastatic lesions. Specific examples of such cancers include, but are not limited to, colorectal cancer, lung cancer, ovarian cancer, uterine cancer, endometrial cancer, peritoneal cancer, fallopian tube cancer, pancreatic cancer, head and neck squamous cell carcinoma, nasopharyngeal cancer, laryngeal cancer, lung adenocarcinoma, liver cancer, breast cancer, brain cancer, renal cancer, renal cell carcinoma, colon cancer, testicular cancer, cervical cancer, bladder cancer, retinoblastoma, glioblastoma, mesothelioma, oral epithelioid cancer, choriocarcinoma and head and neck cancer. More specific examples of such cancers include ovarian cancer, epithelial ovarian cancer, primary peritoneal cancer of the ovary or fallopian tube cancer. In one or more embodiments, the cancer is metastatic or advanced cancer. In one or more embodiments, the patient is a patient with an increased expression level of FRα. In one or more embodiments, the patient is a human. In addition, the patient may be a mammal into which FRα has been introduced (e.g., by administration of FRα or by expression of a FRα transgene). The antibody or the antigen-binding unit thereof or the ADC of the present invention may be administered to a human patient for therapeutic purposes. In addition, the antibody or the antigen-binding unit thereof or the ADC of the present invention may be administered to a non-human mammal expressing FRα (for veterinary purposes or as an animal model of a human disease). Such animal models of human disease can be used to assess the therapeutic efficacy (e.g., dose testing and time course of administration) of the antibody or the antigen-binding unit thereof or the ADC of the present invention.

The specific dose and treatment regimen for any particular patient will depend on a variety of factors including the particular antibody or derivative thereof (e.g., ADC or pharmaceutical composition) used, the age and body weight, general health condition, sex and diet of the patient, and the time of administration, frequency of metabolism, drug combination and the severity of the particular disease being treated. These factors are judged by medical caregivers included within the scope of those of ordinary skills in the art. The dose will also depend on the individual patient to be treated, the route of administration, the type of the formulation, the nature of the compound used, the severity of the disease and the efficacy desired. The dose employed can be determined by pharmacological and pharmacokinetic

principles well known in the art. The effective amount refers to the amount of an active compound or drug that results in a biological or drug response of tissues, systems, animals, individuals and humans which is being sought by researchers, vets, doctors or other clinical doctors, including the treatment of a disease. In one or more embodiments, an effective dose ranges from about 0.1 mg/kg to about 100 mg/kg, and the frequency of administration can be, for example, once a month, once every two weeks, once every three weeks, twice every three weeks, three times every four weeks, once a week, twice a week, etc. For example, the administration may be performed by intravenous infusion, intravenous bolus injection, subcutaneous injection, intramuscular injection, etc. It should be noted that dose values may vary with the type and severity of the condition to be alleviated. It is to be further understood that for any particular patient, specific dosage regimens should be adjusted over time according to the patient's need and the professional judgment of the person administering or supervising the administration of the compositions, and that dose ranges set forth herein are exemplary only and are not intended to limit the scope or practice of the claimed composition.

The modes of administration for the antibody or the antigen-binding unit thereof or the ADC include, but are not limited to, intradermal, intramuscular, intraperitoneal, intravenous, subcutaneous, nasal, epidural and oral administration. The pharmaceutical composition may be administered by any convenient route, e.g., by infusion or bolus injection, by absorption through epithelial or cutaneous mucosa (e.g., oral mucosa or rectal and intestinal mucosa), and may be co-administered with other biologically active agents. Thus, the pharmaceutical composition comprising the antibody of the present invention may be administered orally, rectally, parenterally, intracisternally, intravaginally, intraperitoneally, topically (e.g., by powder, ointment, drop or transdermal patch) or buccally, or by nasal spray.

The term "parenteral" as used herein refers to modes of administration including intravenous, intramuscular, intraperitoneal, intrasternal, subcutaneous and intraarticular injections and infusions.

The mode of administration may be systemic or local. In addition, it may be desirable to introduce the antibody or the antigen-binding unit thereof or the ADC of the present invention into the central nervous system by any suitable route, including intracerebroventricular and intrathecal injections; intracerebroventricular injection may be assisted by an intracerebroventricular catheter connected to, for example, a reservoir (which may be an Ommaya reservoir). Pulmonary administration is also possible, for example by use of an inhaler or nebulizer, and by use of nebulized formulations.

The antibody or the antigen-binding unit thereof or the ADC of the present invention may be administered locally to an area in need of treatment; this may be achieved by (but not limited to) the following: local infusion during surgery (e.g., topical application in combination with a post-operative wound dressing), by injection, by means of a catheter, by means of a suppository, or by means of an implant, the implant being of a porous, non-porous, or gelatinous material, including membranes (such as silicone rubber membranes) or fibers. Preferably, when administering the antibody or the antigen-binding unit thereof or the ADC of the present invention, care must be taken to use materials that do not absorb protein. Methods for treating diseases comprising administering the antibody or the ADC described herein are generally tested *in vitro,* followed by *in-vivo* tests for desired therapeutic or prophylactic activities in an acceptable animal model, and finally administration in human beings. Suitable animal models (including transgenic animals) are well known to those of ordinary skill in the art. For example, *in-vitro* assays for demonstrating therapeutic use of the antibody or the antigen-binding unit thereof or the ADC of the present invention include the effect of the antibody or the antigen-binding unit thereof or the ADC on a cell line or a patient tissue sample. The effect of the antibody or the antigen-binding unit thereof or the ADC on the cell line and/or the tissue sample can be detected using techniques known to those skilled in the art, such as the techniques disclosed elsewhere herein. In accordance with the teachings of the present invention, *in-vitro* assays that can be used to determine whether to administer the antibody or the antigen-binding unit thereof or the ADC include *in-vitro* cell culture experiments, wherein a patient tissue sample is cultured in a culture medium and is exposed to or otherwise administered with the antibody or the antigen-binding unit thereof or the ADC, and the effect of the antibody or the antigen-binding unit thereof or the ADC on the tissue sample is observed.

Various known delivery systems can be used to administer the antibody or the antigen-binding unit thereof, or the polynucleotide encoding the same or the ADC of the present invention, e.g., encapsulation in liposomes, microparticles, microcapsules, recombinant cells capable of expressing the compound, receptor-mediated endocytosis (see, e.g., Wu and Wu, 1987, J. Biol. Chem. 262:4429-4432), construction of nucleic acids as part of a retrovirus or other vectors, or the like.

***Combination therapy***

In one or more embodiments, the antibody or the antigen-binding unit thereof or the ADC of the present invention can be administered in combination with other therapeutic or prophylactic regimens, including administration of one or more antibodies or antigen-binding units thereof or ADCs of the present invention together with or in combination with one or more other therapeutic agents or methods. For combination therapy, the antibody or the antigen-binding unit thereof or the ADC may be administered simultaneously or separately with other therapeutic agents. When administered separately, the antibody or the antigen-binding unit thereof or the ADC of the present invention can be administered before or after administration of another additional therapeutic agent.

In one or more embodiments, when the antibody or the antigen-binding unit thereof or the ADC of the present invention is administered to a patient, the antibody or the antigen-binding unit thereof or the ADC or the pharmaceutical composition or the immunoconjugate disclosed herein and one or more other therapies, such as therapeutic modalities and/or other formulations (e.g., a therapeutic agent), may also be administered in combination to the patient.

In one or more embodiments, the antibody or the antigen-binding unit thereof or the ADC of the present invention can be used with an immune checkpoint inhibitor. In one or more embodiments, the antibody or the antigen-binding unit thereof or the ADC of the present invention is administered in combination with other therapeutic or prophylactic regimens, such as radiotherapy.

Such combination therapies encompass both simultaneous administration (wherein two or more formulations are contained in the same formulation or separate formulations) and separate administration (wherein the antibody or the antigen-binding unit thereof or the ADC of the present invention can be administered prior to, concurrently with, and/or subsequent to the administration of other therapies, e.g., therapeutic modalities or therapeutic agents). The antibody or the antigen-binding unit thereof or the ADC and/or other therapies, e.g., therapeutic agents or therapeutic modalities, can be administered when a disease is active or when the disease is in remission or less active. The antibody or the antigen-binding unit thereof or the ADC can be administered prior to, concurrently with or subsequent to other therapies, or during remission of a disease.

***Preparation method for antibody***

In one or more embodiments, the antibody, the antigen-binding unit or the derivative disclosed herein is modified using techniques well known in the art to reduce the immunogenicity. For example, the antibody can be humanized, primatized or deimmunized, or a chimeric antibody can be prepared. Such antibodies are derived from non-human antibodies, typically murine or primate antibodies, which retain or substantially retain the antigen-binding properties of the parent antibody but are less immunogenic in humans. This can be accomplished by a variety of methods, including: (a) grafting the entire variable region of a non-human origin to the constant region of a human origin to produce a chimeric antibody, wherein methods for producing chimeric antibodies are known in the art, see U.S. Pat. Nos. 5,807,715, 4,816,567 and 4,816,397, the entire contents of which are incorporated herein by reference; (b) grafting at least a portion of one or more non-human complementarity determining regions (CDRs) to the framework region and constant region of a human origin, with or without retention of critical framework residues; or (c) grafting the entire variable region of a non-human origin, but "hiding" the surface residues by replacing them with portions of a human-like origin. Generally, framework residues in the human framework regions will be substituted by corresponding residues from the CDR donor antibody, preferably residues that may improve the antigen binding. Such framework substitutions can be identified by methods well known in the art, for example, by modeling the interaction of the CDR and framework residues to identify framework residues that play an important role in antigen binding and by sequence alignment to identify abnormal framework residues at particular positions (see U.S. Pat. No. 5,585,089, the content of which is incorporated herein by reference in its entirety). Antibodies can be humanized using a variety of techniques well known in the art, such as CDR grafting (EP 239,400; WO 91/09967; U.S. Pat. Nos. 5,225,539, 5,530,101 and 5,585,089), repair or surface rearrangement (EP 592,106; EP 519,596), and chain rearrangement (U.S. Pat. No. 5,565,332), the contents of which are incorporated herein by reference in their entirety.

The production of antibodies with reduced immunogenicity is also achieved via humanization techniques, chimerization techniques and display techniques using appropriate libraries. It should be understood that murine antibodies or antibodies from other species can be humanized or primatized using techniques well known in the art. See, e.g., Winter and Harris Immunol Today 14:43 46 (1993) and Wright et al., Crit. Reviews in Immunol. 12125-168 (1992). The antibodies can be engineered by recombinant DNA techniques to substitute CH1, CH2, CH3, hinge domains and/or framework domains with the corresponding human sequences (see WO 92102190 and U.S. Pat. Nos. 5,530,101; 5,585,089; 5,693,761; 5,693,792; 5,714,350 and 5, 777,085). The use of Ig cDNAs for the construction of chimeric immunoglobulin genes is also known in the art (Liu et al., P.N.A.S. 84:3439 (1987) and J. Immunol. 139:3521 (1987)). mRNA is isolated from hybridomas or other cells that produce the antibody and used to produce cDNA. The cDNA can be amplified by polymerase chain reaction using specific primers (U.S. Pat. Nos. 4,683,195 and 4,683,202). Alternatively, a library is constructed and screened to isolate the sequence of interest. The DNA sequences encoding the variable regions of the antibody are then fused to human constant region sequences. The sequences of the human constant region genes can be found in Kabat et al., Sequences of Proteins of immunological Interest (N.I.H. publication no.91-3242 (1991)). The human C region genes can be readily obtained from known clones. The choice of isotype will be guided by the desired effector functions, such as activity of complement fixation or activity in antibody-dependent cellular cytotoxicity. Preferred isotypes are IgG1 and IgG2. Either of the human light chain constant regions, i.e., k or $\lambda$, can be used, and the chimeric humanized antibody is then expressed using conventional methods.

Deimmunization may also be used to reduce the immunogenicity of the antibody. In the present invention, the term "deimmunization" includes the alteration of the antibody to modify T cell epitopes (see, e.g., Pat. Nos. WO/9852976

A1 and WO/0034317 A2). For example, a heavy chain variable region sequence and a light chain variable region sequence from the original antibody are analyzed, and a "map" of human T cell epitopes from each variable region is generated, showing the positions of the epitopes relative to the complementarity determining regions (CDRs) and other critical residues within the sequence. Individual T cell epitopes from the T cell epitope map are analyzed to identify alternative amino acid substitutions with a lower risk of altering antibody activity. A series of alternative heavy chain variable region sequences and light chain variable region sequences comprising combinations of amino acid substitutions are designed and subsequently incorporated into a series of binding polypeptides. The genes of intact heavy and light chains comprising the modified variable regions and human constant regions are cloned into an expression vector, and the plasmid is then transferred into a cell line to produce an intact antibody. The antibodies are compared in appropriate biochemical and biological experiments to identify the optimal antibody.

Fully human antibodies that recognize selective epitopes can be produced using a technique known as "guided selection". In this method, a selected non-human monoclonal antibody is used to guide the screening of fully human antibodies that recognize the same epitope (see U.S. Pat. No. 5,565,332, which is incorporated herein by reference in its entirety).

For single-chain Fvs (scFvs), reference can be made to the technology for the production of single-chain units (U.S. Pat. No. 4,694,778). Single-chain units are formed by linking the heavy and light chain fragments of the Fv region via an amino acid bridge, resulting in a single-chain fusion peptide. Techniques for the assembly of functional Fv fragments in *E. coli* may also be used (Skerra et al., Science 242: 1038-1041 (1988)).

Examples of techniques that may be used to produce scFvs and antibodies include those described in U.S. Pat. Nos. 4,946,778 and 5,258,498.

In one or more embodiments, the antigen-binding fragment such as Fab, $F(ab')_2$ and Fv can be prepared by cleavage of an intact protein, e.g., by protease or chemical cleavage, including but not limited to: (i) digesting the antibody molecule with pepsin to obtain a $F(ab')_2$ fragment; (ii) obtaining a Fab fragment by reducing the disulfide bond of the $F(ab')_2$ fragment; (iii) treating the antibody molecule with papain and a reducing agent to produce a Fab fragment, and (iv) an Fv fragment.

Using conventional recombinant DNA techniques, one or more CDRs of the antibody of the present invention can be inserted into framework regions, e.g., into human framework regions to construct a humanized antibody. The framework regions may be naturally occurring or consensus framework regions, preferably human framework regions (see Chothia et al., J. Mol. Biol. 278:457-479 (1998), in which a range of human framework regions are listed). Some polynucleotides may encode antibodies produced by the framework region and CDR combinations that specifically bind to at least one epitope of a target antigen. One or more amino acid substitutions may be made within the framework regions, and amino acid substitutions capable of improving the binding of the antibody to the antigen thereof may be selected. Additionally, substitution or deletion of one or more cysteine residues in the variable regions involved in interchain disulfide bond formation can be made in this manner, thereby producing an antibody molecule lacking one or more interchain disulfide bonds. Other alterations to the polynucleotides made within the technology scope of the art are also encompassed by the present invention.

Antibodies can be prepared by using conventional recombinant DNA techniques. Vectors and cell lines for antibody production can be selected, constructed and cultured using techniques well known to those skilled in the art. These techniques are described in various laboratory manuals and main publications, such as Recombinant DNA Technology for Production of Protein Therapeutics in Cultured Mammalian Cells, D. L. Hacker, F. M. Wurm, Reference Module in Life Sciences, 2017, which is incorporated by reference in its entirety, including the supplements.

In one or more embodiments, the anti-FRα antibody or the antigen-binding unit thereof of the present invention is subjected to glycosylation modification. For example, deglycosylated antibodies (i.e., the antibodies lack glycosylation) can be prepared. Glycosylation can be altered, for example, to increase the affinity of an antibody for an antigen. Such modifications can be accomplished, for example, by altering one or more glycosylation sites within the antibody sequence. For example, one or more amino acid substitutions may be made to eliminate one or more glycosylation sites in the variable region, thereby eliminating glycosylation at those sites. Such deglycosylation can increase the affinity of an antibody for an antigen. Such methods are further described in detail in PCT publication WO 2003016466 A2 and U.S. Pat. Nos. 5,714,350 and 6,350,861, each of which is incorporated herein by reference in its entirety.

Antibody (e.g., anti-FRα antibody) genes can be inserted into an expression vector by standard methods (e.g., linking the antibody gene fragment to complementary restriction sites on the vector, or blunt end ligation if no restriction sites are present). The expression vector may be a plasmid, a retrovirus, a YAC, an EBV-derived episome, and the like. To express an antibody (e.g., an anti-FRα antibody), the DNA encoding the full-length light and heavy chains can be inserted into an expression vector, such that the genes are operably linked to transcriptional and translational control sequences. "Operably linked" means that the antibody genes are linked into a vector such that transcriptional and translational control sequences within the vector perform their intended functions of regulating the transcription and translation of the antibody genes.

The consensus sequence J region of the heavy and light chains can be used to design oligonucleotides for

use as primers to introduce useful restriction sites into the J region for subsequent linkage of V region fragments to human C region fragments. The C region cDNA can be modified by site-directed mutagenesis to place a restriction site at a similar position in the human sequence. In one or more embodiments, the expression vector already carries antibody constant region sequences prior to insertion of the antibody-related light or heavy chain gene sequences. For example, one method for converting the anti-FRα antibody-related VH and VL sequences into full-length antibody genes is to insert them into an expression vector that already encodes a heavy chain constant region and a light chain constant region, such that the VH segment is operably linked to the CH segment within the vector and the VL segment is operably linked to the CL segment within the vector.

The recombinant expression vector may encode a signal peptide that facilitates secretion of the heavy and light chains of an antibody from a host cell. Alternatively, the antibody heavy and light chain genes may be cloned into a vector encoding a signal peptide that facilitates secretion of the antibody heavy and light chains from a host cell, such that the signal peptide is linked in frame to the amino termini of the antibody heavy and light chain genes. The signal peptide may be an immunoglobulin signal peptide or a heterologous signal peptide (i.e., a signal peptide from a non-immunoglobulin protein), for example, MDWTWRILFLVAAATGAHS (SEQ ID NO: 21) or MEAPAQLLFLLLLWLPDTTG (SEQ ID NO: 22).

In addition to the antibody heavy and light chain genes, the recombinant expression vector may also carry regulatory sequences that control expression of the antibody chain genes in the host cell. Regulatory sequences include promoters, enhancers and other expression control elements (e.g., polyadenylation signals) that control the transcription or translation of antibody chain genes. Such regulatory sequences are described, for example, in Goeddel, Gene Expression Technology: Methods in Enzymology 185, Academic Press, San Diego, CA, 1990. Those skilled in the art will understand that the design of an expression vector, including the choice of regulatory sequences, may depend on such factors as the selection of a host cell to be transformed, the expression level of the desired protein, and the like. Suitable regulatory sequences for expression in mammalian host cells include viral elements that direct high-level protein expression in mammalian cells, such as promoters and/or enhancers derived from cytomegalovirus (CMV) (e.g., the CMV promoter/enhancer), simian virus 40 (SV40) (e.g., the SV40 promoter/enhancer), adenovirus (e.g., the adenovirus major late promoter (AdMLP)), and polyoma. For further description of viral regulatory elements and sequences thereof, see, e.g., U.S. Pat. Nos. 5,168,062, 4,510,245 and 4,968,615.

In addition to the antibody chain genes and regulatory sequences, the recombinant expression vector may carry other sequences, such as a sequence that regulates replication of the vector in a host cell (e.g., an origin of replication) and a selectable marker gene. The selectable marker gene facilitates selection of a host cell into which the vector has been introduced (see, e.g., U.S. Pat. Nos. 4,399,216, 4,634,665 and 5,179,017). For example, generally a marker gene that imparts resistance to drugs (such as G418, hygromycin or methotrexate) to a host cell into which the vector has been introduced can be selected. Suitable selectable marker genes include the dihydrofolate reductase (DHFR) gene (for use in DHFR-host cells with methotrexate selection/amplification), the neo gene (for G418 selection), and the GS gene. For expression of heavy and light chains, an expression vector encoding the heavy and light chains is transfected into a host cell by standard techniques.

The antibody of the present invention (e.g., anti-FRα antibody) can be prepared by recombinant expression of the heavy and light chain genes of the antibody in a host cell. For example, a host cell is transfected with one or more recombinant expression vectors carrying heavy and light chain DNA fragments encoding the antibody such that the heavy and light chains are expressed in the host cell, the expressed antibody can be secreted into the medium in which the host cell is cultured, and the antibody can be isolated from the medium. "Transfect" refers to a wide variety of techniques commonly used to introduce a foreign DNA into eukaryotic host cells, such as electroporation, lipofection, calcium phosphate precipitation, DEAE-dextran transfection, and the like. Standard recombinant DNA methods for obtaining antibody heavy and light chain genes, incorporating these genes into expression vectors and introducing the vectors into host cells are well known in the art, e.g., Molecular Cloning: A Laboratory Manual (second edition, Eds. Sambrook, Fritsch and Maniatis, Cold Spring Harbor, N. Y., 1989), Current Protocols in Molecular Biology (Eds. Ausubel, F.M. Et al., Greene Publishing Associates, 1989), and those methods described in U.S. Pat. No. 4,816,397. The DNAs expressing the heavy and light chains of the antibody may be placed in the same vector or placed in different vectors; if placed in different vectors, the vectors expressing the heavy and light chains of the antibody may transfect host cells in an appropriate ratio (e.g., Tihomir S. Dodev et al., A tool kit for rapid cloning and expression of recombinant antibodies, Scientific Reports volume 4, Article number: 5885 (2014); Stefan Schlatter et al., On the Optimal Ratio of Heavy to Light Chain Genes for Efficient Recombinant Antibody Production by CHO Cells, Biotechnol Progress, 21: 122-133 (2005); Hadi Bayat et al., Evaluation of different vector design strategies for the expression of recombinant monoclonal antibody in CHO cells, Preparative Biochemistry & Biotechnology, 48(8): 822-829 (2018)). In one or more embodiments, the antibody expression vector includes at least one promoter element, an antibody coding sequence, a transcription termination signal, and a polyA tail. Other elements may include enhancers, Kozak sequences (GCCACC, set forth in SEQ ID NO: 33), and intervening sequences flanked by donor and acceptor sites for RNA splicing. Efficient transcription can be obtained by early and late promoters of SV40, long terminal repeats from retroviruses such as RSV, HTLV1 and

HIVI, and early promoters of cytomegalovirus, and promoters from other cells such as actin promoter can also be used. Suitable expression vectors may include pIRES1neo, pRetro-Off, pRetro-On, pLXSN, pLNCX, pcDNA3.1(+/-), pcDNA/Zeo(+/-), pcDNA3.1/Hygro(+/-), pSVL, pMSG, pRSVcat, pSV2dhfr, pBC12MI, pCS2, pCHO1.0 or the like.

The antibody of the present invention (e.g., anti-FRα antibody) is capable of being expressed in eukaryotic host cells. In certain embodiments, the antibody is expressed in eukaryotic cells, such as mammalian host cells. Exemplary host cells for expressing the antibody of the present invention include Chinese hamster ovary cells (CHO cells) (e.g., CHO-K1 cells) or CHO-S, CHO-dhfr-, CHO/DG44 or ExpiCHO obtained by modification of CHO cells, NSO myeloma cells, COS cells, Cos1 cells, Cos7 cells, SP2 cells, CV1 cells, murine L cells, human embryonic kidney HEK293 cells, or HEK293T, HEK293F or HEK293E cells obtained by modification of HEK293 cells. After a recombinant expression vector encoding an antibody chain gene is introduced into a host cell, the host cell is cultured in a medium for a period of time sufficient to allow the antibody to be expressed in the host cell or allow the antibody to be secreted into the medium to produce the antibody. The antibody can be isolated from the medium using standard protein purification methods.

Antibody-producing cell lines can be selected, constructed and cultured using techniques well known to those skilled in the art. These techniques are described in various laboratory manuals and main publications, such as Recombinant DNA Technology for Production of Protein Therapeutics in Cultured Mammalian Cells, D. L. Hacker, F. M. Wurm, Reference Module in Life Sciences, 2017, which is incorporated by reference in its entirety, including the supplements.

For recombinant expression of the antibody of the present invention (e.g., anti-FRα antibody), a host cell can be co-transfected with two recombinant expression vectors, a first recombinant expression vector encoding the heavy chain of the antibody and a second recombinant expression vector encoding the light chain of the antibody. The two recombinant expression vectors may contain the same selectable marker, or they may each contain separate selectable markers. Alternatively, a host cell may be transfected with a recombinant expression vector encoding the heavy and light chains of the antibody.

The antibody of the present invention (e.g., anti-FRα antibody) can also be produced by chemical synthesis (e.g., by the methods described in Solid Phase Peptide Synthesis, second edition, 1984, The Pierce Chemical Co., Rockford, Ill.). Variant antibodies can also be generated using a cell-free platform (see, e.g., Chu et al., Biochemia No. 2, 2001 (Roche Molecular Biologicals) and Murray et al., 2013, Current Opinion in Chemical Biology, 17: 420-426).

The antibody of the present invention (e.g., anti-FRα antibody) produced by recombinant expression can be purified by any method known in the art for purifying immunoglobulin molecules, for example, by chromatography (e.g., ion exchange, affinity chromatography and sizing column chromatography), centrifugation, differential solubility, or any other standard technique for purifying proteins. For example, protein A or protein G is subjected to affinity chromatography, which provides primarily the IgG fraction in immune serum. In addition, the specific antigen targeted by the immunoglobulin, or an epitope thereof, can be immobilized on a column to purify the immune-specific antibody by immunoaffinity chromatography. The antibody of the present invention (e.g., anti-FRα antibody) can be fused to heterologous polypeptide sequences known in the art to facilitate purification. For purification of immunoglobulins, reference can be made to the D. Wilkinson's article (The Scientist, published by the Scientist, Inc., Philadelphia Pa., Vol. 14, No. 8 (Apr. 17, 2000), pp. 25-28). In addition, mutations can be introduced in the nucleotide sequence encoding the antibody of the present invention using standard techniques known to those skilled in the art, including but not limited to site-directed mutations resulting in amino acid substitutions and PCR-mediated mutations. The variant (including derivative) encodes a substitution of less than 50 amino acids, a substitution of less than 40 amino acids, a substitution of less than 30 amino acids, a substitution of less than 25 amino acids, a substitution of less than 20 amino acids, a substitution of less than 15 amino acids, a substitution of less than 10 amino acids, a substitution of less than 5 amino acids, a substitution of less than 4 amino acids, a substitution of less than 3 amino acids or a substitution of less than 2 amino acids relative to the VH CDR1, VH CDR2 and VH CDR3 of the original heavy chain variable region and the VL CDR1, VL CDR2 or VL CDR3 of the original light chain variable region. Alternatively, mutations can be introduced randomly along all or part of the encoding sequence, for example by saturation mutagenesis, and the resulting mutants can be screened for the biological activity to identify mutants that retain the activity. In one or more embodiments, the substitutions described herein are conservative amino acid substitutions.

The amino acid sequence of the heavy chain of the exemplary Antibody 1 of the present invention is set forth in SEQ ID NO: 19, and the coding gene sequence thereof is set forth in SEQ ID NO: 23.

SEQ ID NO: 19 is shown below:

QVQLVQSGVEVKKPGASVKVSCKASGYSFTGYFMNWVRQAPGQGLEWIGRIHPYD
GDTFYNQNFKDKATLTVDKSTTTAYMELKSLQFDDTAVYYCTRYDGSRAMDYWGQ
GTTVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCDKTHT
CPPCPAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK

SEQ ID NO: 23 is shown below:

CAGGTGCAGCTGGTGCAGTCCGGCGTGGAGGTGAAGAAGCCCGGCGCCAGCGTG
AAGGTGAGCTGCAAGGCCTCCGGCTACTCCTTCACCGGCTACTTCATGAACTGGG
TGAGGCAGGCCCCCGGCCAGGGACTGGAATGGATCGGCAGAATCCACCCCTACG
ACGGCGACACCTTTTACAATCAGAACTTCAAGGATAAGGCCACCCTGACCGTGGA
TAAGTCCACCACCACCGCCTACATGGAGCTGAAGTCCCTGCAGTTTGACGATACC
GCCGTGTACTACTGTACCAGATACGACGGCTCCAGAGCCATGGACTACTGGGGCC

AGGGCACCACCGTGACCGTGTCCTCCGCCAGCACCAAGGGCCCATCGGTCTTCCC
CCTGGCACCCTCCTCCAAGAGCACCTCTGGGGGCACAGCGGCCCTGGGCTGCCTG
GTCAAGGACTACTTCCCCGAACCGGTGACGGTGTCGTGGAACTCAGGCGCCCTGA
CCAGCGGCGTGCACACCTTCCCGGCTGTCCTACAGTCCTCAGGACTCTACTCCCTC
AGCAGCGTGGTGACCGTGCCCTCCAGCAGCTTGGGCACCCAGACCTACATCTGCA
ACGTGAATCACAAGCCCAGCAACACCAAGGTGGACAAGAAAGTTGAGCCCAAAT
CTTGTGACAAAACTCACACATGCCCACCGTGCCCAGCACCTGAACTCCTGGGGGG
ACCGTCAGTCTTCCTCTTCCCCCCAAAACCCAAGGACACCCTCATGATCTCCCGG
ACCCCTGAGGTCACATGCGTGGTGGTGGACGTGAGCCACGAAGACCCTGAGGTC
AAGTTCAACTGGTACGTGGACGGCGTGGAGGTGCATAATGCCAAGACAAAGCCG
CGGGAGGAGCAGTACAACAGCACGTACCGTGTGGTCAGCGTCCTCACCGTCCTGC
ACCAGGACTGGCTGAATGGCAAGGAGTACAAGTGCAAGGTCTCCAACAAAGCCC
TCCCAGCCCCCATCGAGAAAACCATCTCCAAAGCCAAAGGGCAGCCCCGAGAAC
CACAGGTGTACACCCTGCCCCCATCCCGGGATGAGCTGACCAAGAACCAGGTCA
GCCTGACCTGCCTGGTCAAAGGCTTCTATCCCAGCGACATCGCCGTGGAGTGGGA
GAGCAATGGGCAGCCGGAGAACAACTACAAGACCACGCCTCCCGTGCTGGACTC
CGACGGCTCCTTCTTCCTCTACAGCAAGCTCACCGTGGACAAGAGCAGGTGGCAG
CAGGGGAACGTCTTCTCATGCTCCGTGATGCATGAGGCTCTGCACAACCACTACA
CGCAGAAGAGCCTCTCCCTGTCTCCGGGTAAATGA

The amino acid sequence of the light chain of the exemplary Antibody 1 of the present invention is set forth in SEQ ID NO: 20, and the coding gene sequence thereof is set forth in SEQ ID NO: 24.

SEQ ID NO: 20 is shown below:

EIVLTQSPATLSLSPGERATLSCKASQSVSFAGTSLMHWYQQKPGQAPRLLIYRASNL
EAGVPARFSGSGSKTDFTLTISSLEPEDFAVYYCQQSREYPYTFGGGTKVEIKRTVAA
PSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC

SEQ ID NO: 24 is shown below:

GAGATCGTGCTGACCCAGTCCCCCGCCACCCTGAGCCTGAGCCCAGGAGAGAGG
GCCACCCTGTCCTGCAAGGCCTCCCAGAGCGTGAGCTTTGCCGGCACCAGCCTGA
TGCACTGGTATCAACAGAAGCCCGGCCAGGCCCCTAGGCTGCTGATCTACAGAGC
CAGCAACCTGGAGGCCGGCGTGCCTGCTAGGTTTTCCGGCAGCGGCTCCAAGACC
GATTTCACCCTGACCATCAGCAGCCTGGAGCCTGAGGATTTTGCCGTGTACTACT
GCCAGCAGTCCAGGGAGTACCCCTACACCTTCGGCGGCGGCACCAAGGTGGAGA
TCAAGCGTACGGTGGCTGCACCATCTGTCTTCATCTTCCCGCCATCTGATGAGCAG
TTGAAATCTGGAACTGCCTCTGTTGTGTGCCTGCTGAATAACTTCTATCCCAGAGA
GGCCAAAGTACAGTGGAAGGTGGATAACGCCCTCCAATCGGGTAACTCCCAGGA
GAGTGTCACAGAGCAGGACAGCAAGGACAGCACCTACAGCCTCAGCAGCACCCT
GACGCTGAGCAAAGCAGACTACGAGAAACACAAAGTCTACGCCTGCGAAGTCAC
CCATCAGGGCCTGAGCTCGCCCGTCACAAAGAGCTTCAACAGGGGAGAGTGTTG
A

Citation of publications and patent documents in the present invention is not intended as an admission that any is pertinent prior art, nor does it constitutes any admission as to the contents or date of the same. Now, the present invention has been described in the written description, and it should be understood by those skilled in the art that the present invention may be practiced in various embodiments. The above specification and the examples below are intended to be illustrative rather than limiting the claims disclosed herein. All the publications, patents, and patent applications cited in the present invention are incorporated herein by reference in their entirety for all purposes.

## Examples

The materials, reagents, etc. used in the following examples can be commercially available or obtained using known methods unless otherwise stated.

**Example 1:** Preparation of Antigen

Preparation of antigen FRα-His: the gene sequence encoding the antigen FRα-His (the amino acid sequence of the antigen FRα-His is set forth in SEQ ID NO: 28 and is constructed by adding 8× HIS tag (HHHHHHHH) to the C-terminus of the human FRα extracellular domain (set forth in SEQ ID NO: 27, the underlined part is a signal peptide)) was cloned into an expression vector to construct a recombinant expression vector, which was then stably transfected into CHO-K1 cells, cultured and purified to obtain the antigen FRα-His.

Amino acid sequence of human FRα extracellular domain:

MAQRMTTQLLLLLVWVAVVGEAQTRIAWARTELLNVCMNAKHHKEKPGPEDKLH EQCRPWRKNACCSTNTSQEAHKDVSYLYRFNWNHCGEMAPACKRHFIQDTCLYECS PNLGPWIQQVDQSWRKERVLNVPLCKEDCEQWWEDCRTSYTCKSNWHKGWNWTS GFNKCAVGAACQPFHFYFPTPTVLCNEIWTHSYKVSNYSRGSGRCIQMWFDPAQGNP NEEVARFYAAAM (SEQ ID NO: 27)

Amino acid sequence of antigen FRα-His:

MAQRMTTQLLLLLVWVAVVGEAQTRIAWARTELLNVCMNAKHHKEKPGPEDKLH EQCRPWRKNACCSTNTSQEAHKDVSYLYRFNWNHCGEMAPACKRHFIQDTCLYECS PNLGPWIQQVDQSWRKERVLNVPLCKEDCEQWWEDCRTSYTCKSNWHKGWNWTS GFNKCAVGAACQPFHFYFPTPTVLCNEIWTHSYKVSNYSRGSGRCIQMWFDPAQGNP NEEVARFYAAAMHHHHHHHH (SEQ ID NO: 28)

**Example 2:** Anti-FRα Antibody

The heavy chain of the anti-FRα antibody consists of a VH and a CH, and the light chain consists of a VL and a CL; the amino acid sequence of the CH is set forth in SEQ ID NO: 17, and the amino acid sequence of the CL is set forth in SEQ ID NO: 18. VH CDRs and VL CDRs are shown in Table 2, framework regions of the heavy and light chains are shown in Table 3, the amino acid sequences of the variable regions are shown in Table 4, and the amino acid sequences of the constant regions are shown in Table 5. The amino acid sequences of the heavy and light chains of the control antibody huMOV19 are shown in Table 6.

Gene sequences encoding the heavy chain and the light chain of the antibody were cloned into an expression vector to construct a recombinant expression vector, which was then transiently transfected into HEK293F cells, cultured and purified to obtain the antibody. The sequence was consistent with the expected sequence as determined by sequencing.

Table 2. VH CDRs and VL CDRs of anti-FRα antibody

| No. | Type | Amino acid sequence | SEQ ID NO: |
|---|---|---|---|
| 1 | VH CDR1 | GYFMN | 5 |
| | VH CDR2 | RIHPYDGDTFYNQNFKD | 6 |
| | VH CDR3 | YDGSRAMDY | 7 |
| | VL CDR1 | KASQSVSFAGTSLMH | 8 |
| | VL CDR2 | RASNLEA | 9 |
| | VL CDR3 | QQSREYPYT | 10 |

Table 3. Framework regions of anti-FRα antibody

| No. | Type | Amino acid sequence | SEQ ID NO: |
|---|---|---|---|
| 1 | VH FR1 | QVQLVQSGVEVKKPGASVKVSCKASGYSFT | 29 |
| | VB FR2 | WVRQAPGQGLEWIG | 30 |
| | VH FR3 | KATLTVDKSTTTAYMELKSLQFDDTAVYYCTR | 31 |
| | VH FR4 | WGQGTTVTVSS | 11 |
| | VL FR1 | EIVLTQSPATLSLSPGERATLSC | 12 |
| | VL FR2 | WYQQKPGQAPRLLIY | 13 |
| | VL FR3 | GVPARFSGSGSKTDFTLTISSLEPEDFAVYYC | 32 |
| | VL FR4 | FGGGTKVEIK | 14 |

Table 4. Variable regions of anti-FRα antibody

| No. | Type | Amino acid sequence | SEQ ID NO: |
|---|---|---|---|
| 1 | VH | QVQLVQSGVEVKKPGASVKVSCKASGYSFTGYFMNWVRQAPGQGLEWIGRIHPYDGDTFYNQNFKDKATLTVDKSTTTAYMELKSLQFDDTAVYYCTRYDGSRAMDYWGQGTTVTVSS | 15 |
| | VL | EIVLTQSPATLSLSPGERATLSCKASQSVSFAGTSLMHWYQQKPGQAPRLLIYRASNLEAGVPARFSGSGSKTDFTLTISSLEPEDFAVYYCQQSREYPYTFGGGTKVEIK | 16 |

Table 5. Amino acid sequences of constant regions

| Name | Amino acid sequence | SEQ ID NO: |
|---|---|---|
| Heavy chain constant region | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK | 17 |
| Light chain constant region | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC | 18 |

Table 6. Amino acid sequence of control antibody huMOV19

| No. | Type | Amino acid sequence | SEQ ID NO: |
|---|---|---|---|
| huMOV19 | Heavy chain | QVQLVQSGAEVVKPGASVKISCKASGYTFTGYFMNWVKQSPGQSLEWIGRIHPYDGDTFYNQKFQGKATLTVDKSSNTAHMELLSLTSEDFAVYYCTRYDGSRAMDYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK | 34 |
| huMOV19 | Light chain | DIVLTQSPLSLAVSLGQPAIISCKASQSVSFAGTSLMHWYHQKPGQQPRLLIYRASNLEAGVPDRFSGSGSKTDFTLTISPVEAEDAATYYCQQSREYPYTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC | 35 |

**Example 3:** Assays for Affinity Specificity and Species Specificity of Antibody

3.1 Affinity specificity of antibodies

Experimental reagents and consumables:

1. Antigens:

FRα-His (i.e., FOLR1), prepared by Example 1;
FOLR2, purchased from Aero, Catalog No. FO2-H5223, Batch No. 2792C-88HF1-K6;
FOLR3, purchased from R&D, Catalog No. 5319-FR-050, Batch No. RBK0319041.

2. Probe: Protein A Chip sensor, purchased from GE Healthcare, Cas# 29127556.
3. Reagents:

HBS-EP+ (10×): purchased from GE Healthcare, cat# BR-1006-69;
Glycine pH 1.5: purchased from GE Healthcare, cat# BR100354;
Running buffer: 50 mL of HBS-EP+ (10×) was taken, and 450 mL of ultrapure water was added, and the resulting solution was well mixed.
Experimental instrument: Biacore: T200, purchased from GE Healthcare.

Experimental procedures:

A Protein A chip was used for the assay. The antibody was diluted to 5 μg/mL using a running buffer and then passed through the experimental flow channels (Fc2, Fc4) at a flow rate of 10 μL/min, and the capturing lasted for 15 s so that the capture amount was about 440 RU; then the flow rate was adjusted to 30 μL/min, analytes, i.e., antigen dilutions at different concentrations (0 nM, 1.23 nM, 3.7 nM, 11.1 nM, 33.3 nM and 100 nM, diluted with the running buffer), were fed in sequence and each simultaneously passed through the surfaces of the experimental flow channels (Fc2, Fc4) and reference flow channels (Fc1, Fc3), the binding time was 180 s, and the dissociation time was 450 s; finally the chip was regenerated with Glycine pH 1.5 for 60 s, and then the next cycle was performed.

Result processing:

Experiment results were analyzed using data analysis software Evaluation Software 3.1. The sensing signals

acquired from the experimental flow channels for the samples were subjected to double subtraction (minus values of reference flow channels and blank control), and the fitting was performed using a "1:1" kinetic model to obtain the kinetic parameters (Ka: association rate; Kd: dissociation rate; KD: equilibrium dissociation constant) for the binding of each sample to the antigens. The results are shown in Table 7.

Table 7. Equilibrium dissociation constant KD of antibody to antigens

| Antibody | Antigen | | |
|---|---|---|---|
| | FOLR1 | FOLR2 | FOLR3 |
| 1 | 0.703 nM | - | - |
| huMOV19 | 0.468 nM | - | - |
| Note: "-" indicates no binding activity | | | |

As can be seen from the results in Table 7, Antibody 1 could specifically bind to FOLR1.

3.2 Species specificity of antibodies

Each antigen was diluted to 1 μg/mL with PBS and added to a microplate (manufacturer: Corning, Catalog No.: 42592) in a volume of 100 μL per well, and the microplate was coated overnight in a refrigerator (2-8 °C). The coating solution was discarded, and 200 μL of a blocking solution (containing 5% skimmed milk powder, prepared from PBS) was added. The microplate was left to stand in an electric thermostatic incubator at 37 °C for 2 h. The blocking solution was discarded, and the microplate was washed 3 times with 0.05% PBST. Antibody dilutions at different concentrations (at an initial concentration of 6 μg/mL, 3-fold dilution, 8 concentration gradients in total, diluted with PBS) were added at 100 μL per well. The microplate was left to stand in the electric thermostatic incubator at 37 °C for 2 h. The antibody dilutions were discarded, and the microplate was washed 8 times with 0.05% PBST and patted dry. Anti-Human Kappa Light Chains (Bound and Free) peroxidase antibody Produced in goat (manufacturer: sigma, Catalog No.: A7164-1ML) diluted in a 1:10000 ratio was added at 100 μL/well. The microplate was left to stand in the electric thermostatic incubator at 37 °C for 1 h. The antibody dilutions were discarded, and the microplate was washed 8 times with 0.05% PBST and patted dry. Single-component TMB substrate solution I (manufacturer: Huzhou InnoReagents Co., Ltd.; Catalog No.: TMB-S-001) was added at 100 μL/well, and the microplate was left to stand in the electric thermostatic incubator at 37 °C for 10 min in the dark. 0.1 M $H_2SO_4$ was added at 100 μL/well to terminate the reaction. The microplate was read on SpectraMax Plus Absorbance Microplate Reader with an absorption light wavelength of 450 nm.

Table 8. Affinities of antibodies for binding to antigens

| Antigen | $EC_{50}$ (μg/mL) | |
|---|---|---|
| | 1 | huMOV19 |
| FRα-His | 0.069 | 0.09 |
| Cynomolgus monkey FOLR1 (manufacturer: Aero, Catalog No.: F01-C52H8) | 0.066 | 0.087 |
| Mouse FOLR1 (manufacturer: Aero, Catalog No.: F01-M5225) | - | - |
| Rat FOLR1 (manufacturer: sino Biological, Catalog No.: 81073-R08N00507) | - | - |
| Note: "-" indicates no binding | | |

As can be seen from Table 8, Antibody 1 could bind to FRα-His and cynomolgus monkey FOLR1, and did not bind to mouse FOLR1 and rat FOLR1.

**Example 4:** Synthesis Procedures for Compound (1*S*,9*S*)-1-amino-9-ethyl-4,5-difluoro-9-hydroxy-1,2,3,9,12,15-hexahydro-10*H*,13*H*-benzo[*de*]pyrano[3',4':6,7]indolo[1,2-*b*]quinoline-10,13-dione hydrochloride (D-1)

1. Synthesis of *N,N*'-(3,4-difluoro-8-oxo-5,6,7,8-tetrahydronaphthalene-1,7-diyl)diacetamide

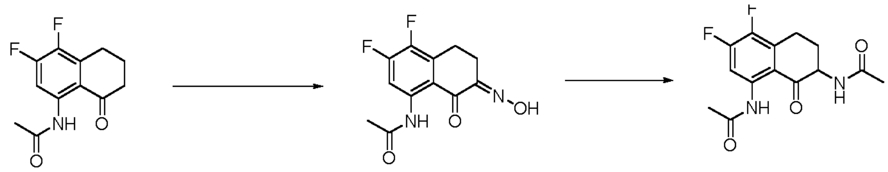

Under a nitrogen atmosphere, a solution of potassium *tert*-butoxide in tetrahydrofuran (42 mL, 1 M) was added to a dry reaction flask, and the mixture was stirred and cooled to 0-5 °C. *N*-(3,4-difluoro-8-oxo-5,6,7,8-tetrahydronaphthalen-1-yl)acetamide (CAS No.: 143655-49-6, 5 g, 21 mmol) dissolved in tetrahydrofuran (25 mL) was slowly added dropwise to the reaction flask, followed by dropwise addition of *tert*-butyl nitrite (4.32 g, 2 eq) (with temperature controlled at 0-5 °C). The resulting mixture was warmed to 15-20 °C and stirred for 2 hours (h). After the completion of the reaction, the mixture was cooled to 0-5 °C. Acetic acid (25 mL) and acetic anhydride (25 mL) were added dropwise (with temperature controlled at no more than 10 °C). After the dropwise addition, the mixture was stirred for 20 minutes (min). Zinc powder (8 eq) was added according to the amount of the *N*-(3,4-difluoro-8-oxo-5,6,7,8-tetrahydronaphthalen-1-yl)acetamide with the temperature maintained at 5-10 °C, and the resulting mixture was stirred at 20-25 °C for 1 h. The mixture was filtered, and the solid was rinsed with ethyl acetate (50 mL). The temperature was reduced to 0-5 °C, and 15% aqueous solution of $Na_2CO_3$ (50 mL) was added dropwise for three washes. Ethyl acetate (25 mL) was added for extraction, and the organic phases were combined and washed with a saturated aqueous solution of NaCl. Ethyl acetate (10 mL) was added, and the mixture was stirred at 40 °C for 30 min, slowly cooled to 0-5 °C and stirred for 2 h. The mixture was filtered, and the solid was washed with ethyl acetate/petroleum ether (1/2, 10 mL). Drying was performed *in vacuo* to obtain a gray powder (2.1 g, 33.7%). LC-MS: $[M+H]^+$ = 297.

2. Synthesis of *N*-(8-amino-5,6-difluoro-1-oxo-1,2,3,4-tetrahydronaphthalen-2-yl)acetamide

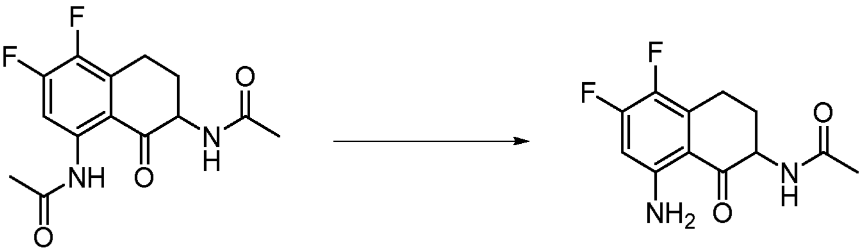

*N,N'*-(3,4-difluoro-8-oxo-5,6,7,8-tetrahydronaphthalene-1,7-diyl)diethylamide (500 mg, 1.68 mmol) was added to a 2 M solution of hydrochloric acid in ethanol (5 mL). The mixture was stirred at 50 °C for 4 h. After the completion of the reaction as detected, water (7.5 mL) was added. The mixture was cooled to 0-5 °C, and triethylamine (1.03 g) was added dropwise. The mixture was stirred for 3 h. The mixture was filtered, and washing was performed successively with 40% cold aqueous solution of ethanol (3 mL) and water (3 mL). Drying was performed *in vacuo* to obtain a gray powder (320 mg, 74.6%). LC-MS: $[M+H]^+$ = 255.

3. Synthesis of *N*-((9*S*)-9-ethyl-4,5-difluoro-9-hydroxy-10,13-dioxo-2,3,9,10,13,15-hexahydro-1*H*,12*H*-benzo[*de*]pyrano[3';4':6,7]indolo[1,2-*b*]quinoline-1-yl)acetamide

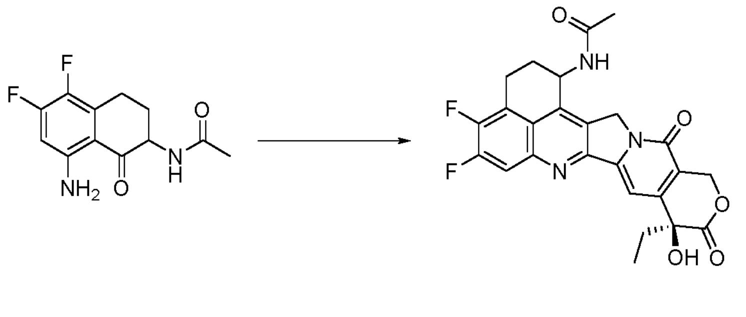

Under a nitrogen atmosphere, *N*-(8-amino-5,6-difluoro-1-oxo-1,2,3,4-tetrahydronaphthalen-2-yl)acetamide (1.1 g, 1 eq), (*S*)-4-ethyl-4-hydroxy-7,8-dihydro-1*H*-pyrano[3,4-*f*]indolizine-3,6,10(4*H*)-trione (1.15 g, 1 eq) and toluene (50 mL) were added to a reaction flask. The mixture was heated to reflux and stirred for 1 h. Then pyridinium 4-toluenesulfonate (100 mg) was added, and the mixture was stirred at reflux for another 20 h. The mixture was cooled to room temperature and stirred for 1 h. The mixture was filtered, and the solid was successively washed with acetone (10 mL) and cold ethanol (5 mL). Drying was performed *in vacuo* to obtain a taupe powder (1.1 g, 53%). LC-MS: $[M+H]^+$ = 482.

4. Synthesis of (1*S*,9*S*)-1-amino-9-ethyl-4,5-difluoro-9-hydroxy-1,2,3,9,12,15-hexahydro-10*H*,13*H*-benzo[*de*]pyrano[3',4':6,7]indolo[1,2-*b*]quinoline-10,13-dione hydrochloride (D-1)

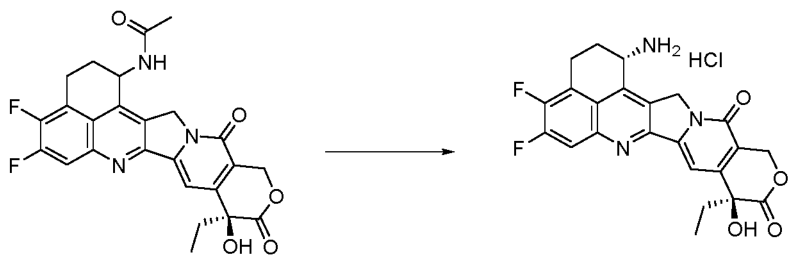

*N*-((9*S*)-9-ethyl-4,5-difluoro-9-hydroxy-10,13-dioxo-2,3,9,10,13,15-hexahydro-1*H*,12*H*-benzo[*de*]pyrano[3',4':6,7]indolo[1,2-*b*]quinoline-1-yl)acetamide (1.1 g, 2.28 mmol) and a 6 M aqueous solution of hydrochloric acid (44 mL) were added to a reaction flask. The mixture was stirred at reflux under a nitrogen atmosphere for 4 h. The mixture was concentrated to remove the solvent, and the residue was purified by HPLC to obtain a white powder (200 mg, 18%). LC-MS: $[M+H]^+$ = 440.

**Example 5:** Synthesis Procedures for 4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11, 14, 17,20,23-octaoxo-27,32,35-triazaheptatriacontan-37-amino)benzyl (4-nitrophenyl) carbonate (CB07)

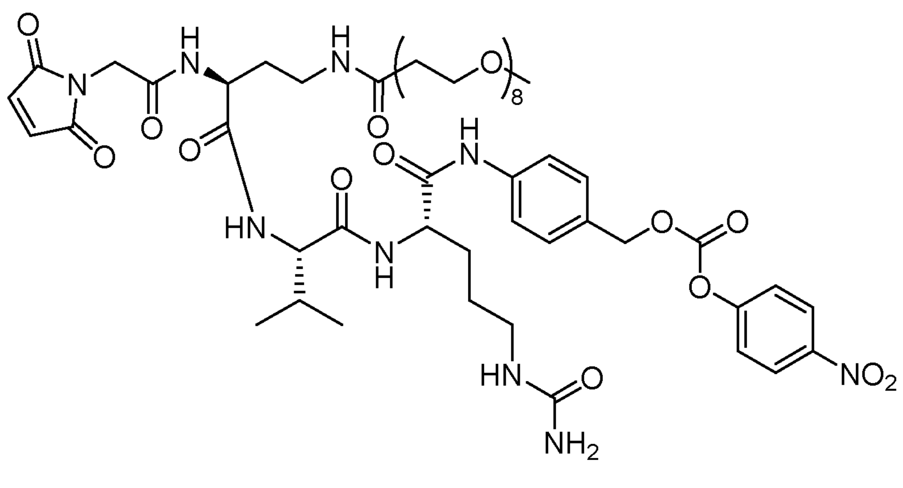

1) Synthesis of (*S*)-30-((((9*H*-fluoren-9-yl)methoxy)carbonyl)amino)-26-oxo-2,5,8,11, 14, 17,20,23-octaoxa-27-aza-hentriacontan-31-oic acid (CB01).

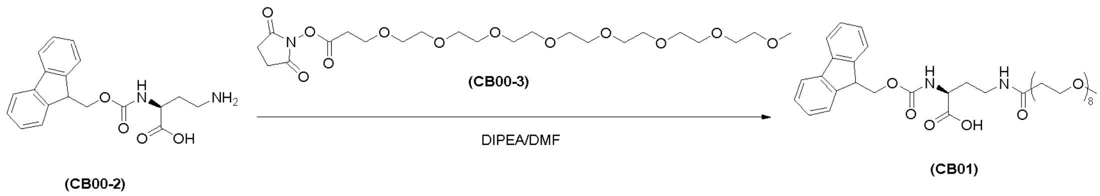


(CB00-2) (CB00-3) (CB01)

Under a nitrogen atmosphere at 0-5 °C, 8.14 g of *N*2-fluorenylmethoxycarbonyl-L-2,4-diaminobutyric acid (CB00-2) was dissolved in 40 mL of dimethylformamide (DMF), and 10 g of *N*-succinimidyl 4,7,10,13,16,19,22,25-octaoxahexacosanoate (CB00-3) and 10 mL of DMF were added. 6.5 mL of DIPEA was added dropwise with the temperature maintained at 0-5 °C. 1 h after the addition, the mixture was stirred at room temperature for 4 h. After the completion of the reaction, DMF was removed under reduced pressure, and the residue was purified by silica gel column chromatography (with dichloromethane and methanol (volume ratio of 20:1) as elution solvents) to obtain CB01 in the form of a pale yellow oil (14.2 g).

2) Synthesis of (9*H*-fluoren-9-yl)methyl [(*S*)-1-[[(*S*)-1-[[4-(hydroxymethyl)phenyl]amino]-1-oxo-5-ureidopentan-2-yl]amino]-3-methyl-1-oxobutan-2-yl]carbamate (CB02).

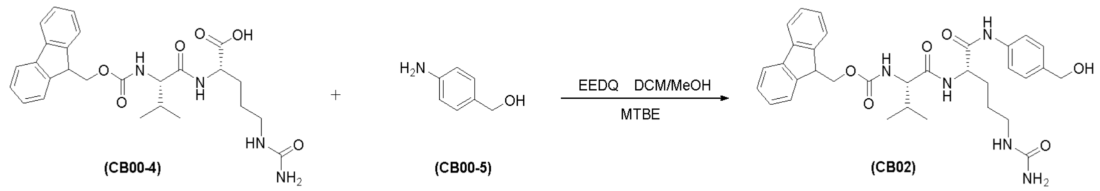


(CB00-4) (CB00-5) (CB02)

Under a nitrogen atmosphere at room temperature, 11 g of (*S*)-2-((*S*)-2-((((9*H*-fluoren-9-yl)methoxy)carbonyl)amino)-3-methylbutanamido)-5-ureidopentanoic acid (CB00-4) and 5.5 g of (4-aminophenyl)methanol (CB00-5) were dissolved in 400 mL of dichloromethane and 200 mL of methanol, and 17 g of 2-ethoxy-1-ethoxycarbonyl-1,2-dihydroquinoline (EEDQ) were added in portions with mechanical stirring. The resulting mixture was reacted for 15 h in the absence of light. After the completion of the reaction, the solvent was removed under reduced pressure to obtain a paste solid, which was subjected to silica gel column chromatography (with dichloromethane and methanol (volume ratio of 20:1) as elution solvents) to obtain CB02 in the form of an off-white solid (11.9 g).

3) Synthesis of (*S*)-2-((*S*)-2-amino-3-methylbutanamido)-*N*-(4-(hydroxymethyl)phenyl)-5-ureidopentanamide (CB03).

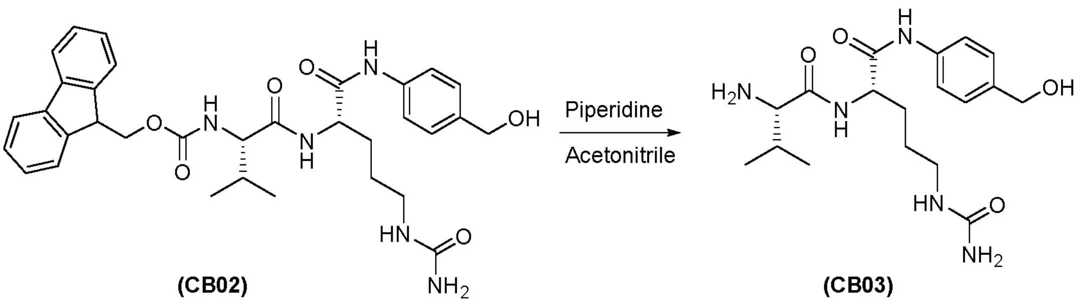


(CB02) (CB03)

Under a nitrogen atmosphere at room temperature, 300 mL of acetonitrile was added to 11.9 g of (9*H*-fluoren-9-yl)methyl [(*S*)-1-[[(*S*)-1-[[4-(hydroxymethyl)phenyl] amino]-1-oxo-5-ureidopentan-2-yl]amino]-3-methyl-1-oxobutan-2-yl]carbamate (CB02), and 18 mL of piperidine was added dropwise with stirring. After the dropwise addition, the mixture was reacted at room temperature for 2 h. After the completion of the reaction, the mixture was distilled under reduced pressure to remove the solvent and piperidine, and the residue was subjected to silica gel column chromatography (with dichloromethane and methanol (volume ratio of 20:1) as elution solvents) to obtain CB03 in the form of a white solid (7.5 g).

4) Synthesis of (9*H*-fluoren-9-yl)methyl ((30*S*,33*S*,36*S*)-41-amino-36-((4-(hydroxymethyl)phenyl)carbamoyl)-33-isopropyl-26,31,34,41-tetraoxo-2,5,8,11,14,17,20,23-octaoxa-27,32,35,40-tetraaza-30-yl)carbamate (CB04).

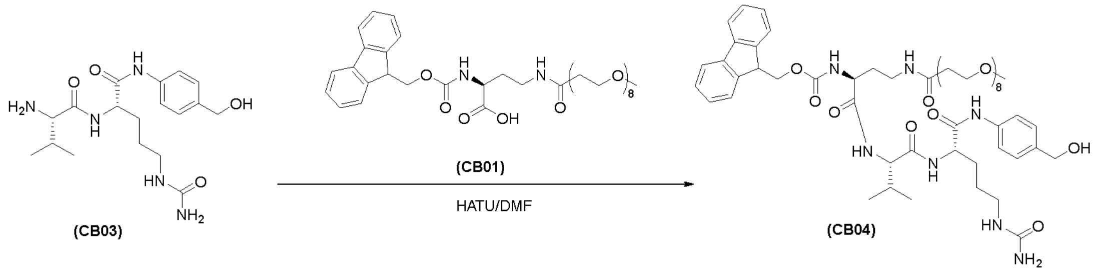

(CB03) (CB04)

Under a nitrogen atmosphere at 0 °C, 14.2 g of (*S*)-30-((((9*H*-fluoren-9-yl)methoxy)carbonyl)amino)-26-oxo-2,5,8,11,14, 17,20,23-octaoxa-27-aza-hentriacontan-31-oic acid (CB01) was dissolved in 100 mL of DMF, and 11 g of *N*,*N*,*N*',*N*'-tetramethyl-*O*-(7-azabenzotriazol-1-yl)uronium hexafluorophosphate (HATU) was added in portions. After 30 min of stirring, 7.5 g of (*S*)-2-((*S*)-2-amino-3-methylbutanamido)-*N*-(4-(hydroxymethyl)phenyl)-5-ureidopentanamide (CB03) was added, and the mixture was reacted for 2.5 h with the temperature maintained at 0 °C. After the completion of the reaction, the mixture was distilled under reduced pressure to remove the solvent, and the residue was subjected to silica gel column chromatography (with dichloromethane and methanol (volume ratio of 10:1) as elution solvents) to obtain CB04 in the form of a solid (9.66 g).

5) Synthesis of *N*-((3*S*)-3-amino-4-(((2*S*)-1-((1-((4-(hydroxymethyl)phenyl)amino)-1-oxo-5-ureidopentan-2-yl)amino)-3-methyl-1-oxobutan-2-yl)amino-oxobutan-2-yl)amino)-4-oxy)-2,5,8,11,14,17,20,23-octaoxahexacosan-26-amide (CB05).

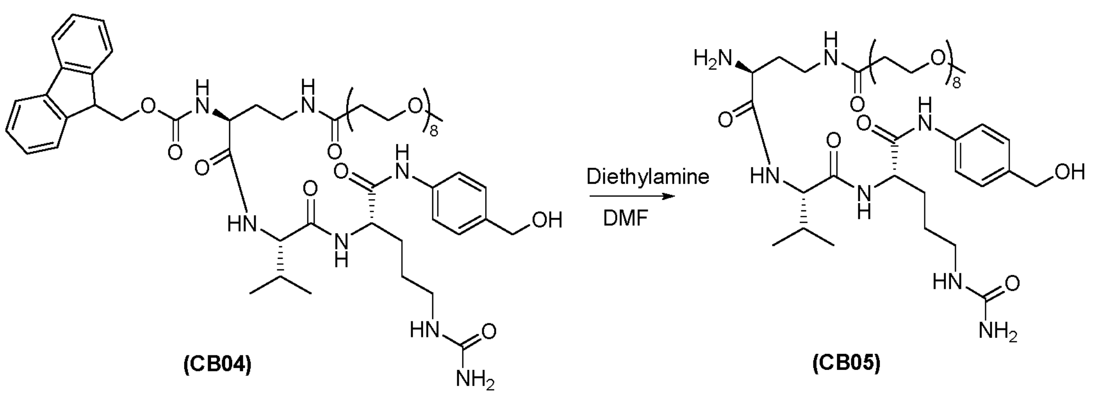

(CB04) (CB05)

Under a nitrogen atmosphere at room temperature, 9.66 g of (9*H*-fluoren-9-yl)methyl ((30*S*,33*S*,36*S*)-41-amino-36-((4-(hydroxymethyl)phenyl)carbamoyl)-33-isopropyl-26,31,34,41-tetraoxo-2,5,8, 11, 14, 17,20,23-octaoxa-27,32,35,40-tetraaza-30-yl)carbamate (CB04) was dissolved in 50 mL of DMF, and 12 mL of diethylamine was added. The mixture was stirred for 1.5 h. After the completion of the reaction, the mixture was distilled under reduced pressure to remove the solvent, and the residue was subjected to silica gel column chromatography (with dichloromethane and methanol (volume ratio of 7.5:1) as elution solvents) to obtain CB05 in the form of a pale yellow solid (7.7 g).

6) Synthesis of *N*-((3*S*)-3-(2-(2,5-dioxo-2,5-dihydro-1*H*-pyrrol-1-yl)acetamido)-4-(((2*S*)-1-((1-((4-(hydroxymethyl)phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06).

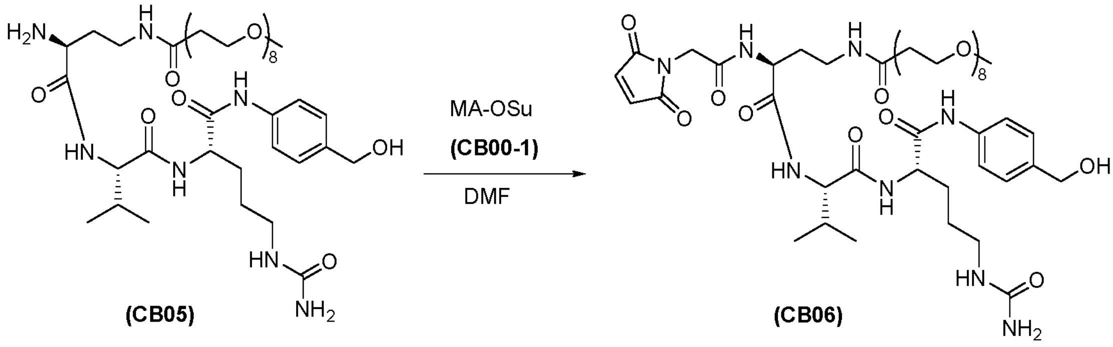


(CB05) (CB06)

7.7 g of *N*-((3*S*)-3-amino-4-(((2*S*)-1-((1-((4-(hydroxymethyl)phenyl)amino)-1-oxo-5-ureidopentan-2-yl)amino)-3-methyl-1-oxobutan-2-yl)amino)-4-oxy)-2,5,8,11,14,17,20,23-octaoxahexacosan-26-amide (CB05) was dissolved in 40 mL of DMF, and succinimidyl maleimidoacetate (CB00-1) was added in portions under a nitrogen atmosphere at 0-5 °C. The mixture was reacted for 4 h with the temperature maintained at 0-5 °C. After the completion of the reaction, the mixture was distilled under reduced pressure to remove the solvent, and the residue was subjected to silica gel column chromatography (with dichloromethane and methanol (volume ratio of 10:1) as elution solvents) to obtain CB06 in the form of a pale yellow solid (9.5 g).

7) Synthesis of 4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8, 11, 14, 17,20,23-octaoxo-27,32,35-triazaheptatriacontan-37-amino)benzyl (4-nitrophenyl) carbonate (CB07).

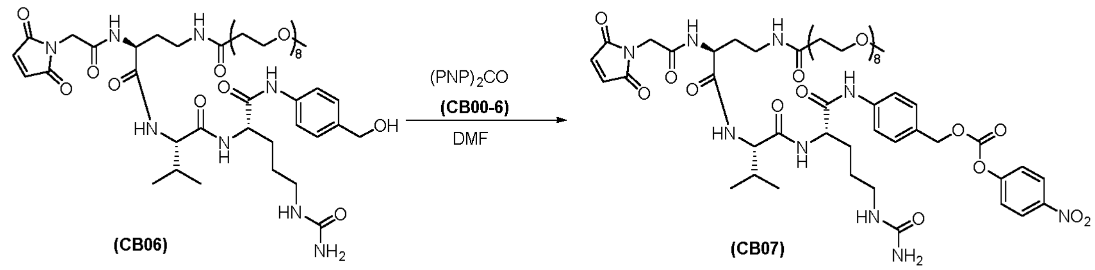


(CB06) (CB07)

9.5 g of *N*-((3*S*)-3-(2-(2,5-dioxo-2,5-dihydro-1*H*-pyrrol-1-yl)acetamido)-4-(((2*S*)-1-((1-((4-(hydroxymethyl)phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06) was dissolved in 50 mL of DMF, and 14.0 g of bis(p-nitrophenyl)carbonate ($(PNP)_2CO$) was added under a nitrogen atmosphere at 0 °C, followed by addition of 8.2 mL of *N*,*N*-diisopropylethylamine (DIPEA) after dissolution. The mixture was reacted at 0 °C for 4 h. After the completion of the reaction, the mixture was distilled under reduced pressure to remove the solvent, and the residue was subjected to silica gel column chromatography (with dichloromethane and methanol (volume ratio of 8:1) as elution solvents) to obtain CB07 in the form of a white solid (2.6 g).

**Example 6:** Synthesis of 4-((18*S*,21*S*,24*S*)-18-(2-(2,5-dioxo-2,5-dihydro-1*H*-pyrrol-1-yl)acetamido)-21-isopropyl-14,19,22-trioxo-24-(3-ureidopropyl)-2,5,8,11 -tetraoxo-15,20,23-triazapentacosan-25-amino)benzyl(4-nitrophenyl)carbonate (CB14)

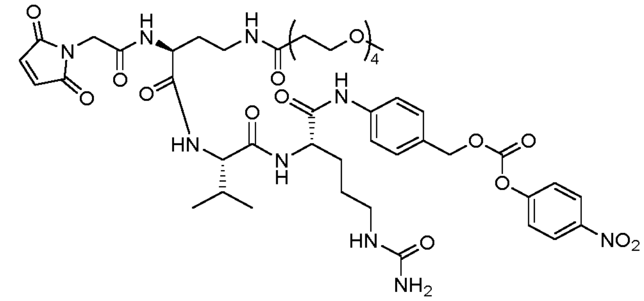

(CB14)

CB14 was prepared by referring to the synthesis of CB07 in Example 5, with N-succinimidyl 4,7,10,13,16,19,22,25-octaoxahexacosanoate replaced by N-succinimidyl 4,7,10,13-tetraoxatetradecanoate. CB14 was eventually obtained in the form of a white solid.

**Example 7:** Synthesis of Intermediate (CB07-Exatecan)

Synthesis of 4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxo-27,32,35-triazaheptatriacontan-37-amido)benzyl ((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indoline[1,2-*b*]quinoline-1-yl)carbamate (CB07-Exatecan).

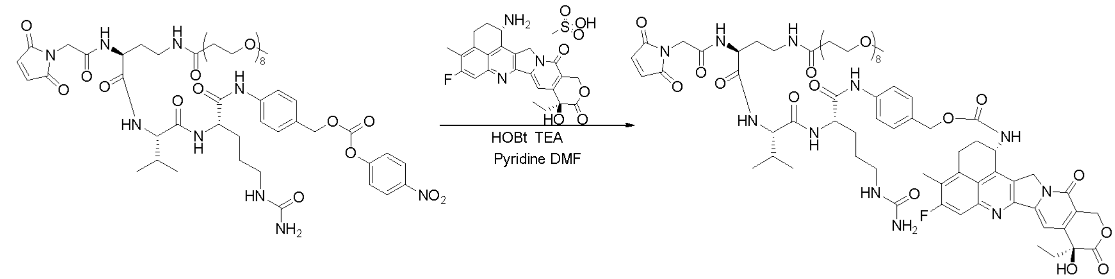


4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxo-27,32,35-triazaheptatriacontan-37-amido)benzyl (4-nitrophenyl) carbonate (CB07) (2.6 g, 2.21 mmol) and *N*,*N*-dimethylformamide (23 mL) were added to a reaction flask R1, stirred under a nitrogen atmosphere and cooled to 0-5 °C. At the same time, (1*S*,9*S*)-1-amino-9-ethyl-5-fluoro-9-hydroxy-4-methyl-1,2,3,9,12,15-hexahydro-10*H*,13*H*-benzo[3',4':6,7]indoline[1,2-*b*]quinoline-10,13-dione methanesulfonate (exatecan methanesulfonate, 0.98 g, 1.84 mmol, Advanced ChemBlocks) and *N,N*-dimethylformamide (5 mL) were added to another reaction flask R2. Triethylamine (230 mg, 2.27 mmol) was added dropwise at 0-5 °C. The mixture was stirred until complete dissolution was achieved. The solution in reaction flask R2 was added dropwise to reaction flask R1. Reaction flask R2 was then washed with *N,N*-dimethylformamide (2 mL), and the washing solution was added to reaction flask R1. 1-Hydroxybenzotriazole (497 mg, 3.68 mmol) and pyridine (1.45 g, 18.4 mmol) were weighed into reaction flask R1. The mixture was stirred at 0-5 °C for 10 min, warmed to room temperature, and stirred for 5.5 h. After the completion of the reaction, the mixture was concentrated under reduced pressure at 35 °C to remove the solvent. The residue was purified by preparative high performance liquid chromatography (prep-HPLC) and lyophilized to obtain a white powder (CB07-Exatecan, 1.6 g, 59%). LC-MS: $[1/2M+H]^+$ = 737.

**Example 8:** Synthesis of Intermediate (CB07-D-1)

Synthesis of 4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxo-27,32,35-triazaheptatriacontan-37-amido)benzyl ((1*S*,9*S*)-9-ethyl-4,5-difluoro-9-hydroxy-10,13-dioxo-2,3,9,10,13,15-hexahydro-1*H*,12*H*-benzo[*de*]pyrano[3',4':6,7]indoline[1,2-*b*]quinoline-1-yl)carbamate (CB07-D-1).

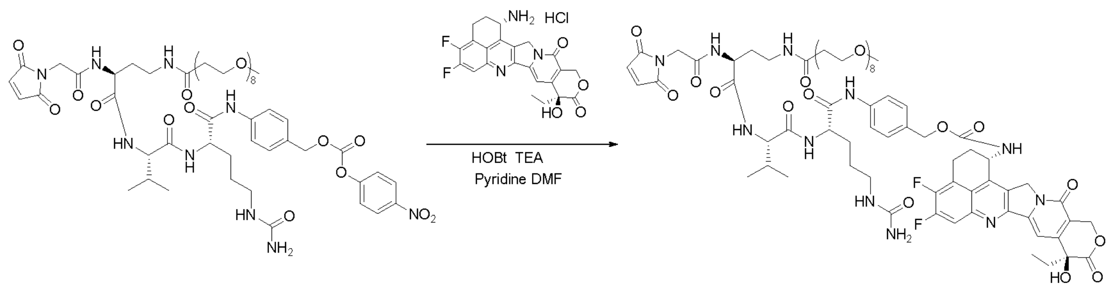


4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxo-27,32,35-triazaheptatriacontan-37-amido)benzyl (4-nitrophenyl) carbonate (CB07) (220 mg, 0.189 mmol) and *N*,*N*-dimethylformamide (5 mL) were added to a reaction flask R1, stirred under a nitrogen atmosphere and cooled to 0-5 °C. At the same time, (1*S*,9*S*)-1-amino-9-ethyl-4,5-difluoro-9-hydroxy-1,2,3,9,12,15-hexahydro-10*H*,13*H*-benzo[*de*]pyrano[3',4':6,7]indolo[1,2-*b*]quinoline-10,13-dione hydrochloride (D-1) (90 mg, 0.189 mmol) and *N,N*-dimethylformamide (5 mL) were added to another reaction flask R2. Three drops of triethylamine were added at 0-5 °C. The mixture was stirred until complete dissolution was achieved. The solution in reaction flask R2 was added dropwise to reaction flask R1, and 1-hydroxybenzotriazole (60 mg, 0.44 mmol) and pyridine (0.5 mL) were weighed into reaction flask R1. The mixture was stirred at 0-5 °C for 10 min, warmed to room temperature, and stirred for 3 h. After the completion of the reaction, the mixture was concentrated under reduced pressure to remove the solvent. The residue was purified by preparative high performance liquid chromatography (prep-HPLC) and lyophilized to obtain a white powder (CB07-D-1, 55 mg, 20%). LC-MS: $[1/2M+H]^+$ = 739.

**Example 9:** Synthesis of Intermediate (CB14-Exatecan)

Synthesis of 4-((18*S*,21*S*,24*S*)-18-(2-(2,5-dioxane-2,5-dihydro-1*H*-pyrrol-1-yl)acetamido)-21-isopropyl-14,19,22-trioxo-24-(3-ureidopropyl)-2,5,8,11-tetraoxo-15,20,23-triazapentacosan-25-amino)-(1*S*,9*S*)-9-ethyl-5-fluoro-9-hydroxy-4-methyl-10,13-dioxo-2,3,9,10,13,15-hexahydro-1*H*,12*H*-benzo[*de*]pyran[3',4':6,7]indoline[1,2-*b*]quinoline-1-carbamate (CB14-Exatecan).

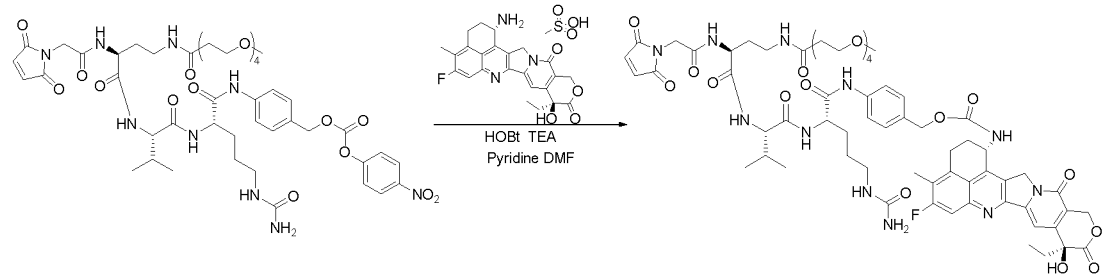


Similarly, 4-((18*S*,21*S*,24*S*)-18-(2-(2,5-dioxo-2,5-dihydro-1*H*-pyrrol-1-yl)acetamido)-21-isopropyl-14,19,22-trioxo-24-(3-ureidopropyl)-2,5, 8,11-tetraoxo-15,20,23-triazapentacosan-25-amino)benzyl(4-nitrophenyl)carbonate (190 mg, 0.19 mmol) and *N*,*N*-dimethylformamide (23 mL) were added to a reaction flask R1, stirred under a nitrogen atmosphere and cooled to 0-5 °C. At the same time, (1*S*,9*S*)-1-amino-9-ethyl-5-fluoro-9-hydroxy-4-methyl-1,2,3,9,12,15-hexahydro-10*H*,13*H*-benzo[3',4':6,7]indoline[1,2-*b*]quinoline-10,13-dione methanesulfonate (exatecan methanesulfonate, 101 mg, 0.19 mmol, Advanced ChemBlocks) and *N,N*-dimethylformamide (5 mL) were added to another reaction flask R2. Triethylamine (3 drops) was added dropwise at 0-5 °C. The mixture was stirred until complete dissolution was achieved. The solution in reaction flask R2 was added dropwise to reaction flask R1. Reaction flask R2 was then washed with *N*,*N*-dimethylformamide (1 mL), and the washing solution was added to reaction flask R1. 1-Hydroxybenzotriazole (60 mg, 0.44 mmol) and pyridine (0.5 mL) were weighed into reaction flask R1. The mixture was stirred at 0-5 °C for 10 min, warmed to room temperature, and stirred for 5.5 h. After the completion of the reaction, the mixture was concentrated under reduced pressure at 35 °C to remove the solvent.

The residue was purified by preparative high performance liquid chromatography (prep-HPLC) and lyophilized to obtain CB 14-Exatecan in the form of a white powder.

**Example 10:** Synthesis of ADCs

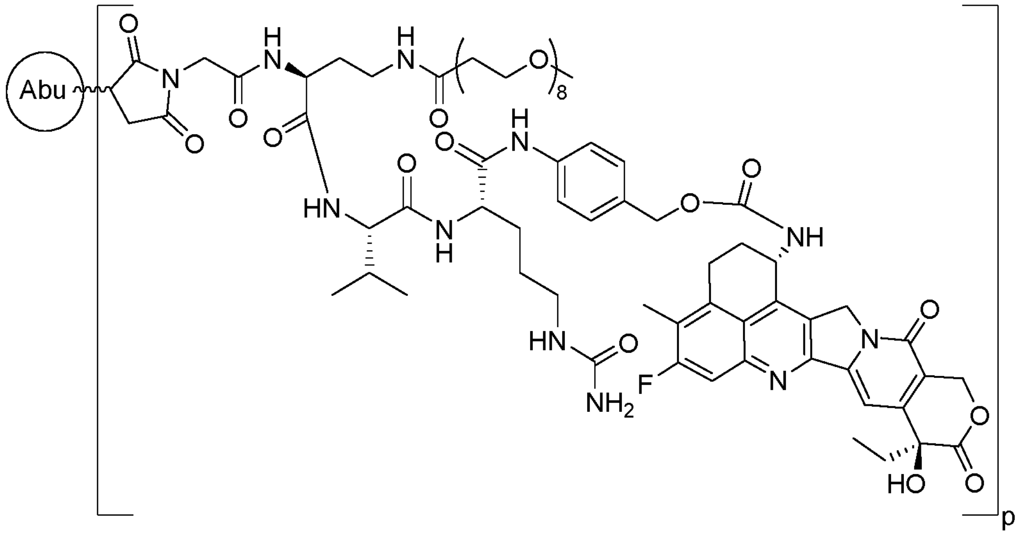

ADC1 conjugation process (batch 1):

Reduction reaction: the concentration of Antibody 1 was adjusted to 18 g/L using a conjugation buffer (10 mM aqueous solution of succinic acid); a 0.1 M aqueous solution of EDTA was added until the final concentration of EDTA reached 2 mM; 5.4 molar equivalents of a 0.2 M aqueous solution of TCEP was added according to the amount of the antibody substance; the pH was adjusted to 7; and then the system was stirred and reacted at 25 °C and 180 rpm for 2 h, such that the interchain disulfide bond of the antibody was reduced to free sulfhydryl.
The buffer was exchanged by ultrafiltration for 10 volumes using an ultrafiltration membrane with 30 KD to remove the reducing agent TCEP in the system.
Conjugation reaction: 15-fold molar equivalents of a 100 mM solution of CB07-Exatecan in dimethylacetamide (DMA) was added according to the amount of the antibody substance. The system was stirred and reacted at 25 °C and 180 rpm for 2 h. Finally, a 0.2 M aqueous solution of *N*-acetylcysteine was added until its final concentration reached 2 mM, and the system was reacted for another 15 min to terminate the conjugation reaction. The reaction mixture was subjected to purification and ultrafiltration to obtain ADC1 at a concentration of 23.56 mg/mL, and the DAR (namely p) was 8 as measured by reversed-phase chromatography. Unless otherwise specified, ADC1 in the following examples is batch 1. ADC2 conjugation process:
Reduction reaction: the concentration of Antibody 1 was adjusted to about 18 g/L using a conjugation buffer (10 mM aqueous solution of succinic acid); a 0.1 M aqueous solution of EDTA was added until the final concentration of EDTA reached 2 mM; 2.5 molar equivalents of a 10 mM aqueous solution of TCEP was added according to the amount of the antibody substance; the pH was adjusted to 7; and then the system was shaken on a shaker and reacted at 25 °C for 2 h, such that the interchain disulfide bond of the antibody was reduced to free sulfhydryl.
The buffer was exchanged by ultrafiltration for 10 volumes to remove the reducing agent TCEP in the system.
Conjugation reaction: 6-fold molar equivalents of a 100 mM solution of CB07-Exatecan in dimethylacetamide (DMA) was added according to the amount of the antibody substance. The system was shaken on the shaker and reacted at 25 °C for 1 h. Finally, a 0.1 M aqueous solution of N-acetylcysteine was added until its final concentration reached 1.5 mM, and the system was reacted for another 15 min to terminate the conjugation reaction. The reaction mixture was subjected to purification to obtain ADC2 at a concentration of 1.34 mg/mL, and the DAR (namely p) was 4 as measured by reversed-phase chromatography.

**Example 11:** *In Vitro* Activity Assay for ADC1

1) Binding activity of ADC1 to JEG-3 cells (FCM)

The binding activity of ADC1 and Antibody 1 to JEG-3 cells was detected by flow cytometry. Experimental procedures:

1. JEG-3 cells were plated in a 96-well plate (manufacturer: Corning, Catalog No.: 3897) at 300,000 cells/well. The plate was centrifuged at 1500 rpm for 5 min using a centrifuge, and the supernatant was removed.
2. Diluted ADC1 and Antibody 1 dilutions were separately added at 200 μL/well, wherein ADC1 and Antibody 1 were separately subjected to two-fold dilution from a concentration of 25 nM for a total of 10 concentration gradients. Three replicate wells were set for each concentration. A blank control and a control incubated with the secondary antibody were set. The plate was incubated on ice for 1 h.
3. The plate was centrifuged at 1500 rpm for 5 min on the centrifuge, and the supernatant was discarded. 200 μL of PBS was added, the resulting solution was pipetted uniformly and centrifuged at 1500 rpm for 5 min, and the supernatant was discarded.
4. Anti-Hu IgG (Fcy-specific) PE (manufacturer: eBioscience, Catalog No.: 12-4998-82) diluted in a 1:500 ratio was added at 100 μL/well, and the plate was incubated on ice for 0.5 h in the dark.
5. The plate was centrifuged at 1500 rpm for 5 min on the centrifuge, and the supernatant was discarded. 200 μL of PBS was added, the resulting solution was pipetted uniformly and centrifuged at 1500 rpm for 5 min, and the supernatant was discarded. The procedure was repeated once.
6. 200 μL of PBS was added, and the resulting solution was pipetted uniformly. The results were detected using CytoFLEX.
7. Data analysis was performed using GraphPad Prism.

Table 9. Binding activity of Antibody 1 and ADC1 to JEG-3 cells

| Sample | $EC_{50}$ |
|---|---|
| Antibody 1 | 1.405 nM |
| ADC1 | 1.592 nM |

The results are shown in Table 9 and show that both Antibody 1 and ADC1 had strong binding activity to JEG3 cells.

2) Binding activity of ADC1 to FRα-His (Biacore)

The affinities of ADC1 and Antibody 1 to FRα-His were evaluated using Biacore T200 surface plasmon resonance (SPR) technique.

Reagents:

HBS-EP+ (10×): purchased from GE Healthcare, cat# BR-1006-69;
Glycine pH 1.5: purchased from GE Healthcare, cat# BR100354;
Running buffer: 50 mL of HBS-EP+ (10×) was taken, and 450 mL of ultrapure water was added, and the resulting solution was well mixed.
Experimental instrument: Biacore: T200, purchased from GE Healthcare.

Experimental procedures:

A Protein A chip was used for the assay. The drug (ADC1 or Antibody 1) was diluted to 5 μg/mL using the running buffer and then passed through the experimental flow channels (Fc2, Fc4) at a flow rate of 10 μL/min, and the capturing lasted for 15 s so that the capture amount was about 440 RU; then the flow rate was adjusted to 30 μL/min, analytes, i.e., FRα-His dilutions at different concentrations (0 nM, 1.23 nM, 3.7 nM, 11.1 nM, 33.3 nM and 100 nM, diluted with the running buffer), were fed in sequence and each simultaneously passed through the surfaces of the experimental flow channels (Fc2, Fc4) and reference flow channels (Fc1, Fc3), the binding time was 180 s, and the dissociation time was 450 s; finally the chip was regenerated with Glycine pH 1.5 for 60 s, and then the next cycle was performed.

Result processing:

Experiment results were analyzed using data analysis software Evaluation Software 3.1. The sensing signals acquired from the experimental flow channels for the samples were subjected to double subtraction (minus values of reference flow channels and blank control), and the fitting was performed using a "1:1" kinetic model to obtain the kinetic

parameters (Ka: association rate; Kd: dissociation rate; KD: equilibrium dissociation constant) for the binding of each sample to the antigens. The results are shown in Table 10. The results show that Antibody 1 and ADC1 had similar affinities to FRα-His.

Table 10. Affinities of Antibody 1 and ADC1 to FRα-His

| Drug | Ka (1/Ms) | Kd (1/s) | KD (M) |
|---|---|---|---|
| ADC1 | 1.178E+06 | 3.848E-04 | 3.265E-10 |
| Antibody 1 | 1.021E+06 | 2.927E-04 | 2.867E-10 |

**Example 12:** Endocytosis of ADC1 in FRα-Expressing Cell Line

The endocytosis of ADC1 in an FRα-expressing cell line KB was observed using a confocal microscope, and FRα-negative cells SNU-1 were used as a control. After the cells were cultured overnight in a cell culture dish, lysosome-stained LysoBrite Red solution (BBI Life Sciences, Shanghai, China) was added, and the cells were incubated for 1.5 h. After washing, the cells were treated with fitc-labeled ADC1 (10 μg/mL) and bound to ADC1 at 4 °C for 20 min. Then, the cells were washed 3 times with PBS to remove unbound ADC1. The cells were then cultured at 37 °C to internalize ADC1. At each time point, the cells were immobilized in 4% paraformaldehyde and observed under a ZEISS LSM 710 confocal microscope. As shown in FIG. 2, there was no fitc-labeled ADC1 in the cells prior to internalization in the cells. However, after 3 h of internalization, more fitc-labeled ADC1 was detected in the cells, and most of them were coincided with lysosomes.

**Example 13:** *In Vitro* Cytotoxicity of ADC1

The *in vitro* cytotoxicity mediated by ADC1 was evaluated using an FRα-positive cell strain JEG-3 and MCF-7. Cells were harvested by trypsin, cultured with ADC1 diluted in gradient, and then incubated at 37 °C. The viability was determined after 5 days using CCK-8. The reading and analysis were performed on SpectraMax Gemini (Molecular Devices) to determine $IC_{50}$ (half maximal inhibitory concentration) values.

Experimental procedures:

1) JEG-3 cells were adjusted to a cell density of 60,000 cells/mL with DMEM + 10% FBS and seeded in a 96-well plate (manufacturer: Corning, Catalog No.: 3599) at 100 μL/well, and the plate was left to stand in Series II Water Jacketed $CO_2$ Incubator at 37 °C with 5% $CO_2$ for 3 h.
MCF-7 cells were adjusted to a cell density of 40,000 cells/mL with DMEM + 2% FBS and seeded in a 96-well plate (manufacturer: Corning, Catalog No.: 3599) at 100 μL/well, and the plate was left to stand in Series II Water Jacketed $CO_2$ Incubator at 37 °C with 5% $CO_2$ for 2 h.
2) ADC1 was diluted with the medium for corresponding cells. JEG-3 cells: ADC1 was subjected to 4-fold dilution from a concentration of 1000 nM for a total of 8 concentration gradients, and added to the cells in a volume of 100 μL per well. MCF-7 cells: ADC1 was subjected to 4-fold dilution from a concentration of 250 nM for a total of 8 concentration gradients, and added to the cells in a volume of 100 μL per well. 3000 nM Exatecan was selected as a lethal control. The medium for corresponding cells was a blank control.
Three replicate wells were set for each concentration.
3) The cells were cultured in Series II Water Jacketed $CO_2$ Incubator at 37 °C with 5% $CO_2$ for 5 days.
4) After 5 days, the culture supernatant was discarded, and RPMI basic medium 1640 (1×) containing 10% CCK-8 was added. The plate was left to stand in Series II Water Jacketed $CO_2$ Incubator at 37 °C with 5% $CO_2$ for about 1 h.
5) The plate was read on a microplate reader SpectraMax M3 with an absorption light wavelength of 450 nm.
6) Data analysis and processing: the Exatecan-treated well data were complete kill controls, and the blank control was a zero kill control. The formula for calculating the cell viability is as follows:

Cell viability (%) = (experimental group - lethal control group)/(blank control group - lethal control group) × 100

7) Data were processed and analyzed using GraphPad Prism.

The results are shown in Table 11. The results show that ADC1 had very high *in vitro* cytotoxicity on the FRα-positive cell strain.

Table 11. *In vitro* cytotoxicity of ADC1 on FRα-positive cell strain

| Cell line | $IC_{50}$ (nM) |
|---|---|
| JEG-3 | 3.611 |
| MCF-7 | 0.645 |

**Example 14:** Bystander Effect of ADC1

To confirm the bystander effect induced by ADC1, we performed an *in vitro* co-culture cell killing assay and a conditioned medium (CM) cytotoxicity assay.

FRα-positive JEG-3 cells and FRα-negative A549 cells (ADC1 had no killing effect on A549 cells) were selected for the assays. JEG-3 cells were treated with ADC1 for 2 days, 3 days and 4 days, respectively. CM was then transferred to A549 cells, and changes in cell viability were monitored. It could be found that the viability of A549 cells was significantly decreased. Experimental procedures:

1) JEG-3 cells were adjusted to a cell density of 100,000 cells/mL with DMEM + 10% FBS and seeded in a 96-well plate (manufacturer: Corning, Catalog No.: 3599) at 100 μL/well, and the plate was left to stand in Series II Water Jacketed $CO_2$ Incubator at 37 °C with 5% $CO_2$ for 2 h.
2) ADC1 was diluted in the DMEM + 10% FBS medium with 4-fold dilution from a concentration of 2000 nM for a total of 10 concentration gradients, and added to the cells in a volume of 100 μL per well.
3) steps 1) and 2) were repeated on Day 2 and Day 3.
4) On Day 5, A549 cells were adjusted to a density of 40,000 cells/mL with a DMEM + 2% FBS medium and seeded in a 96-well plate at 100 μL/well, and the plate was left to stand in Series II Water Jacketed $CO_2$ Incubator at 37 °C with 5% $CO_2$ for 2 h. The culture supernatants obtained in steps 1), 2) and 3) were added at 100 μL per well. 100 μL of 3000 nM Exatecan was added to column 1 and used as a lethal control, and 100 μL of the DMEM + 2% FBS medium was added to column 12 and used as a blank control.
5) The cells were cultured in Series II Water Jacketed $CO_2$ Incubator for 3 days. The culture conditions were 37 °C and 5% $CO_2$.
6) The culture solution was discarded, and a DMEM medium containing 10% CCK-8 was added. The cells were placed in Series II Water Jacketed $CO_2$ Incubator and subjected to color development at 37 °C for 1 h in the dark. The plate was then read on a microplate reader SpectraMax M3 with an absorption light wavelength of 450 nm.
7) Data were processed. The formula for caculating cell viability is as follows:

$$\text{Cell viability (\%)} = (\text{experimental well} - \text{lethal well})/(\text{blank well} - \text{lethal well}) \times 100$$

8) Data analysis was performed using GraphPad Prism.

The results are shown in FIG. 3 and show that the culture supernatant obtained by *in vitro* co-culture of ADC1 and FRα-positive JEG-3 cells had a significant killing effect on A549 cells.

**Example 15:** *In Vivo* Animal Experiments for ADCs

1) Pharmacodynamic evaluation of test drugs in subcutaneous xenograft BALB/c nude female mouse models of HuPrime® ovarian cancer OV3756. Grouping and administration regimens are shown in Table 12.

Table 12. Grouping and administration regimens

| Group | Number of animals | Administration group | Dose (mg/kg) | Mode of administration | Administration period |
|---|---|---|---|---|---|
| 1 | 8 | Vehicle control (normal saline) | -- | i.v. | Single administration |
| 2 | 8 | ADC2 | 5 | i.v. | Single administration |

(continued)

| Group | Number of animals | Administration group | Dose (mg/kg) | Mode of administration | Administration period |
|---|---|---|---|---|---|
| 3 | 8 | ADC1 | 2.5 | i.v. | Single administration |

Tumor tissues were collected from tumor-bearing mice of HuPrime® ovarian cancer xenograft model OV3756, cut into tumor masses of a diameter of 2-3 mm and then subcutaneously inoculated into the right anterior scapula of the female BALB/c nude mice aged 6-7 weeks.

When the mean tumor volume of the tumor-bearing mice reached about 137.55 $mm^3$, the mice were randomly grouped for administration. The day of grouping was defined as Day 0 and the administration was started on Day 0.

After tumor grafting, routine monitoring included the effect of tumor growth and treatment on the normal behavior of the animals, specifically the activity, food and water intake, weight gain or loss (the weight was measured twice a week), the eyes, the coat and other abnormal conditions. The calculation formula for tumor volume: Tumor volume ($mm^3$) = $1/2 \times (a \times b^2)$ (where a represents long diameter and b represents short diameter).

Table 13. Efficacy analysis for each group in the xenograft models of OV3756

| Group | Mean tumor volume (Day 0)[a] | Mean tumor volume (Day 28)[a] | TGI (%)[b] | P Value (relative to control group)[c] |
|---|---|---|---|---|
| 1 | 137.46±9.66 | 1198.78±115.80 | - | - |
| 2 | 137.46±9.84 | 439.00±149.55 | 71.59 | 1.15e-04*** |
| 3 | 137.62±11.18 | 277.59±68.82 | 86.81 | 3.59e-06*** |
| Note: a. Data were expressed as "mean±standard error";<br>b. TGI% = [1 - (Ti - T0)/(Ci - CO)] × 100, where T0 and C0 are the mean tumor volumes of the administration group and vehicle control group on the day of grouping (Day 0), respectively, and Ti and Ci are the mean tumor volumes of the administration group and vehicle control group on Day 25, respectively;<br>c. **P* < 0.05, ***P* < 0.01 and ****P* < 0.001 compared to the tumor volume of the vehicle control group. | | | | |

The tumor growth of each treatment group and the control group of the OV3756 xenograft models is shown in Table 13 and FIG. 4. As can be seen from Table 13 and FIG. 4, ADC1 and ADC2 could significantly inhibit the tumor growth in the OV3756 xenograft models.

2) The research on the *in vivo* anti-tumor therapeutic effect of the experimental drugs in the Balb/c nude mouse JEG-3 subcutaneous model aims to investigate the *in vivo* efficacy of the ADC1 in the JeG-3 model. Grouping and administration regimens are shown in Table 14.

Table 14. Grouping and administration regimens

| Group | Administration group | Administration volume (μL/g) | Dose (mg/kg) | Mode of administration | Administration period | Number of animals | Treatment time |
|---|---|---|---|---|---|---|---|
| 1 | Blank control (Normal saline) | 10 μL/g | - | i.v. | Single administration | 8 | Day 28 |
| 2 | Exatecan Mesylate | 10 μL/g | 0.136 | i.v. | Single administration | 8 | Day 28 |
| 3 | ADC1 | 10 μL/g | 2.5 | i.v. | Single administration | 8 | Day 28 |
| 4 | ADC1 | 10 μL/g | 5 | i.v. | Single administration | 8 | Day 28 |

Female Balb/c nude mice aged 6-8 weeks were selected, and $1 \times 10^6$ JEG-3 cells were suspended in 100 μL of an EMEM medium containing 50% matrigel, and inoculated subcutaneously on the right side of the mice. When the mean tumor volume reached about 118 $mm^3$, the mice were randomly grouped (with 8 in each group) according to the body weight and tumor volume of the animal and administered.

After the administration was started, the tumor volume was measured twice weekly. The calculation formula for tumor volume is as follows: Tumor volume ($mm^3$) = 0.5a × $b^2$ (where a represents long diameter of tumor and b represents short diameter of tumor).

The results are shown in FIG. 5 and show that the treatment with ADC1 had a significant tumor inhibitory effect and was dose dependent ($p < 0.001$) compared to the control group. On Day 8 after administration, the TGI was 98.98% (2.5 mg/kg) and 100.00% (5 mg/kg). It is noted that ADC1 caused complete tumor regression, which was maintained to the end of the experiment.

3) Pharmacodynamic evaluation of experimental drug in subcutaneous xenograft NOD/SCID mouse models of HuPrime® lung cancer LU11554

Tumor tissues were collected from tumor-bearing mice of HuPrime® lung cancer xenograft model LU11554, cut into tumor masses of a diameter of 2-3 mm and then subcutaneously inoculated into the right anterior scapula of the NOD/SCID mice.

When the mean tumor volume of the tumor-bearing mice reached about 165.15 $mm^3$, the mice were randomly grouped according to Table 15. The day of grouping was defined as Day 0 and the administration was started on Day 0. The administration method, administration dose, and route of administration are detailed in Table 15.

Table 15. Route, dose and regimen of administration in subcutaneous xenograft models of HuPrime® lung cancer LU11554

| Group | Number of animals | Administration group | Dose (mg/kg) | Mode of administration | Administration volume (μL/g) | Administration period |
|---|---|---|---|---|---|---|
| 1 | 8 | Vehicle control | -- | *i.v.* | 10 | Day 0, Day 14 |
| 2 | 8 | ADC1 (batch 2, with a DAR of 7.4) | 1 | *i.v.* | 10 | Day 0, Day 14 |
| 3 | 8 | ADC1 (batch 2, with a DAR of 7.4) | 3 | *i.v.* | 10 | Day 0, Day 14 |
| 4 | 8 | ADC1 (batch 2, with a DAR of 7.4) | 5 | *i.v.* | 10 | Day 0, Day 14 |
| Note: The day of grouping was defined as Day 0 and the administration was started on Day 0. After tumor grafting, routine monitoring included the effect of tumor growth and treatment on the normal behavior of the animals, specifically the activity, food and water intake, weight gain or loss (the weight was measured twice a week), the eyes, the coat and other abnormal conditions. The calculation formula for tumor volume: Tumor volume ($mm^3$) = 1/2 × (a × $b^2$) (where a represents long diameter and b represents short diameter). Mice were euthanized when their body weight decreased by more than 20% once. | | | | | | |

The tumor growth of each treatment group and the control group of the LU11554 xenograft models is shown in Table 16 and FIG. 6.

Table 16. Efficacy analysis for each group in xenograft models of LU11554 (tumor volume

| Experimental group | Mean tumor volume (Day 0)[a] | Mean tumor volume (Day 24)[a] | TGI (%)[b] | *P* Value (relative to control group)[c] |
|---|---|---|---|---|
| Group 1 | 165.18±10.70 | 1189.51±73.14 | - | - |
| Group 2 | 164.96±10.73 | 1172.94±136.42 | 1.60 | 9.99e-01$^{ns}$ |
| Group 3 | 165.30±10.66 | 812.00±84.87 | 36.87 | 4.76e-02* |
| Group 4 | 165.16±11.19 | 672.27±75.32 | 50.49 | 4.40e-03** |
| Note: a. Data were expressed as "mean±standard error";<br>b. TGI% = [1 - (Ti - T0)/(Ci - CO)] × 100, where T0 and C0 are the mean tumor volumes of the administration group and vehicle control group on the day of grouping (Day 0), respectively, and Ti and Ci are the mean tumor volumes of the administration group and vehicle control group on Day 24, respectively;<br>c. $^{ns}P > 0.05$ $*P < 0.05$, $**P < 0.01$ and $***P < 0.001$ compared to the tumor volume of the vehicle control group. | | | | |

The tumor volume was used as an index, and the mean tumor volume of the control group (Group 1) in this

model reached 1189.51 mm$^3$ 24 days after administration. After 24 days of administration of 1 mg/kg, 3 mg/kg and 5 mg/kg ADC1 (i.e., Group 2, Group 3 and Group 4), the mean tumor volumes were 1172.94 mm$^3$, 812.00 mm$^3$ and 672.27 mm$^3$, respectively, and the relative tumor growth inhibition TGI were 1.60% (*P* = 9.99e-01$^{ns}$), 36.87% (*P* = 4.76e-02*) and 50.49% (*P* = 4.40e-03**), respectively.

4) Pharmacodynamic evaluation of experimental drug in subcutaneous xenograft NOD/SCID mouse models of HuPrime® lung cancer LU5197

Tumor tissues were collected from tumor-bearing mice of HuPrime® lung cancer xenograft model LU5197, cut into tumor masses of a diameter of 2-3 mm and then subcutaneously inoculated into the right anterior scapula of the NOD/SCID mice.

When the mean tumor volume of the tumor-bearing mice reached about 146.50 mm$^3$, the mice were randomly grouped according to Table 17. The day of grouping was defined as Day 0 and the administration was started on Day 0. The administration method, administration dose, and route of administration are detailed in Table 17.

Table 17. Route, dose and regimen of administration in subcutaneous xenograft models of HuPrime® lung cancer LU5197

| Group | Number of animals | Administration group | Dose (mg/kg) | Mode of administration | Administration volume (μL/g) | Administration period |
|---|---|---|---|---|---|---|
| 1 | 8 | Vehicle control | -- | *i.v.* | 10 | Day 0, Day 14 |
| 2 | 8 | ADC1 (batch 2, with a DAR of 7.4) | 1 | *i.v.* | 10 | Day 0, Day 14 |
| 3 | 8 | ADC1 (batch 2, with a DAR of 7.4) | 3 | *i.v.* | 10 | Day 0, Day 14 |
| 4 | 8 | ADC1 (batch 2, with a DAR of 7.4) | 5 | *i.v.* | 10 | Day 0, Day 14 |
| Note: The day of grouping was defined as Day 0 and the administration was started on Day 0. After tumor grafting, routine monitoring included the effect of tumor growth and treatment on the normal behavior of the animals, specifically the activity, food and water intake, weight gain or loss (the weight was measured twice a week), the eyes, the coat and other abnormal conditions. The calculation formula for tumor volume: Tumor volume (mm$^3$) = 1/2 × (a × b$^2$) (where a represents long diameter and b represents short diameter). Mice were euthanized when their body weight decreased by more than 20% once. | | | | | | |

The tumor growth of each treatment group and the control group of the LU5197 xenograft models is shown in Table 18 and FIG. 7.

Table 18. Efficacy analysis for each group in xenograft models of LU5197 (tumor volume)

| Experimental group | Mean tumor volume (Day 0)$^a$ | Mean tumor volume (Day 28)$^a$ | TGI (%)$^b$ | *P* Value (relative to control group)$^c$ |
|---|---|---|---|---|
| Group 1 | 146.51±4.99 | 595.89±36.72 | - | - |
| Group 2 | 146.57±4.90 | 541.48±96.09 | 12.12 | 4.56e-01$^{ns}$ |
| Group 3 | 146.50±4.48 | 277.93±22.54 | 70.75 | 6.62e-05*** |
| Group 4 | 146.45±4.92 | 204.69±21.79 | 87.04 | 1.33e-07*** |
| Note: a. Data were expressed as "mean±standard error";<br>b. TGI% = [1 - (Ti - T0)/(Ci - CO)] × 100, where T0 and C0 are the mean tumor volumes of the administration group and vehicle control group on the day of grouping (Day 0), respectively, and Ti and Ci are the mean tumor volumes of the administration group and vehicle control group on Day 28, respectively; c. $^{ns}$*P* > 0.05 **P* < 0.05, ***P* < 0.01 and ****P* < 0.001 compared to the tumor volume of the vehicle control group. | | | | |

The tumor volume was used as an index, and the mean tumor volume of the control group (Group 1) in this model reached 595.89 mm$^3$ 28 days after administration. After 28 days of administration of 1 mg/kg, 3 mg/kg and 5 mg/kg ADC1 (i.e., Group 2, Group 3 and Group 4), the mean tumor volumes were 541.48 mm$^3$, 277.93 mm$^3$ and 204.69 mm$^3$, respectively, and the relative tumor growth inhibition TGI were 12.12% (*P* = 4.56e-01$^{ns}$), 70.75% (*P* = 6.62e-05***)

and 87.04% (*P* = 1.33e-07***), respectively.

**Example 16:** Pharmacokinetic Study

After cynomolgus monkeys were observed and given ADC1 for injection or Exatecan Mesylate by a single intravenous infusion, blood samples were collected at different time points. The analyte ADC1 and total antibody concentrations in the serum were determined by ELISA assay, the analyte Exatecan concentration in the plasma was determined by LC-MS/MS assay, and the major pharmacokinetic parameters were calculated. 24 common cynomolgus monkeys aged 3-5 years (half male and half female) were adopted and randomly divided into 4 groups, and each group consisted of 3 female animals and 3 male animals. Animals in each group were not fasted and given 1 mg/kg, 3 mg/kg and 10 mg/kg of ADC1 for injection and 0.25 mg/kg of Exatecan Mesylate by a single intravenous infusion, respectively. Blood samples were collected until 504 hours after the end of the administration. The major pharmacokinetic parameters, i.e., $AUC_{(0-t)}$, $AUC_{(0-\infty)}$, $T_{1/2}$, $C_{max}$, $T_{max}$, CL, MRT, etc., were calculated from the plasma concentration data with a non-compartmental model using Phoenix WinNonlin® 7.0 software. The results are shown in Tables 19, 20 and 21.

Table 19. Major pharmacokinetic parameters of ADC1 in serum after giving ADC1 for injection or Exatecan Mesylate to cynomolgus monkeys by single intravenous infusion (Mean ± SD)

| Group | Test sample | Route of administration | Administration dose | Sex of animals | $T_{1/2}$ | $T_{max}$ | $C_{max}$ | $AUC_{(0-t)}$ | $AUC_{(0-\infty)}$ | Vd | Cl | $MRT_{(0-t)}$ | $MRT_{(0-\infty)}$ |
|---|---|---|---|---|---|---|---|---|---|---|---|---|---|
| | | | mg/kg | | h | h | ng/mL | h*ng/mL | h*ng/mL | mL/kg | mL/h/kg | h | h |
| G1 | ADC1 for injection | Intravenous infusion for 30 min | 1 | Male | 52.74 ± 14.91 | 2.389 ± 3.128 | 38183.94 ± 1543.66 | 1891367.17 ± 124655.81 | 1931462.33 ± 145905.21 | 39.79 ± 12.47 | 0.52± 0.04 | 50.33± 7.55 | 55.42± 7.32 |
| | | | | Female | 37.77 ± 6.33 | 2.194 ± 1.717 | 35521.65 ± 6293.82 | 1321591.40 ± 61070.44 | 1332246.74 ± 58284.73 | 41.11 ± 8.32 | 0.75± 0.03 | 37.55± 3.20 | 39.63± 3.98 |
| G2 | ADC1 for injection | Intravenous infusion for 30 min | 3 | Male | 24.34 ± 3.79 | 0.694 ± 0.268 | 120869.31 ± 2953.01 | 4143659.03 ± 563169.94 | 4151564.96 ± 564747.57 | 25.47 ± 3.53 | 0.73± 0.09 | 32.23± 3.07 | 32.63± 3.13 |
| | | | | Female | 61.79 ± 36.83 | 0.694 ± 0.268 | 123727.47 ±17904.88 | 4371589.25 ± 1244537.68 | 4384750.20 ± 1248070.85 | 57.48 ± 28.28 | 0.73± 0.23 | 41.56± 13.12 | 42.92± 13.82 |
| G3 | ADC1 for injection | Intravenous infusion for 30 min | 10 | Male | 58.28 ± 15.41 | 2.194 ± 1.717 | 447572.03 ±43879.24 | 17877194.14 ± 6796831.15 | 17917623.87 ± 6818357.91 | 49.71 ± 12.63 | 0.63± 0.30 | 49.89± 13.74 | 50.96± 14.09 |
| | | | | Female | 54.01 ± 22.55 | 2.194 ± 1.717 | 463717.61 ± 8601.50 | 14460166.60 ± 3855727.81 | 14527741.27 ± 3864992.91 | 52.72 ± 11.18 | 0.72± 0.17 | 43.03 ± 20.20 | 44.71 ± 20.51 |

Table 20. Major pharmacokinetic parameters of total antibody in serum after giving ADC1 for injection or Exatecan Mesylate to cynomolgus monkeys by single intravenous infusion (Mean ± SD)

| Group | Test sample | Route of administration | Administration dose | Sex of animals | $T_{1/2}$ | $T_{max}$ | $C_{max}$ | $AUC_{(0-t)}$ | $AUC_{(0-\infty)}$ | Vd | Cl | $MRT_{(0-t)}$ | $MRT_{(0-\infty)}$ |
|---|---|---|---|---|---|---|---|---|---|---|---|---|---|
| | | | mg/kg | | h | h | ng/mL | h*ng/mL | h*ng/mL | mL/kg | mL/h/kg | h | h |
| G1 | ADC1 for injection | Intravenous infusion for 30 min | 1 | Male | 39.77 ± 11.75 | 0.583 ± 0.000 | 35556.30 ± 1687.77 | 1344523.16 ± 249124.70 | 1359644.51 ± 247018.98 | 42.10 ± 8.58 | 0.75± 0.13 | 43.74± 5.34 | 46.39± 4.98 |
| | | | | Female | 31.67 ± 3.78 | 0.583 ± 0.000 | 36978.27 ± 3564.40 | 1075796.86 ± 57354.48 | 1084360.43 ± 61170.18 | 42.39 ± 709 | 0.92± 0.05 | 33.82± 3.72 | 35.50± 3.95 |
| G2 | ADC1 for injection | Intravenous infusion for 30 min | 3 | Male | 24.68 ± 2.97 | 0.528 ± 0.048 | 131341.11 ± 4347.65 | 3178467.95 ± 502689.99 | 3237368.77 ± 439991.51 | 33.40 ± 5.92 | 0.94± 0.12 | 27.80± 4.29 | 30.22± 1.80 |
| | | | | Female | 41.34 ± 19.35 | 0.528 ± 0.048 | 142109.12 ± 16423.52 | 3613156.17 ± 777608.21 | 3649757.11 ± 748382.38 | 46.98 ± 14.93 | 0.85± 0.20 | 33.83± 12.33 | 35.87± 11.91 |
| G3 | ADC1 for injection | Intravenous infusion for 30 min | 10 | Male | 52.58 ± 21.49 | 0.528 ± 0.048 | 493805.51 ± 47587.05 | 12978144.84 ± 3135076.27 | 13014384.96 ± 3160088.51 | 57.79 ± 15.34 | 0.81 ± 0.23 | 41.62 ± 9.36 | 42.72 ± 10.06 |
| | | | | Female | 50.74 ± 29.88 | 0.555 ± 0.048 | 502153.41 ± 31145.44 | 14228936.52 ± 7574517.87 | 14481257.10 ± 7550300.91 | 49.59 ± 9.89 | 0.80 ± 0.32 | 44.19 ± 33.90 | 48.17 ± 35.10 |

Table 21. Major pharmacokinetic parameters of Exatecan in plasma after giving ADC1 for injection or Exatecan Mesylate to cynomolgus monkeys by single intravenous infusion (Mean ± SD)

| Group | Test sample | Route of administration | Administration dose | Sex of animals | $T_{1/2}$ | $T_{max}$ | $C_{max}$ | $AUC_{(0-t)}$ | $AUC_{(0-\infty)}$ | Vd | Cl | $MRT_{(0-t)}$ | $MRT_{(0-\infty)}$ |
|---|---|---|---|---|---|---|---|---|---|---|---|---|---|
| | | | mg/kg | | h | h | ng/mL | h*ng/mL | h*ng/mL | L/kg | L/h/kg | h | h |
| G1 | ADC1 for injection | Intravenous infusion for 30 min | 1 | Male | NA | 1.00± 0.00 | 0.236± 0.068 | 1.49± 1.56 | NA | NA | NA | 4.24± 4.63 | NA |
| | | | | Female | NA | 1.00± 0.00 | 0.176± 0.057 | 0.87± 0.71 | NA | NA | NA | 3.16± 2.28 | NA |
| G2 | ADC1 for injection | Intravenous infusion for 30 min | 3 | Male | 38.28± 12.18 | 0.86± 0.24 | 0.881± 0.149 | 13.21± 3.53 | 22.39± 8.99 | 7741.37 ± 2114.55 | 148.85 ± 56.91 | 18.88± 1.38 | 52.64± 17.40 |
| | | | | Female | 89.69± 66.20 | 0.72± 0.24 | 0.727± 0.075 | 8.45± 2.96 | 38.18± 25.13 | 9654.34± 1713.58 | 104.74± 63.67 | 13.28± 5.01 | 127.14± 96.06 |
| G3 | ADC1 for injection | Intravenous infusion for 30 min | 10 | Male | 28.46± 9.66 | 0.72± 0.24 | 3.383± 0.328 | 44.53± 2.45 | 51.44± 0.10 | 7985.39 ± 2720.63 | 194.40 ± 0.38 | 22.86 ± 6.09 | 36.53± 13.94 |
| | | | | Female | 22.39± 1.42 | 0.72± 0.24 | 2.933± 0.730 | 65.42± 9.95 | 69.36± 10.45 | 4719.89 ± 717.86 | 146.34 ± 21.50 | 25.84± 1.20 | 31.65± 1.27 |
| G4 | Exatecan Mesylate | Intravenous infusion for 30 min | 0.25 | Male | 2.81± 0.01 | 0.50± 0.00 | 349.808± 69.543 | 231.77± 60.82 | 232.74± 60.95 | 4.55± 1.09 | 1.12± 0.27 | 0.46± 0.02 | 0.51± 0.03 |
| | | | | Female | 2.16± 0.68 | 0.50± 0.00 | 237.567 ± 29.481 | 155.05± 10.95 | 155.68 ± 11.13 | 5.07± 1.84 | 1.61± 0.12 | 0.39± 0.04 | 0.43± 0.04 |

NA: not applicable.

Within the dose range of 1-10 mg/kg, after male and female cynomolgus monkeys were given ADC1 for injection by a single intravenous infusion without fasting, the systemic exposure $AUC_{(0-t)}$ of ADC1 and total antibody in the serum and Exatecan in the plasma had no significant sex difference, and the systemic exposure increased along with the increase of the administration dose; after male and female cynomolgus monkeys were given 0.25 mg/kg of Exatecan Mesylate by a single intravenous infusion without fasting, the systemic exposure $AUC_{(0-t)}$ of Exatecan in the plasma had no significant sex difference.

When cynomolgus monkeys were given ADC1 for injection and Exatecan Mesylate at a dose ratio of 1:0.025 (the dose level of Exatecan Mesylate corresponding to 10 mg/kg of ADC1 for injection was about 0.25 mg/kg) by a single intravenous infusion without fasting, the ratio of Exatecan systemic exposure in the plasma of male and female animals was 1:5.20 and 1:2.37, respectively, indicating that less Exatecan is dissociated after administration of ADC1 for injection into the body.

**Example 17:** Clinical Study on ADC1

The study was a multicenter, open, dose-escalation and dose-extension phase I clinical study exploring the safety, tolerability and PK characteristics of ADC1 in patients with advanced solid tumors.

The study was divided into two parts:

First part: dose escalation study. The "3 + 3" dose escalation rule was used to explore the safety, tolerability and pharmacokinetic characteristics of ADC1.

A total of 5 dose groups were set, i.e., a 1.5 mg/kg group, a 3.0 mg/kg group, a 4.5 mg/kg group, a 6.5 mg/kg group and a 8.5 mg/kg group, and the dose escalation study was performed according to the standard "3 + 3" rule.

Second part: dose extension study. This study was performed to further explore the safety and clinical efficacy of ADC1 in the treatment of advanced solid tumors.

or

First part: dose escalation study. Rapid titration and the "3 + 3" dose escalation rule were used to explore the safety, tolerability and pharmacokinetic characteristics of ADC1.

A total of 6 dose groups were set, i.e., a 1.2 mg/kg group, a 2.4 mg/kg group, a 3.5 mg/kg group, a 5.0 mg/kg group, a 6.5 mg/kg group and a 8.0 mg/kg group. Among these dose groups, the 1.2 mg/kg group was subjected to dose escalation using a rapid titration method; the 2.4 mg/kg group, 3.5 mg/kg group, 5.0 mg/kg group, 6.5 mg/kg group and 8.0 mg/kg group were subjected to dose escalation following the standard "3 + 3" rule.

Administration regimen: the administration was performed by intravenous infusion once every 3 weeks (Q3W), with an infusion time of ≥ 90 min being recommended for the first week, and subsequent cycles could be accomplished within 30 min if no infusion reaction occurred.

Definition of dose-limiting toxicity (DLT):

AEs were graded according to National Cancer Institute Common Terminology Criteria for Adverse Events (NCI CTCAE), version 5.0. DLT refers to any grade 3 or higher toxicity related to a test drug that occurs within 21 days after a subject is first given the drug and meets the following definitions:

■ definition of hepatotoxicity DLT:

·Grade 4 AST or ALT elevation
·subjects with hepatocellular carcinoma or liver metastasis, e.g., AST or ALT ≤ 3-fold ULN at baseline, AST or ALT elevation > 5-fold ULN in a DLT evaluation period and lasting > 7 days
·subjects with hepatocellular carcinoma or liver metastasis, e.g., AST or ALT > 3-fold ULN at baseline, AST or ALT elevation > 8-fold ULN in a DLT evaluation period and lasting > 7 days
·Subjects without liver metastases, AST or ALT elevation > 5-fold ULN and lasting > 7 days
·AST or ALT > 5-fold upper limit of normal (ULN), with ≥ Grade 2 blood bilirubin elevation

■ definition of hematological toxicity DLT:

·Grade 4 neutropenia lasting > 7 days
·≥ Grade 3 neutropenia with fever

·Grade 4 anemia
·Grade 4 thrombocytopenia
·$\geq$ Grade 3 thrombocytopenia lasting > 7 days
·$\geq$ Grade 3 thrombocytopenia with bleeding
·Grade 4 lymphopenia lasting for $\geq$ 14 days

■ definition of non-hepatotoxicity and non-hematological toxicity DLT:

·Other $\geq$ Grade 3 non-hepatotoxicity and non-hematological toxicity

■ the following TEAEs are not considered DLT:

·Grade 3 fatigue lasting < 7 days
·Grade 3 nausea, vomiting, diarrhea or anorexia being relieved to $\leq$ Grade 2 within 3 days
·abnormalities in laboratory examinations without clinically relevant symptoms or signs, including elevated ALP, elevated uric acid, elevated blood amylase or elevated lipase to Grade 3 or 4, with Grade 1 hyponatremia at baseline escalating to Grade 3 but lasting < 72 hours
·Grade 3 lymphopenia

Evaluation of tolerability and safety:

Tolerability evaluation indexes: dose-limiting toxicity (DLT) events and their incidence.
Safety evaluation indexes: vital signs, physical examination, laboratory examination (blood routine examination, blood biochemistry, cardiac function, thyroid function, coagulation routine, urinalysis routine, stool routine, and pregnancy test), ECOG score, electrocardiogram, adverse events (including immune-related adverse events), and the like.

Pharmacokinetic evaluation:

Subjects in all dose groups were required for blood sample collection at specified time points during treatment (first 6 treatment cycles), and the plasma concentrations (Ctrough) were monitored for 2 h before administration. Concentration levels of ADC1, Exatecan and total antibody in the serum were examined to investigate pharmacokinetic (PK) characteristics of ADC1.
The following PK parameters were calculated from the actual administration doses and actual sampling time by the non-compartmental model:
Single administration: $C_{max}$, $T_{max}$, $T_{1/2}$, CL, Vd, Ke, MRT, $AUC_{(0-\tau)}$, and $AUC_{(0-\infty)}$;
Multiple administrations: $C_{max, ss}$, $C_{avg, ss}$, $C_{min, ss}$, $AUC_{(0-\tau)ss}$, $AUC_{(0-\infty)ss}$, $T_{max, ss}$, $T_{1/2, ss}$, CL, $V_{ss}$, Ke, MRT, accumulation index ($R_{ac}$), and fluctuation index DF.

Immunogenicity evaluation:

Subjects in all dose groups were required for blood sample collection at specified time points during treatment to detect anti-drug and neutralizing antibodies in the serum.
The immunogenicity evaluation indexes are as follows:

sample positive rate and individual positivity rate for anti-ADC1 antibody (ADA);
whether ADA-positive samples will continue to be detected for a neutralizing antibody (Nab); and
number and percentage of ADA antibody- and Nab-positive subjects.

Pharmacodynamic evaluation:

1. Detect the level of FR$\alpha$ in the serum prior to administration;
2. Archive tumor tissue samples or tumor tissue samples freshly collected during the screening phase for folate receptor expression levels.

Clinical efficacy evaluation:

Objective response rate (ORR), duration of response (DOR), disease control rate (DCR), progression-free survival (PFS), and overall survival (OS) as defined by RECIST 1. 1.

Table 22. Clinical data (immature, administration of ADC1, batch 2)

| Dose | Type of cancer | Duration of treatment | Drug-related adverse events | Expression of FRα | Efficacy evaluation |
|---|---|---|---|---|---|
| 1.2 mg/kg | Breast cancer | 2 cycles | - | NA | SD (-12%) |
| 2.4 mg/kg | Ovarian cancer | 4 cycles* | Grade 4 leukopenia<br>Grade 4 neutropenia<br>Grade 3 thrombocytopenia<br>Grade 3 anemia | 3+ | PR (-64.5%) |
| | Ovarian cancer | 2 cycles* | Grade 3 leukopenia<br>Grade 4 neutropenia<br>Grade 4 thrombocytopenia (DLT) | 3+ | PR (-40.7%) |
| | Ovarian cancer | 1 cycle* | Grade 1 thrombocytopenia | 3+ | |

*Patient continued to receive treatment after the received treatment cycles.

## Claims

1. An antibody or an antigen-binding unit thereof, wherein the antibody or the antigen-binding unit thereof specifically binds to folate receptor α and comprises one or more of (a)-(f):

(a) a VH CDR1 comprising an amino acid sequence set forth in SEQ ID NO: 5;
(b) a VH CDR2 comprising an amino acid sequence set forth in SEQ ID NO: 6;
(c) a VH CDR3 comprising an amino acid sequence set forth in SEQ ID NO: 7;
(d) a VL CDR1 comprising an amino acid sequence set forth in SEQ ID NO: 8;
(e) a VL CDR2 comprising an amino acid sequence set forth in SEQ ID NO: 9; and
(f) a VL CDR3 comprising an amino acid sequence set forth in SEQ ID NO: 10; optionally, comprises one or more of (g)-(n):
(g) a VH FR1 comprising an amino acid sequence set forth in SEQ ID NO: 1 or SEQ ID NO: 29, or an amino acid sequence having at least 90% sequence identity compared with the amino acid sequence set forth in SEQ ID NO: 1 or SEQ ID NO: 29, or an amino acid sequence having substitutions, deletions or insertions at one or more sites compared with the amino acid sequence set forth in SEQ ID NO: 1 or SEQ ID NO: 29;
(h) a VH FR2 comprising an amino acid sequence set forth in SEQ ID NO: 2 or SEQ ID NO: 30, or an amino acid sequence having at least 90% sequence identity compared with the amino acid sequence set forth in SEQ ID NO: 2 or SEQ ID NO: 30, or an amino acid sequence having substitutions, deletions or insertions at one or more sites compared with the amino acid sequence set forth in SEQ ID NO: 2 or SEQ ID NO: 30;
(i) a VH FR3 comprising an amino acid sequence set forth in SEQ ID NO: 3 or SEQ ID NO: 31, or an amino acid sequence having at least 90% sequence identity compared with the amino acid sequence set forth in SEQ ID NO: 3 or SEQ ID NO: 31, or an amino acid sequence having substitutions, deletions or insertions at one or more sites compared with the amino acid sequence set forth in SEQ ID NO: 3 or SEQ ID NO: 31;
(j) a VH FR4 comprising an amino acid sequence set forth in SEQ ID NO: 11, or an amino acid sequence having at least 90% sequence identity compared with the amino acid sequence set forth in SEQ ID NO: 11, or an amino acid sequence having substitutions, deletions or insertions at one or more sites compared with the amino acid sequence set forth in SEQ ID NO: 11;
(k) a VL FR1 comprising an amino acid sequence set forth in SEQ ID NO: 12, or an amino acid sequence having at least 90% sequence identity compared with the amino acid sequence set forth in SEQ ID NO: 12, or an amino acid sequence having substitutions, deletions or insertions at one or more sites compared with the amino acid sequence set forth in SEQ ID NO: 12;
(l) a VL FR2 comprising an amino acid sequence set forth in SEQ ID NO: 13, or an amino acid sequence having at least 90% sequence identity compared with the amino acid sequence set forth in SEQ ID NO: 13, or an amino

acid sequence having substitutions, deletions or insertions at one or more sites compared with the amino acid sequence set forth in SEQ ID NO: 13;
(m) a VL FR3 comprising an amino acid sequence set forth in SEQ ID NO: 4 or SEQ ID NO: 32, or an amino acid sequence having at least 90% sequence identity compared with the amino acid sequence set forth in SEQ ID NO: 4 or SEQ ID NO: 32, or an amino acid sequence having substitutions, deletions or insertions at one or more sites compared with the amino acid sequence set forth in SEQ ID NO: 4 or SEQ ID NO: 32; and
(n) a VL FR4 comprising an amino acid sequence set forth in SEQ ID NO: 14, or an amino acid sequence having at least 90% sequence identity compared with the amino acid sequence set forth in SEQ ID NO: 14, or an amino acid sequence having substitutions, deletions or insertions at one or more sites compared with the amino acid sequence set forth in SEQ ID NO: 14.

**2.** The antibody or the antigen-binding unit thereof according to claim 1, wherein the antibody or the antigen-binding unit thereof comprises a VH CDR1 set forth in SEQ ID NO: 5, a VH CDR2 set forth in SEQ ID NO: 6 and a VH CDR3 set forth in SEQ ID NO: 7.

**3.** The antibody or the antigen-binding unit thereof according to claim 1, wherein the antibody or the antigen-binding unit thereof comprises a VL CDR1 set forth in SEQ ID NO: 8, a VL CDR2 set forth in SEQ ID NO: 9 and a VL CDR3 set forth in SEQ ID NO: 10.

**4.** The antibody or the antigen-binding unit thereof according to any one of claims 1-3, wherein the antibody or the antigen-binding unit thereof comprises the VH CDR1 set forth in SEQ ID NO: 5, the VH CDR2 set forth in SEQ ID NO: 6, the VH CDR3 set forth in SEQ ID NO: 7, the VL CDR1 set forth in SEQ ID NO: 8, the VL CDR2 set forth in SEQ ID NO: 9 and the VL CDR3 set forth in SEQ ID NO: 10.

**5.** An antibody or an antigen-binding unit thereof, wherein the antibody or the antigen-binding unit thereof specifically binds to folate receptor $\alpha$ and comprises a heavy chain variable region and a light chain variable region, wherein:

the heavy chain variable region comprises an amino acid sequence set forth in SEQ ID NO: 15, or an amino acid sequence having at least 90% sequence identity compared with the amino acid sequence set forth in SEQ ID NO: 15, or an amino acid sequence having substitutions, deletions or insertions at one or more sites compared with the amino acid sequence set forth in SEQ ID NO: 15; and/or
the light chain variable region comprises an amino acid sequence set forth in SEQ ID NO: 16, or an amino acid sequence having at least 90% sequence identity compared with the amino acid sequence set forth in SEQ ID NO: 16, or an amino acid sequence having substitutions, deletions or insertions at one or more sites compared with the amino acid sequence set forth in SEQ ID NO: 16.

**6.** The antibody or the antigen-binding unit thereof according to any one of claims 1-5, wherein the antibody or the antigen-binding unit thereof is of an IgG isotype selected from IgG1 subtype, IgG2 subtype, IgG3 subtype or IgG4 subtype.

**7.** The antibody or the antigen-binding unit thereof according to any one of claims 1-5, wherein the antibody or the antigen-binding unit thereof further comprises a heavy chain constant region and a light chain constant region, wherein:

the heavy chain constant region comprises an amino acid sequence set forth in SEQ ID NO: 17, or an amino acid sequence having at least 90% sequence identity compared with the amino acid sequence set forth in SEQ ID NO: 17, or an amino acid sequence having substitutions, deletions or insertions at one or more sites compared with the amino acid sequence set forth in SEQ ID NO: 17; and/or
the light chain constant region comprises an amino acid sequence set forth in SEQ ID NO: 18, or an amino acid sequence having at least 90% sequence identity compared with the amino acid sequence set forth in SEQ ID NO: 18, or an amino acid sequence having substitutions, deletions or insertions at one or more sites compared with the amino acid sequence set forth in SEQ ID NO: 18.

**8.** An antibody or an antigen-binding unit thereof, wherein the antibody or the antigen-binding unit thereof specifically binds to folate receptor $\alpha$ and comprises a heavy chain and a light chain, wherein:

the heavy chain comprises an amino acid sequence set forth in SEQ ID NO: 19, or an amino acid sequence having at least 90% sequence identity compared with the amino acid sequence set forth in SEQ ID NO: 19, or

an amino acid sequence having substitutions, deletions or insertions at one or more sites compared with the amino acid sequence set forth in SEQ ID NO: 19; and/or the light chain comprises an amino acid sequence set forth in SEQ ID NO: 20, or an amino acid sequence having at least 90% sequence identity compared with the amino acid sequence set forth in SEQ ID NO: 20, or an amino acid sequence having substitutions, deletions or insertions at one or more sites compared with the amino acid sequence set forth in SEQ ID NO: 20.

**9.** The antibody or the antigen-binding unit thereof according to any one of claims 1-8, wherein the substitution is a conservative amino acid substitution.

**10.** A biomaterial, being

(1) a polynucleotide, wherein the polynucleotide encodes the antibody or the antigen-binding unit thereof according to any one of claims 1-9; or
(2) an expression vector, wherein the expression vector comprises a polynucleotide encoding the antibody or the antigen-binding unit thereof according to any one of claims 1-9; or
(3) a cell, wherein the cell comprises one or more polynucleotides encoding the antibody or the antigen-binding unit thereof according to any one of claims 1-9.

**11.** An antibody-drug conjugate comprising the antibody or the antigen-binding unit thereof according to any one of claims 1-9 conjugated to a drug via a linker, or a pharmaceutically acceptable salt or solvate thereof, wherein alternatively, the linker is a cleavable linker.

**12.** An antibody-drug conjugate having a structure shown as Formula I or a stereoisomer thereof, or a pharmaceutically acceptable salt or solvate thereof:

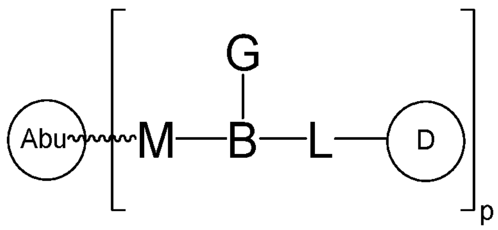

Formula I

wherein,

Abu is an antibody or an antigen-binding unit thereof that binds to folate receptor $\alpha$; alternatively, Abu is the antibody or the antigen-binding unit thereof according to any one of claims 1-9;
D is a drug;
M is

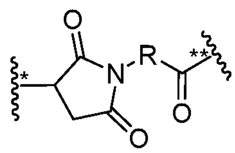

,

wherein * links to Abu, ** links to B, and R is selected from:$-(CH_2)_r-$, $-(CHR^m)_r-$, C3-C8 carbocyclyl, $-O-(CH_2)_r-$, arylene, $-(CH_2)_r$-arylene-, -arylene-$(CH_2)_r-$, $-(CH_2)_r$-(C3-C8 carbocyclyl)-, -(C3-C8 carbocyclyl)-$(CH_2)_r-$, C3-C8 heterocyclyl,$-(CH_2)_r$-(C3-C8 heterocyclyl)-, -(C3-C8 heterocyclyl)-$(CH_2)_r-$, $-(CH_2)_rC(O)NR^m(CH_2)_r-$,$-(CH_2CH_2O)_r-$, $-(CH_2CH_2O)_r$-CH2-, $-(CH_2)_rC(O)NR^m(CH_2CH_2O)_r-$,$-(CH_2)_rC(O)NR^m(CH_2CH_2O)_r-CH_2-$, $-(CH_2CH_2O)_rC(O)NR_m(CH_2CH_2O)_r-$,$-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10; or R is $-(CH_2)_r-$, or r is 1 or 5;

B is

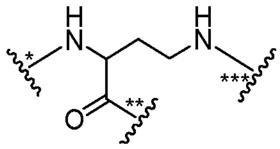 , or 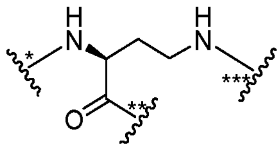 ;

wherein * links to M, ** links to L, and *** links to G;

L is -(AA)i-(FF)f-, wherein AA is an amino acid or polypeptide, and i is 1, 2, 3, 4, 5, 6, 7, 8, 9, 10, 11, 12, 13, 14, 15, 16, 17, 18, 19 or 20; or each AA is independently selected from the following amino acid or peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu and Gly-Phe-Leu-Gly; or AA is Val-Cit, and i is 1; each FF is independently

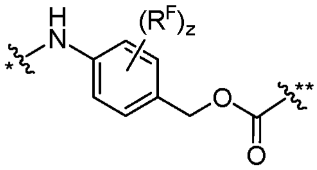 ,

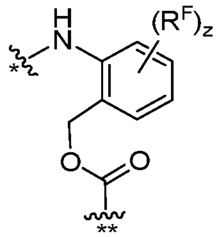 or 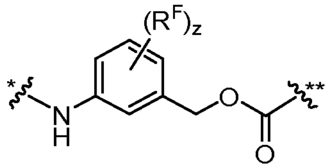 ,

wherein each $R^F$ is independently C1-C6 alkyl, C1-C6 alkoxy, $-NO_2$ or halogen; z is 0, 1, 2, 3 or 4, wherein * links to AA, and ** links to D; or each FF is independently

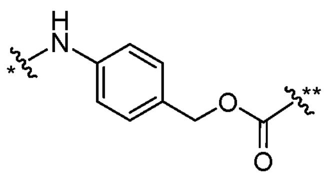 , 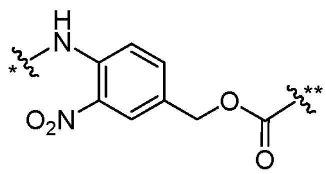 ,

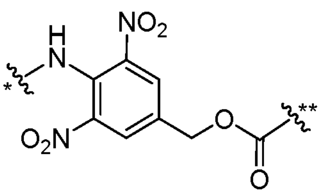 , 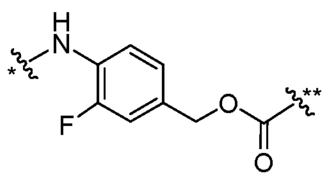 , 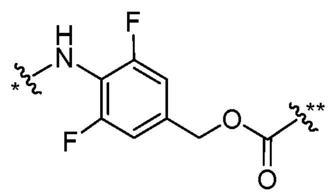 ,

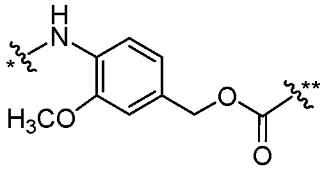 , 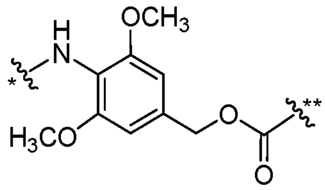 , 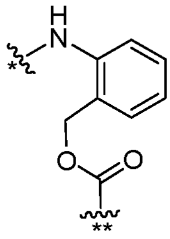 , 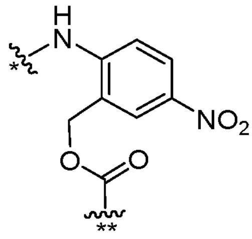 ,

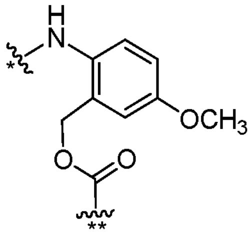 , 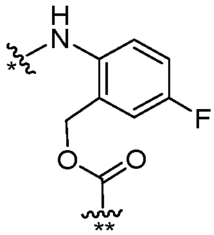 , 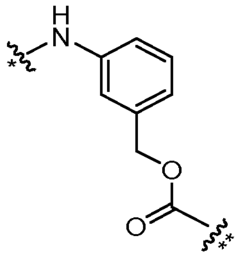 , 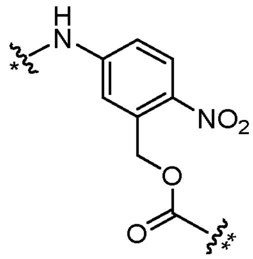 ,

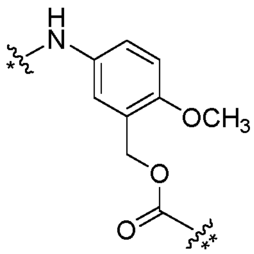 or 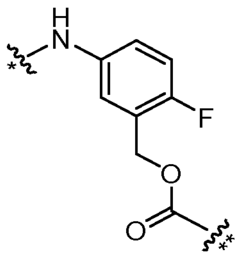 ;

f is 1, 2, 3, 4, 5, 6, 7, 8, 9 or 10, wherein * links to AA, and ** links to D; or FF is

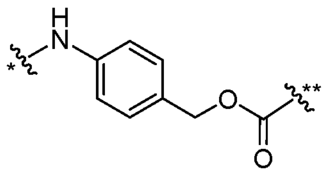 ,

and f is 1; wherein * links to AA, and ** links to D; or L is

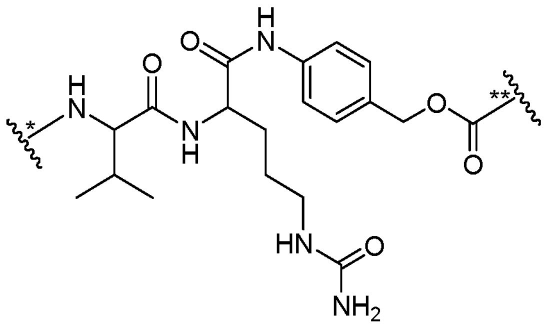 ,

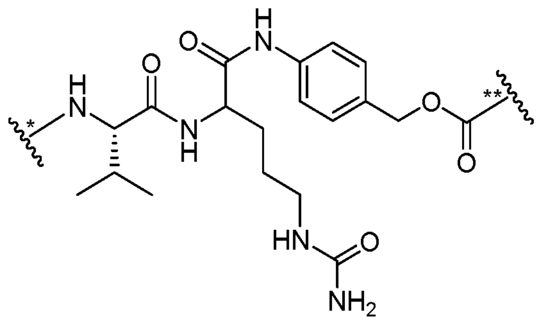

or

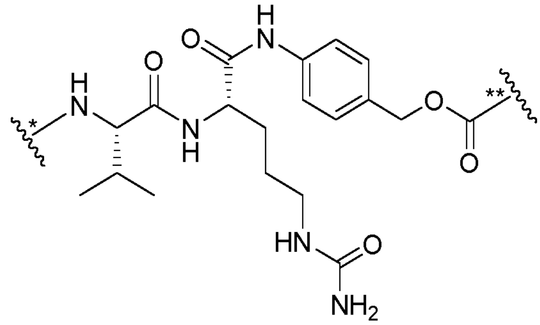

,

wherein * links to B, and ** links to D;
Gis

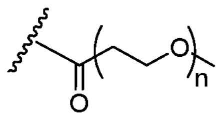

,

wherein n is 1-24; or n is 4-12; or n is 4-8; or n is 4 or 8;
p is 1-10; or p is 2-8; or p is 4-8; or p is 6-8; or p is 7-8; or p is 7.4; or p is 7; or p is 8.

**13.** An antibody-drug conjugate having a structure shown as Formula I-1, I-2, I-2-1, I-3, I-4, I-4-1, I-5, I-5-1, I-6, I-6-1, I-7, I-7-1, I-8, I-8-1, I-9, I-9-1, I-10, I-10-1, I-11 or I-11-1 or a stereoisomer thereof, or a pharmaceutically acceptable salt or solvate thereof, wherein the Formula 1-1 is:

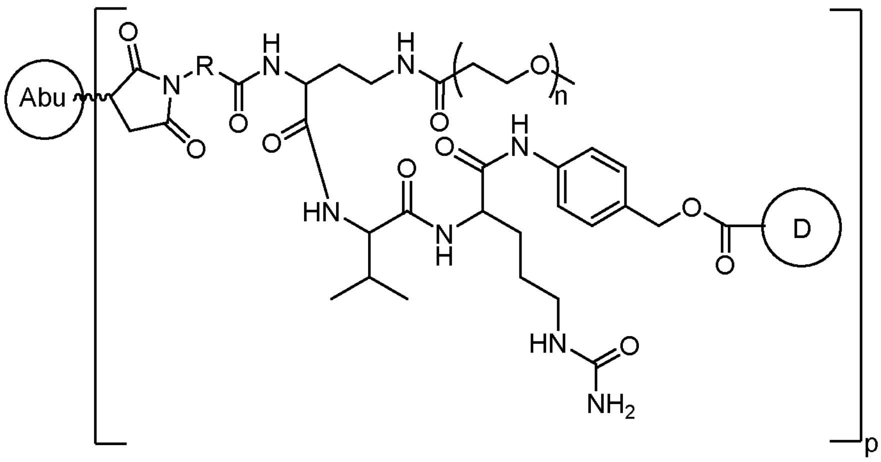

Formula I-1

the Formulas I-2 and I-2-1 are:

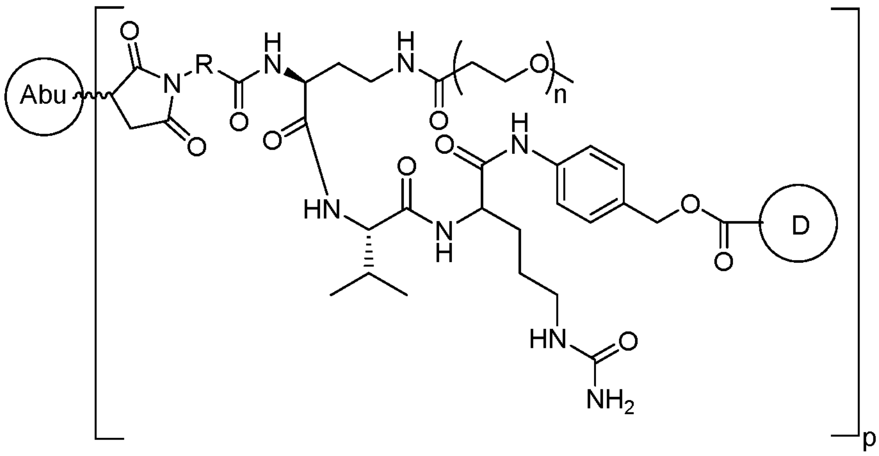

Formula I-2

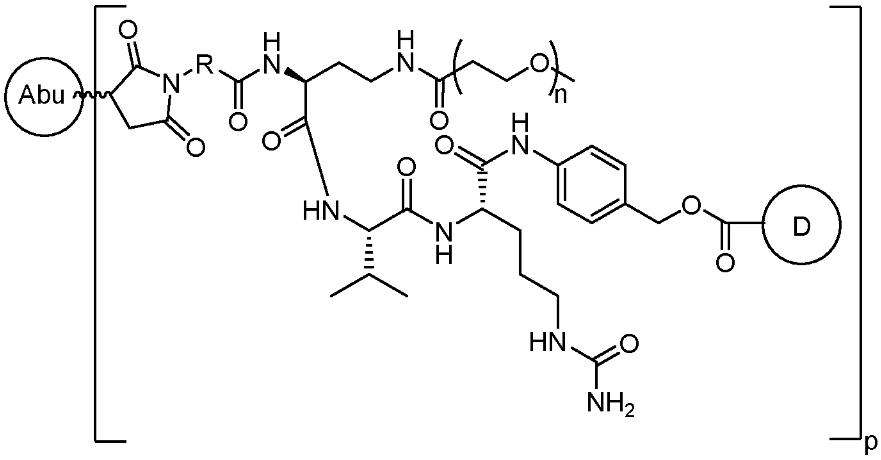

Formula I-2-1

the Formula I-3 is:

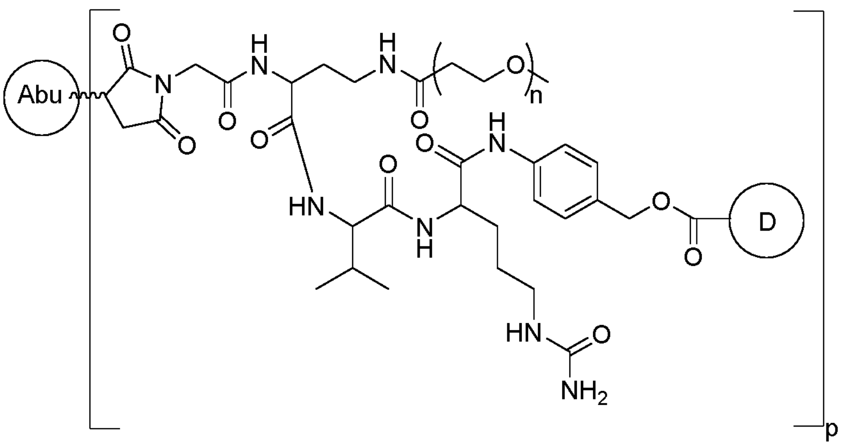

Formula I-3

the Formulas I-4 and I-4-1 are:

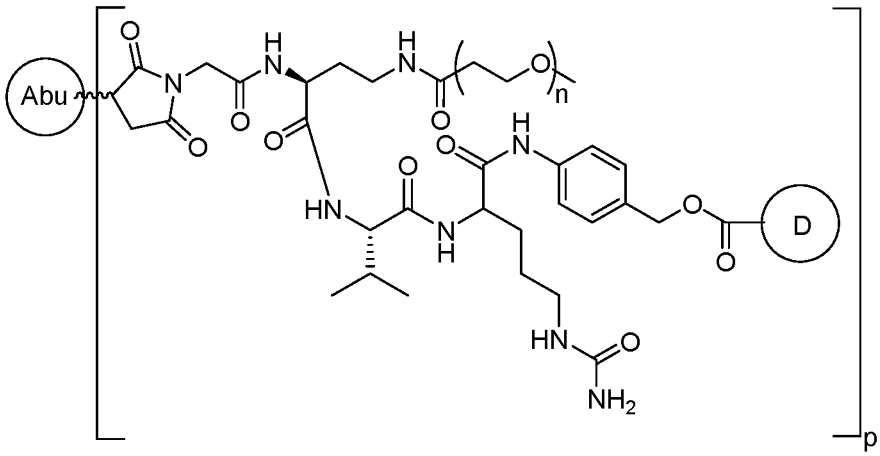

Formula I-4

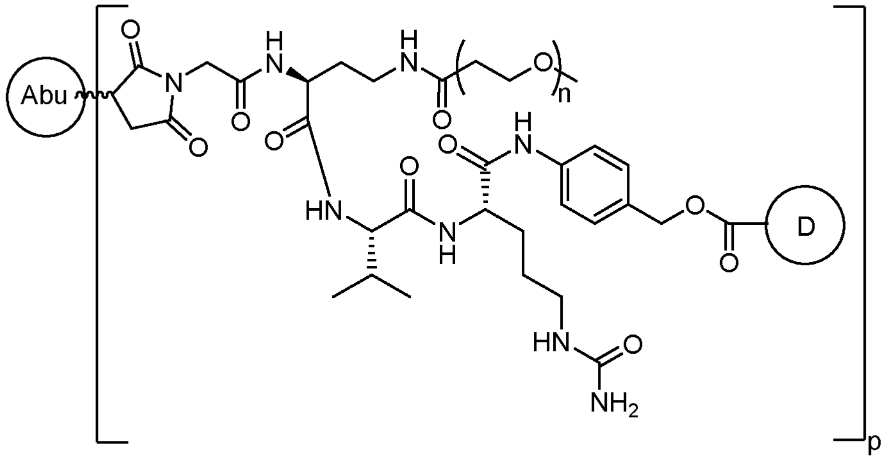

Formula I-4-1

the Formulas I-5 and I-5-1 are:

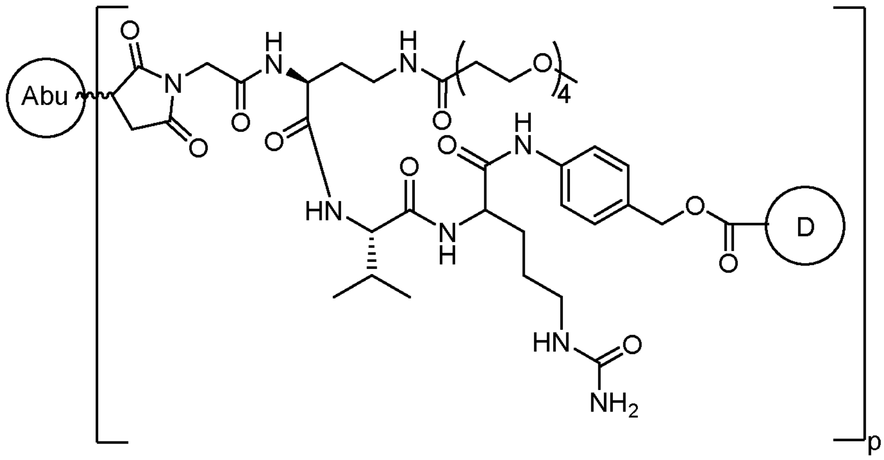

Formula I-5

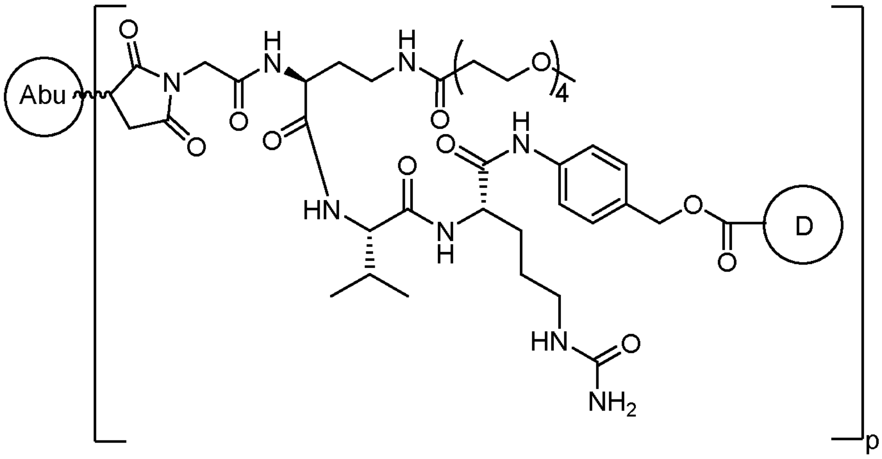

Formula I-5-1

the Formulas I-6 and I-6-1 are:

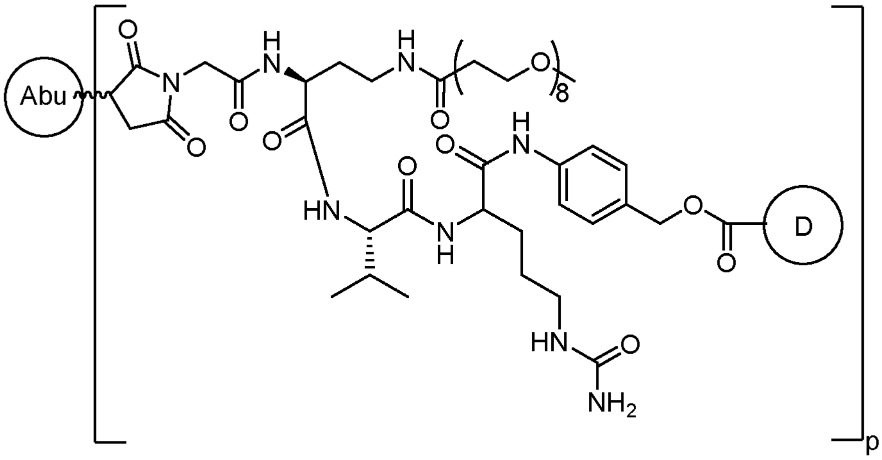

Formula I-6

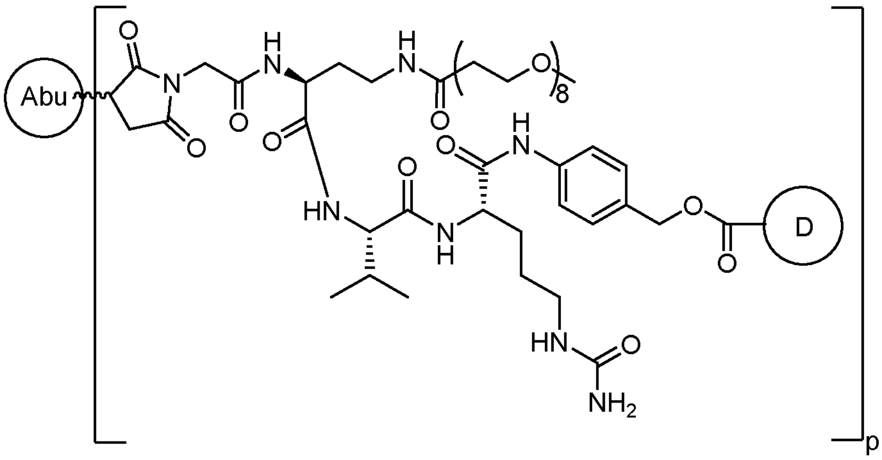

Formula I-6-1

the Formulas I-7 and I-7-1 are:

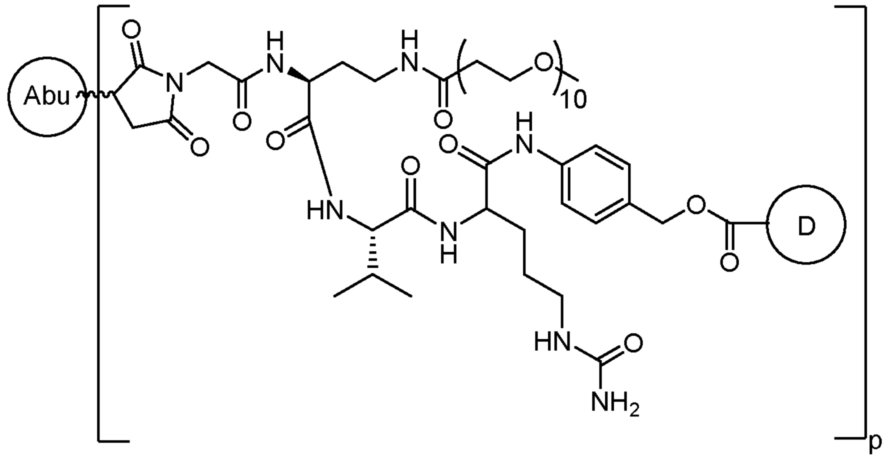

Formula I-7

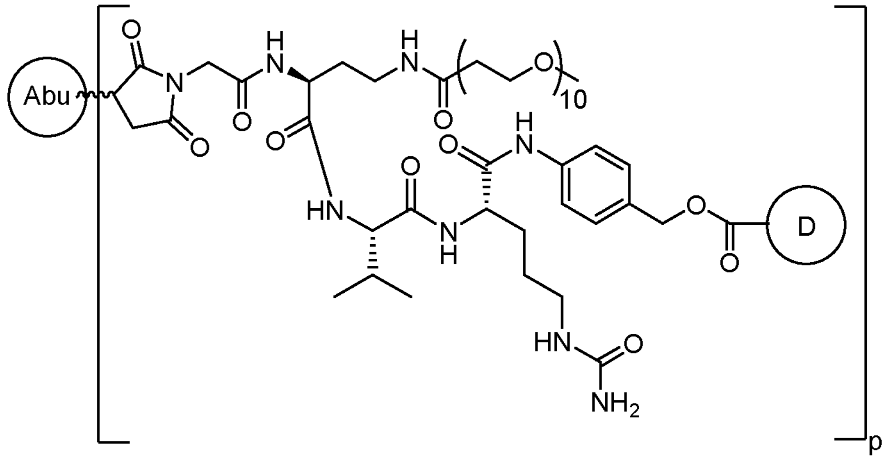

Formula I-7-1

the Formulas I-8 and I-8-1 are:

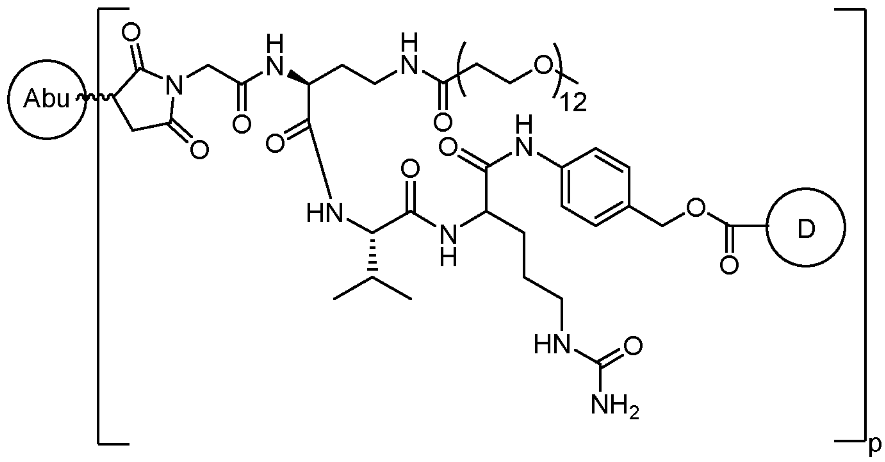

Formula I-8

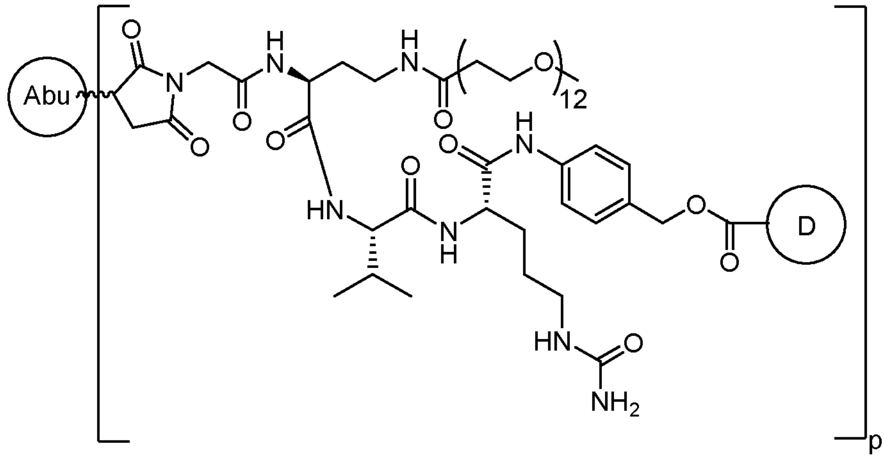

Formula I-8-1

the Formulas I-9 and I-9-1 are:

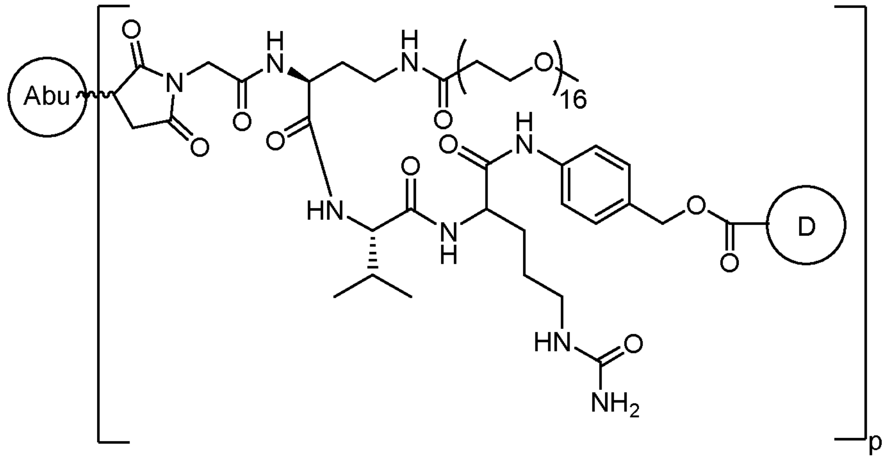

Formula I-9

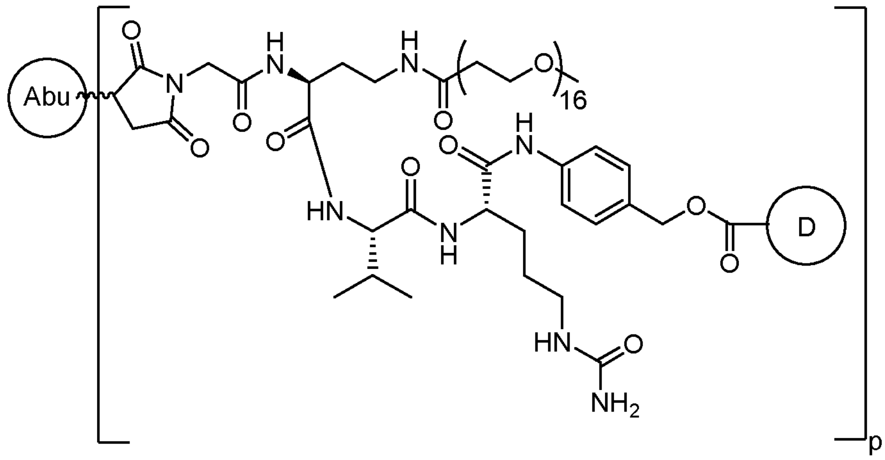

Formula I-9-1

the Formulas I-10 and I-10-1 are:

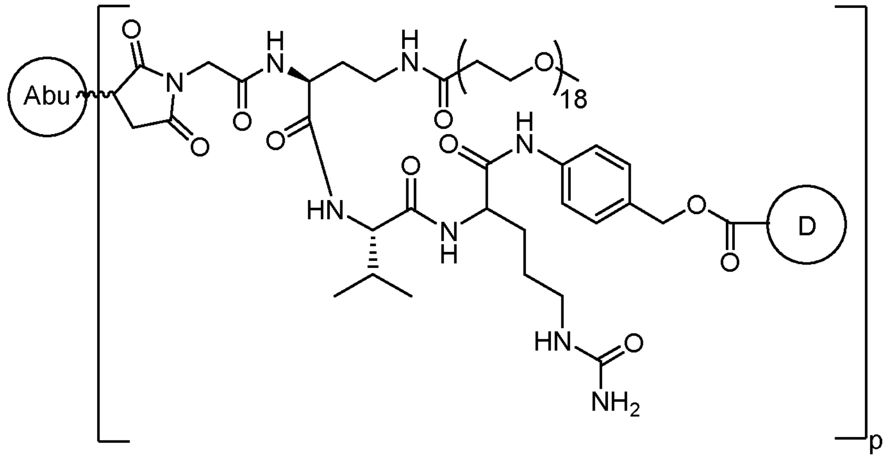

Formula I-10

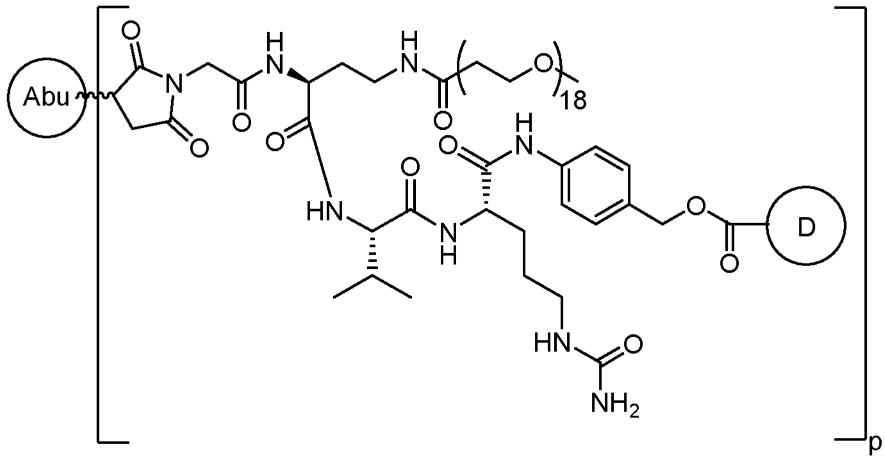

or

Formula I-10-1

the Formulas I-11 and I-11-1 are:

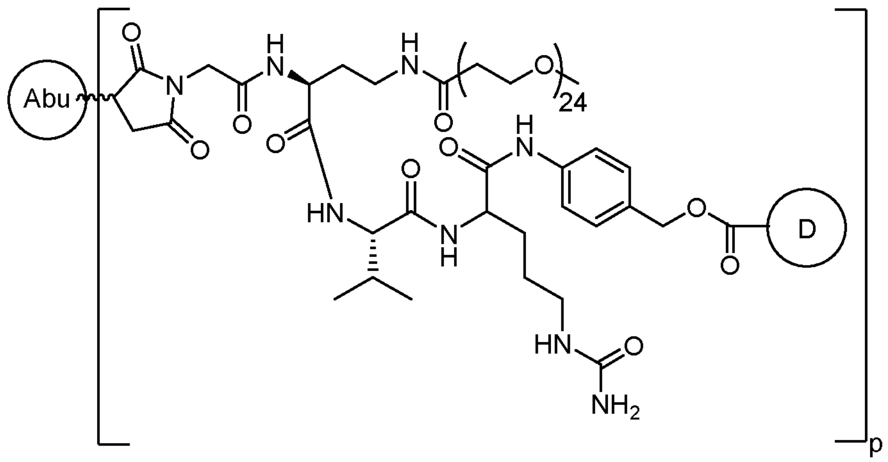

Formula I-11

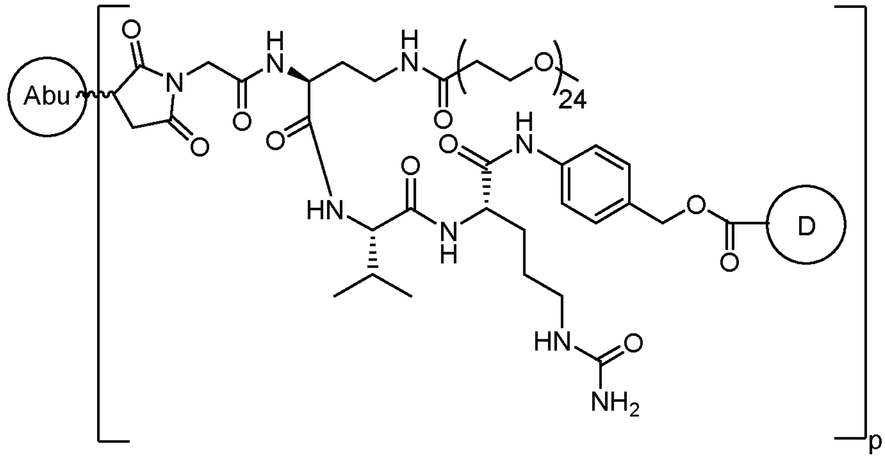

Formula I-11-1

wherein

Abu is the antibody or the antigen-binding unit thereof according to any one of claims 1-9;
R is selected from: $-(CH_2)_r-$, $-(CHR^m)_r-$, C3-C8 carbocyclyl, $-O-(CH_2)_r-$, arylene, $-(CH_2)_r$-arylene-, -arylene-$(CH_2)_r-$, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$,$-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$,$-(CH_2)_rC(O)NR^m(CH_2CH_2O)_r-CH_2-$, $-(CH_2CH_2O)_rC(O)NR^m(CH_2CH_2O)_r-$,$-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10; or R is $-(CH_2)_r-$, or r is 1 or 5;
D is a drug;
n is an integer from 1 to 24; or n is 4-12; or n is 4-8; or n is 4 or 8;
p is 1-10; or p is 2-8; or p is 4-8; or p is 6-8; or p is 7-8; or p is 7; or p is 8; or p is 7.4.

**14.** The antibody-drug conjugate according to any one of claims 11-13, wherein the drug is an anti-cancer drug, a cytotoxic drug, a cell differentiation factor, a stem cell trophic factor, a steroid drug, a drug for treating autoimmune diseases, an anti-inflammatory drug or a drug for treating infectious diseases; or the drug is an anti-cancer drug; or the drug is a tubulin inhibitor, a DNA damaging agent or a DNA topoisomerase inhibitor; or the tubulin inhibitor is selected from dolastatin, auristatins and maytansinoids; or the drug is an auristatin selected from MMAE, MMAF or AF; or the drug is a DNA damaging agent selected from calicheamicins, duocarmycins and an anthramycin derivative PBD; or the drug is a DNA topoisomerase inhibitor or a salt thereof selected from irinotecan, irinotecan hydrochloride, an exatecan derivative, camptothecin, 9-aminocamptothecin, 9-nitrocamptothecin, 10-hydroxycamptothecin, 9-chloro-10-hydroxycamptothecin, a camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-N-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, *N*-[2-(dimethylamino)ethyl]-4-acridinecarboxamide dihydrochloride and *N*-[2-(dimethylamino)ethyl]-4-acridinecarboxamide; or the DNA topoisomerase inhibitor is camptothecin, 10-hydroxycamptothecin, topotecan, belotecan, irinotecan, 22-hydroxyacuminatine or exatecan; or the pharmaceutically acceptable salt or solvate thereof.

**15.** The antibody-drug conjugate according to any one of claims 11-13, wherein the drug is

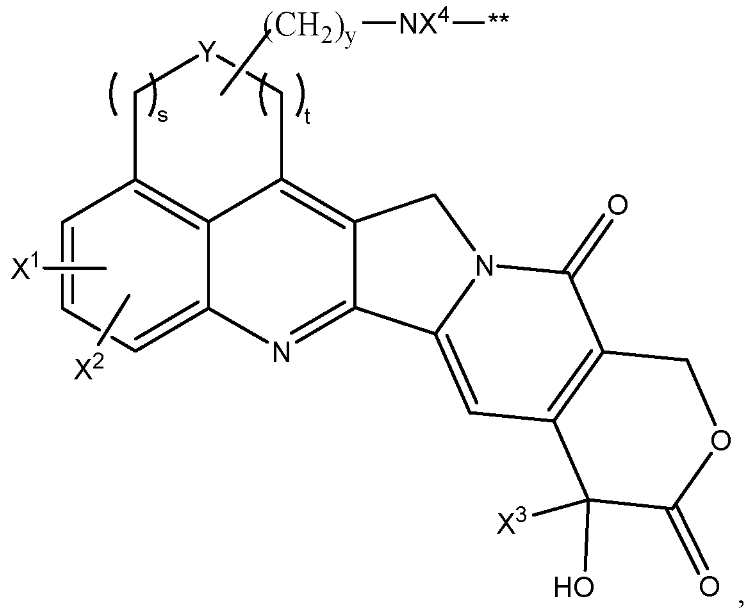

wherein $X^1$ and $X^2$ are each independently:

H,
hydroxy,
C1-C6 alkyl,
C1-C6 alkyl substituted with one or more hydroxy, halogen, nitro or cyano groups,
C2-C6 alkenyl,
C2-C6 alkynyl,
C1-C6 alkoxy,
C1-C6 aminoalkoxy,
halogen,
nitro,
cyano,
thiol,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocyclic ring, wherein the heterocyclic ring is optionally substituted with one or more C1-C6 alkyl, C1-C6 alkoxy, amino, halogen, nitro or cyano groups,
C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or a protecting group,
amino-substituted heterocyclyl optionally substituted at a nitrogen atom of the heterocyclyl moiety or at the amino moiety with a protecting group or one or more C1-C6 alkyl groups, heterocyclylamino optionally substituted at a nitrogen atom of the heterocyclyl moiety or at the amino moiety with a protecting group or C1-C6 alkyl, carbamoyl optionally substituted with a carbamoyl-protecting group or C1-C6 alkyl, morpholin-1-yl, or piperidin-1-yl;
$X^3$ is C1-C6 alkyl;
$X^4$ is H, $-(CH_2)_q-CH_3$, $-(CHR^n)_q-CH_3$, C3-C8 carbocyclyl, $-O-(CH_2)_q-CH_3$, arylene-$CH_3$,$-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_qC(O)NR^n(CH_2)_q-CH_3$, $-(CH_{2C}H_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$,$-(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or-$(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10; or $X^4$ is H or C1-C6 alkyl;

** links to other moieties of the antibody-drug conjugate;
y is 0, 1 or 2;
Y is O, S or $CR^1R^2$, wherein $R^1$ and $R^2$ are each independently H or C1-C6 alkyl;
s and t are each independently 0, 1 or 2, but not both 0;
or the drug is

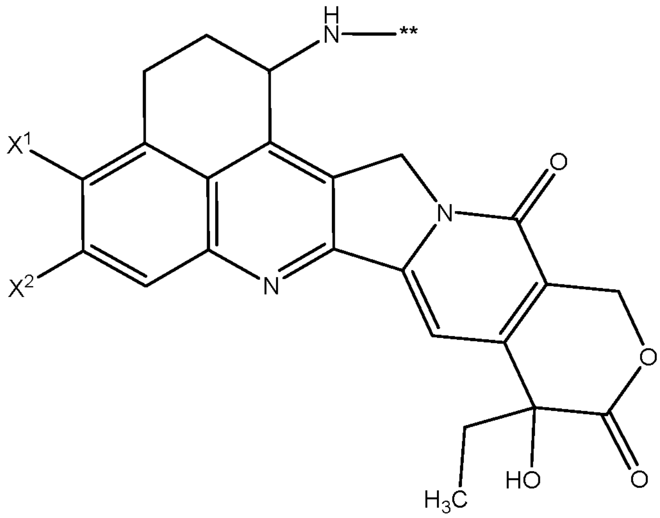

or

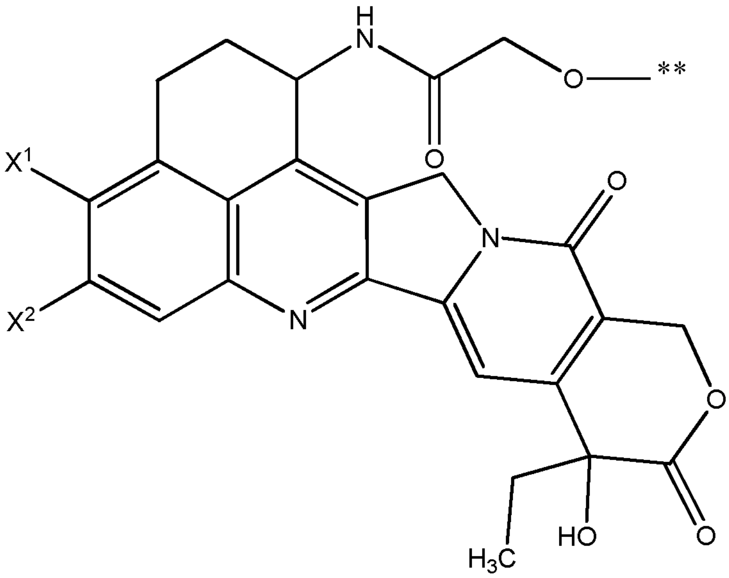

,

wherein $X^1$ and $X^2$ are each independently C1-C6 alkyl, halogen or -OH; or the C1-C6 alkyl is $-CH_3$; or the halogen is F; ** links to other moieties of the antibody-drug conjugate;
or the drug is

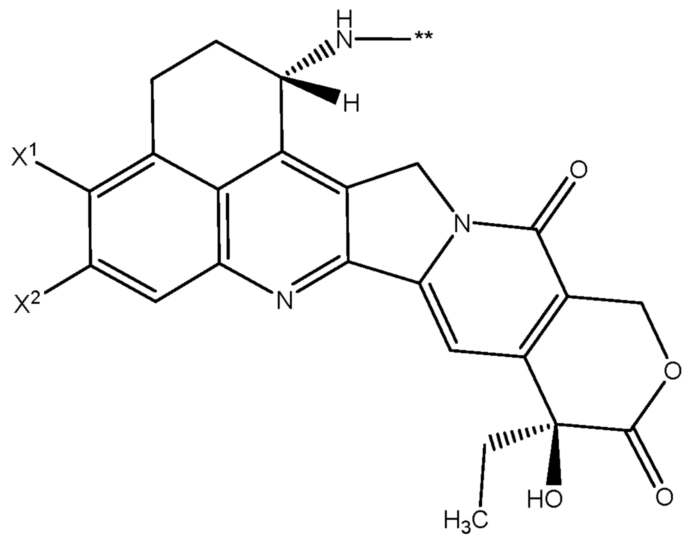

or

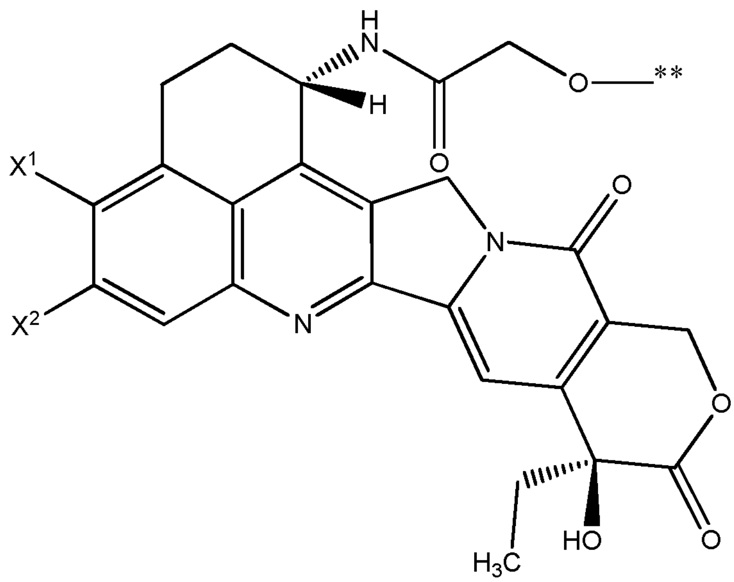

,

wherein $X^1$ and $X^2$ are each independently C1-C6 alkyl, halogen or -OH; or the C1-C6 alkyl is $-CH_3$; or the halogen is F; ** links to other moieties of the antibody-drug conjugate.

**16.** An antibody-drug conjugate having a structure shown as Formula I-12, I-12-1, I-13, I-13-1, I-14, I-14-1, I-15, I-15-1, I-16, I-16-1, I-17, I-17-1, I-18, I-18-1, I-19, I-19-1, I-20, I-20-1, I-21, I-21-1, I-22, I-22-1, I-23, I-23-1, I-24, I-24-1, I-25 or I-25-1 or a stereoisomer thereof, or a pharmaceutically acceptable salt or solvate thereof, wherein I-12, I-12-1, I-13, I-13-1, I-14, I-14-1, I-15, I-15-1, I-16, I-16-1, I-17, I-17-1, I-18, I-18-1, I-19, I-19-1, I-20, I-20-1, I-21, I-21-1, I-22, I-22-1, I-23, I-23-1, I-24, I-24-1, I-25 or I-25-1 is:

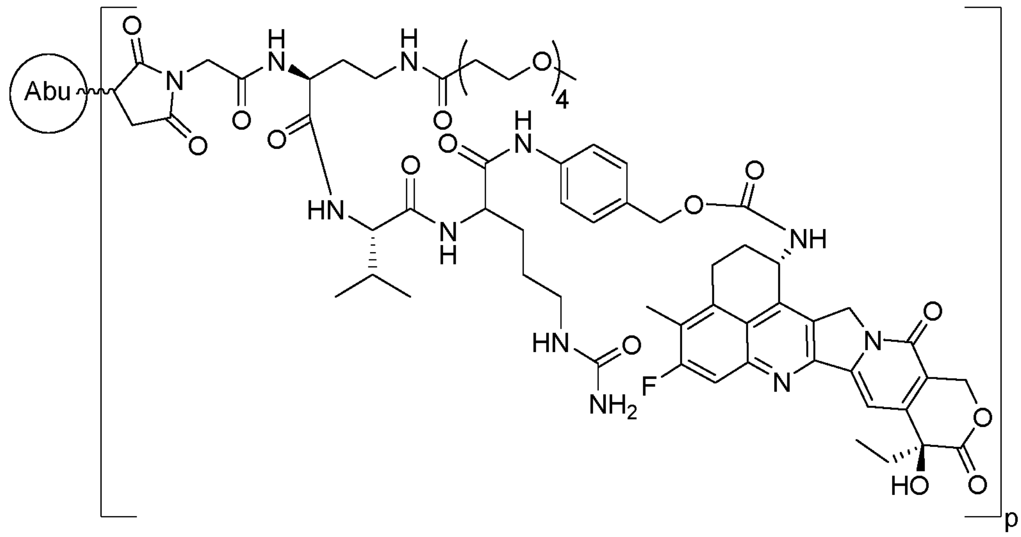

Formula I-12

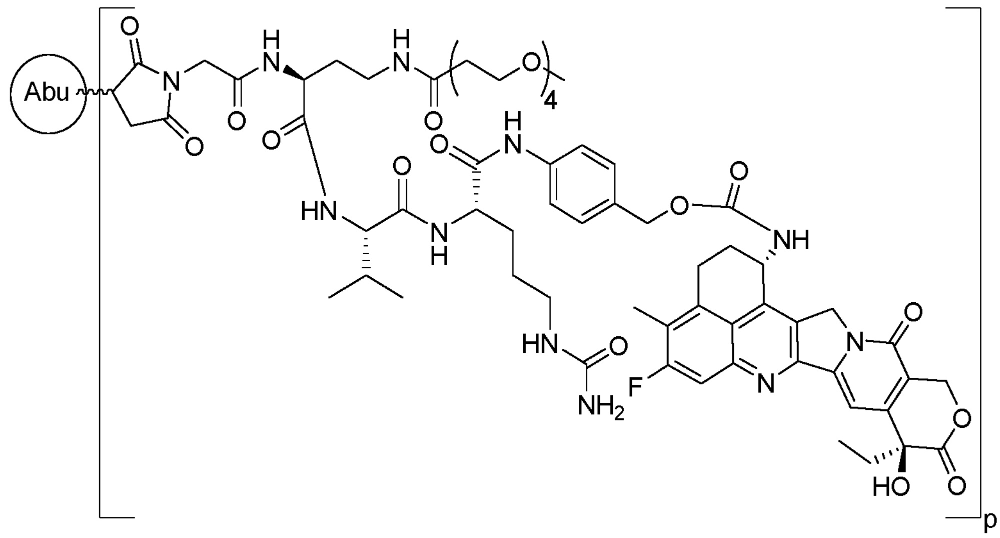

Formula I-12-1

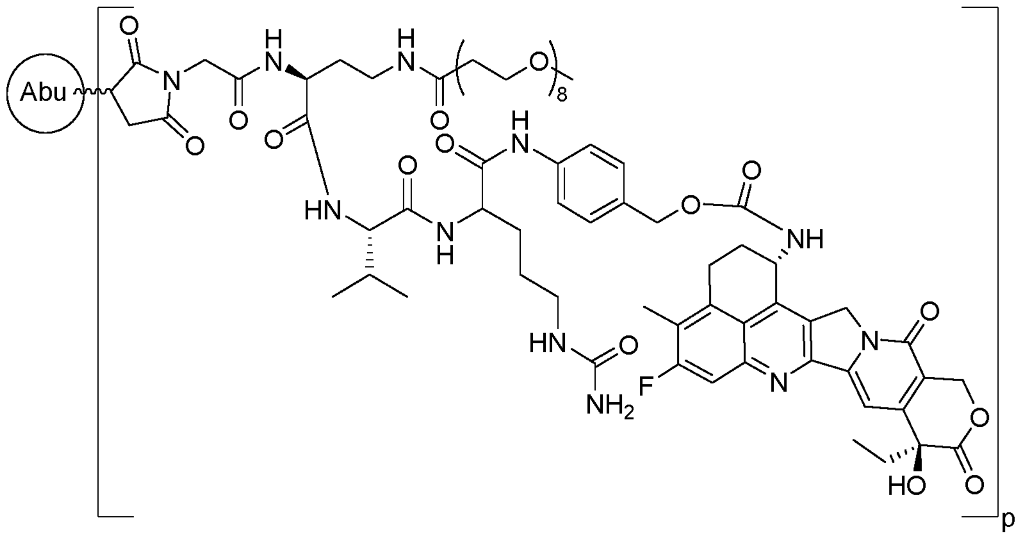

Formula I-13

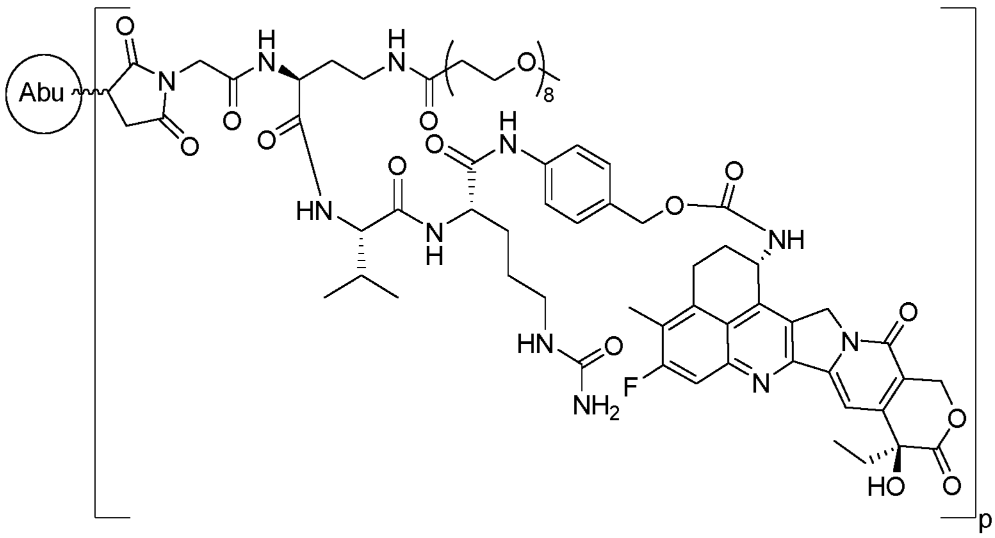

Formula I-13-1

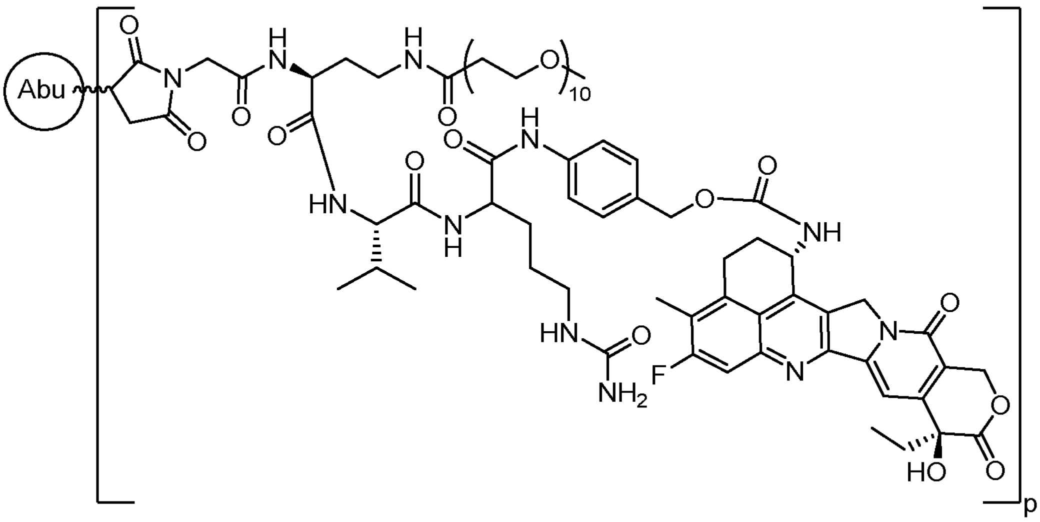

Formula I-14

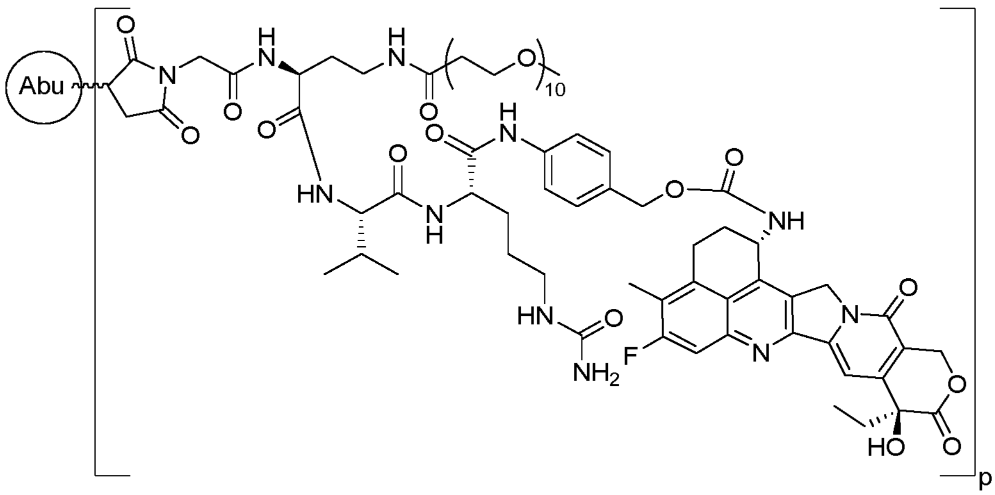

Formula I-14-1

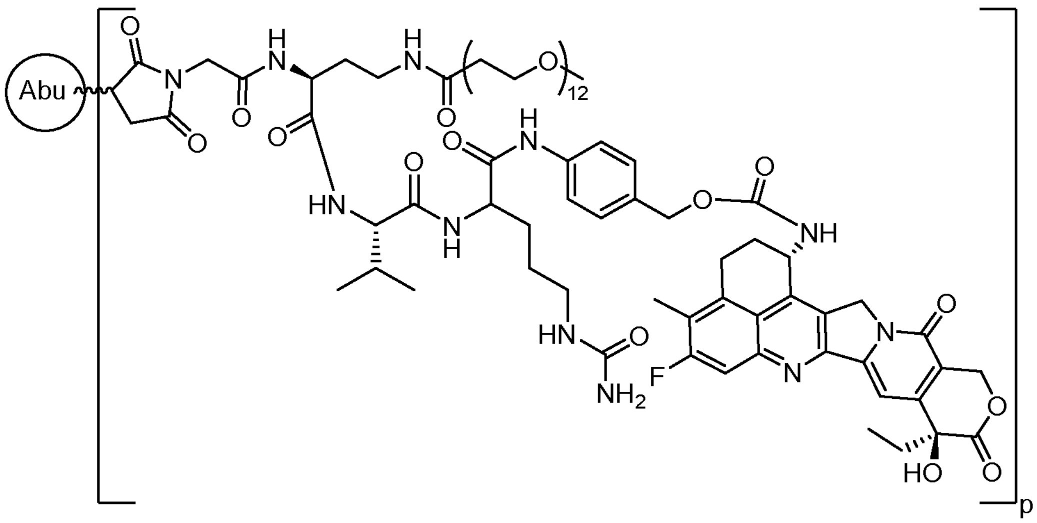

Formula I-15

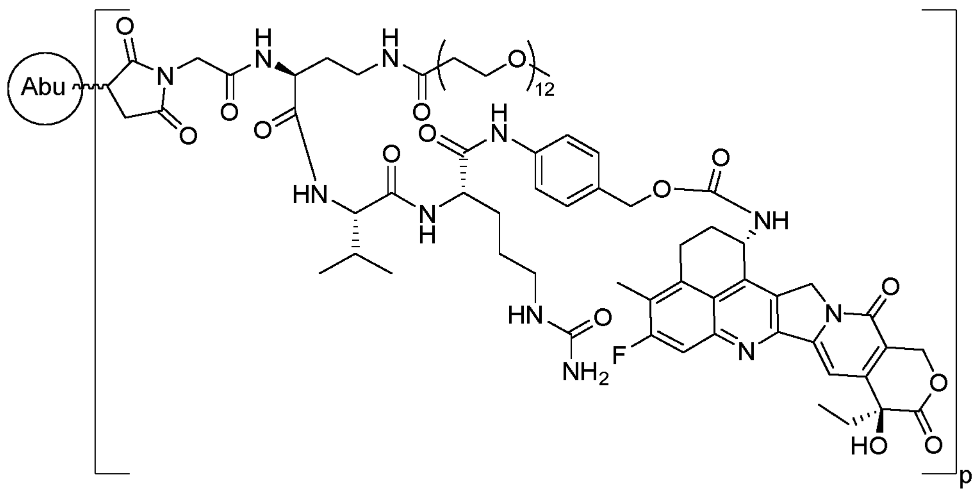

Formula I-15-1

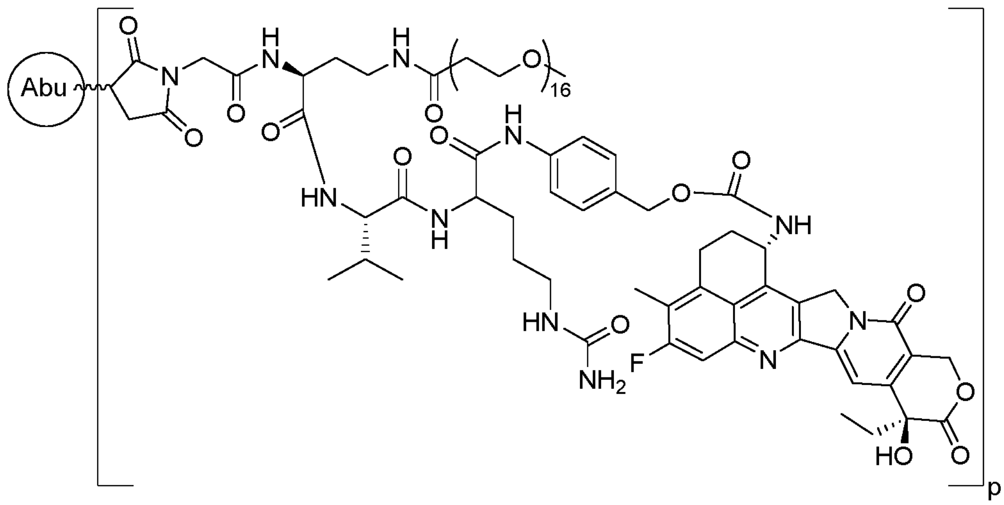

Formula I-16

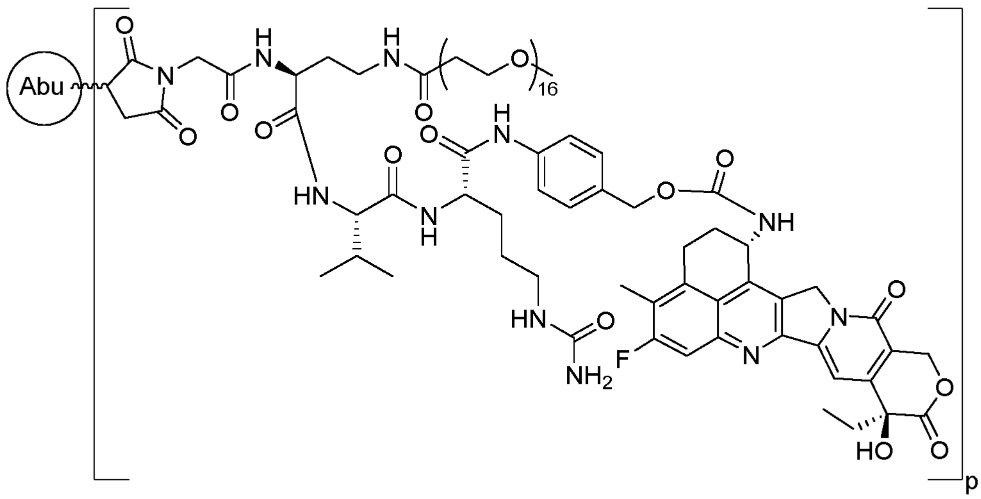

Formula I-16-1

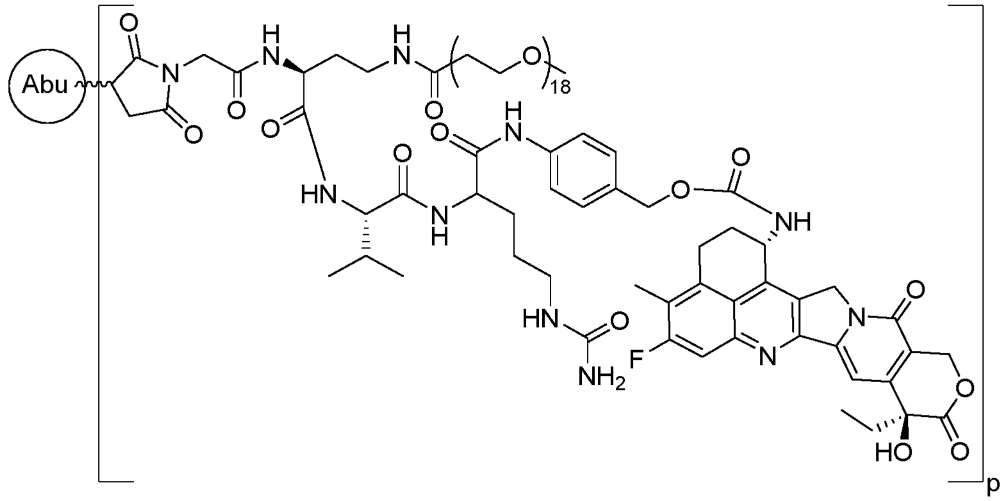

Formula I-17

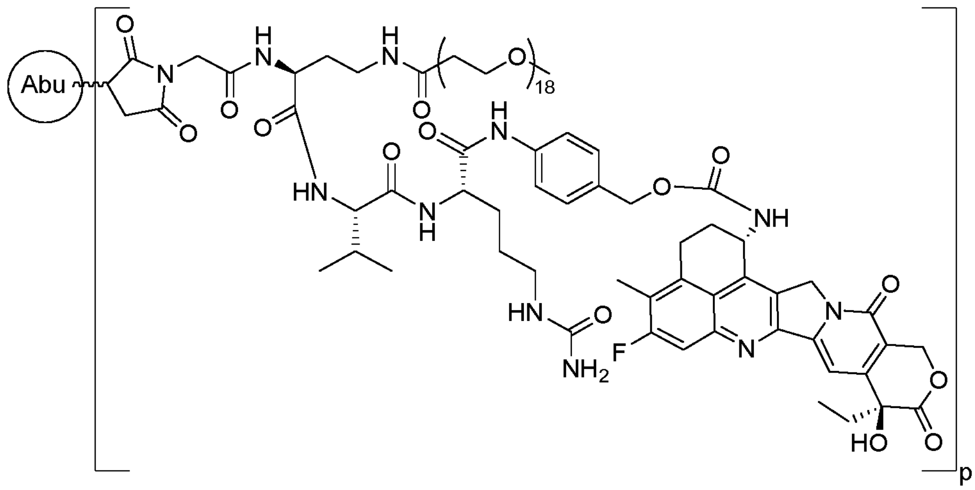

Formula I-17-1

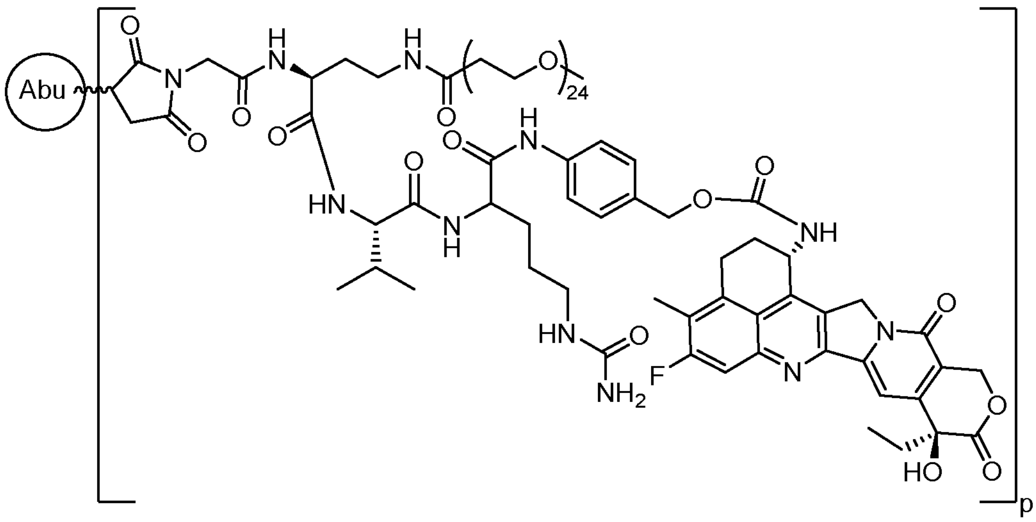

Formula I-18

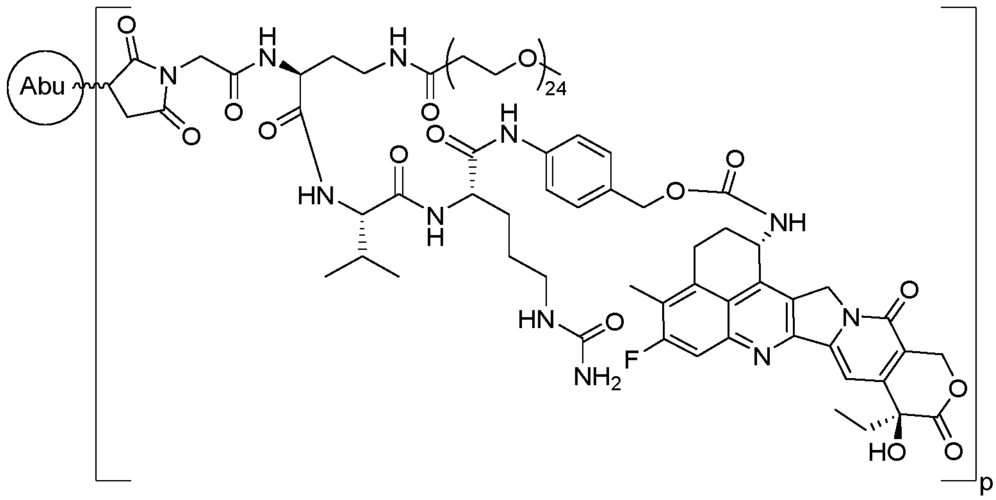

Formula I-18-1

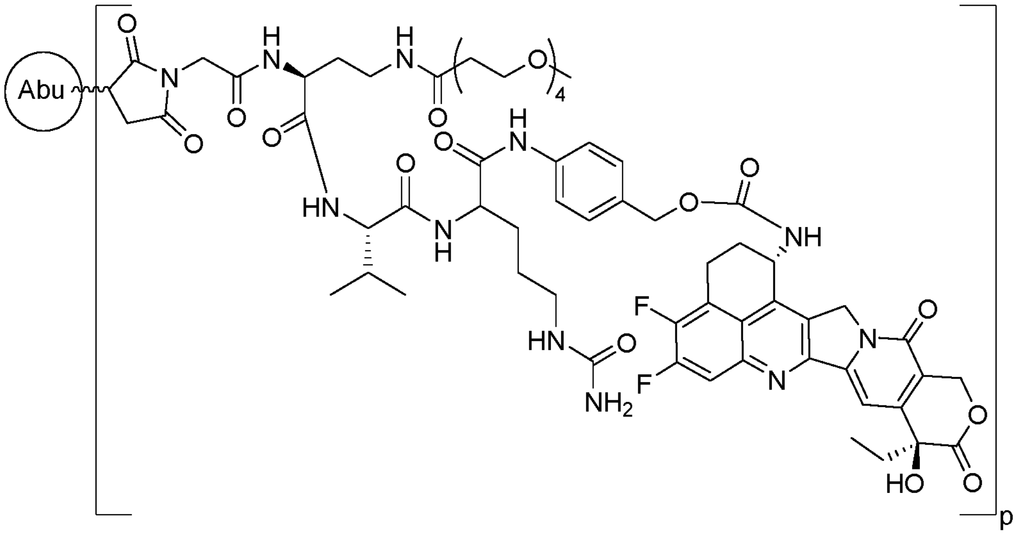

Formula I-19

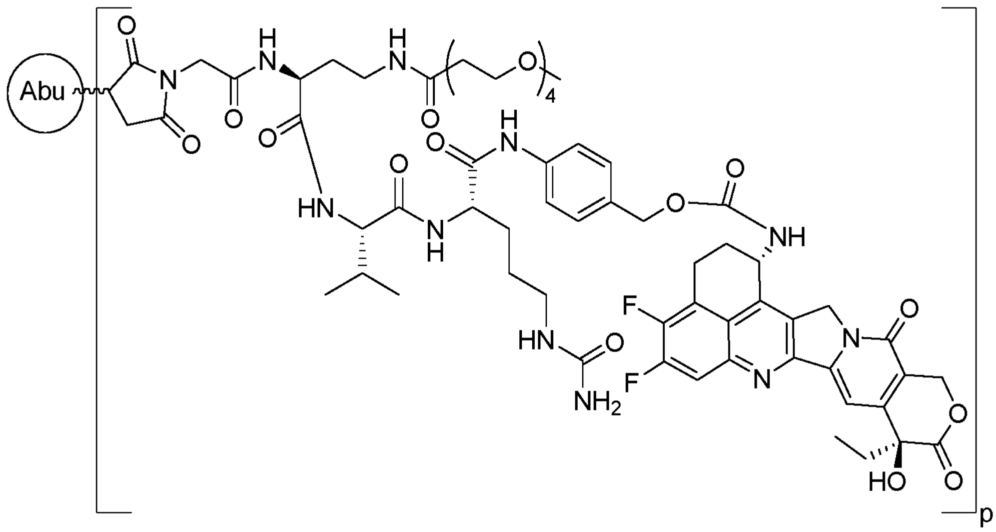

Formula I-19-1

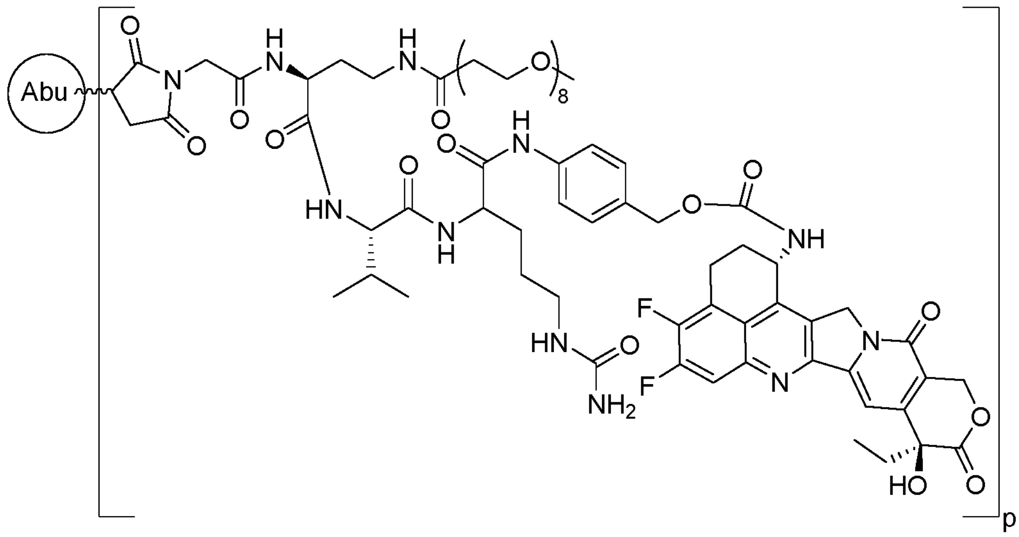

Formula I-20

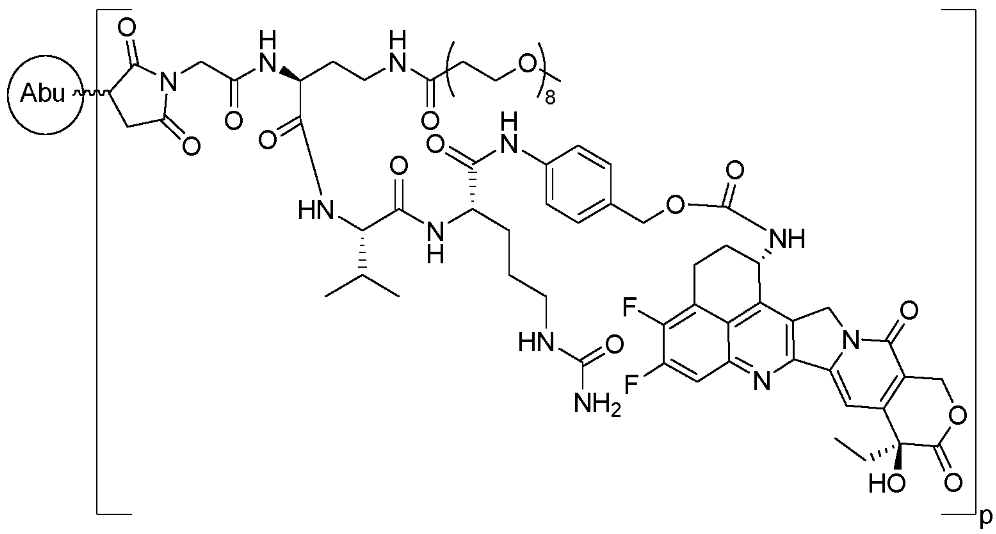

Formula I-20-1

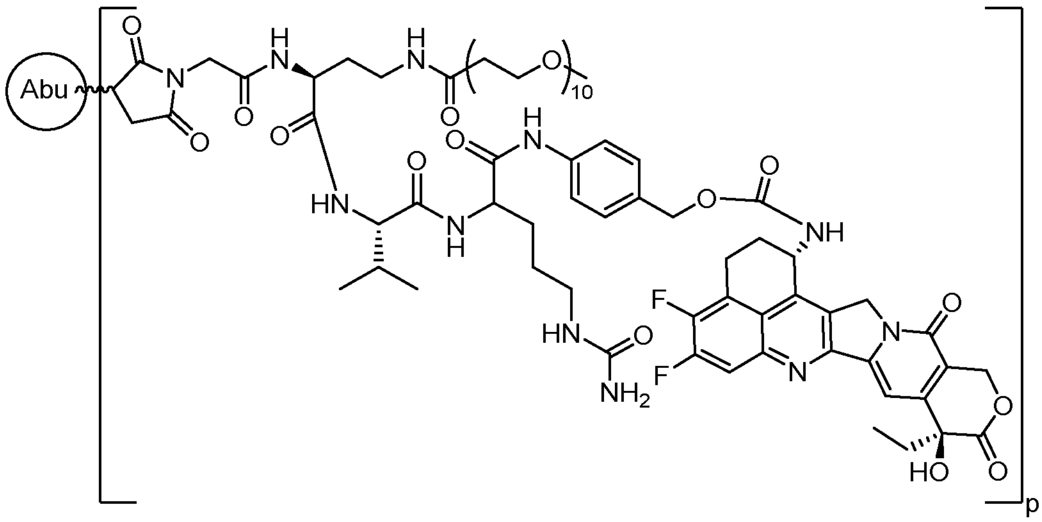

Formula I-21

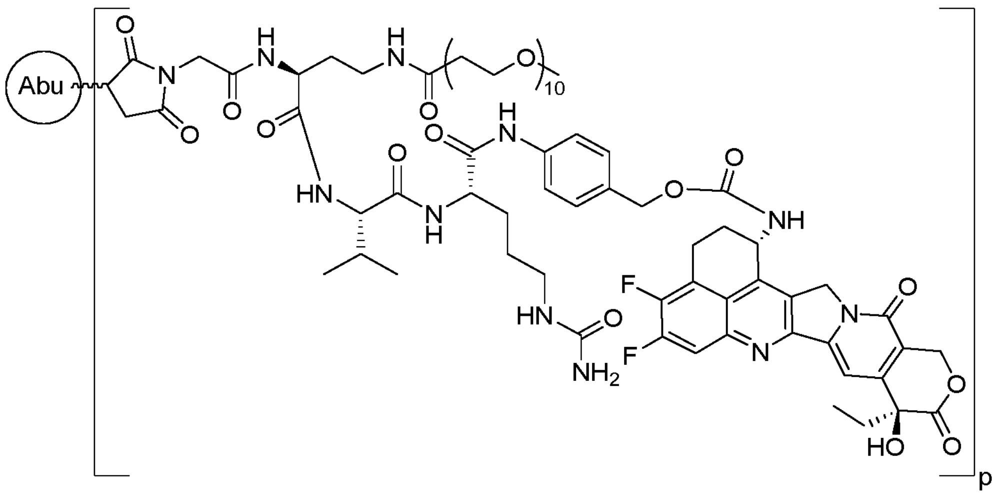

Formula I-21-1

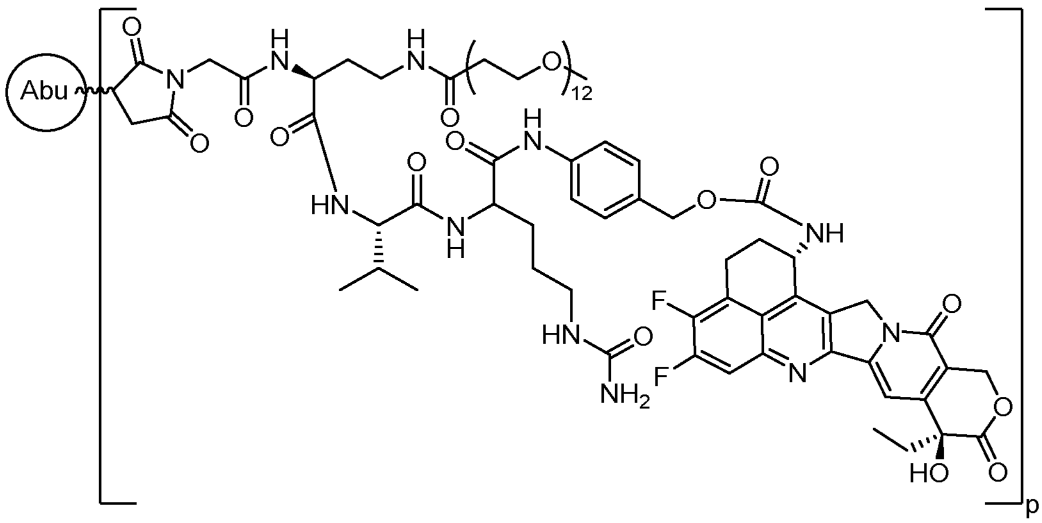

Formula I-22

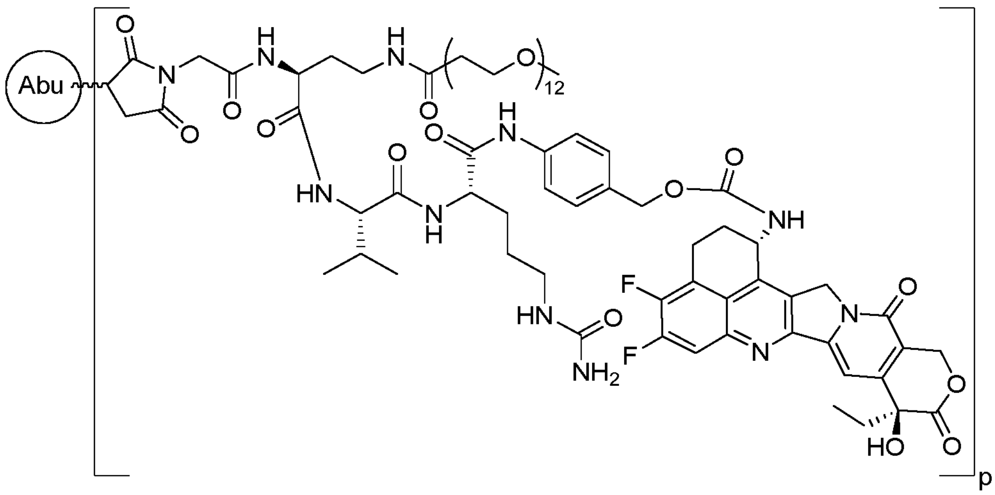

Formula I-22-1

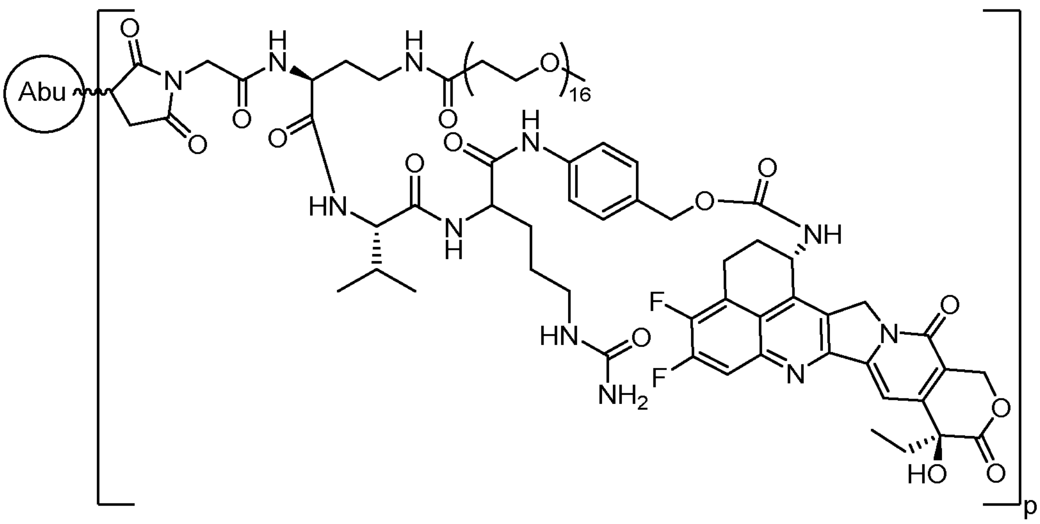

Formula I-23

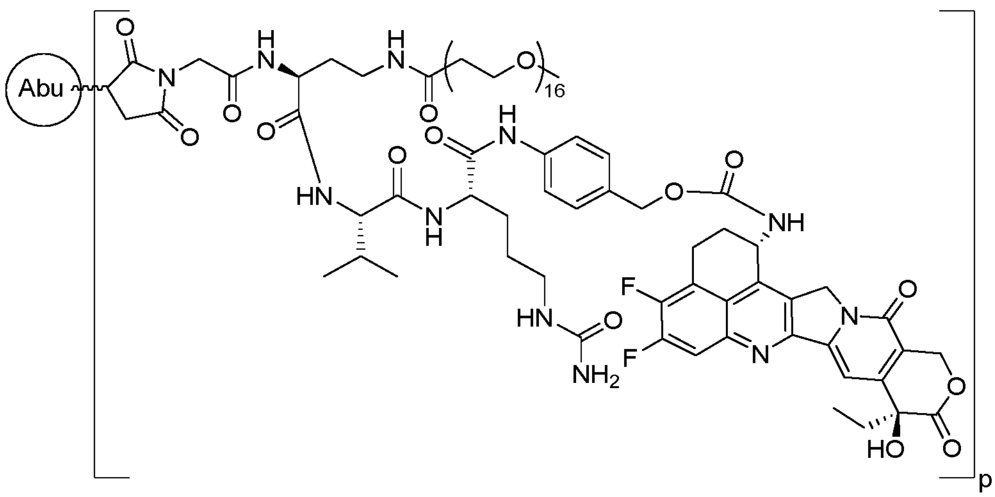

Formula I-23-1

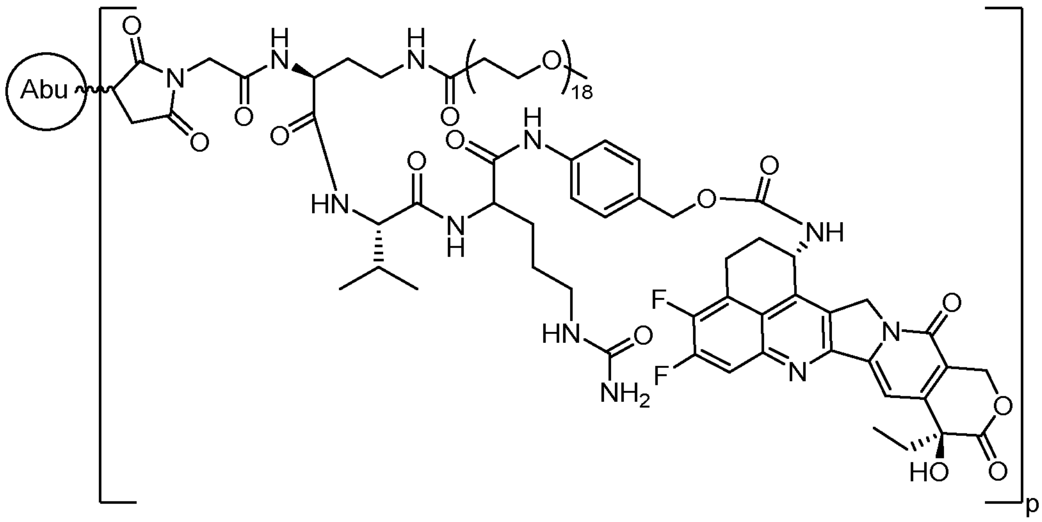

Formula I-24

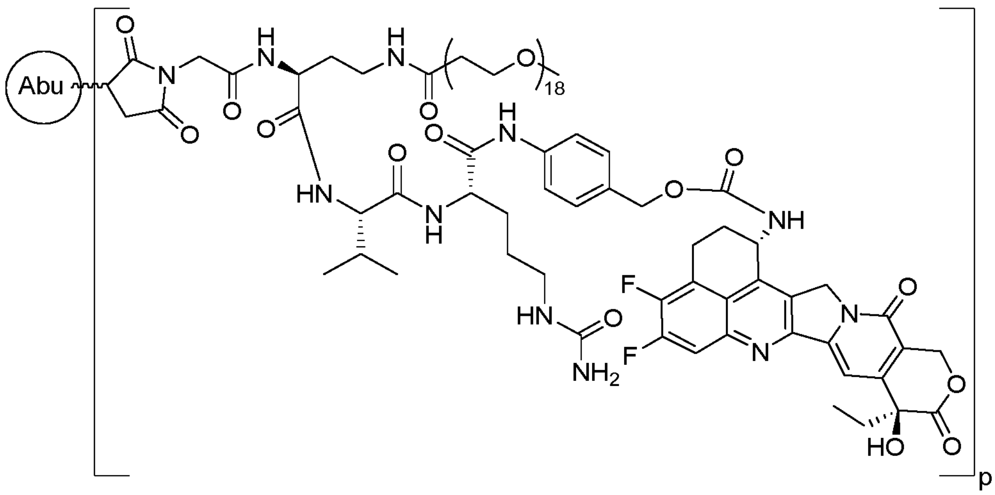

Formula I-24-1

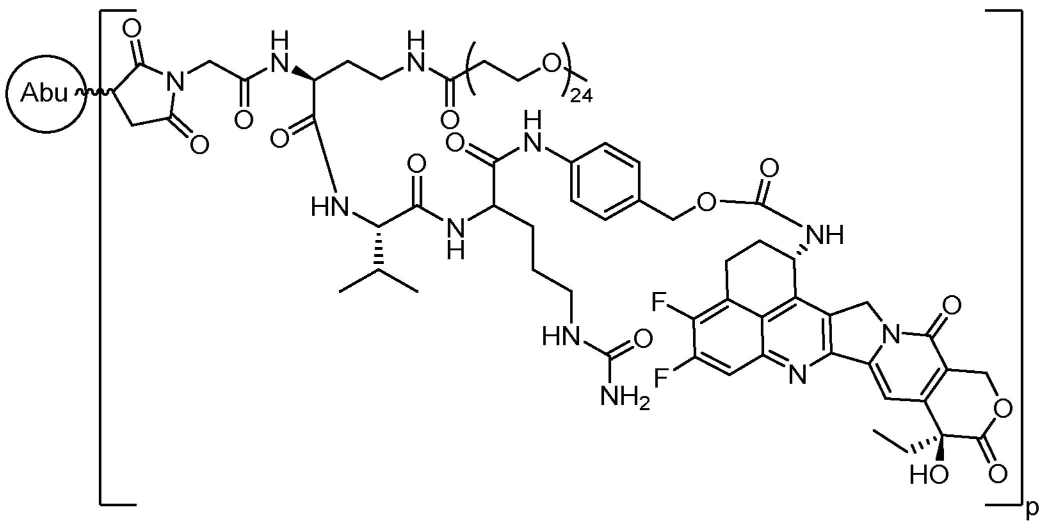

Formula I-25

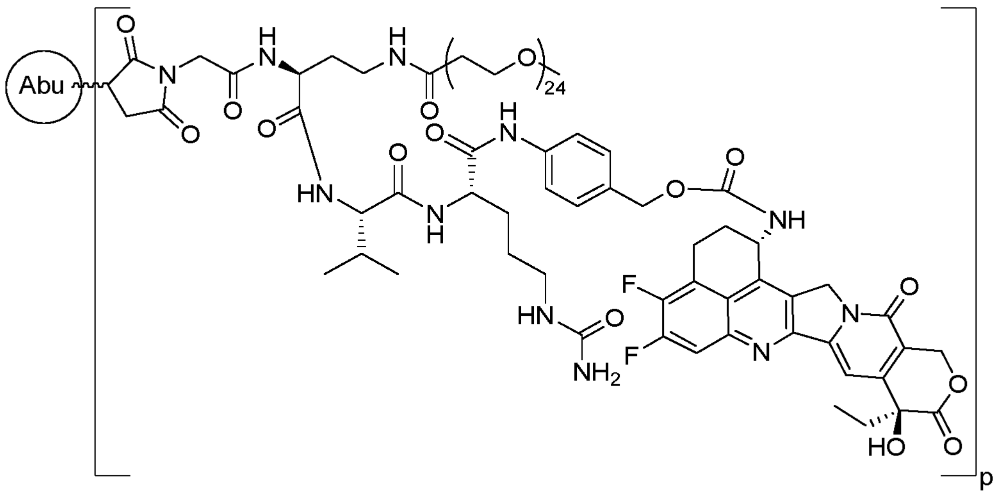

Formula I-25-1

wherein

Abu is the antibody or the antigen-binding unit thereof according to any one of claims 1-9;
p is 1-10; or p is 2-8; or p is 4-8; or p is 6-8; or p is 7-8; or p is 8.

**17.** A pharmaceutical composition, comprising the antibody or the antigen-binding unit thereof according to any one of claims 1-9 or the antibody-drug conjugate according to any one of claims 11-16, and a pharmaceutically acceptable carrier, an excipient and/or an adjuvant.

**18.** A pharmaceutical composition, comprising the antibody or the antigen-binding unit thereof according to any one of claims 1-9 or the antibody-drug conjugate according to any one of claims 11-16, and one or more additional anti-cancer drugs.

**19.** Use of the antibody or the antigen-binding unit thereof according to any one of claims 1-9, the antibody-drug conjugate according to any one of claims 11-16 or the pharmaceutical composition according to claim 17 or 18 in the treatment and/or prevention of a disease or in the manufacture of a drug for treating and/or preventing a disease, wherein alternatively, the disease is a disease associated with the expression of folate receptor $\alpha$; or the disease is a disease associated with the overexpression of folate receptor $\alpha$; or the disease is a tumor; or the drug for treating a tumor further comprises an additional anti-cancer drug.

**20.** A method for treating and/or preventing a disease, comprising: administering to a patient in need thereof an effective amount of the antibody or the antigen-binding unit thereof according to any one of claims 1-9, the antibody-drug conjugate according to any one of claims 11-16 or the pharmaceutical composition according to claim 17 or 18.

**21.** The method according to claim 20, wherein the disease is a disease associated with the expression of folate receptor $\alpha$; or the disease is a disease associated with the overexpression of folate receptor $\alpha$; or the disease is a tumor; or the disease is cancer; or the cancer is colorectal cancer, lung cancer, ovarian cancer, uterine cancer, endometrial cancer, peritoneal cancer, fallopian tube cancer, pancreatic cancer, head and neck squamous cell carcinoma, nasopharyngeal cancer, laryngeal cancer, lung adenocarcinoma, liver cancer, breast cancer, brain cancer, renal cancer, renal cell carcinoma, colon cancer, testicular cancer, cervical cancer, bladder cancer, retinoblastoma, glioblastoma, mesothelioma, oral epithelioid cancer, choriocarcinoma or head and neck cancer.

**22.** The method according to claim 20 or 21, wherein the antibody or the antigen-binding unit thereof or the antibody-drug conjugate is administered at a single dose of 1 mg/kg-10 mg/kg, or 50 mg-1000 mg, or 100 mg-600 mg; or an administration cycle is once a week, once every 2 weeks, once every 3 weeks, once every 4 weeks, once every 5 weeks or once every 6 weeks; or a mode of administration is by injection; or a mode of administration is intravenous injection; or a mode of administration is intravenous infusion.

**23.** A kit, comprising the antibody or the antigen-binding unit thereof according to any one of claims 1-9, the antibody-drug conjugate according to any one of claims 11-16 or the pharmaceutical composition according to claim 17 or 18, and instructions guiding administration to a patient.

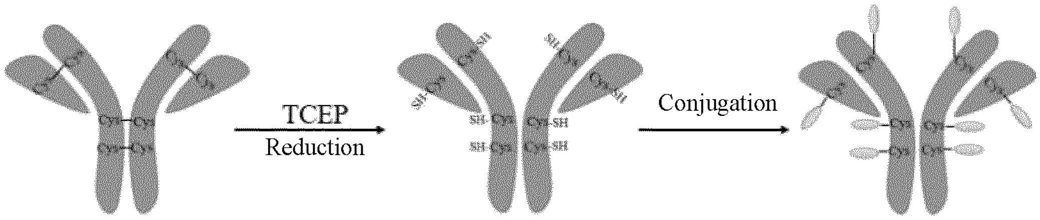
TCEP
Reduction
Conjugation

FIG. 1

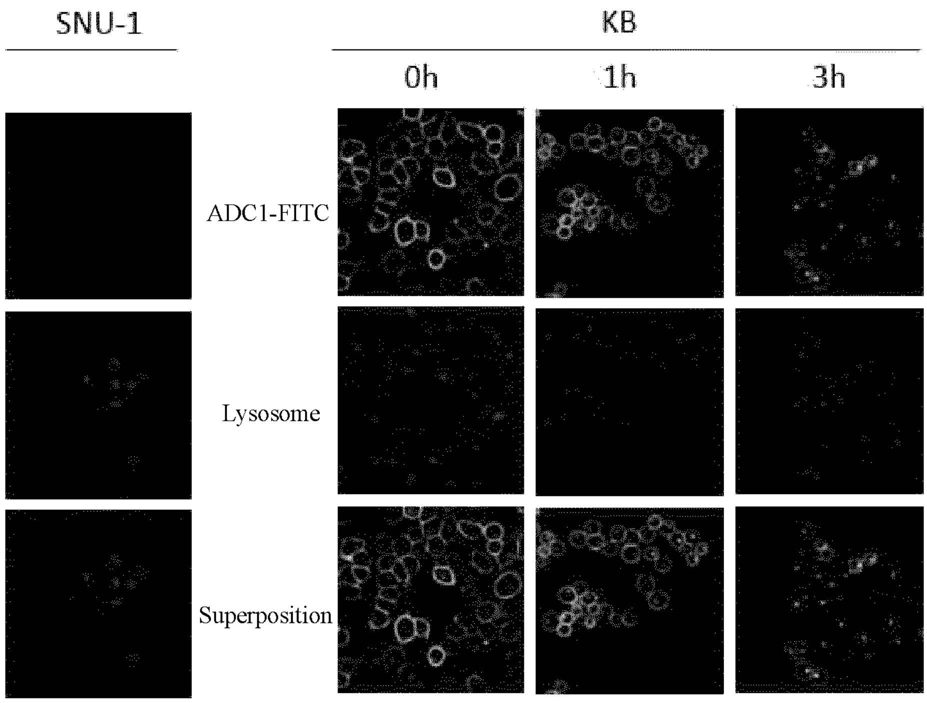
SNU-1
KB
0h
1h
3h
ADC1-FITC
Lysosome
Superposition

FIG. 2

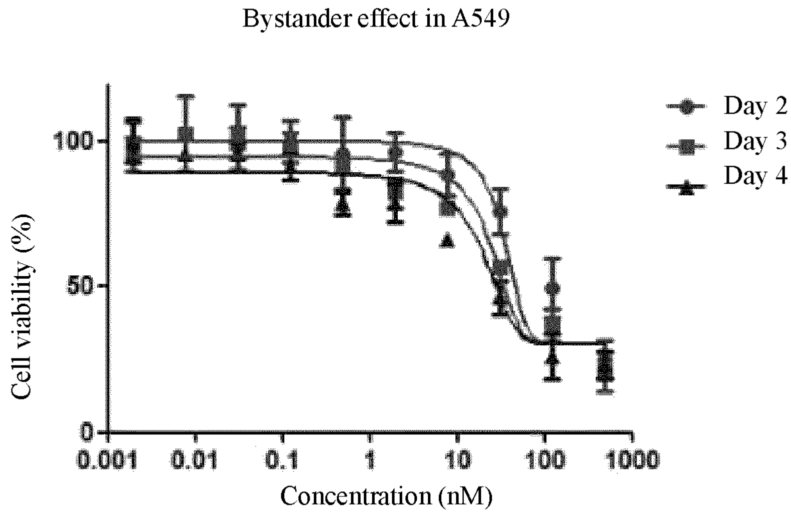
Bystander effect in A549
Cell viability (%)
100
50
0
0.001
0.01
0.1
1
10
100
1000
Concentration (nM)
Day 2
Day 3
Day 4

FIG. 3

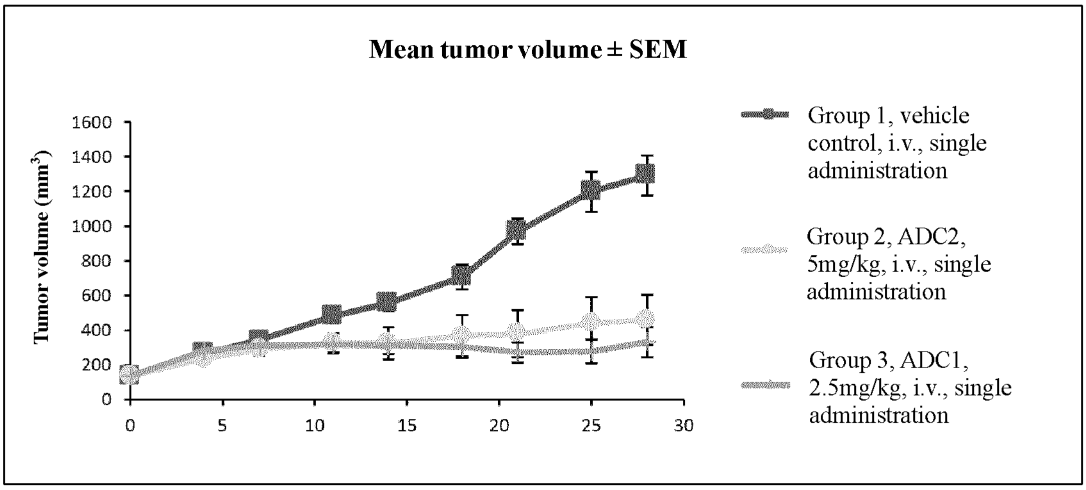
Mean tumor volume ± SEM
Tumor volume (mm3)
1600
1400
1200
1000
800
600
400
200
0
0
5
10
15
20
25
30
Group 1, vehicle control, i.v., single administration
Group 2, ADC2, 5mg/kg, i.v., single administration
Group 3, ADC1, 2.5mg/kg, i.v., single administration

FIG. 4

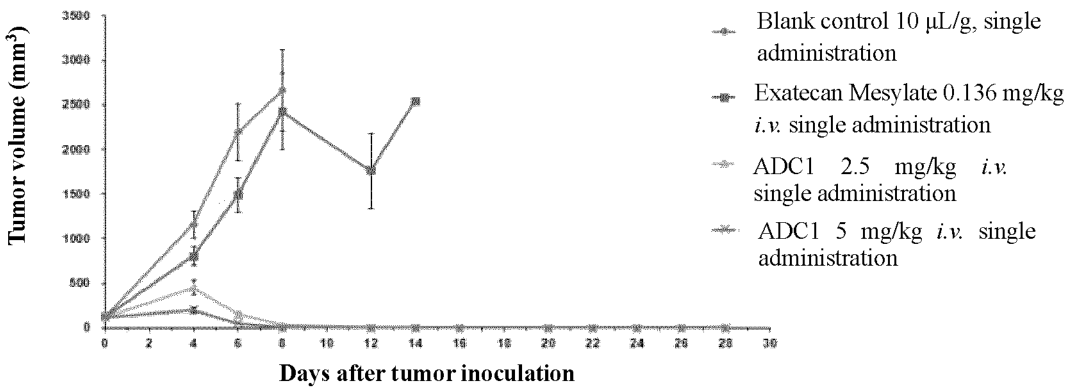


FIG. 5

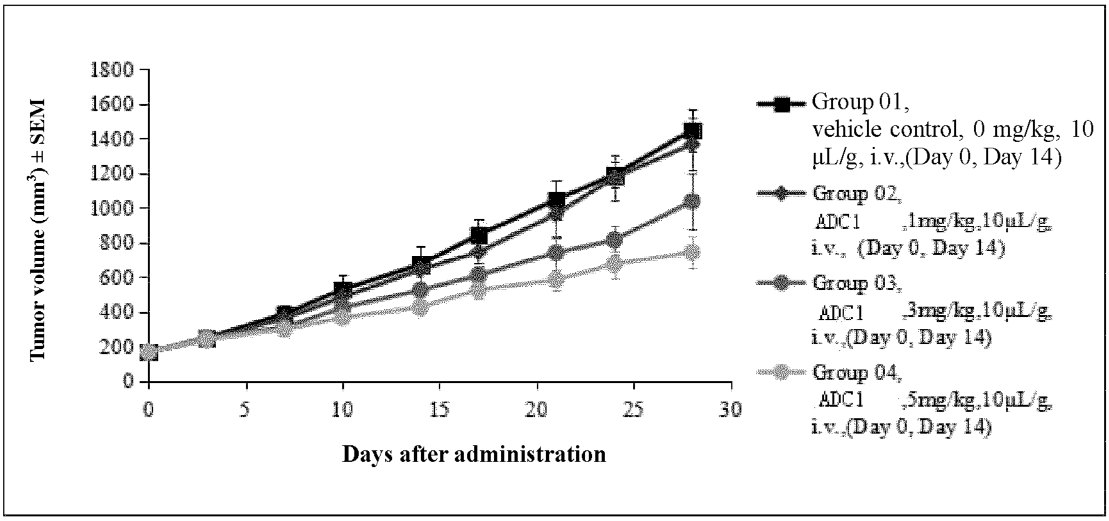


FIG. 6

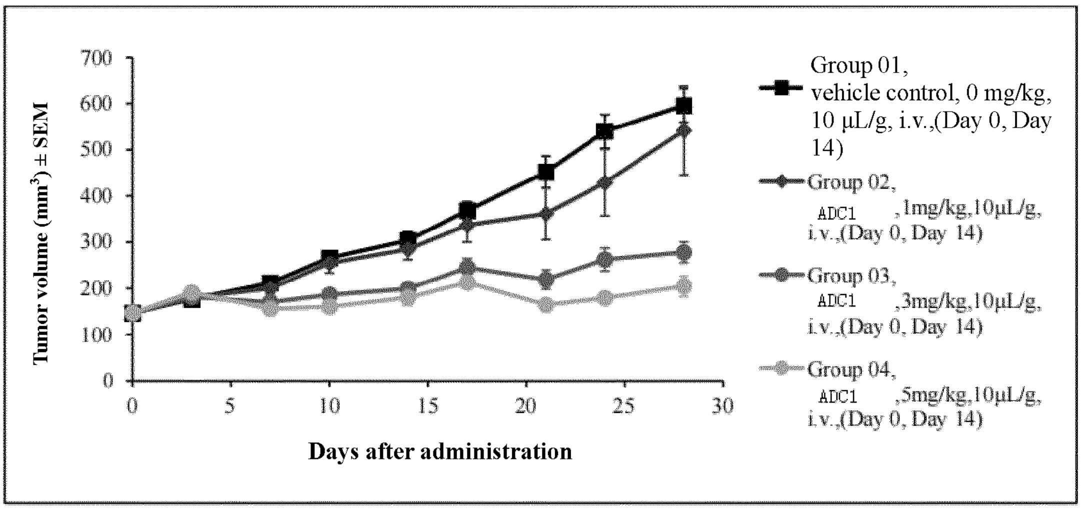


FIG. 7

**INTERNATIONAL SEARCH REPORT**

International application No.
**PCT/CN2022/141572**

**A. CLASSIFICATION OF SUBJECT MATTER**

C07K16/32(2006.01)i;A61K47/68(2017.01)i;A61K31/4745(2006.01)i;A61P35/00(2006.01)i

According to International Patent Classification (IPC) or to both national classification and IPC

**B. FIELDS SEARCHED**

Minimum documentation searched (classification system followed by classification symbols)

C07K,A61K,A61P

Documentation searched other than minimum documentation to the extent that such documents are included in the fields searched

Electronic data base consulted during the international search (name of data base and, where practicable, search terms used)

CNABS, DWPI, CNTXT, EPTXT, WOTXT, USTXT, CNKI, ISI web of science , baidu, Pudmed, STN on web, 百奥泰生物制药股份有限公司, 汤伟佳, Folr1, FRα, 叶酸受体1, 偶联物, 缀合物, conjugate, 喜树碱, 依喜替康, exatecan, 框架, Fr1-4, 聚乙二醇, 连接子, linker, PEG, SEQ ID NOs: 1-32, 171335-80-1/rn

**C. DOCUMENTS CONSIDERED TO BE RELEVANT**

| Category* | Citation of document, with indication, where appropriate, of the relevant passages | Relevant to claim No. |
|---|---|---|
| PX | CN 115429893 A (BIO-THERA SOLUTIONS, LTD.) 06 December 2022 (2022-12-06) claims 1-35, and description paragraphs 412-413, SEQ ID NOs: 23-24 | 1-23 |
| X | CN 105308072 A (IMMUNOGEN, INC.) 03 February 2016 (2016-02-03) claims 1-12, and description paragraphs 112-114, 134-139, 174, 181, 223-244 | 1-4, 6, 9-11, 14, 17-23 |
| Y | CN 105308072 A (IMMUNOGEN, INC.) 03 February 2016 (2016-02-03) claims 1-12, and description paragraphs 112-114, 134-139, 174, 181, 223-244 | 1-23 |
| X | WO 2015054400 A2 (IMMUNOGEN, INC.) 16 April 2015 (2015-04-16) description pages 58-59 | 1-4, 6, 9-11, 14, 17-23 |
| Y | WO 2015054400 A2 (IMMUNOGEN, INC.) 16 April 2015 (2015-04-16) description pages 58-59 | 1-23 |
| X | WO 2015031815 A2 (IMMUNOGEN, INC.) 05 March 2015 (2015-03-05) description paragraphs 365-368 | 1-4, 6, 9-11, 14, 17-23 |
| Y | WO 2015031815 A2 (IMMUNOGEN, INC.) 05 March 2015 (2015-03-05) description paragraphs 365-368 | 1-23 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 March 2023** | **14 March 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

INTERNATIONAL SEARCH REPORT

International application No.
**PCT/CN2022/141572**

**C. DOCUMENTS CONSIDERED TO BE RELEVANT**

| Category* | Citation of document, with indication, where appropriate, of the relevant passages | Relevant to claim No. |
|---|---|---|
| Y | CN 112739717 A (GENSUN BIOPHARMA INC.) 30 April 2021 (2021-04-30)<br>description paragraphs 68, 140, 141, 149 | 1-23 |
| Y | US 2021093733 A1 (OBI PHARMA, INC.) 01 April 2021 (2021-04-01)<br>description, page 58 | 12-23 |
| Y | WO 2019243825 A1 (CURADEV PHARMA LIMITED) 26 December 2019 (2019-12-26)<br>description pages 128,133 | 12-23 |
| A | WO 2014057687 A1 (DAIICHI SANKYO COMPANY, LIMITED) 17 April 2014 (2014-04-17)<br>entire document | 1-23 |

Form PCT/ISA/210 (second sheet) (July 2022)

**INTERNATIONAL SEARCH REPORT**

International application No.
**PCT/CN2022/141572**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2022/141572**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19-22**
because they relate to subject matter not required to be searched by this Authority, namely:
Claims 19-22 relate to a method for treating a disease (PCT Rule 39.1(iv)). However, the search opinion was still provided on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2022/141572**

| Patent document cited in search report | Publication date (day/month/year) | Patent family member(s) | Publication date (day/month/year) |
|---|---|---|---|
| CN 115429893 A | 06 December 2022 | WO 2022253284 A1 | 08 December 2022 |
| CN 105308072 A | 03 February 2016 | MX 2015015735 A | 31 March 2016 |
| | | JP 2018197249 A | 13 December 2018 |
| | | RU 2015149285 A | 19 June 2017 |
| | | US 2014363451 A1 | 11 December 2014 |
| | | US 2020046634 A1 | 13 February 2020 |
| | | AU 2014265587 A1 | 19 November 2015 |
| | | US 2017239367 A1 | 24 August 2017 |
| | | KR 20160020421 A | 23 February 2016 |
| | | AU 2021250837 A1 | 04 November 2021 |
| | | EP 2997044 A2 | 23 March 2016 |
| | | EP 2997044 A4 | 22 March 2017 |
| | | JP 2016520082 A | 11 July 2016 |
| | | SG 11201509043 RA | 30 December 2015 |
| | | KR 20220054700 A | 03 May 2022 |
| | | HK 1222340 A1 | 30 June 2017 |
| | | WO 2014186403 A2 | 20 November 2014 |
| | | WO 2014186403 A3 | 08 January 2015 |
| | | JP 2021102648 A | 15 July 2021 |
| | | SG 10201701096 UA | 30 March 2017 |
| | | CA 2911499 A1 | 20 November 2014 |
| | | BR 112015028244 A2 | 19 September 2017 |
| | | AU 2019250121 A1 | 31 October 2019 |
| | | US 2022378694 A1 | 01 December 2022 |
| | | IL 268180 A | 26 September 2019 |
| | | MX 2019008028 A | 06 September 2019 |
| WO 2015054400 A2 | 16 April 2015 | HRP 20192140 T1 | 06 March 2020 |
| | | SG 10202102555 TA | 29 April 2021 |
| | | AU 2020264270 A1 | 26 November 2020 |
| | | RU 2016114708 A | 15 November 2017 |
| | | RU 2016114708 A3 | 31 July 2018 |
| | | RU 2696579 C2 | 05 August 2019 |
| | | ZA 201902009 B | 28 October 2020 |
| | | NZ 757964 A | 01 July 2022 |
| | | KR 20200058592 A | 27 May 2020 |
| | | US 2015132323 A1 | 14 May 2015 |
| | | SG 10201907042 PA | 27 September 2019 |
| | | KR 20160079797 A | 06 July 2016 |
| | | KR 102115217 B1 | 26 May 2020 |
| | | KR 102115217 B9 | 24 December 2021 |
| | | US 2021155688 A1 | 27 May 2021 |
| | | WO 2015054400 A3 | 25 June 2015 |
| | | JP 2021178847 A | 18 November 2021 |
| | | LT 3055332 T | 10 April 2020 |
| | | MX 2016004411 A | 05 July 2016 |
| | | EP 3653228 A1 | 20 May 2020 |
| | | SG 11201602361 UA | 28 April 2016 |
| | | HUE 046696 T2 | 30 March 2020 |
| | | HK 1226671 A1 | 06 October 2017 |
| | | RS 59644 B1 | 31 January 2020 |

Form PCT/ISA/210 (patent family annex) (July 2022)

**INTERNATIONAL SEARCH REPORT**
**Information on patent family members**

International application No.
**PCT/CN2022/141572**

| Patent document cited in search report | Publication date (day/month/year) | Patent family member(s) | Publication date (day/month/year) |
|---|---|---|---|
| | | BR 112016007479 A2 | 12 September 2017 |
| | | AU 2014331964 A1 | 28 April 2016 |
| | | AU 2014331964 B2 | 13 September 2018 |
| | | AU 2014331964 C1 | 31 October 2019 |
| | | ES 2759426 T3 | 11 May 2020 |
| | | IL 265586 A | 30 May 2019 |
| | | PL 3055332 T3 | 28 February 2020 |
| | | KR 20220136468 A | 07 October 2022 |
| | | EP 3055332 A2 | 17 August 2016 |
| | | EP 3055332 A4 | 31 May 2017 |
| | | EP 3055332 B1 | 25 September 2019 |
| | | NZ 718766 A | 28 February 2020 |
| | | KR 20210118220 A | 29 September 2021 |
| | | CA 2926325 A1 | 16 April 2015 |
| | | CY 1122449 T1 | 27 January 2021 |
| | | IL 244772 A0 | 21 April 2016 |
| | | IL 244772 B | 30 May 2019 |
| | | AU 2018278880 A1 | 03 January 2019 |
| | | DK 3055332 T3 | 09 December 2019 |
| | | JP 2019031568 A | 28 February 2019 |
| | | JP 6581279 B2 | 25 September 2019 |
| | | SI 3055332 T1 | 28 February 2020 |
| | | IL 298044 A | 01 January 2023 |
| | | PT 3055332 T | 09 December 2019 |
| | | KR 20210041124 A | 14 April 2021 |
| | | KR 102303874 B1 | 17 September 2021 |
| | | JP 2016534039 A | 04 November 2016 |
| | | JP 6463744 B2 | 06 February 2019 |
| | | UA 119541 C2 | 10 July 2019 |
| | | JP 2019218386 A | 26 December 2019 |
| WO 2015031815 A2 | 05 March 2015 | CA 2921975 A1 | 05 March 2015 |
| | | SG 10201907501 QA | 30 October 2019 |
| | | US 2015093388 A1 | 02 April 2015 |
| | | US 9637547 B2 | 02 May 2017 |
| | | US 2017306041 A1 | 26 October 2017 |
| | | US 10017578 B2 | 10 July 2018 |
| | | ES 2883191 T3 | 07 December 2021 |
| | | NZ 717140 A | 27 May 2022 |
| | | SG 10202100275 TA | 25 February 2021 |
| | | JP 2021035968 A | 04 March 2021 |
| | | EP 3038650 A2 | 06 July 2016 |
| | | EP 3038650 A4 | 08 March 2017 |
| | | EP 3038650 B1 | 26 May 2021 |
| | | JP 2016536330 A | 24 November 2016 |
| | | PT 3038650 T | 25 August 2021 |
| | | BR 112016003665 A2 | 05 December 2017 |
| | | AU 2020203507 A1 | 18 June 2020 |
| | | EP 3925980 A1 | 22 December 2021 |
| | | DK 3038650 T3 | 16 August 2021 |
| | | SG 11201601037 YA | 30 March 2016 |

Form PCT/ISA/210 (patent family annex) (July 2022)

**INTERNATIONAL SEARCH REPORT**
**Information on patent family members**

International application No.
**PCT/CN2022/141572**

| Patent document cited in search report | Publication date (day/month/year) | Patent family member(s) | Publication date (day/month/year) |
|---|---|---|---|
| | | JP 2019059756 A | 18 April 2019 |
| | | JP 6983746 B2 | 17 December 2021 |
| | | US 2020325240 A1 | 15 October 2020 |
| | | US 11198736 B2 | 14 December 2021 |
| | | US 2022169745 A1 | 02 June 2022 |
| | | RU 2016109642 A | 06 October 2017 |
| | | RU 2016109642 A3 | 25 September 2018 |
| | | RU 2725825 C2 | 06 July 2020 |
| | | RS 62240 B1 | 30 September 2021 |
| | | IL 244061 A0 | 21 April 2016 |
| | | IL 244061 B | 01 July 2022 |
| | | HK 1222402 A1 | 30 June 2017 |
| | | ZA 201907806 B | 26 January 2022 |
| | | SI 3038650 T1 | 30 November 2021 |
| | | MX 2016002537 A | 08 September 2016 |
| | | MX 371496 B | 31 January 2020 |
| | | WO 2015031815 A3 | 07 May 2015 |
| | | KR 20160055831 A | 18 May 2016 |
| | | KR 102428492 B1 | 03 August 2022 |
| | | AU 2014312086 A1 | 10 March 2016 |
| | | AU 2014312086 B2 | 12 March 2020 |
| | | UA 120166 C2 | 25 October 2019 |
| | | LT 3038650 T | 10 September 2021 |
| | | US 2019023805 A1 | 24 January 2019 |
| | | US 10544230 B2 | 28 January 2020 |
| | | PL 3038650 T3 | 22 November 2021 |
| | | KR 20220110871 A | 09 August 2022 |
| | | HRP 20211317 T1 | 26 November 2021 |
| | | MX 2020001160 A | 12 March 2020 |
| | | HUE 055856 T2 | 28 December 2021 |
| | | IL 293871 A | 01 August 2022 |
| CN 112739717 A | 30 April 2021 | JP 2021532170 A | 25 November 2021 |
| | | WO 2020006516 A1 | 02 January 2020 |
| | | US 2020002417 A1 | 02 January 2020 |
| | | US 10597453 B2 | 24 March 2020 |
| | | CA 3105101 A1 | 02 January 2020 |
| | | US 2021009696 A1 | 14 January 2021 |
| | | US 2021246214 A1 | 12 August 2021 |
| | | JP 2021529557 A | 04 November 2021 |
| | | KR 20210028222 A | 11 March 2021 |
| | | US 2020277388 A1 | 03 September 2020 |
| | | US 11518813 B2 | 06 December 2022 |
| | | JP 2021529556 A | 04 November 2021 |
| | | US 2020002426 A1 | 02 January 2020 |
| | | US 11001635 B2 | 11 May 2021 |
| | | WO 2020006511 A1 | 02 January 2020 |
| | | WO 2020006509 A1 | 02 January 2020 |
| | | EP 3814381 A1 | 05 May 2021 |
| | | EP 3814381 A4 | 10 August 2022 |
| | | AU 2019293047 A1 | 28 January 2021 |

Form PCT/ISA/210 (patent family annex) (July 2022)

**INTERNATIONAL SEARCH REPORT**
**Information on patent family members**

International application No.
**PCT/CN2022/141572**

| Patent document cited in search report | Publication date (day/month/year) | Patent family member(s) | Publication date (day/month/year) |
|---|---|---|---|
| | | BR 112020026862 A2 | 20 April 2021 |
| | | US 2020181272 A1 | 11 June 2020 |
| | | US 2020002439 A1 | 02 January 2020 |
| | | US 10647773 B2 | 12 May 2020 |
| | | EP 3813864 A1 | 05 May 2021 |
| | | EP 3813864 A4 | 20 July 2022 |
| | | EP 3813880 A1 | 05 May 2021 |
| | | EP 3813880 A4 | 13 July 2022 |
| US 2021093733 A1 | 01 April 2021 | None | |
| WO 2019243825 A1 | 26 December 2019 | TW 202016081 A | 01 May 2020 |
| WO 2014057687 A1 | 17 April 2014 | LT 2907824 T | 11 June 2018 |
| | | US 2016279259 A1 | 29 September 2016 |
| | | US 9808537 B2 | 07 November 2017 |
| | | CA 3021435 A1 | 17 April 2014 |
| | | CA 3021435 C | 12 October 2021 |
| | | BR 122021014396 B1 | 05 July 2022 |
| | | JP 6030267 B1 | 24 November 2016 |
| | | JP 2017036274 A | 16 February 2017 |
| | | JP 2022008581 A | 13 January 2022 |
| | | JP 7170812 B2 | 14 November 2022 |
| | | JP 2023011799 A | 24 January 2023 |
| | | SI 2907824 T1 | 29 June 2018 |
| | | BR 112015006521 A2 | 05 September 2017 |
| | | BR 112015006521 B1 | 03 March 2022 |
| | | NZ 746440 A | 29 November 2019 |
| | | SG 10201804788 XA | 30 July 2018 |
| | | PT 2907824 T | 07 June 2018 |
| | | PT 3342785 T | 25 February 2020 |
| | | JP 5953378 B2 | 20 July 2016 |
| | | JPWO 2014057687 A1 | 05 September 2016 |
| | | KR 20220100727 A | 15 July 2022 |
| | | KR 102456726 B1 | 20 October 2022 |
| | | US 2020282073 A1 | 10 September 2020 |
| | | ES 2773710 T3 | 14 July 2020 |
| | | CA 2885800 A1 | 17 April 2014 |
| | | CA 2885800 C | 04 December 2018 |
| | | AU 2020200548 A1 | 13 February 2020 |
| | | AU 2020200548 B2 | 25 August 2022 |
| | | US 2015297748 A1 | 22 October 2015 |
| | | US 10195288 B2 | 05 February 2019 |
| | | MX 2020010964 A | 09 November 2020 |
| | | HRP 20180870 T1 | 13 July 2018 |
| | | TR 201809636 T4 | 23 July 2018 |
| | | TW 202233186 A | 01 September 2022 |
| | | KR 20180030734 A | 23 March 2018 |
| | | KR 101901558 B1 | 21 September 2018 |
| | | KR 20150067149 A | 17 June 2015 |
| | | KR 101841818 B1 | 26 March 2018 |
| | | CY 1120363 T1 | 10 July 2019 |
| | | SG 11201502887 WA | 28 May 2015 |

Form PCT/ISA/210 (patent family annex) (July 2022)

**INTERNATIONAL SEARCH REPORT**
**Information on patent family members**

International application No.
**PCT/CN2022/141572**

| Patent document cited in search report | Publication date (day/month/year) | Patent family member(s) | | | Publication date (day/month/year) |
|---|---|---|---|---|---|
| | | KR | 20190135559 | A | 06 December 2019 |
| | | KR | 102087017 | B1 | 10 March 2020 |
| | | KR | 20180105271 | A | 27 September 2018 |
| | | KR | 102052319 | B1 | 05 December 2019 |
| | | TW | 201811746 | A | 01 April 2018 |
| | | TWI | 650312 | B | 11 February 2019 |
| | | TW | 201420117 | A | 01 June 2014 |
| | | TWI | 615152 | B | 21 February 2018 |
| | | EP | 3632471 | A1 | 08 April 2020 |
| | | TW | 202033499 | A | 16 September 2020 |
| | | TWI | 757740 | B | 11 March 2022 |
| | | DK | 3342785 | T3 | 23 March 2020 |
| | | PL | 3342785 | T3 | 07 September 2020 |
| | | RS | 57278 | B1 | 31 August 2018 |
| | | KR | 20220029776 | A | 08 March 2022 |
| | | KR | 102417310 | B1 | 05 July 2022 |
| | | ZA | 201501825 | B | 25 May 2022 |
| | | DK | 2907824 | T3 | 23 July 2018 |
| | | PH | 12018501433 | A1 | 27 February 2019 |
| | | PL | 2907824 | T3 | 31 August 2018 |
| | | KR | 20210132240 | A | 03 November 2021 |
| | | KR | 102369419 | B1 | 02 March 2022 |
| | | HK | 1256631 | A1 | 27 September 2019 |
| | | IL | 238143 | A0 | 31 May 2015 |
| | | IL | 238143 | B | 30 January 2020 |
| | | KR | 20210038728 | A | 07 April 2021 |
| | | KR | 102320907 | B1 | 02 November 2021 |
| | | SI | 3342785 | T1 | 28 February 2020 |
| | | JP | 2016196484 | A | 24 November 2016 |
| | | JP | 6186045 | B2 | 23 August 2017 |
| | | KR | 20200026323 | A | 10 March 2020 |
| | | KR | 102237639 | B1 | 07 April 2021 |
| | | NZ | 705394 | A | 26 October 2018 |
| | | IL | 271760 | A | 27 February 2020 |
| | | IL | 271760 | B | 01 August 2022 |
| | | RU | 2015113767 | A | 10 December 2016 |
| | | RU | 2664465 | C2 | 17 August 2018 |
| | | EP | 2907824 | A1 | 19 August 2015 |
| | | EP | 2907824 | A4 | 08 June 2016 |
| | | EP | 2907824 | B1 | 11 April 2018 |
| | | AU | 2013328111 | A1 | 12 March 2015 |
| | | AU | 2013328111 | B2 | 02 November 2017 |
| | | RS | 60000 | B1 | 30 April 2020 |
| | | MX | 2015003903 | A | 17 July 2015 |
| | | MX | 364484 | B | 29 April 2019 |
| | | ES | 2671644 | T3 | 07 June 2018 |
| | | TW | 201920098 | A | 01 June 2019 |
| | | TWI | 696611 | B | 21 June 2020 |
| | | NZ | 740948 | A | 29 November 2019 |
| | | NZ | 746439 | A | 29 November 2019 |

Form PCT/ISA/210 (patent family annex) (July 2022)

**INTERNATIONAL SEARCH REPORT**
**Information on patent family members**

International application No.
**PCT/CN2022/141572**

| Patent document cited in search report | Publication date (day/month/year) | Patent family member(s) | | | Publication date (day/month/year) |
|---|---|---|---|---|---|
| | | HK | 1210477 | A1 | 22 April 2016 |
| | | EP | 3342785 | A1 | 04 July 2018 |
| | | EP | 3342785 | B1 | 25 December 2019 |
| | | JP | 2020180124 | A | 05 November 2020 |
| | | JP | 6952835 | B2 | 27 October 2021 |
| | | BR | 122021014365 | B1 | 05 July 2022 |
| | | HUE | 039000 | T2 | 28 December 2018 |
| | | KR | 20220146669 | A | 01 November 2022 |
| | | JP | 2018188455 | A | 29 November 2018 |
| | | JP | 6715888 | B2 | 01 July 2020 |
| | | PH | 12015500667 | A1 | 18 May 2015 |
| | | LT | 3342785 | T | 10 February 2020 |
| | | MX | 2019004502 | A | 21 August 2019 |
| | | ZA | 201900820 | B | 31 August 2022 |
| | | US | 2019008981 | A1 | 10 January 2019 |
| | | US | 10973924 | B2 | 13 April 2021 |
| | | IL | 294622 | A | 01 September 2022 |
| | | AU | 2022203560 | A1 | 16 June 2022 |
| | | AU | 2018200308 | A1 | 01 February 2018 |
| | | AU | 2018200308 | B2 | 07 November 2019 |
| | | HRP | 20200353 | T1 | 12 June 2020 |
| | | HUE | 048748 | T2 | 28 August 2020 |
| | | RU | 2018128384 | A | 02 October 2018 |
| | | RU | 2018128384 | A3 | 20 December 2021 |
| | | JP | 2018008982 | A | 18 January 2018 |
| | | JP | 6371452 | B2 | 08 August 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5892019 A
- US 5648260 A
- US 5624821 A
- WO 2014036495 A
- WO 2015031815 A
- US 5807715 A
- US 4816567 A
- US 4816397 A
- US 5585089 A
- EP 239400 A
- WO 9109967 A
- US 5225539 A
- US 5530101 A
- EP 592106 A
- EP 519596 A
- US 5565332 A
- WO 92102190 A
- US 5693761 A
- US 5693792 A
- US 5714350 A
- US 5777085 A
- US 4683195 A
- US 4683202 A
- WO 9852976 A1
- WO 0034317 A2
- US 4694778 A
- US 4946778 A
- US 5258498 A
- WO 2003016466 A2
- US 6350861 B
- US 5168062 A
- US 4510245 A
- US 4968615 A
- US 4399216 A
- US 4634665 A
- US 5179017 A


### Non-patent literature cited in the description


- **SAMBROOK et al.** Molecular Cloning: A Laboratory Manual. Cold Spring Harbor Laboratory Press, 1989
- **KABAT, E. et al.** Sequences of Proteins of Immunological Interest. U.S. Department of Health and Human Services, 1983
- **CHOTHIA ; LESK.** *J. Mol. Biol.,* 1987, vol. 196, 901-917
- **KABAT.** Sequences of Proteins of Immunological Interest. National Institutes of Health, 1987
- **CHOTHIA ; LESK'S.** *J. Mol. Biol.,* 1987, vol. 196, 901-917
- **CHOTHIA et al.** *Nature,* 1989, vol. 342, 878-883
- **WINNAKER.** From Genes to Clones. Verlagsgesellschaft, 1987
- **KABAT et al.** Sequences of Proteins of Immunological Interest. U.S. Dept. of Health and Human Services, 1983
- **CHOTHIA et al.** *J. Mol. Biol.,* 1987, vol. 196, 901-917
- **KABAT et al.** Sequence of Proteins of Immunological Interest. U.S. Dept. of Health and Human Services, 1983
- **ROUX et al.** *J. Immunol.,* 1998, vol. 161, 4083
- *Nature,* 1993, vol. 361, 186-87
- **DAVIES et al.** *Annual Rev Biochem,* 1990, vol. 59, 439-473
- Immunology-A Synthesis. Sinauer Associates, 1991
- Current Protocols in Molecular Biology. 2007
- Proteins: Structures and Molecular Principles. W. H. Freeman and Company, 1984
- Introduction to Protein Structure. Garland Publishing, 1991
- **THORNTON et al.** *Nature,* 1991, vol. 354, 105
- **OUYANG.** *J. Methods Mol Biol,* 2013, vol. 1045, 275-83
- McGraw-Hill Dictionary of Chemical Terms. McGraw-Hill, 1985
- **J. R. CROWTHER.** ELISA: Theory and Practice: Methods in Molecular Biology. Human Press, 1995, vol. 42
- **E. DIAMANDIS ; T. CHRISTOPOULUS.** Immunoassay. Academic Press, Inc, 1996
- **P. TIJSSEN.** Practice and Theory of Enzyme Immunoassays. Elsevier Science Publishers, 1985
- Remington's Pharmaceutical Sciences: The Science And Practice Of Pharmacy. Mack Pub. Co, 1995
- Drug Absorption Enhancement: Concepts, Possibilities, Limitations, And Trends. Harwood Academic Publishers, 1994

- Peptide And Protein Drug Delivery, Advances In Parenteral Sciences. M. Dekker, 1991, vol. 4
- **WU ; WU.** *J. Biol. Chem.,* 1987, vol. 262, 4429-4432
- **WINTER ; HARRIS.** *Immunol Today,* 1993, vol. 14, 43-46
- **WRIGHT et al.** *Crit. Reviews in Immunol.,* 1992, 12125-168
- **LIU et al.** *P.N.A.S.,* 1987, vol. 84, 3439
- *J. Immunol.,* 1987, vol. 139, 3521
- **KABAT et al.** Sequences of Proteins of immunological Interest. N.I.H, 1991
- **SKERRA et al.** *Science,* 1988, vol. 242, 1038-1041
- **CHOTHIA et al.** *J. Mol. Biol.,* 1998, vol. 278, 457-479
- Recombinant DNA Technology for Production of Protein Therapeutics in Cultured Mammalian Cells. **D. L. HACKER ; F. M. WURM.** Reference Module in Life Sciences. 2017
- **GOEDDEL.** Gene Expression Technology: Methods in Enzymology. Academic Press, 1990, vol. 185
- Molecular Cloning: A Laboratory Manual. Cold Spring Harbor, 1989
- Current Protocols in Molecular Biology. Greene Publishing Associates, 1989
- **TIHOMIR S. DODEV et al.** A tool kit for rapid cloning and expression of recombinant antibodies. *Scientific Reports,* 2014, vol. 4
- **STEFAN SCHLATTER et al.** On the Optimal Ratio of Heavy to Light Chain Genes for Efficient Recombinant Antibody Production by CHO Cells. *Biotechnol Progress,* 2005, vol. 21, 122-133
- **HADI BAYAT et al.** Evaluation of different vector design strategies for the expression of recombinant monoclonal antibody in CHO cells. *Preparative Biochemistry & Biotechnology,* 2018, vol. 48 (8), 822-829
- **D. L. HACKER ; F. M. WURM.** Recombinant DNA Technology for Production of Protein Therapeutics in Cultured Mammalian Cells. *Reference Module in Life Sciences,* 2017
- Solid Phase Peptide Synthesis. The Pierce Chemical Co, 1984
- **CHU et al.** Biochemia. Roche Molecular Biologicals, 2001
- **MURRAY et al.** *Current Opinion in Chemical Biology,* 2013, vol. 17, 420-426
- **D. WILKINSON.** The Scientist. Scientist, Inc, 17 April 2000, vol. 14, 25-28
- *CHEMICAL ABSTRACTS,* 143655-49-6